(19) Europäisches Patentamt
European Patent Office
Office européen des brevets

(11) EP 4 772 501 A1

(12) EUROPEAN PATENT APPLICATION
published in accordance with Art. 153(4) EPC

(43) Date of publication:
**08.07.2026 Bulletin 2026/28**

(21) Application number: **24870571.7**

(22) Date of filing: **19.09.2024**

(51) International Patent Classification (IPC):
***C07D 307/64*** *(2006.01)* ***C07D 307/60*** *(2006.01)*
***A01N 43/08*** *(2006.01)* ***A01P 21/00*** *(2006.01)*

(86) International application number:
**PCT/CN2024/119810**

(87) International publication number:
**WO 2025/067027 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311268630**

(71) Applicant: **Nankai University**
**Tianjin 300071 (CN)**

(72) Inventors:
- **XI, Zhen**
  **Tianjin 300071 (CN)**
- **WANG, Dawei**
  **Tianjin 300071 (CN)**
- **WU, Haixiang**
  **Tianjin 300071 (CN)**

(74) Representative: **reuteler & cie SA**
**Chemin de la Vuarpillière 29**
**1260 Nyon (CH)**

# (54) AMIDE COMPOUND AND PREPARATION METHOD THEREFOR, AND AGRICULTURAL APPLICATION

(57) The present invention relates to the field of pesticide chemistry, and provides an amide compound and a preparation method therefor, an agricultural application, and a biostimulant. The compound has the structure as shown in formula (I). The compound can effectively promote seed germination, increase crop yield, regulate plant growth, enhance plant disease resistance, and perform abiotic stress management on plants.

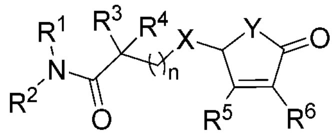
formula (I)

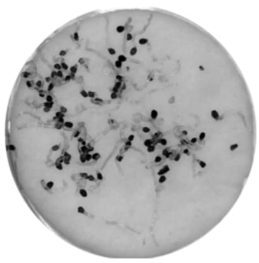

FIG.1



Processed by Luminess, 75001 PARIS (FR)

## Description

### Cross Reference to Related Applications

The present application claims the benefit of Chinese patent application 202311268630.2, filed on 28/09/2023, the contents of which are incorporated herein by reference.

### Technical Field

The invention relates to the field of pesticide chemistry, and particularly relates to an amide compound and preparation method therefor, and agricutural application.

### Background

Economic losses to grain production from weed damage occur worldwide every year as much as $ 950 billion, and a huge seed pool in the soil is a direct cause of weed damage. Germination is a necessary condition for the seed plant to perform life activities. Promoting seed germination can improve seed germination rate, reduce seed usage amount in agricultural production, improve inoculation rate of cistanche and cynomorium songaricum, and protect wild traditional Chinese medicine resources; on the other hand, the weeds can be controlled by applying the post-emergence herbicide to the weeds after emergence.

Strigolactones are a class of natural products isolated from plants containing structural units of alkenyl ether butenolide. Researches show that the strigolactones can promote germination of roots parasitic weeds such as seeds of Orobanchus, striga asiatica and the like, and promote germination of arbuscular mycorrhizal fungi spores and branching of hyphae. In addition, as a newly discovered plant hormone, Strigolactones can also regulate multiple processes of plant growth and development, such as inhibiting plant branching, controlling the germination of axillary buds, inhibiting the growth of plant lateral roots and promoting the elongation of main roots, etc. ("Strigolactones: plant hormones with promising features") (Bouwmeester, H. J. etc., (Angew.Chem. Int.Ed., 2019, Vol. 58, p12778 -12786).The existing strigolactone compounds are difficult to obtain and have poor stability, which limits their application in agricultural production.

"Tentative molecular mechanism for germination stimulation of striga and orobanche seeds by strigol and its synthetic analogs"(Mangnus, E. M.; Zwanenburg, B.;(J. Agric. Food Chem.),1992, Vol. 40,p1066-1070) indicate that the alkenyl ether butenolide structural units in strigolactones are critical for maintaining the activity of the compounds, which would result in loss of activity if the alkenyl ether bond was changed to methylene. At present, all reported strigolactone analogs or mimics have an alkenyl ether butenolide structural unit with poor stability (such as WO2018060865A1 and WO2012146374A3), and the structural fragment is also an important factor which causes the poor stability of the compounds and restricts the field application of the compounds.

Currently, most highly active strigolactone agonists such as nijmegen-1, MP1, etc. are not practical for use in production due to their poor stability. Therefore, there is a need for a new compound that has good stability and can be effectively applied to agricultural growth.

### Disclosure of Invention

The invention aims to provide a novel amide compound with at least one of the effects of germinating seeds, improving crop yield, regulating plant growth, regulating and controlling soil microbial population structure and improving soil fertility.

In order to achieve the above object, a first aspect of the present invention provides an amide compound having a structure represented by formula (I),

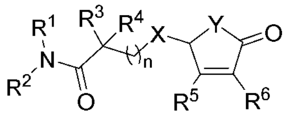 formula (I),

wherein, in formula (I),

$R^1$ and $R^2$ are each independently selected from H, substituted or unsubstituted $C_{1-12}$ alkyl, substituted or unsubstituted $C_{3-12}$ cycloalkyl,

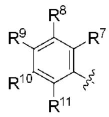
,

substituted or unsubstituted $C_{1-8}$ heterocycle containing a heteroatom selected from at least one of N, O and S, wherein $R^7$, $R^8$, $R^9$, $R^{10}$ and $R^{11}$ are each independently selected from H, halogen, nitro, cyano, phenyl, benzyl, $C_{1-6}$ alkyl, $C_{3-6}$ cycloalkyl, $C_{1-6}$ alkyl substituted by 1-9 halogens, $C_{1-6}$ alkoxy, $C_{1-6}$ alkoxy substituted by 1-9 halogens, $C_{1-6}$ alkylthio, $C_{1-6}$ alkylthio substituted by 1-9 halogens, -COO-$C_{1-6}$ alkyl and-$S(O)_2$-$C_{1-6}$ alkyl, or any two adjacent of $R^7$, $R^8$, $R^9$, $R^{10}$ and $R^{11}$ are cyclized together to form a 3-8 membered ring structure;and the substituents optionally present on $R^1$ and $R^2$ are each independently selected from the group consisting of $C_{1-12}$ alkyl, phenyl;
$R^3$, $R^4$, $R^5$ and $R^6$ are each independently selected from H, $C_{1-12}$ alkyl and $C_{3-12}$ cycloalkyl;
n is 0, 1, 2, 3 or 4;
X is O, S, -S(O)- or -$S(O)_2$-; Y is O, NH, S, $CH_2$ or -$N(CH_3)$-.

The second aspect of the present invention provides a process for producing the amide compound described in the first aspect, which comprises: carrying out contact reaction on the compound shown in a formula (V-1) and the compound shown in a formula (V-2);

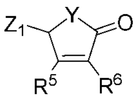 formula (V-1), 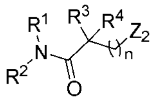 formula (V-2)

wherein the substitution of the substituents in the formulae (V-1) and (V-2) corresponds to the same substitution in the amide compound described in the first aspect, and one of $Z_1$ and $Z_2$ is -Br and the other is -XH, and X is defined as corresponding to the definition in the amide compound described in the first aspect.

The third aspect of the present invention provides a process for producing the amide compound described in the first aspect, which comprises: carrying out coupling reaction on a compound shown in a formula (II) and a compound shown in a formula (III);

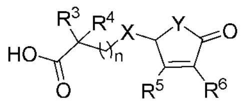 formula (II), 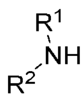 formula (III);

wherein the substitution of the substituents in the formulae (III) and (II) is the same as the substitution in the amide compound described in the first aspect.

The fourth aspect of the invention provides the amide compound of the first aspect for use in agriculture.

In a fifth aspect, the present invention provides a biostimulant, which comprises a plant growth regulating effective amount of at least one of the amide compounds described in the first aspect, and optionally an adjuvant.

The amide compound provided by the invention can regulate and control the microbial population structure of soil and improve the soil fertility.

The amide compound provided by the invention can effectively promote the germination of seeds, regulate the growth and development of plants and improve the quality and yield of crops.

Particularly, in the compound provided by the invention and having the structure shown in the formula (I), when X and Y are both O, the compound provided by the invention can effectively improve the yield of crops.

In the compound with the structure shown in the formula (I), when X and Y are not O simultaneously, the compound provided by the invention can effectively promote the germination of seeds, regulate the growth and development of plants and improve the quality and yield of crops.

Particularly, compared with the compounds containing alkenyl ether substituted butenolide ring structures in the prior art, the amide compounds provided by the invention have good seed germination promoting activity, good stability, plant growth regulating activity and crop yield increasing promoting activity, so that the amide compounds can be well applied to fields.

## Drawings

FIG. 1 shows the germination of *Phelipanche aegyptiaca* seeds after treatment with $10^{-7}$ mol/L of compound 1 according to the present invention; FIG. 2 shows the germination of *Phelipanche aegyptiaca* seeds after treatment with a

blank control (CK) according to the present invention; FIG. 3 shows the germination of wheat seeds after treatment with a blank control (CK) according to the present invention; FIG. 4 shows the germination of wheat seeds after treatment with 1 ppm of compound 1 according to the present invention; FIG. 5 shows the germination of wheat seeds after treatment with 1 ppm of compound 768 according to the present invention; FIG. 6 shows the drought resistance effect on wheat after treatment with $1\times10^{-6}$ mol/L of compound 1 according to the present invention; FIG. 7 shows the drought resistance effect on wheat after treatment with a blank control (CK) according to the present invention; FIG. 8 shows the crop yield increase in a corn test field after treatment with a blank control (shown as A in FIG. 8) and $4\times10^{-5}$ mol/L of compound 1 (shown as B in FIG. 8) according to the present invention; FIG. 9 shows the root growth of *Arabidopsis thaliana* after treatment with a blank control according to the present invention; FIG. 10 shows the root growth of *Arabidopsis thaliana* after treatment with 1 μM of compound 880 according to the present invention; FIG. 11 shows the growth of wheat after treatment with a blank control according to the present invention; FIG. 12 shows the growth of wheat after treatment with 1 ppm of compound 1 according to the present invention.

**Detailed Description**

The endpoints of the ranges and any values disclosed herein are not limited to the precise range or value, and these ranges or values should be understood to encompass values close to these ranges or values. For numerical ranges, each range between its endpoints and individual point values, and each individual point value can be combined with each other to give one or more new numerical ranges, and such numerical ranges should be construed as specifically disclosed herein.

The $C_{1-12}$ alkyl in the present invention represents an alkyl group having 1-12 carbon atoms in total, and includes a straight-chain alkyl group, a branched-chain alkyl group, for example, a straight-chain alkyl group having 1, 2, 3, 4, 5, 6, 7, 8, 9, 10, 11 or 12 carbon atoms in total, a branched-chain alkyl group, for example, a methyl group, an ethyl group, a n-propyl group, an isopropyl group, a n-butyl group, an isobutyl group, a tert-butyl group, a n-pentyl group, an isopentyl group, a n-hexyl group, etc. The explanations for "$C_{1-6}$ alkyl", "$C_{1-4}$ alkyl" and "$C_{1-3}$ alkyl" are similar, except that the total number of carbon atoms is different.

The $C_{3-12}$ cycloalkyl in the present invention means a cycloalkyl group having 3 to 12 carbon atoms in total, for example, a cycloalkyl group having 3, 4, 5, 6, 7, 8, 9, 10, 11 or 12 carbon atoms in total, and the number of carbon atoms in the ring is not particularly limited, and examples thereof include cyclopropyl, methylcyclopropyl, ethylcyclopropyl, cyclopentyl, methylcyclopentyl and cyclohexyl. The same applies to "$C_{3-8}$ cycloalkyl", "$C_{3-6}$ cycloalkyl", and the like, except that the number of carbon atoms is different.

The "$C_{1-8}$ heterocycle containing a heteroatom selected from at least one of N, O and S" in the present invention means a cyclic structure having a total number of carbon atoms of 1 to 8, and the ring-forming atoms contain at least one hetero atom selected from N, O and S, and may be a saturated structure or an unsaturated structure. Examples of the ring include a three-membered ring, a four-membered ring, a five-membered ring, a six-membered ring, a seven-membered ring and an eight-membered ring.

The halogen in the invention comprises fluorine, chlorine, bromine and iodine.

The "$C_{1-6}$ alkyl substituted by 1-9 halogens" in the invention represents that 1 to 9 hydrogen atoms on the $C_{1-6}$ alkyl group are substituted by halogen atoms, wherein the substitution may occur on 1, 2 or 3 hydrogen atoms on the same carbon atom, or on hydrogen atoms on different carbon atoms. Examples include, but are not limited to, trifluoromethyl, difluoromethyl, monofluoromethyl, monofluoroethyl, difluoroethyl, and trifluoroethyl.

The $C_{1-6}$ alkoxy in the invention represents a group represented by $-O-R_a$, wherein $R_a$ is $C_{1-6}$ alkyl.

The alkylthio in the invention represents a group represented by $-S-R_b$, wherein $R_b$ is $C_{1-6}$ alkyl.

The "$C_{1-6}$ alkoxy substituted by 1-9 halogens" in the invention represents that 1 to 9 hydrogen atoms on the $C_{1-6}$ alkoxy group are substituted by halogen atoms, wherein the substitution may occur on 1, 2 or 3 hydrogen atoms on the same carbon atom, or on hydrogen atoms on different carbon atoms. The term "$C_{1-6}$ alkylthio substituted by 1-9 halogens" has a similar definition thereto.

The "$-COO-C_{1-6}$ alkyl" in the invention represents that an ester group is directly connected with a core structure, and the other end of a single bond O atom on the ester group is connected with the $C_{1-6}$ alkyl.

The "$-S(O)_2-C_{1-6}$ alkyl" in the invention represents that the sulfonyl is directly connected with a core structure.

The "$-S(O)_2-C_{1-6}$ alkyl" in the invention represents that the sulfonyl is directly connected with a core structure.

The expression "any two adjacent of $R^7$, $R^8$, $R^9$, $R^{10}$ and $R^{11}$ are cyclized together to form a 3-8 membered ring structure" as used herein means that any two adjacent of $R^7$, $R^8$, $R^9$, $R^{10}$ and $R^{11}$ are cyclized together with 2 carbon atoms on the benzene ring structure in which they are located to form a ring containing 3, 4, 5, 6, 7 or 8 atoms.

In the present invention, when a specific substitution position is not defined, the term "substituted or unsubstituted" as used herein means that the group can be substituted with a substituent defined later, or a group having no substituent defined later; when substituted with a substituent, the substituent may be located at any position that can be substituted. For example, the substituted or unsubstituted $C_{1-12}$ alkyl in the present invention means that any substitutable

position on $C_{1-12}$ alkyl may be substituted.

In the present invention, the wavy line indicates the attachment position, for example, in

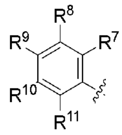

,

the wavy line is shown to indicate the attachment position, the group is chemically bonded to the core structure through the site where the wavy line is located.

"$-(CH_2)_z$-phenyl" means that the methylene group is directly attached to the core structure and the end group is a phenyl group.

The expression "an unsaturated five-membered ring group containing a heteroatom selected from class B" means that the group is a five-membered ring group, and the five-membered ring group is an unsaturated group, wherein the group contains at least one double bond structure, and the ring-forming atoms of the five-membered ring group contain at least one heteroatom selected from class B heteroatoms. "an unsaturated six-membered ring group containing a heteroatom selected from class B" has a similar interpretation.

The expression " a saturated four-membered ring group containing a heteroatom of class B " means that the group is a four-membered cyclic group which is a saturated group and which contains at least one heteroatom selected from class B in its ring-forming atoms.

"a saturated $C_{4-8}$ cycloalkyl" means a cycloalkyl group having 4 to 8 carbon atoms, and the cycloalkyl group is a saturated cycloalkyl group, and the ring-forming atoms are all C atoms.

The present invention has no particular requirement for the attachment site of groups such as "benzothiazolyl, benzoxazolyl, benzopyrazolyl, phenylpropylthiophenyl, N-methyl-benzopyrrolyl" to the core structure. They can be connected to the core structure at any position that allows for connection with the core structure, as long as a stable chemical bond can be formed. That is, any hydrogen atom at any position can be removed to form the aforementioned group and connect to the core structure.

As described above, the first aspect of the present invention provides an amide compound.

Preferably, in formula (1), X and Y are not both O.

Preferably, in formula (I), $R^1$ and $R^2$ are each independently selected from H,

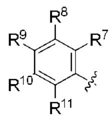

,

$C_{1-6}$ alkyl, $-(CH_2)_z$-phenyl, an unsaturated five-membered ring group containing a heteroatom selected from class B, a saturated four-membered ring group containing a heteroatom of class B, an unsaturated six-membered ring group containing a heteroatom of class B, a saturated $C_{4-8}$ cycloalkyl, benzothiazolyl, benzoxazolyl, benzopyrazolyl, phenylpropylthiophenyl, N-methyl-benzopyrolyl,

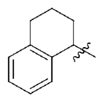

;

z is an integer from 1 to 6; each of said heteroatom selected from class B is each independently at least one selected from O, S, N; and optionally, the heteroatom selected from class B contains $C_{1-3}$ alkyl as a substituent; wherein $R^7$, $R^8$, $R^9$, $R^{10}$ and $R^{11}$ are independently selected from H, halogen, nitro, cyano, phenyl, benzyl, $C_{1-6}$ alkyl, $C_{3-6}$ cycloalkyl, $C_{1-6}$ alkyl substituted by 1-6 halogens, $C_{1-6}$ alkoxy, $C_{1-6}$ alkoxy substituted by 1-6 halogens, $C_{1-6}$ alkylthio, $C_{1-6}$ alkylthio substituted by 1-3 halogens, $-COO-C_{1-6}$ alkyl and $-S(O)_2-C_{1-6}$ alkyl, or any two adjacent of $R^7$, $R^8$ and $R^9$ are cyclized together to form a $C_{3-6}$ saturated cycloalkane structure with or without O atom; or any two adjacent of $R^7$, $R^8$ and $R^9$ are cyclized together to form an unsaturated naphthenic structure of $C_{3-6}$ containing or not containing class A heteroatoms, wherein the class A heteroatoms are selected from at least one of O, S, N, and optionally, $C_{1-3}$ alkyl is contained on the class A heteroatoms as a substituent; $R^3$, $R^4$, $R^5$ and $R^6$ are each independently selected from H, $C_{1-6}$ alkyl and $C_{3-6}$ cycloalkyl; n is 0, 1, 2 or 3; X is S, -S(O)- or $-S(O)_2-$; Y is O, NH, $-CH_2-$ or - $N(CH_3)$-.

According to a particularly preferred embodiment, in formula (I), $R^1$ is

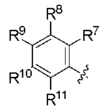

,

wherein, $R^7$, $R^8$, $R^9$, $R^{10}$ and $R^{11}$ are each independently selected from H, halogen, nitro, cyano, phenyl, benzyl, $C_{1-6}$ alkyl, $C_{3-6}$ cycloalkyl, $C_{1-6}$ alkyl substituted by 1-6 halogens, $C_{1-6}$ alkoxy, $C_{1-6}$ alkoxy substituted by 1-6 halogens, $C_{1-6}$ alkylthio, $C_{1-6}$ alkylthio substituted by 1-3 halogens, -COO-$C_{1-6}$ alkyl and-$S(O)_2$-$C_{1-6}$ alkyl, or any two adjacent of $R^7$, $R^8$ and $R^9$ are cyclized together to form a $C_{3-6}$ saturated cycloalkane structure with or without O atom; or any two adjacent of $R^7$, $R^8$ and $R^9$ are cyclized together to form an unsaturated naphthenic structure of $C_{3-6}$ containing or not containing class A heteroatoms, wherein the class A heteroatoms are selected from at least one of O, S, N, and optionally, $C_{1-3}$ alkyl is contained on the class A heteroatoms as a substituent; $R^2$, $R^5$ and $R^6$ are each independently selected from H, methyl, ethyl, n-propyl, isopropyl, tert-butyl; or $R^2$ is phenyl; $R^3$ and $R^4$ are both H; n is 0, 1 or 2; X is S, -S(O)- or -$S(O)_2$-; Y is O, NH, -$CH_2$- or -$N(CH_3)$-.

According to a more preferred embodiment, the compound of formula (I) has the structure of a compound of formula (I-A):

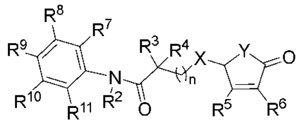

formula (I-A),

wherein, in formula (I-A), the substitution of n, X, Y, $R^2$, $R^3$, $R^4$, $R^5$, $R^6$, $R^7$, $R^8$, $R^9$, $R^{10}$ and $R^{11}$ is as follows: compound 1: n is 0, X is S, Y is O, $R^6$ is Me, and $R^2$, $R^3$, $R^4$, $R^5$, $R^7$, $R^8$, $R^9$, $R^{10}$ and $R^{11}$ are all H; compound 2: n is 0, X is S, Y is O, $R^6$ is Me, $R^7$ is F, and $R^2$, $R^3$, $R^4$, $R^5$, $R^8$, $R^9$, $R^{10}$ and $R^{11}$ are all H; compound 3: n is 0, X is S, Y is O, $R^6$ is Me, $R^8$ is F, and $R^2$, $R^3$, $R^4$, $R^5$, $R^7$, $R^9$, $R^{10}$ and $R^{11}$ are all H; compound 4: n is 0, X is S, Y is O, $R^6$ is Me, $R^9$ is F, and $R^2$, $R^3$, $R^4$, $R^5$, $R^7$, $R^8$, $R^{10}$ and $R^{11}$ are all H; compound 5: n is 0, X is S, Y is O, $R^6$ is Me, $R^7$ is Cl, and $R^2$, $R^3$, $R^4$, $R^5$, $R^8$, $R^9$, $R^{10}$ and $R^{11}$ are all H; compound 6: n is 0, X is S, Y is O, $R^6$ is Me, $R^8$ is Cl, and $R^2$, $R^3$, $R^4$, $R^5$, $R^7$, $R^9$, $R^{10}$ and $R^{11}$ are all H; compound 7: n is 0, X is S, Y is O, $R^6$ is Me, $R^9$ is Cl, and $R^2$, $R^3$, $R^4$, $R^5$, $R^7$, $R^8$, $R^{10}$ and $R^{11}$ are all H; compound 8: n is 0, X is S, Y is O, $R^6$ is Me, $R^7$ is Br, and $R^2$, $R^3$, $R^4$, $R^5$, $R^8$, $R^9$, $R^{10}$ and $R^{11}$ are all H; compound 9: n is 0, X is S, Y is O, $R^6$ is Me, $R^8$ is Br, and $R^2$, $R^3$, $R^4$, $R^5$, $R^7$, $R^9$, $R^{10}$ and $R^{11}$ are all H; compound 10: n is 0, X is S, Y is O, $R^6$ is Me, $R^9$ is Br, and $R^2$, $R^3$, $R^4$, $R^5$, $R^7$, $R^8$, $R^{10}$ and $R^{11}$ are all H; compound 11: n is 0, X is S, Y is O, $R^6$ is Me, $R^7$ is I, and $R^2$, $R^3$, $R^4$, $R^5$, $R^8$, $R^9$, $R^{10}$ and $R^{11}$ are all H; compound 12: n is 0, X is S, Y is O, $R^6$ is Me, $R^8$ is I, and $R^2$, $R^3$, $R^4$, $R^5$, $R^7$, $R^9$, $R^{10}$ and $R^{11}$ are all H; compound 13: n is 0, X is S, Y is O, $R^6$ is Me, $R^9$ is I, and $R^2$, $R^3$, $R^4$, $R^5$, $R^7$, $R^8$, $R^{10}$ and $R^{11}$ are all H; compound 14: n is 0, X is S, Y is O, $R^6$ is Me, $R^7$ is $NO_2$, and $R^2$, $R^3$, $R^4$, $R^5$, $R^8$, $R^9$, $R^{10}$ and $R^{11}$ are all H; compound 15: n is 0, X is S, Y is O, $R^6$ is Me, $R^8$ is $NO_2$, and $R^2$, $R^3$, $R^4$, $R^5$, $R^7$, $R^9$, $R^{10}$ and $R^{11}$ are all H; compound 16: n is 0, X is S, Y is O, $R^6$ is Me, $R^9$ is $NO_2$, and $R^2$, $R^3$, $R^4$, $R^5$, $R^7$, $R^8$, $R^{10}$ and $R^{11}$ are all H; compound 17: n is 0, X is S, Y is O, $R^6$ is Me, $R^7$ is $CF_3$, and $R^2$, $R^3$, $R^4$, $R^5$, $R^8$, $R^9$, $R^{10}$ and $R^{11}$ are all H; compound 18: n is 0, X is S, Y is O, $R^6$ is Me, $R^8$ is $CF_3$, and $R^2$, $R^3$, $R^4$, $R^5$, $R^7$, $R^9$, $R^{10}$ and $R^{11}$ are all H; compound 19: n is 0, X is S, Y is O, $R^6$ is Me, $R^9$ is $CF_3$, and $R^2$, $R^3$, $R^4$, $R^5$, $R^7$, $R^8$, $R^{10}$ and $R^{11}$ are all H; compound 20: n is 0, X is S, Y is O, $R^6$ is Me, $R^7$ is $OCF_3$, and $R^2$, $R^3$, $R^4$, $R^5$, $R^8$, $R^9$, $R^{10}$ and $R^{11}$ are all H; compound 21: n is 0, X is S, Y is O, $R^6$ is Me, $R^8$ is $OCF_3$, and $R^2$, $R^3$, $R^4$, $R^5$, $R^7$, $R^9$, $R^{10}$ and $R^{11}$ are all H; compound 22: n is 0, X is S, Y is O, $R^6$ is Me, $R^9$ is $OCF_3$, and $R^2$, $R^3$, $R^4$, $R^5$, $R^7$, $R^8$, $R^{10}$ and $R^{11}$ are all H; compound 23: n is 0, X is S, Y is O, $R^6$ is Me, $R^7$ is $OCHF_2$, and $R^2$, $R^3$, $R^4$, $R^5$, $R^8$, $R^9$, $R^{10}$ and $R^{11}$ are all H; compound 24: n is 0, X is S, Y is O, $R^6$ is Me, $R^8$ is $OCHF_2$, and $R^2$, $R^3$, $R^4$, $R^5$, $R^7$, $R^9$, $R^{10}$ and $R^{11}$ are all H; compound 25: n is 0, X is S, Y is O, $R^6$ is Me, $R^9$ is $OCHF_2$, and $R^2$, $R^3$, $R^4$, $R^5$, $R^7$, $R^8$, $R^{10}$ and $R^{11}$ are all H; compound 26: n is 0, X is S, Y is O, $R^6$ is Me, $R^9$ is $SCF_3$, and $R^2$, $R^3$, $R^4$, $R^5$, $R^7$, $R^8$, $R^{10}$ and $R^{11}$ are all H; compound 27: n is 0, X is S, Y is O, $R^6$ is Me, $R^7$ is $OCH_3$, and $R^2$, $R^3$, $R^4$, $R^5$, $R^8$, $R^9$, $R^{10}$ and $R^{11}$ are all H; compound 28: n is 0, X is S, Y is O, $R^6$ is Me, $R^8$ is $OCH_3$, and $R^2$, $R^3$, $R^4$, $R^5$, $R^7$, $R^9$, $R^{10}$ and $R^{11}$ are all H; compound 29: n is 0, X is S, Y is O, $R^6$ is Me, $R^9$ is $OCH_3$, and $R^2$, $R^3$, $R^4$, $R^5$, $R^7$, $R^8$, $R^{10}$ and $R^{11}$ are all H; compound 30: n is 0, X is S, Y is O, $R^6$ is Me, $R^9$ is $COOC_2H_5$, and $R^2$, $R^3$, $R^4$, $R^5$, $R^7$, $R^8$, $R^{10}$ and $R^{11}$ are all H; compound 31: n is 0, X is S, Y is O, $R^6$ is Me, $R^7$ is CN, and $R^2$, $R^3$, $R^4$, $R^5$, $R^8$, $R^9$, $R^{10}$ and $R^{11}$ are all H; compound 32: n is 0, X is S, Y is O, $R^6$ is Me, $R^8$ is CN, and $R^2$, $R^3$, $R^4$, $R^5$, $R^7$, $R^9$, $R^{10}$ and $R^{11}$ are all H; compound 33: n is 0, X is S, Y is O, $R^6$ is Me, $R^9$ is CN, and $R^2$, $R^3$, $R^4$, $R^5$, $R^7$, $R^8$, $R^{10}$ and $R^{11}$ are all H; compound 34: n is 0, X is S, Y is O, $R^6$ and $R^7$ are Me, and $R^2$, $R^3$, $R^4$, $R^5$, $R^8$, $R^9$, $R^{10}$ and $R^{11}$ are all H; compound 35: n is 0, X is S, Y is O, $R^6$ and $R^8$ are Me, and $R^2$, $R^3$, $R^4$, $R^5$, $R^7$, $R^9$, $R^{10}$ and $R^{11}$ are all H; compound 36: n is 0, X is S, Y is O, $R^6$ and $R^9$ are Me, and $R^2$, $R^3$, $R^4$, $R^5$, $R^7$, $R^8$, $R^{10}$ and $R^{11}$ are all H; compound 37: n is 0, X is S, Y is O, $R^6$ is Me, $R^7$ is $CH_3SO_2$, and $R^2$, $R^3$, $R^4$, $R^5$, $R^8$, $R^9$, $R^{10}$ and $R^{11}$ are all H; compound 38: n is 0, X is S, Y is O, $R^6$ is Me, $R^8$ is $CH_3SO_2$, and $R^2$, $R^3$, $R^4$, $R^5$, $R^7$, $R^9$, $R^{10}$ and $R^{11}$ are all H; compound 39: n is 0, X is S, Y is O, $R^6$ is Me, $R^9$ is $CH_3SO_2$, and $R^2$, $R^3$, $R^4$, $R^5$, $R^7$, $R^8$, $R^{10}$ and $R^{11}$ are all H; compound 40: n is 0, X is S, Y is O, $R^6$ is Me, $R^7$ is $C_2H_5SO_2$, and $R^2$, $R^3$, $R^4$, $R^5$, $R^8$, $R^9$, $R^{10}$ and $R^{11}$ are all H;compound 874: n is 0, X is S, Y is O, $R^6$ is Me, $R^7$ is $CO_2CH_3$, and $R^2$, $R^3$, $R^4$, $R^5$, $R^8$, $R^9$, $R^{10}$ and $R^{11}$ are all H; compound 875: n is 0, X is S, Y is O, $R^6$ is Me, $R^8$ is $CO_2CH_3$, and $R^2$, $R^3$,

$R^4$, $R^5$, $R^7$, $R^9$, $R^{10}$ and $R^{11}$ are all H; compound 876: n is 0, X is S, Y is O, $R^6$ is Me, $R^9$ is $CO_2CH_3$, and $R^2$, $R^3$, $R^4$, $R^5$, $R^7$, $R^8$, $R^{10}$ and $R^{11}$ are all H; compound 877: n is 0, X is S, Y is O, $R^6$ is Me, $R^7$ is Ph, and $R^2$, $R^3$, $R^4$, $R^5$, $R^8$, $R^9$, $R^{10}$ and $R^{11}$ are all H; compound 878: n is 0, X is S, Y is O, $R^6$ is Me, $R^8$ is Ph, and $R^2$, $R^3$, $R^4$, $R^5$, $R^7$, $R^9$, $R^{10}$ and $R^{11}$ are all H; compound 879: n is 0, X is S, Y is O, $R^6$ is Me, $R^9$ is Ph, and $R^2$, $R^3$, $R^4$, $R^5$, $R^7$, $R^8$, $R^{10}$ and $R^{11}$ are all H; compound 880: n is 0, X is S, Y is O, $R^6$ is Me, $R^7$ is Bn, and $R^2$, $R^3$, $R^4$, $R^5$, $R^8$, $R^9$, $R^{10}$ and $R^{11}$ are all H; compound 881: n is 0, X is S, Y is O, $R^6$ is Me, $R^8$ is Bn, and $R^2$, $R^3$, $R^4$, $R^5$, $R^7$, $R^9$, $R^{10}$ and $R^{11}$ are all H; compound 882: n is 0, X is S, Y is O, $R^6$ is Me, $R^9$ is Bn, and $R^2$, $R^3$, $R^4$, $R^5$, $R^7$, $R^8$, $R^{10}$ and $R^{11}$ are all H; compound 41: n is 0, X is S, Y is O, $R^6$ is Me, $R^7$ and $R^8$ are both Cl, and $R^2$, $R^3$, $R^4$, $R^5$, $R^9$, $R^{10}$ and $R^{11}$ are all H; compound 42: n is 0, X is S, Y is O, $R^6$ is Me, $R^7$ and $R^9$ are both Cl, and $R^2$, $R^3$, $R^4$, $R^5$, $R^8$, $R^{10}$ and $R^{11}$ are all H; compound 43: n is 0, X is S, Y is O, $R^6$ is Me, $R^7$ and $R^{10}$ are both Cl, and $R^2$, $R^3$, $R^4$, $R^5$, $R^8$, $R^9$ and $R^{11}$ are all H; compound 44: n is 0, X is S, Y is O, $R^6$ is Me, $R^7$ and $R^{11}$ are both Cl, and $R^2$, $R^3$, $R^4$, $R^5$, $R^8$, $R^9$ and $R^{10}$ are all H; compound 45: n is 0, X is S, Y is O, $R^6$ is Me, $R^8$ and $R^9$ are both Cl, and $R^2$, $R^3$, $R^4$, $R^5$, $R^7$, $R^{10}$ and $R^{11}$ are all H; compound 46: n is 0, X is S, Y is O, $R^6$ and $R^7$ are Me, $R^9$ is Cl, and $R^2$, $R^3$, $R^4$, $R^5$, $R^8$, $R^{10}$ and $R^{11}$ are all H; compound 47: n is 0, X is S, Y is O, $R^6$, $R^7$ and $R^8$ are all Me, and $R^2$, $R^3$, $R^4$, $R^5$, $R^9$, $R^{10}$ and $R^{11}$ are all H; compound 48: n is 0, X is S, Y is O, $R^6$, $R^7$ and $R^9$ are all Me, and $R^2$, $R^3$, $R^4$, $R^5$, $R^8$, $R^{10}$ and $R^{11}$ are all H; compound 49: n is 0, X is S, Y is O, $R^6$, $R^7$ and $R^{10}$ are all Me, and $R^2$, $R^3$, $R^4$, $R^5$, $R^8$, $R^9$ and $R^{11}$ are all H; compound 50: n is 0, X is S, Y is O, $R^6$, $R^7$ and $R^{11}$ are all Me, and $R^2$, $R^3$, $R^4$, $R^5$, $R^8$, $R^9$ and $R^{10}$ are all H; compound 51: n is 0, X is S, Y is O, $R^6$, $R^7$, $R^8$ and $R^{11}$ are all Me, and $R^2$, $R^3$, $R^4$, $R^5$, $R^9$ and $R^{10}$ are all H; compound 52: n is 0, X is S, Y is O, $R^6$, $R^7$, $R^9$ and $R^{11}$ are all Me, and $R^2$, $R^3$, $R^4$, $R^5$, $R^8$ and $R^{10}$ are all H; compound 53: n is 0, X is S, Y is O, $R^6$ is Me, $R^7$ and $R^9$ are both $CF_3$, and $R^2$, $R^3$, $R^4$, $R^5$, $R^8$, $R^{10}$ and $R^{11}$ are all H; compound 54: n is 0, X is S, Y is O, $R^6$ is Me, $R^8$ and $R^{10}$ are both $CF_3$, and $R^2$, $R^3$, $R^4$, $R^5$, $R^7$, $R^9$ and $R^{11}$ are all H; compound 55: n is 0, X is S, Y is O, $R^6$ is Me, $R^2$, $R^3$, $R^4$ and $R^5$ are all H, and $R^7$, $R^8$, $R^9$, $R^{10}$ and $R^{11}$ are all F; compound 56: n is 0, X is S, Y is O, $R^6$ is Me, $R^7$ is $CH_3CH_2$, and $R^2$, $R^3$, $R^4$, $R^5$, $R^8$, $R^9$, $R^{10}$ and $R^{11}$ are all H; compound 57: n is 0, X is S, Y is O, $R^6$ is Me, $R^8$ is $CH_3CH_2$, and $R^2$, $R^3$, $R^4$, $R^5$, $R^7$, $R^9$, $R^{10}$ and $R^{11}$ are all H; compound 58: n is 0, X is S, Y is O, $R^6$ is Me, $R^9$ is $CH_3CH_2$, and $R^2$, $R^3$, $R^4$, $R^5$, $R^7$, $R^8$, $R^{10}$ and $R^{11}$ are all H; compound 59: n is 0, X is S, Y is O, $R^6$ is Me, $R^7$ is $CH(CH_3)_2$, and $R^2$, $R^3$, $R^4$, $R^5$, $R^8$, $R^9$, $R^{10}$ and $R^{11}$ are all H; compound 60: n is 0, X is S, Y is O, $R^6$ is Me, $R^8$ is $CH(CH_3)_2$, and $R^2$, $R^3$, $R^4$, $R^5$, $R^7$, $R^9$, $R^{10}$ and $R^{11}$ are all H; compound 61: n is 0, X is S, Y is O, $R^6$ is Me, $R^9$ is $CH(CH_3)_2$, and $R^2$, $R^3$, $R^4$, $R^5$, $R^7$, $R^8$, $R^{10}$ and $R^{11}$ are all H; compound 62: n is 0, X is S, Y is O, $R^6$ is Me, $R^7$ is $CH_3(CH_2)_2$, $R^2$, $R^3$, $R^4$, $R^5$, $R^8$, $R^9$, $R^{10}$ and $R^{11}$ are all H; compound 63: n is 0, X is S, Y is O, $R^6$ is Me, $R^7$ is

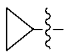,

and $R^2$, $R^3$, $R^4$, $R^5$, $R^8$, $R^9$, $R^{10}$ and $R^{11}$ are all H; compound 64: n is 0, X is S, Y is O, $R^6$ is Me, $R^7$ is

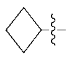,

and $R^2$, $R^3$, $R^4$, $R^5$, $R^8$, $R^9$, $R^{10}$ and $R^{11}$ are all H; compound 65: n is 0, X is S, Y is O, $R^6$ is Me, $R^7$ is $CO_2CH_3$, and $R^2$, $R^3$, $R^4$, $R^5$, $R^8$, $R^9$, $R^{10}$ and $R^{11}$ are all H; compound 66: n is 0, X is S, Y is O, $R^6$ is Me, $R^7$ and $R^8$ are cyclized to form

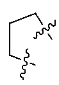,

and $R^2$, $R^3$, $R^4$, $R^5$, $R^9$, $R^{10}$ and $R^{11}$ are all H; compound 67: n is 0, X is S, Y is O, $R^6$ is Me, $R^8$ and $R^9$ are cyclized to form

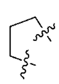,

and $R^2$, $R^3$, $R^4$, $R^5$, $R^7$, $R^{10}$ and $R^{11}$ are all H; compound 68: n is 0, X is S, Y is O, $R^6$ is Me, $R^8$ and $R^9$ are cyclized to form and $R^2$, $R^3$, $R^4$, $R^5$, $R^7$, $R^{10}$ and $R^{11}$ are all H; compound 69: n is 0, X is S, Y is O, $R^6$ is Me, $R^7$ and $R^8$ are cyclized to form

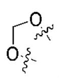,

and $R^2$, $R^3$, $R^4$, $R^5$, $R^9$, $R^{10}$ and $R^{11}$ are all H; compound 70: n is 0, X is S, Y is O, $R^6$ is Me, $R^8$ and $R^9$ are cyclized to form

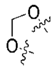,

and $R^2$, $R^3$, $R^4$, $R^5$, $R^7$, $R^{10}$ and $R^{11}$ are all H; compound 71: n is 0, X is S, Y is O, $R^6$ is Me, $R^8$ and $R^9$ are cyclized to form

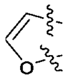,

and $R^2$, $R^3$, $R^4$, $R^5$, $R^7$, $R^{10}$ and $R^{11}$ are H; compound 72: n is 0, X is S, Y is O, $R^6$ is Me, $R^7$ and $R^8$ are cyclized to form

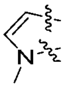,

and $R^2$, $R^3$, $R^4$, $R^5$, $R^9$, $R^{10}$ and $R^{11}$ are all H; compound 73: n is 0, X is S, Y is O, $R^6$ is Me, $R^7$ and $R^8$ are cyclized to form

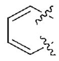,

and $R^2$, $R^3$, $R^4$, $R^5$, $R^9$, $R^{10}$ and $R^{11}$ are all H; compound 74: n is 0, X is S, Y is O, $R^6$ is Me, $R^8$ and $R^9$ are cyclized to form

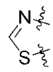,

and $R^2$, $R^3$, $R^4$, $R^5$, $R^7$, $R^{10}$ and $R^{11}$ are all H; compound 75: n is 0, X is S, Y is O, $R^6$ is Me, $R^8$ and $R^9$ are cyclized to form

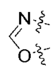,

and $R^2$, $R^3$, $R^4$, $R^5$, $R^7$, $R^{10}$ and $R^{11}$ are all H; compound 76: n is 0, X is S, Y is O, $R^6$ is Me, $R^8$ and $R^9$ are cyclized to form

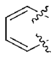,

and $R^2$, $R^3$, $R^4$, $R^5$, $R^7$, $R^{10}$ and $R^{11}$ are all H; compound 77: n is 0, X is S, Y is O, $R^5$ is Me, and $R^2$, $R^3$, $R^4$, $R^6$, $R^7$, $R^8$, $R^9$, $R^{10}$ and $R^{11}$ are all H; compound 78: n is 0, X is S, Y is O, $R^5$ is Me, $R^7$ is F, and $R^2$, $R^3$, $R^4$, $R^6$, $R^8$, $R^9$, $R^{10}$ and $R^{11}$ are all H; compound 79: n is 0, X is S, Y is O, $R^5$ is Me, $R^8$ is F, and $R^2$, $R^3$, $R^4$, $R^6$, $R^7$, $R^9$, $R^{10}$ and $R^{11}$ are all H; compound 80: n is 0, X is S, Y is O, $R^5$ is Me, $R^9$ is F, and $R^2$, $R^3$, $R^4$, $R^6$, $R^7$, $R^8$, $R^{10}$ and $R^{11}$ are all H; compound 81: n is 0, X is S, Y is O, $R^5$ is Me, $R^7$ is Cl, and $R^2$, $R^3$, $R^4$, $R^6$, $R^8$, $R^9$, $R^{10}$ and $R^{11}$ are all H; compound 82: n is 0, X is S, Y is O, $R^5$ is Me, $R^8$ is Cl, and $R^2$, $R^3$, $R^4$, $R^6$, $R^7$, $R^9$, $R^{10}$ and $R^{11}$ are all H; compound 83: n is 0, X is S, Y is O, $R^5$ is Me, $R^9$ is Cl, and $R^2$, $R^3$, $R^4$, $R^6$, $R^7$, $R^8$, $R^{10}$ and $R^{11}$ are all H; compound 84: n is 0, X is S, Y is O, $R^5$ is Me, $R^7$ is Br, and $R^2$, $R^3$, $R^4$, $R^6$, $R^8$, $R^9$, $R^{10}$ and $R^{11}$ are all H; compound 85: n is 0, X is S, Y is O, $R^5$ is Me, $R^8$ is Br, and $R^2$, $R^3$, $R^4$, $R^6$, $R^7$, $R^9$, $R^{10}$ and $R^{11}$ are all H; compound 86: n is 0, X is S, Y is O, $R^5$ is Me, $R^9$ is Br, and $R^2$, $R^3$, $R^4$, $R^6$, $R^7$, $R^8$, $R^{10}$ and $R^{11}$ are all H; compound 87: n is 0, X is S, Y is O, $R^5$ is Me, $R^7$ is I, and $R^2$, $R^3$, $R^4$, $R^6$, $R^8$, $R^9$, $R^{10}$ and $R^{11}$ are all H; compound 88: n is 0, X is S, Y is O, $R^5$ is Me, $R^8$ is I, and $R^2$, $R^3$, $R^4$, $R^6$, $R^7$, $R^9$, $R^{10}$ and $R^{11}$ are all H; compound 89: n is 0, X is S, Y is O, $R^5$ is Me, $R^9$ is I, and $R^2$, $R^3$, $R^4$, $R^6$, $R^7$, $R^8$, $R^{10}$ and $R^{11}$ are all H; compound 90: n is 0, X is S, Y is O, $R^5$ is Me, $R^7$ is $NO_2$, and $R^2$, $R^3$, $R^4$, $R^6$, $R^8$, $R^9$, $R^{10}$ and $R^{11}$ are all H; compound 91: n is 0, X is S, Y is O, $R^5$ is Me, $R^8$ is $NO_2$, and $R^2$, $R^3$, $R^4$, $R^6$, $R^7$, $R^9$, $R^{10}$ and $R^{11}$ are all H; compound 92: n is 0, X is S, Y is O, $R^5$ is Me, $R^9$ is $NO_2$, and $R^2$, $R^3$, $R^4$, $R^6$, $R^7$, $R^8$, $R^{10}$ and $R^{11}$ are all H; compound 93: n is 0, X is S, Y is O, $R^5$ is Me, $R^7$ is $CF_3$, and $R^2$, $R^3$, $R^4$, $R^6$, $R^8$, $R^9$, $R^{10}$ and $R^{11}$ are all H; compound 94: n is 0, X is S, Y is O, $R^5$ is Me, $R^8$ is $CF_3$, and $R^2$, $R^3$, $R^4$, $R^6$, $R^7$, $R^9$, $R^{10}$ and $R^{11}$ are all H; compound 95: n is 0, X is S, Y is O, $R^5$ is Me, $R^9$ is $CF_3$, and $R^2$, $R^3$, $R^4$, $R^6$, $R^7$, $R^8$, $R^{10}$ and $R^{11}$ are all H; compound 96: n is 0, X is S, Y is O, $R^5$ is Me, $R^7$ is $OCF_3$, and $R^2$, $R^3$, $R^4$, $R^6$, $R^8$, $R^9$, $R^{10}$ and $R^{11}$ are all H; compound 97: n is 0, X is S, Y is O, $R^5$ is Me, $R^8$ is $OCF_3$, and $R^2$, $R^3$, $R^4$, $R^6$, $R^7$, $R^9$, $R^{10}$ and $R^{11}$ are all H; compound 98: n is 0, X is S, Y is O, $R^5$ is Me, $R^9$ is $OCF_3$, and $R^2$, $R^3$, $R^4$, $R^6$, $R^7$, $R^8$, $R^{10}$ and $R^{11}$ are all H; compound 99: n is 0, X is S, Y is O, $R^5$ is Me, $R^7$ is $OCHF_2$, and $R^2$, $R^3$, $R^4$, $R^6$, $R^8$, $R^9$, $R^{10}$ and $R^{11}$ are all H; compound 100: n is 0, X is S, Y is O, $R^5$ is Me, $R^8$ is $OCHF_2$, and $R^2$, $R^3$, $R^4$, $R^6$, $R^7$, $R^9$, $R^{10}$ and $R^{11}$ are all H;compound 101: n is 0, X is S, Y is O, $R^5$ is Me, $R^9$ is $OCHF_2$, and $R^2$,

$R^3$, $R^4$, $R^6$, $R^7$, $R^8$, $R^{10}$ and $R^{11}$ are all H; compound 102: n is 0, X is S, Y is O, $R^5$ is Me, $R^9$ is $SCF_3$, and $R^2$, $R^3$, $R^4$, $R^6$, $R^7$, $R^8$, $R^{10}$ and $R^{11}$ are all H; compound 103: n is 0, X is S, Y is O, $R^5$ is Me, $R^7$ is $OCH_3$, and $R^2$, $R^3$, $R^4$, $R^6$, $R^8$, $R^9$, $R^{10}$ and $R^{11}$ are all H; compound 104: n is 0, X is S, Y is O, $R^5$ is Me, $R^8$ is $OCH_3$, and $R^2$, $R^3$, $R^4$, $R^6$, $R^7$, $R^9$, $R^{10}$ and $R^{11}$ are all H; compound 105: n is 0, X is S, Y is O, $R^5$ is Me, $R^9$ is $OCH_3$, and $R^2$, $R^3$, $R^4$, $R^6$, $R^7$, $R^8$, $R^{10}$ and $R^{11}$ are all H; compound 106: n is 0, X is S, Y is O, $R^5$ is Me, $R^9$ is $OC_2H_5$, and $R^2$, $R^3$, $R^4$, $R^6$, $R^8$, $R^7$, $R^{10}$ and $R^{11}$ are all H; compound 107: n is 0, X is S, Y is O, $R^5$ is Me, $R^7$ is CN, and $R^2$, $R^3$, $R^4$, $R^6$, $R^8$, $R^9$, $R^{10}$ and $R^{11}$ are all H; compound 108: n is 0, X is S, Y is O, $R^5$ is Me, $R^8$ is CN, and $R^2$, $R^3$, $R^4$, $R^6$, $R^7$, $R^9$, $R^{10}$ and $R^{11}$ are all H; compound 109: n is 0, X is S, Y is O, $R^5$ is Me, $R^9$ is CN, and $R^2$, $R^3$, $R^4$, $R^6$, $R^7$, $R^8$, $R^{10}$ and $R^{11}$ are all H; compound 110: n is 0, X is S, Y is O, $R^5$ and $R^7$ are Me, and $R^2$, $R^3$, $R^4$, $R^6$, $R^8$, $R^9$, $R^{10}$ and $R^{11}$ are all H; compound 111: n is 0, X is S, Y is O, $R^5$ and $R^8$ are Me, and $R^2$, $R^3$, $R^4$, $R^6$, $R^7$, $R^9$, $R^{10}$ and $R^{11}$ are all H; compound 112: n is 0, X is S, Y is O, $R^5$ and $R^9$ are Me, and $R^2$, $R^3$, $R^4$, $R^6$, $R^7$, $R^8$, $R^{10}$ and $R^{11}$ are all H; compound 113: n is 0, X is S, Y is O, $R^5$ is Me, $R^7$ is $CH_3SO_2$, and $R^2$, $R^3$, $R^4$, $R^6$, $R^8$, $R^9$, $R^{10}$ and $R^{11}$ are all H; compound 114: n is 0, X is S, Y is O, $R^5$ is Me, $R^8$ is $CH_3SO_2$, and $R^2$, $R^3$, $R^4$, $R^6$, $R^7$, $R^9$, $R^{10}$ and $R^{11}$ are all H; compound 115: n is 0, X is S, Y is O, $R^5$ is Me, $R^9$ is $CH_3SO_2$, and $R^2$, $R^3$, $R^4$, $R^6$, $R^7$, $R^8$, $R^{10}$ and $R^{11}$ are all H; compound 116: n is 0, X is S, Y is O, $R^5$ is Me, $R^7$ is $C_2H_5SO_2$, and $R^2$, $R^3$, $R^4$, $R^6$, $R^8$, $R^9$, $R^{10}$ and $R^{11}$ are all H; compound 117: n is 0, X is S, Y is O, $R^5$ is Me, $R^7$ and $R^8$ are both Cl, and $R^2$, $R^3$, $R^4$, $R^6$, $R^9$, $R^{10}$ and $R^{11}$ are all H; compound 118: n is 0, X is S, Y is O, $R^5$ is Me, $R^7$ and $R^9$ are both Cl, and $R^2$, $R^3$, $R^4$, $R^6$, $R^8$, $R^{10}$ and $R^{11}$ are all H; compound 119: n is 0, X is S, Y is O, $R^5$ is Me, $R^7$ and $R^{10}$ are both Cl, and $R^2$, $R^3$, $R^4$, $R^6$, $R^8$, $R^9$ and $R^{11}$ are all H; compound 120: n is 0, X is S, Y is O, $R^5$ is Me, $R^7$ and $R^{11}$ are both Cl, and $R^2$, $R^3$, $R^4$, $R^6$, $R^8$, $R^9$ and $R^{10}$ are all H; compound 121: n is 0, X is S, Y is O, $R^5$ is Me, $R^8$ and $R^9$ are both Cl, and $R^2$, $R^3$, $R^4$, $R^6$, $R^7$, $R^{10}$ and $R^{11}$ are all H; compound 122: n is 0, X is S, Y is O, $R^5$ and $R^7$ are Me, $R^9$ is Cl, and $R^2$, $R^3$, $R^4$, $R^6$, $R^8$, $R^{10}$ and $R^{11}$ are all H; compound 123: n is 0, X is S, Y is O, $R^5$, $R^7$ and $R^8$ are all Me, and $R^2$, $R^3$, $R^4$, $R^6$, $R^9$, $R^{10}$ and $R^{11}$ are all H; compound 124: n is 0, X is S, Y is O, $R^5$, $R^7$ and $R^9$ are all Me, and $R^2$, $R^3$, $R^4$, $R^6$, $R^8$, $R^{10}$ and $R^{11}$ are all H; compound 125: n is 0, X is S, Y is O, $R^5$, $R^7$ and $R^{10}$ are all Me, and $R^2$, $R^3$, $R^4$, $R^6$, $R^8$, $R^9$ and $R^{11}$ are all H; compound 126: n is 0, X is S, Y is O, $R^5$, $R^7$ and $R^{11}$ are all Me, and $R^2$, $R^3$, $R^4$, $R^6$, $R^8$, $R^9$ and $R^{10}$ are all H; compound 127: n is 0, X is S, Y is O, $R^5$, $R^7$, $R^8$ and $R^{11}$ are all Me, and $R^2$, $R^3$, $R^4$, $R^6$, $R^9$ and $R^{10}$ are all H; compound 128: n is 0, X is S, Y is O, $R^5$, $R^7$, $R^9$ and $R^{11}$ are all Me, and $R^2$, $R^3$, $R^4$, $R^6$, $R^8$ and $R^{10}$ are all H; compound 129: n is 0, X is S, Y is O, $R^5$ is Me, $R^7$ and $R^9$ are both $CF_3$, and $R^2$, $R^3$, $R^4$, $R^6$, $R^8$, $R^{10}$ and $R^{11}$ are all H; compound 130: n is 0, X is S, Y is O, $R^5$ is Me, $R^8$ and $R^{10}$ are both $CF_3$, and $R^2$, $R^3$, $R^4$, $R^6$, $R^7$, $R^9$ and $R^{11}$ are all H; compound 131: n is 0, X is S, Y is O, $R^5$ is Me, $R^2$, $R^3$, $R^4$ and $R^6$ are all H, and $R^7$, $R^8$, $R^9$, $R^{10}$ and $R^{11}$ are all F; compound 132: n is 0, X is S, Y is O, $R^5$ is Me, $R^7$ is $CH_3CH_2$, and $R^2$, $R^3$, $R^4$, $R^6$, $R^8$, $R^9$, $R^{10}$ and $R^{11}$ are all H; compound 133: n is 0, X is S, Y is O, $R^5$ is Me, $R^8$ is $CH_3CH_2$, and $R^2$, $R^3$, $R^4$, $R^6$, $R^7$, $R^9$, $R^{10}$ and $R^{11}$ are all H; Compound 134: n is 0, X is S, Y is O, $R^5$ is Me, $R^9$ is $CH_3CH_2$, and $R^2$, $R^3$, $R^4$, $R^6$, $R^7$, $R^8$, $R^{10}$ and $R^{11}$ are all H;compound 135: n is 0, X is S, Y is O, $R^5$ is Me, $R^7$ is $CH(CH_3)_2$, and $R^2$, $R^3$, $R^4$, $R^6$, $R^8$, $R^9$, $R^{10}$ and $R^{11}$ are all H;compound 136: n is 0, X is S, Y is O, $R^5$ is Me, $R^8$ is $CH(CH_3)_2$, and $R^2$, $R^3$, $R^4$, $R^6$, $R^7$, $R^9$, $R^{10}$ and $R^{11}$ are all H;compound 137: n is 0, X is S, Y is O, $R^5$ is Me, $R^9$ is $CH(CH_3)_2$, and $R^2$, $R^3$, $R^4$, $R^6$, $R^7$, $R^8$, $R^{10}$ and $R^{11}$ are all H;compound 138: n is 0, X is S, Y is O, $R^5$ is Me, $R^7$ is $CH_3(CH_2)_2$, $R^2$, $R^3$, $R^4$, $R^6$, $R^8$, $R^9$, $R^{10}$ and $R^{11}$ are all H;compound 139: n is 0, X is S, Y is O, $R^5$ is Me, $R^7$ is

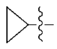,

and $R^2$, $R^3$, $R^4$, $R^6$, $R^8$, $R^9$, $R^{10}$ and $R^{11}$ are all H;compound 140: n is 0, X is S, Y is O, $R^5$ is Me, $R^7$ is

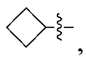,

and $R^2$, $R^3$, $R^4$, $R^6$, $R^8$, $R^9$, $R^{10}$ and $R^{11}$ are all H;compound 141: n is 0, X is S, Y is O, $R^5$ is Me, $R^7$ is $CO_2CH_3$, and $R^2$, $R^3$, $R^4$, $R^6$, $R^8$, $R^9$, $R^{10}$ and $R^{11}$ are all H; compound 142: n is 0, X is S, Y is O, $R^5$ is Me, $R^7$ and $R^8$ are cyclized to form

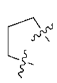,

and $R^2$, $R^3$, $R^4$, $R^6$, $R^9$, $R^{10}$ and $R^{11}$ are all H;compound 143: n is 0, X is S, Y is O, $R^5$ is Me, $R^8$ and $R^9$ are cyclized to form

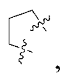,

and $R^2$, $R^3$, $R^4$, $R^6$, $R^7$, $R^{10}$ and $R^{11}$ are all H;compound 144: n is 0, X is S, Y is O, $R^5$ is Me, $R^8$ and $R^9$ are cyclized to form

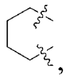,

and $R^2$, $R^3$, $R^4$, $R^6$, $R^7$, $R^{10}$ and $R^{11}$ are all H;compound 145: n is 0, X is S, Y is O, $R^5$ is Me, $R^7$ and $R^8$ are cyclized to form

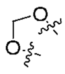,

and $R^2$, $R^3$, $R^4$, $R^6$, $R^9$, $R^{10}$ and $R^{11}$ are all H;compound 146: n is 0, X is S, Y is O, $R^5$ is Me, $R^8$ and $R^9$ are cyclized to form

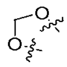,

and $R^2$, $R^3$, $R^4$, $R^6$, $R^7$, $R^{10}$ and $R^{11}$ are all H;compound 147: n is 0, X is S, Y is O, $R^5$ is Me, $R^7$ and $R^8$ are cyclized to form

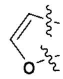

, and $R^2$, $R^3$, $R^4$, $R^6$, $R^9$, $R^{10}$ and $R^{11}$ are all H;compound 148: n is 0, X is S, Y is O, $R^5$ is Me, $R^7$ and $R^8$ are cyclized to

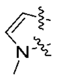,

and $R^2$, $R^3$, $R^4$, $R^6$, $R^9$, $R^{10}$ and $R^{11}$ are all H;compound 149: n is 0, X is S, Y is O, $R^5$ is Me, $R^7$ and $R^8$ are cyclized to form

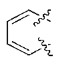,

and $R^2$, $R^3$, $R^4$, $R^6$, $R^9$, $R^{10}$ and $R^{11}$ are all H;compound 150: n is 0, X is S, Y is O, $R^5$ is Me, $R^7$ and $R^8$ are cyclized to form

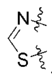,

and $R^2$, $R^3$, $R^4$, $R^6$, $R^9$, $R^{10}$ and $R^{11}$ are all H;compound 151: n is 0, X is S, Y is O, $R^5$ is Me, $R^7$ and $R^8$ are cyclized to form

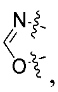,

and $R^2$, $R^3$, $R^4$, $R^6$, $R^9$, $R^{10}$ and $R^{11}$ are all H;compound 152: n is 0, X is S, Y is O, $R^5$ is Me, $R^8$ and $R^9$ are cyclized to form

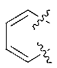,

and $R^2$, $R^3$, $R^4$, $R^6$, $R^7$, $R^{10}$ and $R^{11}$ are all H;compound 153: n is 0, X is S, Y is O, $R^5$ and $R^6$ are Me, and $R^2$, $R^3$, $R^4$, $R^7$, $R^8$, $R^9$, $R^{10}$ and $R^{11}$ are H;compound 154: n is 0, X is S, Y is O, $R^5$ and $R^6$ are both Me, $R^7$ is F, and $R^2$, $R^3$, $R^4$, $R^8$, $R^9$, $R^{10}$ and $R^{11}$ are all H;compound 155: n is 0, X is S, Y is O, $R^5$ and $R^6$ are both Me, $R^8$ is F, and $R^2$, $R^3$, $R^4$, $R^7$, $R^9$, $R^{10}$ and $R^{11}$ are all H;compound 156: n is 0, X is S, Y is O, $R^5$ and $R^6$ are both Me, $R^9$ is F, and $R^2$, $R^3$, $R^4$, $R^7$, $R^8$, $R^{10}$ and $R^{11}$ are all H;compound 157: n is 0, X is S, Y is O, $R^5$ and $R^6$ are both Me, $R^7$ is Cl, and $R^2$, $R^3$, $R^4$, $R^8$, $R^9$, $R^{10}$ and $R^{11}$ are all H;compound 158: n is 0, X is S, Y is O, $R^5$ and $R^6$ are both Me, $R^8$ is Cl, and $R^2$, $R^3$, $R^4$, $R^7$, $R^9$, $R^{10}$ and $R^{11}$ are all H;compound 159: n is 0, X is S, Y is O, $R^5$ and $R^6$ are both Me, $R^9$ is Cl, and $R^2$, $R^3$, $R^4$, $R^7$, $R^8$, $R^{10}$ and $R^{11}$ are all H;compound 160: n is 0, X is S, Y is O, $R^5$ and $R^6$ are both Me, $R^7$ is Br, $R^2$, $R^3$, $R^4$, $R^8$, $R^9$, $R^{10}$ and $R^{11}$ are all H;compound 161: n is 0, X is S, Y is O, $R^5$ and $R^6$ are both Me, $R^8$ is Br, $R^2$, $R^3$, $R^4$, $R^7$, $R^9$, $R^{10}$ and $R^{11}$ are all H;compound 162: n is 0, X is S, Y is O, $R^5$ and $R^6$ are both Me, $R^9$ is Br, $R^2$, $R^3$, $R^4$, $R^7$, $R^8$, $R^{10}$ and $R^{11}$ are all H;compound 163: n is 0, X is S, Y is O, $R^5$

and $R^6$ are both Me, $R^7$ is I, and $R^2$, $R^3$, $R^4$, $R^8$, $R^9$, $R^{10}$ and $R^{11}$ are all H;compound 164: n is 0, X is S, Y is O, $R^5$ and $R^6$ are both Me, $R^8$ is I, and $R^2$, $R^3$, $R^4$, $R^7$, $R^9$, $R^{10}$ and $R^{11}$ are all H;compound 165: n is 0, X is S, Y is O, $R^5$ and $R^6$ are both Me, $R^9$ is I, and $R^2$, $R^3$, $R^4$, $R^7$, $R^8$, $R^{10}$ and $R^{11}$ are all H;compound 166: n is 0, X is S, Y is O, $R^5$ and $R^6$ are both Me, $R^7$ is $NO_2$, $R^2$, $R^3$, $R^4$, $R^8$, $R^9$, $R^{10}$ and $R^{11}$ are all H;compound 167: n is 0, X is S, Y is O, $R^5$ and $R^6$ are both Me, $R^8$ is $NO_2$, $R^2$, $R^3$, $R^4$, $R^7$, $R^9$, $R^{10}$ and $R^{11}$ are all H; compound 168: n is 0, X is S, Y is O, $R^5$ and $R^6$ are both Me, $R^9$ is $NO_2$, $R^2$, $R^3$, $R^4$, $R^7$, $R^8$, $R^{10}$ and $R^{11}$ are all H;compound 169: n is 0, X is S, Y is O, $R^5$ and $R^6$ are both Me, $R^7$ is $CF_3$, $R^2$, $R^3$, $R^4$, $R^8$, $R^9$, $R^{10}$ and $R^{11}$ are all H;compound 170: n is 0, X is S, Y is O, $R^5$ and $R^6$ are both Me, $R^8$ is $CF_3$, $R^2$, $R^3$, $R^4$, $R^7$, $R^9$, $R^{10}$ and $R^{11}$ are all H;compound 171: n is 0, X is S, Y is O, $R^5$ and $R^6$ are both Me, $R^9$ is $CF_3$, $R^2$, $R^3$, $R^4$, $R^7$, $R^8$, $R^{10}$ and $R^{11}$ are all H;compound 172: n is 0, X is S, Y is O, $R^5$ and $R^6$ are both Me, $R^7$ is $OCF_3$, $R^2$, $R^3$, $R^4$, $R^8$, $R^9$, $R^{10}$ and $R^{11}$ are all H;compound 173: n is 0, X is S, Y is O, $R^5$ and $R^6$ are both Me, $R^8$ is $OCF_3$, $R^2$, $R^3$, $R^4$, $R^7$, $R^9$, $R^{10}$ and $R^{11}$ are all H;compound 174: n is 0, X is S, Y is O, $R^5$ and $R^6$ are both Me, $R^9$ is $OCF_3$, $R^2$, $R^3$, $R^4$, $R^7$, $R^8$, $R^{10}$ and $R^{11}$ are all H;compound 175: n is 0, X is S, Y is O, $R^5$ and $R^6$ are both Me, $R^7$ is $OCHF_2$, $R^2$, $R^3$, $R^4$, $R^8$, $R^9$, $R^{10}$ and $R^{11}$ are all H;compound 176: n is 0, X is S, Y is O, $R^5$ and $R^6$ are both Me, $R^8$ is $OCHF_2$, $R^2$, $R^3$, $R^4$, $R^7$, $R^9$, $R^{10}$ and $R^{11}$ are all H;compound 177: n is 0, X is S, Y is O, $R^5$ and $R^6$ are both Me, $R^9$ is $OCHF_2$, $R^2$, $R^3$, $R^4$, $R^7$, $R^8$, $R^{10}$ and $R^{11}$ are all H;compound 178: n is 0, X is S, Y is O, $R^5$ and $R^6$ are both Me, $R^9$ is $SCF_3$, $R^2$, $R^3$, $R^4$, $R^7$, $R^8$, $R^{10}$ and $R^{11}$ are all H;compound 179: n is 0, X is S, Y is O, $R^5$ and $R^6$ are both Me, $R^7$ is $OCH_3$, $R^2$, $R^3$, $R^4$, $R^8$, $R^9$, $R^{10}$ and $R^{11}$ are all H;compound 180: n is 0, X is S, Y is O, $R^5$ and $R^6$ are both Me, $R^8$ is $OCH_3$, $R^2$, $R^3$, $R^4$, $R^7$, $R^9$, $R^{10}$ and $R^{11}$ are all H;compound 181: n is 0, X is S, Y is O, $R^5$ and $R^6$ are both Me, $R^9$ is $OCH_3$, $R^2$, $R^3$, $R^4$, $R^7$, $R^8$, $R^{10}$ and $R^{11}$ are all H;compound 182: n is 0, X is S, Y is O, $R^5$ and $R^6$ are both Me, $R^9$ is $OC_2H_5$, $R^2$, $R^3$, $R^4$, $R^8$, $R^7$, $R^{10}$ and $R^{11}$ are all H;compound 183: n is 0, X is S, Y is O, $R^5$ and $R^6$ are both Me, $R^7$ is CN, and $R^2$, $R^3$, $R^4$, $R^8$, $R^9$, $R^{10}$ and $R^{11}$ are all H;compound 184: n is 0, X is S, Y is O, $R^5$ and $R^6$ are both Me, $R^8$ is CN, and $R^2$, $R^3$, $R^4$, $R^7$, $R^9$, $R^{10}$ and $R^{11}$ are all H;compound 185: n is 0, X is S, Y is O, $R^5$ and $R^6$ are both Me, $R^9$ is CN, and $R^2$, $R^3$, $R^4$, $R^7$, $R^8$, $R^{10}$ and $R^{11}$ are all H;compound 186: n is 0, X is S, Y is O, $R^5$, $R^6$ and $R^7$ are Me, and $R^2$, $R^3$, $R^4$, $R^8$, $R^9$, $R^{10}$ and $R^{11}$ are all H;compound 187: n is 0, X is S, Y is O, $R^5$, $R^6$ and $R^8$ are Me, and $R^2$, $R^3$, $R^4$, $R^7$, $R^9$, $R^{10}$ and $R^{11}$ are all H; compound 188: n is 0, X is S, Y is O, $R^5$, $R^6$ and $R^9$ are Me, and $R^2$, $R^3$, $R^4$, $R^7$, $R^8$, $R^{10}$ and $R^{11}$ are all H;compound 189: n is 0, X is S, Y is O, $R^5$ and $R^6$ are both Me, $R^7$ is $CH_3SO_2$, $R^2$, $R^3$, $R^4$, $R^8$, $R^9$, $R^{10}$ and $R^{11}$ are all H;compound 190: n is 0, X is S, Y is O, $R^5$ and $R^6$ are both Me, $R^8$ is $CH_3SO_2$, $R^2$, $R^3$, $R^4$, $R^7$, $R^9$, $R^{10}$ and $R^{11}$ are all H;compound 191: n is 0, X is S, Y is O, $R^5$ and $R^6$ are both Me, $R^9$ is $CH_3SO_2$, $R^2$, $R^3$, $R^4$, $R^7$, $R^8$, $R^{10}$ and $R^{11}$ are all H;compound 192: n is 0, X is S, Y is O, $R^5$ and $R^6$ are both Me, $R^7$ is $C_2H_5SO_2$, $R^2$, $R^3$, $R^4$, $R^8$, $R^9$, $R^{10}$ and $R^{11}$ are all H;compound 193: n is 0, X is S, Y is O, $R^5$ and $R^6$ are both Me, $R^7$ and $R^8$ are both Cl, and $R^2$, $R^3$, $R^4$, $R^9$, $R^{10}$ and $R^{11}$ are all H;compound 194: n is 0, X is S, Y is O, $R^5$ and $R^6$ are both Me, $R^7$ and $R^9$ are both Cl, and $R^2$, $R^3$, $R^4$, $R^8$, $R^{10}$ and $R^{11}$ are all H;compound 195: n is 0, X is S, Y is O, $R^5$ and $R^6$ are both Me, $R^7$ and $R^{10}$ are both Cl, and $R^2$, $R^3$, $R^4$, $R^8$, $R^9$ and $R^{11}$ are all H;compound 196: n is 0, X is S, Y is O, $R^5$ and $R^6$ are both Me, $R^7$ and $R^{11}$ are both Cl, and $R^2$, $R^3$, $R^4$, $R^8$, $R^9$ and $R^{10}$ are all H;compound 197: n is 0, X is S, Y is O, $R^5$ and $R^6$ are both Me, $R^8$ and $R^9$ are both Cl, and $R^2$, $R^3$, $R^4$, $R^7$, $R^{10}$ and $R^{11}$ are all H;compound 198: n is 0, X is S, Y is O, $R^5$, $R^6$ and $R^7$ are Me, $R^9$ is Cl, and $R^2$, $R^3$, $R^4$, $R^8$, $R^{10}$ and $R^{11}$ are all H;compound 199: n is 0, X is S, Y is O, $R^5$, $R^6$, $R^7$ and $R^8$ are all Me, and $R^2$, $R^3$, $R^4$, $R^9$, $R^{10}$ and $R^{11}$ are all H;compound 200: n is 0, X is S, Y is O, $R^5$, $R^6$, $R^7$ and $R^9$ are all Me, and $R^2$, $R^3$, $R^4$, $R^8$, $R^{10}$ and $R^{11}$ are all H;compound 201: n is 0, X is S, Y is O, $R^5$, $R^6$, $R^7$ and $R^{10}$ are all Me, and $R^2$, $R^3$, $R^4$, $R^8$, $R^9$ and $R^{11}$ are all H;compound 202: n is 0, X is S, Y is O, $R^5$, $R^6$, $R^7$ and $R^{11}$ are all Me, and $R^2$, $R^3$, $R^4$, $R^8$, $R^9$ and $R^{10}$ are all H;compound 203: n is 0, X is S, Y is O, $R^5$, $R^6$, $R^7$, $R^8$ and $R^{11}$ are all Me, and $R^2$, $R^3$, $R^4$, $R^9$ and $R^{10}$ are all H;compound 204: n is 0, X is S, Y is O, $R^5$, $R^6$, $R^7$, $R^9$ and $R^{11}$ are all Me, and $R^2$, $R^3$, $R^4$, $R^8$ and $R^{10}$ are all H;compound 205: n is 0, X is S, Y is O, $R^5$ and $R^6$ are both Me, $R^7$ and $R^9$ are both $CF_3$, and $R^2$, $R^3$, $R^4$, $R^8$, $R^{10}$ and $R^{11}$ are all H;compound 206: n is 0, X is S, Y is O, $R^5$ and $R^6$ are both Me, $R^8$ and $R^{10}$ are both $CF_3$, and $R^2$, $R^3$, $R^4$, $R^7$, $R^9$ and $R^{11}$ are all H;compound 207: n is 0, X is S, Y is O, $R^5$ and $R^6$ are Me, $R^2$, $R^3$ and $R^4$ are H, and $R^7$, $R^8$, $R^9$, $R^{10}$ and $R^{11}$ are F;compound 208: n is 0, X is S, Y is O, $R^5$ and $R^6$ are both Me, $R^7$ is $CH_3CH_2$, $R^2$, $R^3$, $R^4$, $R^8$, $R^9$, $R^{10}$ and $R^{11}$ are all H;compound 209: n is 0, X is S, Y is O, $R^5$ and $R^6$ are both Me, $R^8$ is $CH_3CH_2$, $R^2$, $R^3$, $R^4$, $R^7$, $R^9$, $R^{10}$ and $R^{11}$ are all H;compound 210: n is 0, X is S, Y is O, $R^5$ and $R^6$ are both Me, $R^9$ is $CH_3CH_2$, $R^2$, $R^3$, $R^4$, $R^7$, $R^8$, $R^{10}$ and $R^{11}$ are all H;compound 211: n is 0, X is S, Y is O, $R^5$ and $R^6$ are both Me, $R^7$ is $CH(CH_3)_2$, $R^2$, $R^3$, $R^4$, $R^8$, $R^9$, $R^{10}$ and $R^{11}$ are all H;compound 212: n is 0, X is S, Y is O, $R^5$ and $R^6$ are both Me, $R^8$ is $CH(CH_3)_2$, $R^2$, $R^3$, $R^4$, $R^7$, $R^9$, $R^{10}$ and $R^{11}$ are all H;compound 213: n is 0, X is S, Y is O, $R^5$ and $R^6$ are both Me, $R^9$ is $CH(CH_3)_2$, $R^2$, $R^3$, $R^4$, $R^7$, $R^8$, $R^{10}$ and $R^{11}$ are all H;compound 214: n is 0, X is S, Y is O, $R^5$ and $R^6$ are both Me, $R^7$ is $CH_3(CH_2)_2$, $R^2$, $R^3$, $R^4$, $R^8$, $R^9$, $R^{10}$ and $R^{11}$ are all H;compound 215: n is 0, X is S, Y is O, $R^5$ and $R^6$ are both Me, $R^7$ is $C(CH_3)_3$, $R^2$, $R^3$, $R^4$, $R^8$, $R^9$, $R^{10}$ and $R^{11}$ are all H;compound 216: n is 0, X is S, Y is O, $R^5$ and $R^6$ are both Me, $R^7$ is

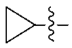,

$R^2$, $R^3$, $R^4$, $R^8$, $R^9$, $R^{10}$ and $R^{11}$ are all H;compound 217: n is 0, X is S, Y is O, $R^5$ and $R^6$ are both Me, $R^7$ is

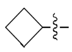,

$R^2$, $R^3$, $R^4$, $R^8$, $R^9$, $R^{10}$ and $R^{11}$ are all H;compound 218: n is 0, X is S, Y is O, $R^5$ and $R^6$ are both Me, $R^7$ is $CO_2CH_3$, $R^2$, $R^3$, $R^4$, $R^8$, $R^9$, $R^{10}$ and $R^{11}$ are all H;compound 219: n is 0, X is S, Y is O, $R^5$ and $R^6$ are both Me, $R^7$ and $R^8$ are cyclized to form

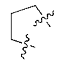,

and $R^2$, $R^3$, $R^4$, $R^9$, $R^{10}$ and $R^{11}$ are all H;compound 220: n is 0, X is S, Y is O, $R^5$ and $R^6$ are both Me, $R^8$ and $R^9$ are cyclized to form

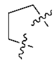,

and $R^2$, $R^3$, $R^4$, $R^7$, $R^{10}$ and $R^{11}$ are all H;compound 221: n is 0, X is S, Y is O, $R^5$ and $R^6$ are both Me, $R^8$ and $R^9$ are cyclized to form

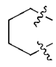,

and $R^2$, $R^3$, $R^4$, $R^7$, $R^{10}$ and $R^{11}$ are all H;compound 222: n is 0, X is S, Y is O, $R^5$ and $R^6$ are both Me, $R^7$ and $R^8$ are cyclized to form

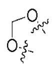, ;

and $R^2$, $R^3$, $R^4$, $R^9$, $R^{10}$ and $R^{11}$ are all H;compound 223: n is 0, X is S, Y is O, $R^5$ and $R^6$ are both Me, $R^8$ and $R^9$ are cyclized to form

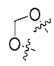,

and $R^2$, $R^3$, $R^4$, $R^7$, $R^{10}$ and $R^{11}$ are all H;compound 224: n is 0, X is S, Y is O, $R^5$ and $R^6$ are both Me, $R^7$ and $R^8$ are cyclized to form

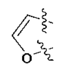,

and $R^2$, $R^3$, $R^4$, $R^9$, $R^{10}$ and $R^{11}$ are all H;compound 225: n is 0, X is S, Y is O, $R^5$ and $R^6$ are both Me, $R^7$ and $R^8$ are cyclized to form

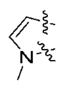

, and $R^2$, $R^3$, $R^4$, $R^9$, $R^{10}$ and $R^{11}$ are all H;compound 226: n is 0, X is S, Y is O, $R^5$ and $R^6$ are both Me, $R^7$ and $R^8$ are cyclized to form

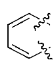,

and $R^2$, $R^3$, $R^4$, $R^9$, $R^{10}$ and $R^{11}$ are all H; compound 227: n is 0, X is S, Y is O, $R^5$ and $R^6$ are both Me, $R^7$ and $R^8$ are cyclized to form

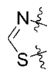,

and $R^2$, $R^3$, $R^4$, $R^9$, $R^{10}$ and $R^{11}$ are all H;compound 228: n is 0, X is S, Y is O, $R^5$ and $R^6$ are both Me, $R^7$ and $R^8$ are cyclized to form

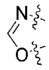,

and $R^2$, $R^3$, $R^4$, $R^9$, $R^{10}$ and $R^{11}$ are all H;compound 229: n is 0, X is S, Y is O, $R^5$ and $R^6$ are both Me, $R^8$ and $R^9$ are cyclized to form

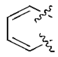,

and $R^2$, $R^3$, $R^4$, $R^7$, $R^{10}$ and $R^{11}$ are all H;compound 230: n is 0, X is S, Y is $CH_2$, $R^6$ is Me, and $R^2$, $R^3$, $R^4$, $R^5$, $R^7$, $R^8$, $R^9$, $R^{10}$ and $R^{11}$ are all H;compound 231: n is 0, X is S, Y is $CH_2$, $R^6$ is Me, $R^7$ is F, and $R^2$, $R^3$, $R^4$, $R^5$, $R^8$, $R^9$, $R^{10}$ and $R^{11}$ are all H;compound 232: n is 0, X is S, Y is $CH_2$, $R^6$ is Me, $R^8$ is F, and $R^2$, $R^3$, $R^4$, $R^5$, $R^7$, $R^9$, $R^{10}$ and $R^{11}$ are all H;compound 233: n is 0, X is S, Y is $CH_2$, $R^6$ is Me, $R^9$ is F, and $R^2$, $R^3$, $R^4$, $R^5$, $R^7$, $R^8$, $R^{10}$ and $R^{11}$ are all H;compound 234: n is 0, X is S, Y is $CH_2$, $R^6$ is Me, $R^7$ is Cl, and $R^2$, $R^3$, $R^4$, $R^5$, $R^8$, $R^9$, $R^{10}$ and $R^{11}$ are all H;compound 235: n is 0, X is S, Y is $CH_2$, $R^6$ is Me, $R^8$ is Cl, and $R^2$, $R^3$, $R^4$, $R^5$, $R^7$, $R^9$, $R^{10}$ and $R^{11}$ are all H;compound 236: n is 0, X is S, Y is $CH_2$, $R^6$ is Me, $R^9$ is Cl, and $R^2$, $R^3$, $R^4$, $R^5$, $R^7$, $R^8$, $R^{10}$ and $R^{11}$ are all H;compound 237: n is 0, X is S, Y is $CH_2$, $R^6$ is Me, $R^7$ is Br, and $R^2$, $R^3$, $R^4$, $R^5$, $R^8$, $R^9$, $R^{10}$ and $R^{11}$ are all H;compound 238: n is 0, X is S, Y is $CH_2$, $R^6$ is Me, $R^8$ is Br, and $R^2$, $R^3$, $R^4$, $R^5$, $R^7$, $R^9$, $R^{10}$ and $R^{11}$ are all H;compound 239: n is 0, X is S, Y is $CH_2$, $R^6$ is Me, $R^9$ is Br, and $R^2$, $R^3$, $R^4$, $R^5$, $R^7$, $R^8$, $R^{10}$ and $R^{11}$ are all H;compound 240: n is 0, X is S, Y is $CH_2$, $R^6$ is Me, $R^7$ is I, and $R^2$, $R^3$, $R^4$, $R^5$, $R^8$, $R^9$, $R^{10}$ and $R^{11}$ are all H;compound 241: n is 0, X is S, Y is $CH_2$, $R^6$ is Me, $R^8$ is I, and $R^2$, $R^3$, $R^4$, $R^5$, $R^7$, $R^9$, $R^{10}$ and $R^{11}$ are all H;compound 242: n is 0, X is S, Y is $CH_2$, $R^6$ is Me, $R^9$ is I, and $R^2$, $R^3$, $R^4$, $R^5$, $R^7$, $R^8$, $R^{10}$ and $R^{11}$ are all H;compound 243: n is 0, X is S, Y is $CH_2$, $R^6$ is Me, $R^7$ is $NO_2$, and $R^2$, $R^3$, $R^4$, $R^5$, $R^8$, $R^9$, $R^{10}$ and $R^{11}$ are all H;compound 244: n is 0, X is S, Y is $CH_2$, $R^6$ is Me, $R^8$ is $NO_2$, and $R^2$, $R^3$, $R^4$, $R^5$, $R^7$, $R^9$, $R^{10}$ and $R^{11}$ are all H;compound 245: n is 0, X is S, Y is $CH_2$, $R^6$ is Me, $R^9$ is $NO_2$, and $R^2$, $R^3$, $R^4$, $R^5$, $R^7$, $R^8$, $R^{10}$ and $R^{11}$ are all H;compound 246: n is 0, X is S, Y is $CH_2$, $R^6$ is Me, $R^7$ is $CF_3$, and $R^2$, $R^3$, $R^4$, $R^5$, $R^8$, $R^9$, $R^{10}$ and $R^{11}$ are all H;compound 247: n is 0, X is S, Y is $CH_2$, $R^6$ is Me, $R^8$ is $CF_3$, and $R^2$, $R^3$, $R^4$, $R^5$, $R^7$, $R^9$, $R^{10}$ and $R^{11}$ are all H;compound 248: n is 0, X is S, Y is $CH_2$, $R^6$ is Me, $R^9$ is $CF_3$, and $R^2$, $R^3$, $R^4$, $R^5$, $R^7$, $R^8$, $R^{10}$ and $R^{11}$ are all H; compound 249: n is 0, X is S, Y is $CH_2$, $R^6$ is Me, $R^7$ is $OCF_3$, and $R^2$, $R^3$, $R^4$, $R^5$, $R^8$, $R^9$, $R^{10}$ and $R^{11}$ are all H;compound 250: n is 0, X is S, Y is $CH_2$, $R^6$ is Me, $R^8$ is $OCF_3$, and $R^2$, $R^3$, $R^4$, $R^5$, $R^7$, $R^9$, $R^{10}$ and $R^{11}$ are all H;compound 251: n is 0, X is S, Y is $CH_2$, $R^6$ is Me, $R^9$ is $OCF_3$, and $R^2$, $R^3$, $R^4$, $R^5$, $R^7$, $R^8$, $R^{10}$ and $R^{11}$ are all H;compound 252: n is 0, X is S, Y is $CH_2$, $R^6$ is Me, $R^7$ is $OCHF_2$, and $R^2$, $R^3$, $R^4$, $R^5$, $R^8$, $R^9$, $R^{10}$ and $R^{11}$ are all H;compound 253: n is 0, X is S, Y is $CH_2$, $R^6$ is Me, $R^8$ is $OCHF_2$, and $R^2$, $R^3$, $R^4$, $R^5$, $R^7$, $R^9$, $R^{10}$ and $R^{11}$ are all H;compound 254: n is 0, X is S, Y is $CH_2$, $R^6$ is Me, $R^9$ is $OCHF_2$, and $R^2$, $R^3$, $R^4$, $R^5$, $R^7$, $R^8$, $R^{10}$ and $R^{11}$ are all H;compound 255: n is 0, X is S, Y is $CH_2$, $R^6$ is Me, $R^9$ is $SCF_3$, and $R^2$, $R^3$, $R^4$, $R^5$, $R^7$, $R^8$, $R^{10}$ and $R^{11}$ are all H;compound 256: n is 0, X is S, Y is $CH_2$, $R^6$ is Me, $R^7$ is $OCH_3$, and $R^2$, $R^3$, $R^4$, $R^5$, $R^8$, $R^9$, $R^{10}$ and $R^{11}$ are all H;compound 257: n is 0, X is S, Y is $CH_2$, $R^6$ is Me, $R^8$ is $OCH_3$, and $R^2$, $R^3$, $R^4$, $R^5$, $R^7$, $R^9$, $R^{10}$ and $R^{11}$ are all H;compound 258: n is 0, X is S, Y is $CH_2$, $R^6$ is Me, $R^9$ is $OCH_3$, and $R^2$, $R^3$, $R^4$, $R^5$, $R^7$, $R^8$, $R^{10}$ and $R^{11}$ are all H;compound 259: n is 0, X is S, Y is $CH_2$, $R^6$ is Me, $R^9$ is $OC_2H_5$, $R^2$, $R^3$, $R^4$, $R^5$, $R^7$, $R^8$, $R^{10}$ and $R^{11}$ are all H;compound 260: n is 0, X is S, Y is $CH_2$, $R^6$ is Me, $R^7$ is CN, and $R^2$, $R^3$, $R^4$, $R^5$, $R^8$, $R^9$, $R^{10}$ and $R^{11}$ are all H;compound 261: n is 0, X is S, Y is $CH_2$, $R^6$ is Me, $R^8$ is CN, and $R^2$, $R^3$, $R^4$, $R^5$, $R^7$, $R^9$, $R^{10}$ and $R^{11}$ are all H;compound 262: n is 0, X is S, Y is $CH_2$, $R^6$ is Me, $R^9$ is CN, and $R^2$, $R^3$, $R^4$, $R^5$, $R^7$, $R^8$, $R^{10}$ and $R^{11}$ are all H;compound 263: n is 0, X is S, Y is $CH_2$, $R^6$ and $R^7$ are Me, and $R^2$, $R^3$, $R^4$, $R^5$, $R^8$, $R^9$, $R^{10}$ and $R^{11}$ are all H;compound 264: n is 0, X is S, Y is $CH_2$, $R^6$ and $R^8$ are Me, and $R^2$, $R^3$, $R^4$, $R^5$, $R^7$, $R^9$, $R^{10}$ and $R^{11}$ are all H;compound 265: n is 0, X is S, Y is $CH_2$, $R^6$ and $R^9$ are Me, and $R^2$, $R^3$, $R^4$, $R^5$, $R^7$, $R^8$, $R^{10}$ and $R^{11}$ are all H;compound 266: n is 0, X is S, Y is $CH_2$, $R^6$ is Me, $R^7$ is $CH_3SO_2$, $R^2$, $R^3$, $R^4$, $R^5$, $R^8$, $R^9$, $R^{10}$ and $R^{11}$ are all H;compound 267: n is 0, X is S, Y is $CH_2$, $R^6$ is Me, $R^8$ is $CH_3SO_2$, $R^2$, $R^3$, $R^4$, $R^5$, $R^7$, $R^9$, $R^{10}$ and $R^{11}$ are all H;compound 268: n is 0, X is S, Y is $CH_2$, $R^6$ is Me, $R^9$ is $CH_3SO_2$, $R^2$, $R^3$, $R^4$, $R^5$, $R^7$, $R^8$, $R^{10}$ and $R^{11}$ are all H;compound 269: n is 0, X is S, Y is $CH_2$, $R^6$ is Me, $R^7$ is $C_2H_5SO_2$, $R^2$, $R^3$, $R^4$, $R^5$, $R^8$, $R^9$, $R^{10}$ and $R^{11}$ are all H;compound 270: n is 0, X is S, Y is $CH_2$, $R^6$ is Me, $R^7$ and $R^8$ are both Cl, and $R^2$, $R^3$, $R^4$, $R^5$, $R^9$, $R^{10}$ and $R^{11}$ are all H;compound 271: n is 0, X is S, Y is $CH_2$, $R^6$ is Me, $R^7$ and $R^9$ are both Cl, and $R^2$, $R^3$, $R^4$, $R^5$, $R^8$, $R^{10}$ and $R^{11}$ are all H;compound 272: n is 0, X is S, Y is $CH_2$, $R^6$ is Me, $R^7$ and $R^{10}$ are both Cl, and $R^2$, $R^3$, $R^4$, $R^5$, $R^8$, $R^9$ and $R^{11}$ are all H;compound 273: n is 0, X is S, Y is $CH_2$, $R^6$ is Me, $R^7$ and $R^{11}$ are both Cl, and $R^2$, $R^3$, $R^4$, $R^5$, $R^8$, $R^9$ and $R^{10}$ are all H;compound 274: n is 0, X is S, Y is $CH_2$, $R^6$ is Me, $R^8$ and $R^9$ are both Cl, and $R^2$, $R^3$, $R^4$, $R^5$, $R^7$, $R^{10}$ and $R^{11}$ are all H;compound 275: n is 0, X is S, Y is $CH_2$, $R^6$ and $R^7$ are Me, $R^9$ is Cl, and $R^2$, $R^3$, $R^4$, $R^5$, $R^8$, $R^{10}$ and $R^{11}$ are all H;compound 276: n is 0, X is S, Y is $CH_2$, $R^6$, $R^7$ and $R^8$ are all Me, and $R^2$, $R^3$, $R^4$, $R^5$, $R^9$, $R^{10}$ and $R^{11}$ are all H;compound 277: n is 0, X is S, Y is $CH_2$, $R^6$, $R^7$ and $R^9$ are all Me, and $R^2$, $R^3$, $R^4$, $R^5$, $R^8$, $R^{10}$ and $R^{11}$ are all H;compound 278: n is 0, X is S, Y is $CH_2$, $R^6$, $R^7$ and $R^{10}$ are all Me, and $R^2$, $R^3$, $R^4$, $R^5$, $R^8$, $R^9$ and $R^{11}$ are all H;compound 279: n is 0, X is S, Y is $CH_2$, $R^6$, $R^7$ and $R^{11}$ are all Me, and $R^2$, $R^3$, $R^4$, $R^5$, $R^8$, $R^9$ and $R^{10}$ are all H;compound 280: n is 0, X is S, Y is $CH_2$, $R^6$, $R^7$, $R^8$ and $R^{11}$ are all Me, and

$R^2$, $R^3$, $R^4$, $R^5$, $R^9$ and $R^{10}$ are all H;compound 281: n is 0, X is S, Y is $CH_2$, $R^6$, $R^7$, $R^9$ and $R^{11}$ are all Me, and $R^2$, $R^3$, $R^4$, $R^5$, $R^8$ and $R^{10}$ are all H;compound 282: n is 0, X is S, Y is $CH_2$, $R^6$ is Me, $R^7$ and $R^9$ are both $CF_3$, and $R^2$, $R^3$, $R^4$, $R^5$, $R^8$, $R^{10}$ and $R^{11}$ are all H;compound 283: n is 0, X is S, Y is $CH_2$, $R^6$ is Me, $R^8$ and $R^{10}$ are both $CF_3$, and $R^2$, $R^3$, $R^4$, $R^5$, $R^7$, $R^9$ and $R^{11}$ are all H;compound 284: n is 0, X is S, Y is $CH_2$, $R^6$ is Me, $R^2$, $R^3$, $R^4$ and $R^5$ are all H, and $R^7$, $R^8$, $R^9$, $R^{10}$ and $R^{11}$ are all F;compound 285: n is 0, X is S, Y is $CH_2$, $R^6$ is Me, $R^7$ is $CH_3CH_2$, $R^2$, $R^3$, $R^4$, $R^5$, $R^8$, $R^9$, $R^{10}$ and $R^{11}$ are all H;compound 286: n is 0, X is S, Y is $CH_2$, $R^6$ is Me, $R^8$ is $CH_3CH_2$, $R^2$, $R^3$, $R^4$, $R^5$, $R^7$, $R^9$, $R^{10}$ and $R^{11}$ are all H;compound 287: n is 0, X is S, Y is $CH_2$, $R^6$ is Me, $R^9$ is $CH_3CH_2$, $R^2$, $R^3$, $R^4$, $R^5$, $R^7$, $R^8$, $R^{10}$ and $R^{11}$ are all H;compound 288: n is 0, X is S, Y is $CH_2$, $R^6$ is Me, $R^7$ is $CH(CH_3)_2$, $R^2$, $R^3$, $R^4$, $R^5$, $R^8$, $R^9$, $R^{10}$ and $R^{11}$ are all H;compound 289: n is 0, X is S, Y is $CH_2$, $R^6$ is Me, $R^8$ is $CH(CH_3)_2$, $R^2$, $R^3$, $R^4$, $R^5$, $R^7$, $R^9$, $R^{10}$ and $R^{11}$ are all H;compound 290: n is 0, X is S, Y is $CH_2$, $R^6$ is Me, $R^9$ is $CH(CH_3)_2$, $R^2$, $R^3$, $R^4$, $R^5$, $R^7$, $R^8$, $R^{10}$ and $R^{11}$ are all H;compound 291: n is 0, X is S, Y is $CH_2$, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;Compound 292: n is 0, X is S, Y is $CH_2$, $R^6$ is Me, $R^7$ is $C(CH_3)_3$, $R^2$, $R^3$, $R^4$, $R^5$, $R^8$, $R^9$, $R^{10}$ and $R^{11}$ are all H;compound 293: n is 0, X is S, Y is $CH_2$, $R^6$ is Me, $R^7$ is

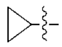,

$R^2$, $R^3$, $R^4$, $R^5$, $R^8$, $R^9$, $R^{10}$ and $R^{11}$ are all H;compound 294: n is 0, X is S, Y is $CH_2$, $R^6$ is Me, $R^7$ is

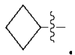,

$R^2$, $R^3$, $R^4$, $R^5$, $R^8$, $R^9$, $R^{10}$ and $R^{11}$ are all H;compound 295: n is 0, X is S, Y is $CH_2$, $R^6$ is Me, $R^7$ is $CO_2CH_3$, $R^2$, $R^3$, $R^4$, $R^5$, $R^8$, $R^9$, $R^{10}$ and $R^{11}$ are all H;compound 296: n is 0, X is S, Y is $CH_2$, $R^6$ is Me, $R^7$ and $R^8$ are cyclized to form

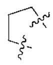,

and $R^2$, $R^3$, $R^4$, $R^5$, $R^9$, $R^{10}$ and $R^{11}$ are all H;compound 297: n is 0, X is S, Y is $CH_2$, $R^6$ is Me, $R^8$ and $R^9$ are cyclized to form

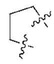,

and $R^2$, $R^3$, $R^4$, $R^5$, $R^7$, $R^{10}$ and $R^{11}$ are all H;compound 298: n is 0, X is S, Y is $CH_2$, $R^6$ is Me, $R^8$ and $R^9$ are cyclized to form

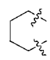,

and $R^2$, $R^3$, $R^4$, $R^5$, $R^7$, $R^{10}$ and $R^{11}$ are all H;compound 299: n is 0, X is S, Y is $CH_2$, $R^6$ is Me, $R^7$ and $R^8$ are cyclized to form

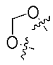,

and $R^2$, $R^3$, $R^4$, $R^5$, $R^9$, $R^{10}$ and $R^{11}$ are all H;compound 300: n is 0, X is S, Y is $CH_2$, $R^6$ is Me, $R^8$ and $R^9$ are cyclized to form

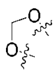,

and $R^2$, $R^3$, $R^4$, $R^5$, $R^7$, $R^{10}$ and $R^{11}$ are all H;compound 300: n is 0, X is S, Y is $CH_2$, $R^6$ is Me, $R^8$ and $R^9$ are cyclized to form

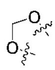,

and $R^2$, $R^3$, $R^4$, $R^5$, $R^7$, $R^{10}$ and $R^{11}$ are all H;compound 301: n is 0, X is S, Y is $CH_2$, $R^6$ is Me, $R^7$ and $R^8$ are cyclized to form

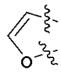,

and $R^2$, $R^3$, $R^4$, $R^5$, $R^9$, $R^{10}$ and $R^{11}$ are all H;compound 302: n is 0, X is S, Y is $CH_2$, $R^6$ is Me, $R^7$ and $R^8$ are cyclized to form

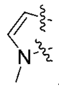,

and $R^2$, $R^3$, $R^4$, $R^5$, $R^9$, $R^{10}$ and $R^{11}$ are all H;compound 303: n is 0, X is S, Y is $CH_2$, $R^6$ is Me, $R^7$ and $R^8$ are cyclized to form

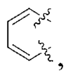,

and $R^2$, $R^3$, $R^4$, $R^5$, $R^9$, $R^{10}$ and $R^{11}$ are all H;compound 304: n is 0, X is S, Y is $CH_2$, $R^6$ is Me, $R^7$ and $R^8$ are cyclized to form

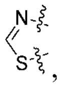,

and $R^2$, $R^3$, $R^4$, $R^5$, $R^9$, $R^{10}$ and $R^{11}$ are all H;compound 305: n is 0, X is S, Y is $CH_2$, $R^6$ is Me, $R^7$ and $R^8$ are cyclized to form

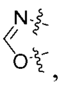,

and $R^2$, $R^3$, $R^4$, $R^5$, $R^9$, $R^{10}$ and $R^{11}$ are all H;compound 306: n is 0, X is S, Y is $CH_2$, $R^6$ is Me, $R^8$ and $R^9$ are cyclized to form

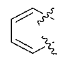,

and $R^2$, $R^3$, $R^4$, $R^5$, $R^7$, $R^{10}$ and $R^{11}$ are all H;compound 307: n is 0, X is S, Y is NMe, $R^6$ is Me, $R^2$, $R^3$, $R^4$, $R^5$, $R^7$, $R^8$, $R^9$, $R^{10}$ and $R^{11}$ are all H;compound 308: n is 0, X is S, Y is NMe, $R^6$ is Me, $R^7$ is F, and $R^2$, $R^3$, $R^4$, $R^5$, $R^8$, $R^9$, $R^{10}$ and $R^{11}$ are all H;compound 309: n is 0, X is S, Y is NMe, $R^6$ is Me, $R^8$ is F, and $R^2$, $R^3$, $R^4$, $R^5$, $R^7$, $R^9$, $R^{10}$ and $R^{11}$ are all H;compound 310: n is 0, X is S, Y is NMe, $R^6$ is Me, $R^9$ is F, and $R^2$, $R^3$, $R^4$, $R^5$, $R^7$, $R^8$, $R^{10}$ and $R^{11}$ are all H;compound 311: n is 0, X is S, Y is NMe, $R^6$ is Me, $R^7$ is Cl, and $R^2$, $R^3$, $R^4$, $R^5$, $R^8$, $R^9$, $R^{10}$ and $R^{11}$ are all H;compound 312: n is 0, X is S, Y is NMe, $R^6$ is Me, $R^8$ is Cl, and $R^2$, $R^3$, $R^4$, $R^5$, $R^7$, $R^9$, $R^{10}$ and $R^{11}$ are all H;compound 313: n is 0, X is S, Y is NMe, $R^6$ is Me, $R^9$ is Cl, and $R^2$, $R^3$, $R^4$, $R^5$, $R^7$, $R^8$, $R^{10}$ and $R^{11}$ are all H;compound 314: n is 0, X is S, Y is NMe, $R^6$ is Me, $R^7$ is Br, and $R^2$, $R^3$, $R^4$, $R^5$, $R^8$, $R^9$, $R^{10}$ and $R^{11}$ are all H;compound 315: n is 0, X is S, Y is NMe, $R^6$ is Me, $R^8$ is Br, and $R^2$, $R^3$, $R^4$, $R^5$, $R^7$, $R^9$, $R^{10}$ and $R^{11}$ are all H;compound 316: n is 0, X is S, Y is NMe, $R^6$ is Me, $R^9$ is Br, and $R^2$, $R^3$, $R^4$, $R^5$, $R^7$, $R^8$, $R^{10}$ and $R^{11}$ are all H;compound 317: n is 0, X is S, Y is NMe, $R^6$ is Me, $R^7$ is I, and $R^2$, $R^3$, $R^4$, $R^5$, $R^8$, $R^9$, $R^{10}$ and $R^{11}$ are all H;compound 318: n is 0, X is S, Y is NMe, $R^6$ is Me, $R^8$ is I, and $R^2$, $R^3$, $R^4$, $R^5$, $R^7$, $R^9$, $R^{10}$ and $R^{11}$ are all H;compound 319: n is 0, X is S, Y is NMe, $R^6$ is Me, $R^9$ is I, and $R^2$, $R^3$, $R^4$, $R^5$, $R^7$, $R^8$, $R^{10}$ and $R^{11}$ are all H;compound 320: n is 0, X is S, Y is NMe, $R^6$ is Me, $R^7$ is $NO_2$, and $R^2$, $R^3$, $R^4$, $R^5$, $R^8$, $R^9$, $R^{10}$ and $R^{11}$ are all H;compound 321: n is 0, X is S, Y is NMe, $R^6$ is Me, $R^8$ is $NO_2$, and $R^2$, $R^3$, $R^4$, $R^5$, $R^7$, $R^9$, $R^{10}$ and $R^{11}$ are all H;compound 322: n is 0, X is S, Y is NMe, $R^6$ is Me, $R^9$ is $NO_2$, and $R^2$, $R^3$, $R^4$, $R^5$, $R^7$, $R^8$, $R^{10}$ and $R^{11}$ are all H;compound 323: n is 0, X is S, Y is NMe, $R^6$ is Me, $R^7$ is $CF_3$, and $R^2$, $R^3$, $R^4$, $R^5$, $R^8$, $R^9$, $R^{10}$ and $R^{11}$ are all H;compound 324: n is 0, X is S, Y is NMe, $R^6$ is Me, $R^8$ is $CF_3$, and $R^2$, $R^3$, $R^4$, $R^5$, $R^7$, $R^9$, $R^{10}$ and $R^{11}$ are all H;compound 325: n is 0, X is S, Y is NMe, $R^6$ is Me, $R^9$ is $CF_3$, and $R^2$, $R^3$, $R^4$, $R^5$, $R^7$, $R^8$, $R^{10}$ and $R^{11}$ are all H;compound 326: n is 0, X is S, Y is NMe, $R^6$ is Me, $R^7$ is $OCF_3$, and $R^2$, $R^3$, $R^4$, $R^5$, $R^8$, $R^9$, $R^{10}$ and $R^{11}$ are all H;compound 327: n is 0, X is S, Y is NMe, $R^6$ is Me, $R^8$ is $OCF_3$, and $R^2$, $R^3$, $R^4$, $R^5$, $R^7$, $R^9$, $R^{10}$ and $R^{11}$ are all H;compound 328: n is 0, X is S, Y is NMe, $R^6$ is Me, $R^9$ is $OCF_3$, and $R^2$, $R^3$, $R^4$, $R^5$, $R^7$, $R^8$, $R^{10}$ and $R^{11}$ are all H;compound 329: n is 0, X is S, Y is NMe, $R^6$ is Me, $R^7$ is $OCHF_2$, and $R^2$, $R^3$, $R^4$, $R^5$, $R^8$, $R^9$, $R^{10}$ and $R^{11}$ are all H;compound 330: n is 0, X is S, Y is NMe, $R^6$ is Me, $R^8$ is $OCHF_2$, and $R^2$, $R^3$, $R^4$, $R^5$, $R^7$, $R^9$, $R^{10}$ and $R^{11}$ are all H;compound 331: n is 0, X is S, Y is NMe, $R^6$ is Me, $R^9$ is $OCHF_2$, and $R^2$, $R^3$, $R^4$, $R^5$, $R^7$, $R^8$, $R^{10}$ and $R^{11}$ are all H;compound 332: n is 0, X is S, Y is NMe, $R^6$ is Me, $R^9$ is $SCF_3$, and $R^2$, $R^3$, $R^4$, $R^5$, $R^7$, $R^8$, $R^{10}$ and $R^{11}$ are all H;compound 333: n is 0, X is S, Y is NMe, $R^6$ is Me, $R^7$ is $OCH_3$, and $R^2$, $R^3$, $R^4$, $R^5$, $R^8$, $R^9$, $R^{10}$ and $R^{11}$ are all H;compound 334: n is 0, X is S, Y is NMe, $R^6$ is Me, $R^8$ is $OCH_3$, and $R^2$, $R^3$, $R^4$, $R^5$, $R^7$, $R^9$, $R^{10}$ and $R^{11}$ are all H;compound 335: n is 0, X is S, Y is NMe, $R^6$ is Me, $R^9$ is $OCH_3$, and $R^2$, $R^3$, $R^4$, $R^5$, $R^7$, $R^8$, $R^{10}$ and $R^{11}$ are all H;compound 336: n is 0, X is S, Y is NMe,

$R^6$ is Me, $R^9$ is $OC_2H_5$, and $R^2$, $R^3$, $R^4$, $R^5$, $R^7$, $R^8$, $R^{10}$ and $R^{11}$ are all H;compound 337: n is 0, X is S, Y is NMe, $R^6$ is Me, $R^7$ is CN, and $R^2$, $R^3$, $R^4$, $R^5$, $R^8$, $R^9$, $R^{10}$ and $R^{11}$ are all H;compound 338: n is 0, X is S, Y is NMe, $R^6$ is Me, $R^8$ is CN, and $R^2$, $R^3$, $R^4$, $R^5$, $R^7$, $R^9$, $R^{10}$ and $R^{11}$ are all H;compound 339: n is 0, X is S, Y is NMe, $R^6$ is Me, $R^9$ is CN, and $R^2$, $R^3$, $R^4$, $R^5$, $R^7$, $R^8$, $R^{10}$ and $R^{11}$ are all H;compound 340: n is 0, X is S, Y is NMe, $R^6$ is Me, $R^7$ is $CH_3$, and $R^2$, $R^3$, $R^4$, $R^5$, $R^8$, $R^9$, $R^{10}$ and $R^{11}$ are all H;compound 341: n is 0, X is S, Y is NMe, $R^6$ is Me, $R^8$ is $CH_3$, and $R^2$, $R^3$, $R^4$, $R^5$, $R^7$, $R^9$, $R^{10}$ and $R^{11}$ are all H;compound 342: n is 0, X is S, Y is NMe, $R^6$ is Me, $R^9$ is $CH_3$, and $R^2$, $R^3$, $R^4$, $R^5$, $R^7$, $R^8$, $R^{10}$ and $R^{11}$ are all H;compound 343: n is 0, X is S, Y is NMe, $R^6$ is Me, $R^7$ is $CH_3SO_2$, and $R^2$, $R^3$, $R^4$, $R^5$, $R^8$, $R^9$, $R^{10}$ and $R^{11}$ are all H;compound 344: n is 0, X is S, Y is NMe, $R^6$ is Me, $R^8$ is $CH_3SO_2$, and $R^2$, $R^3$, $R^4$, $R^5$, $R^7$, $R^9$, $R^{10}$ and $R^{11}$ are all H;compound 345: n is 0, X is S, Y is NMe, $R^6$ is Me, $R^9$ is $CH_3SO_2$, and $R^2$, $R^3$, $R^4$, $R^5$, $R^7$, $R^8$, $R^{10}$ and $R^{11}$ are all H;compound 346: n is 0, X is S, Y is NMe, $R^6$ is Me, $R^7$ is $C_2H_5SO_2$, $R^2$, $R^3$, $R^4$, $R^5$, $R^8$, $R^9$, $R^{10}$ and $R^{11}$ are all H;compound 347: n is 0, X is S, Y is NMe, $R^6$ is Me, $R^7$ and $R^8$ are both Cl, and $R^2$, $R^3$, $R^4$, $R^5$, $R^9$, $R^{10}$ and $R^{11}$ are all H;compound 348: n is 0, X is S, Y is NMe, $R^6$ is Me, $R^7$ and $R^9$ are both Cl, and $R^2$, $R^3$, $R^4$, $R^5$, $R^8$, $R^{10}$ and $R^{11}$ are all H;compound 349: n is 0, X is S, Y is NMe, $R^6$ is Me, $R^7$ and $R^{10}$ are both Cl, and $R^2$, $R^3$, $R^4$, $R^5$, $R^8$, $R^9$ and $R^{11}$ are all H;compound 350: n is 0, X is S, Y is NMe, $R^6$ is Me, $R^7$ and $R^{11}$ are both Cl, and $R^2$, $R^3$, $R^4$, $R^5$, $R^8$, $R^9$ and $R^{10}$ are all H;compound 351: n is 0, X is S, Y is NMe, $R^6$ is Me, $R^8$ and $R^9$ are both Cl, and $R^2$, $R^3$, $R^4$, $R^5$, $R^7$, $R^{10}$ and $R^{11}$ are all H;compound 352: n is 0, X is S, Y is NMe, $R^6$ and $R^7$ are Me, $R^9$ is Cl, and $R^2$, $R^3$, $R^4$, $R^5$, $R^8$, $R^{10}$ and $R^{11}$ are all H;compound 353: n is 0, X is S, Y is NMe, $R^6$, $R^7$ and $R^8$ are Me, and $R^2$, $R^3$, $R^4$, $R^5$, $R^9$, $R^{10}$ and $R^{11}$ are all H;compound 354: n is 0, X is S, Y is NMe, $R^6$, $R^7$ and $R^9$ are Me, and $R^2$, $R^3$, $R^4$, $R^5$, $R^8$, $R^{10}$ and $R^{11}$ are all H;compound 355: n is 0, X is S, Y is NMe, $R^6$, $R^7$ and $R^{10}$ are Me, and $R^2$, $R^3$, $R^4$, $R^5$, $R^8$, $R^9$ and $R^{11}$ are all H;compound 356: n is 0, X is S, Y is NMe, $R^6$, $R^7$ and $R^{11}$ are Me, and $R^2$, $R^3$, $R^4$, $R^5$, $R^8$, $R^9$ and $R^{10}$ are all H;compound 357: n is 0, X is S, Y is NMe, $R^6$, $R^7$, $R^8$ and $R^{11}$ are Me, and $R^2$, $R^3$, $R^4$, $R^5$, $R^9$ and $R^{10}$ are all H;compound 358: n is 0, X is S, Y is NMe, $R^6$, $R^7$, $R^9$ and $R^{11}$ are Me, and $R^2$, $R^3$, $R^4$, $R^5$, $R^8$ and $R^{10}$ are all H;compound 359: n is 0, X is S, Y is NMe, $R^6$ is Me, $R^7$ and $R^9$ are both $CF_3$, $R^2$, $R^3$, $R^4$, $R^5$, $R^8$, $R^{10}$ and $R^{11}$ are all H;compound 360: n is 0, X is S, Y is NMe, $R^6$ is Me, $R^8$ and $R^{10}$ are both $CF_3$, $R^2$, $R^3$, $R^4$, $R^5$, $R^7$, $R^9$ and $R^{11}$ are all H;compound 361: n is 0, X is S, Y is NMe, $R^6$ is Me, $R^2$, $R^3$, $R^4$ and $R^5$ are all H, and $R^7$, $R^8$, $R^9$, $R^{10}$ and $R^{11}$ are all F;compound 362: n is 0, X is S, Y is NMe, $R^6$ is Me, $R^7$ is $CH_3CH_2$, and $R^2$, $R^3$, $R^4$, $R^5$, $R^8$, $R^9$, $R^{10}$ and $R^{11}$ are all H;compound 363: n is 0, X is S, Y is NMe, $R^6$ is Me, $R^8$ is $CH_3CH_2$, and $R^2$, $R^3$, $R^4$, $R^5$, $R^7$, $R^9$, $R^{10}$ and $R^{11}$ are all H;compound 364: n is 0, X is S, Y is NMe, $R^6$ is Me, $R^9$ is $CH_3CH_2$, and $R^2$, $R^3$, $R^4$, $R^5$, $R^7$, $R^8$, $R^{10}$ and $R^{11}$ are all H;compound 365: n is 0, X is S, Y is NMe, $R^6$ is Me, $R^7$ is $CH(CH_3)_2$, $R^2$, $R^3$, $R^4$, $R^5$, $R^8$, $R^9$, $R^{10}$ and $R^{11}$ are all H;compound 366: n is 0, X is S, Y is NMe, $R^6$ is Me, $R^8$ is $CH(CH_3)_2$, $R^2$, $R^3$, $R^4$, $R^5$, $R^7$, $R^9$, $R^{10}$ and $R^{11}$ are all H;compound 367: n is 0, X is S, Y is NMe, $R^6$ is Me, $R^9$ is $CH(CH_3)_2$, $R^2$, $R^3$, $R^4$, $R^5$, $R^7$, $R^8$, $R^{10}$ and $R^{11}$ are all H;compound 368: n is 0, X is S, Y is NMe, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;compound 369: n is 0, X is S, Y is NMe, $R^6$ is Me, $R^7$ is $C(CH_3)_3$, $R^2$, $R^3$, $R^4$, $R^5$, $R^8$, $R^9$, $R^{10}$ and $R^{11}$ are all H;compound 370: n is 0, X is S, Y is NMe, $R^6$ is Me, $R^7$ is

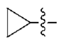

, and $R^2$, $R^3$, $R^4$, $R^5$, $R^8$, $R^9$, $R^{10}$ and $R^{11}$ are all H;compound 371: n is 0, X is S, Y is NMe, $R^6$ is Me, $R^7$ is

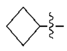,

and $R^2$, $R^3$, $R^4$, $R^5$, $R^8$, $R^9$, $R^{10}$ and $R^{11}$ are all H;compound 372: n is 0, X is S, Y is NMe, $R^6$ is Me, $R^7$ is $CO_2CH_3$, and $R^2$, $R^3$, $R^4$, $R^5$, $R^8$, $R^9$, $R^{10}$ and $R^{11}$ are all H;compound 373: n is 0, X is S, Y is NMe, $R^6$ is Me, $R^7$ and $R^8$ are cyclized to form

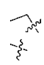,

and $R^2$, $R^3$, $R^4$, $R^5$, $R^9$, $R^{10}$ and $R^{11}$ are all H;compound 374: n is 0, X is S, Y is NMe, $R^6$ is Me, $R^8$ and $R^9$ are cyclized to form

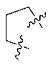,

and $R^2$, $R^3$, $R^4$, $R^5$, $R^7$, $R^{10}$ and $R^{11}$ are all H;compound 375: n is 0, X is S, Y is NMe, $R^6$ is Me, $R^8$ and $R^9$ are cyclized to form

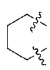,

and $R^2$, $R^3$, $R^4$, $R^5$, $R^7$, $R^{10}$ and $R^{11}$ are all H;compound 376: n is 0, X is S, Y is NMe, $R^6$ is Me, $R^7$ and $R^8$ are cyclized to form

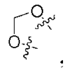,

and $R^2$, $R^3$, $R^4$, $R^5$, $R^9$, $R^{10}$ and $R^{11}$ are all H;compound 377: n is 0, X is S, Y is NMe, $R^6$ is Me, $R^8$ and $R^9$ are cyclized to form

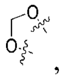,

and $R^2$, $R^3$, $R^4$, $R^5$, $R^7$, $R^{10}$ and $R^{11}$ are all H;compound 378: n is 0, X is S, Y is NMe, $R^6$ is Me, $R^7$ and $R^8$ are cyclized to form

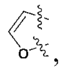,

and $R^2$, $R^3$, $R^4$, $R^5$, $R^9$, $R^{10}$ and $R^{11}$ are all H;compound 379: n is 0, X is S, Y is NMe, $R^6$ is Me, $R^7$ and $R^8$ are cyclized to form

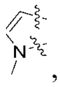,

and $R^2$, $R^3$, $R^4$, $R^5$, $R^9$, $R^{10}$ and $R^{11}$ are all H;compound 380: n is 0, X is S, Y is NMe, $R^6$ is Me, $R^7$ and $R^8$ are cyclized to form

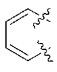,

and $R^2$, $R^3$, $R^4$, $R^5$, $R^9$, $R^{10}$ and $R^{11}$ are all H;compound 381: n is 0, X is S, Y is NMe, $R^6$ is Me, $R^7$ and $R^8$ are cyclized to form

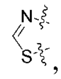,

and $R^2$, $R^3$, $R^4$, $R^5$, $R^9$, $R^{10}$ and $R^{11}$ are all H;compound 382: n is 0, X is S, Y is NMe, $R^6$ is Me, $R^7$ and $R^8$ are cyclized to form

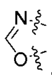, a

nd $R^2$, $R^3$, $R^4$, $R^5$, $R^9$, $R^{10}$ and $R^{11}$ are all H;compound 383: n is 0, X is S, Y is NMe, $R^6$ is Me, $R^8$ and $R^9$ are cyclized to form

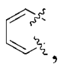,

and $R^2$, $R^3$, $R^4$, $R^5$, $R^7$, $R^{10}$ and $R^{11}$ are all H;compound 873: n is 0, X is S, Y is NH, $R^6$ is Me, and $R^2$, $R^3$, $R^4$, $R^5$, $R^7$, $R^8$, $R^9$, $R^{10}$ and $R^{11}$ are all H;compound 883: n is 0, X is S, Y is S, $R^6$ is Me, $R^2$, $R^3$, $R^4$, $R^5$, $R^7$, $R^8$, $R^9$, $R^{10}$ and $R^{11}$ are all H;compound 384: n is 0, X is S, Y is O, $R^2$ and $R^6$ are Me, and $R^3$, $R^4$, $R^5$, $R^7$, $R^8$, $R^9$, $R^{10}$ and $R^{11}$ are all H;compound 385: n is 0, X is S, Y is O, $R^2$ and $R^6$ are Me, $R^7$ is F, and $R^3$, $R^4$, $R^5$, $R^8$, $R^9$, $R^{10}$ and $R^{11}$ are all H;compound 386: n is 0, X is S, Y is O, $R^2$ and $R^6$ are Me, $R^8$ is F, and $R^3$, $R^4$, $R^5$, $R^7$, $R^9$, $R^{10}$ and $R^{11}$ are all H;compound 387: n is 0, X is S, Y is O, $R^2$ and $R^6$ are Me, $R^9$ is F, and $R^3$, $R^4$, $R^5$, $R^7$, $R^8$, $R^{10}$ and $R^{11}$ are all H;compound 388: n is 0, X is S, Y is O, $R^2$ and $R^6$ are Me, $R^7$ is Cl, and $R^3$, $R^4$, $R^5$, $R^8$, $R^9$, $R^{10}$ and $R^{11}$ are all H;compound 389: n is 0, X is S, Y is O, $R^2$ and $R^6$ are Me, $R^8$ is Cl, and $R^3$, $R^4$, $R^5$, $R^7$, $R^9$, $R^{10}$ and $R^{11}$ are all H;compound 390: n is 0, X is S, Y is O, $R^2$ and $R^6$ are Me, $R^9$ is Cl, and $R^3$, $R^4$, $R^5$, $R^7$, $R^8$, $R^{10}$ and $R^{11}$ are all H;compound 391: n is 0, X is S, Y is O, $R^2$ and $R^6$ are Me, $R^7$ is Br, and $R^3$, $R^4$, $R^5$, $R^8$, $R^9$, $R^{10}$ and $R^{11}$ are all H;compound 392: n is 0, X is S, Y is O, $R^2$ and $R^6$ are Me, $R^8$ is Br, and $R^3$, $R^4$, $R^5$, $R^7$, $R^9$, $R^{10}$ and $R^{11}$ are all H;compound 393: n is 0, X is S, Y is O, $R^2$ and $R^6$ are Me, $R^9$ is Br, and $R^3$, $R^4$, $R^5$, $R^7$, $R^8$, $R^{10}$ and $R^{11}$ are all H;compound 394: n is 0, X is S, Y is O, $R^2$ and $R^6$ are Me, $R^7$ is I, and $R^3$, $R^4$, $R^5$, $R^8$, $R^9$, $R^{10}$ and $R^{11}$ are all H;compound 395: n is 0, X is S, Y is O, $R^2$ and $R^6$ are Me, $R^8$ is I, and $R^3$, $R^4$, $R^5$, $R^7$, $R^9$, $R^{10}$ and $R^{11}$ are all H;compound 396: n is 0, X is S, Y is O, $R^2$ and $R^6$ are Me, $R^9$ is I, and $R^3$, $R^4$, $R^5$, $R^7$, $R^8$, $R^{10}$ and $R^{11}$ are all H;compound 397: n is 0, X is S, Y is O, $R^2$ and $R^6$ are Me, $R^7$ is $NO_2$, and $R^3$, $R^4$, $R^5$, $R^8$, $R^9$, $R^{10}$ and $R^{11}$ are all H;compound 398: n is 0, X is S, Y is O, $R^2$ and $R^6$ are Me, $R^8$ is $NO_2$, and $R^3$, $R^4$, $R^5$, $R^7$, $R^9$, $R^{10}$ and $R^{11}$ are all H;compound 399: n is 0, X is S, Y is O, $R^2$ and $R^6$ are Me, $R^9$ is $NO_2$, and $R^3$, $R^4$, $R^5$, $R^7$, $R^8$, $R^{10}$ and $R^{11}$

are all H;compound 400: n is 0, X is S, Y is O, $R^2$ and $R^6$ are Me, $R^7$ is $CF_3$, and $R^3$, $R^4$, $R^5$, $R^8$, $R^9$, $R^{10}$ and $R^{11}$ are all H;compound 401: n is 0, X is S, Y is O, $R^2$ and $R^6$ are Me, $R^8$ is $CF_3$, and $R^3$, $R^4$, $R^5$, $R^7$, $R^9$, $R^{10}$ and $R^{11}$ are all H;compound 402: n is 0, X is S, Y is O, $R^2$ and $R^6$ are Me, $R^9$ is $CF_3$, and $R^3$, $R^4$, $R^5$, $R^7$, $R^8$, $R^{10}$ and $R^{11}$ are all H;compound 403: n is 0, X is S, Y is O, $R^2$ and $R^6$ are Me, $R^7$ is $OCF_3$, and $R^3$, $R^4$, $R^5$, $R^8$, $R^9$, $R^{10}$ and $R^{11}$ are all H;compound 404: n is 0, X is S, Y is O, $R^2$ and $R^6$ are Me, $R^8$ is $OCF_3$, and $R^3$, $R^4$, $R^5$, $R^7$, $R^9$, $R^{10}$ and $R^{11}$ are all H;compound 405: n is 0, X is S, Y is O, $R^2$ and $R^6$ are Me, $R^9$ is $OCF_3$, and $R^3$, $R^4$, $R^5$, $R^7$, $R^8$, $R^{10}$ and $R^{11}$ are all H;compound 406: n is 0, X is S, Y is O, $R^2$ and $R^6$ are Me, $R^7$ is $OCHF_2$, and $R^3$, $R^4$, $R^5$, $R^8$, $R^9$, $R^{10}$ and $R^{11}$ are all H;compound 407: n is 0, X is S, Y is O, $R^2$ and $R^6$ are Me, $R^8$ is $OCHF_2$, and $R^3$, $R^4$, $R^5$, $R^7$, $R^9$, $R^{10}$ and $R^{11}$ are all H;compound 408: n is 0, X is S, Y is O, $R^2$ and $R^6$ are Me, $R^9$ is $OCHF_2$, and $R^3$, $R^4$, $R^5$, $R^7$, $R^8$, $R^{10}$ and $R^{11}$ are all H;compound 409: n is 0, X is S, Y is O, $R^2$ and $R^6$ are Me, $R^9$ is $SCF_3$, and $R^3$, $R^4$, $R^5$, $R^7$, $R^8$, $R^{10}$ and $R^{11}$ are all H;compound 410: n is 0, X is S, Y is O, $R^2$ and $R^6$ are Me, $R^7$ is $OCH_3$, and $R^3$, $R^4$, $R^5$, $R^8$, $R^9$, $R^{10}$ and $R^{11}$ are all H;compound 411: n is 0, X is S, Y is O, $R^2$ and $R^6$ are Me, $R^8$ is $OCH_3$, and $R^3$, $R^4$, $R^5$, $R^7$, $R^9$, $R^{10}$ and $R^{11}$ are all H;compound 412: n is 0, X is S, Y is O, $R^2$ and $R^6$ are Me, $R^9$ is $OCH_3$, and $R^3$, $R^4$, $R^5$, $R^7$, $R^8$, $R^{10}$ and $R^{11}$ are all H;compound 413: n is 0, X is S, Y is O, $R^2$ and $R^6$ are Me, $R^9$ is $OC_2H_5$, and $R^3$, $R^4$, $R^5$, $R^7$, $R^8$, $R^{10}$ and $R^{11}$ are all H;compound 414: n is 0, X is S, Y is O, $R^2$ and $R^6$ are Me, $R^7$ is CN, and $R^3$, $R^4$, $R^5$, $R^8$, $R^9$, $R^{10}$ and $R^{11}$ are all H;compound 415: n is 0, X is S, Y is O, $R^2$ and $R^6$ are Me, $R^8$ is CN, and $R^3$, $R^4$, $R^5$, $R^7$, $R^9$, $R^{10}$ and $R^{11}$ are all H;compound 416: n is 0, X is S, Y is O, $R^2$ and $R^6$ are Me, $R^9$ is CN, and $R^3$, $R^4$, $R^5$, $R^7$, $R^8$, $R^{10}$ and $R^{11}$ are all H;compound 417: n is 0, X is S, Y is O, $R^2$ and $R^6$ are Me, $R^7$ is $CH_3$, and $R^3$, $R^4$, $R^5$, $R^8$, $R^9$, $R^{10}$ and $R^{11}$ are all H;compound 418: n is 0, X is S, Y is O, $R^2$ and $R^6$ are Me, $R^8$ is $CH_3$, and $R^3$, $R^4$, $R^5$, $R^7$, $R^9$, $R^{10}$ and $R^{11}$ are all H;compound 419: n is 0, X is S, Y is O, $R^2$ and $R^6$ are Me, $R^9$ is $CH_3$, and $R^3$, $R^4$, $R^5$, $R^7$, $R^8$, $R^{10}$ and $R^{11}$ are all H;compound 420: n is 0, X is S, Y is O, $R^2$ and $R^6$ are Me, $R^7$ is $CH_3SO_2$, and $R^3$, $R^4$, $R^5$, $R^8$, $R^9$, $R^{10}$ and $R^{11}$ are all H;compound 421: n is 0, X is S, Y is O, $R^2$ and $R^6$ are Me, $R^8$ is $CH_3SO_2$, and $R^3$, $R^4$, $R^5$, $R^7$, $R^9$, $R^{10}$ and $R^{11}$ are all H;compound 422: n is 0, X is S, Y is O, $R^2$ and $R^6$ are Me, $R^9$ is $CH_3SO_2$, and $R^3$, $R^4$, $R^5$, $R^7$, $R^9$, $R^{10}$ and $R^{11}$ are all H;compound 423: n is 0, X is S, Y is O, $R^2$ and $R^6$ are Me, $R^7$ is $C_2H_5SO_2$, and $R^3$, $R^4$, $R^5$, $R^8$, $R^9$, $R^{10}$ and $R^{11}$ are all H;compound 424: n is 0, X is S, Y is O, $R^2$ and $R^6$ are Me, $R^7$ and $R^8$ are both Cl, and $R^3$, $R^4$, $R^5$, $R^9$, $R^{10}$ and $R^{11}$ are all H;compound 425: n is 0, X is S, Y is O, $R^2$ and $R^6$ are Me, $R^7$ and $R^9$ are both Cl, and $R^3$, $R^4$, $R^5$, $R^8$, $R^{10}$ and $R^{11}$ are all H;compound 426: n is 0, X is S, Y is O, $R^2$ and $R^6$ are Me, $R^7$ and $R^{10}$ are both Cl, and $R^3$, $R^4$, $R^5$, $R^8$, $R^9$ and $R^{11}$ are all H;compound 427: n is 0, X is S, Y is O, $R^2$ and $R^6$ are Me, $R^7$ and $R^{11}$ are both Cl, and $R^3$, $R^4$, $R^5$, $R^8$, $R^9$ and $R^{10}$ are all H; compound 428: n is 0, X is S, Y is O, $R^2$ and $R^6$ are Me, $R^8$ and $R^9$ are both Cl, and $R^3$, $R^4$, $R^5$, $R^7$, $R^{10}$ and $R^{11}$ are all H;compound 429: n is 0, X is S, Y is O, $R^2$, $R^6$ and $R^7$ are Me, $R^9$ is Cl, and $R^3$, $R^4$, $R^5$, $R^8$, $R^{10}$ and $R^{11}$ are all H;compound 430: n is 0, X is S, Y is O, $R^2$, $R^6$, $R^7$ and $R^8$ are Me, and $R^3$, $R^4$, $R^5$, $R^9$, $R^{10}$ and $R^{11}$ are all H;compound 431: n is 0, X is S, Y is O, $R^2$, $R^6$, $R^7$ and $R^9$ are Me, and $R^3$, $R^4$, $R^5$, $R^8$, $R^{10}$ and $R^{11}$ are all H;compound 432: n is 0, X is S, Y is O, $R^2$, $R^6$, $R^7$ and $R^{10}$ are Me, and $R^3$, $R^4$, $R^5$, $R^8$, $R^9$ and $R^{11}$ are all H;compound 433: n is 0, X is S, Y is O, $R^2$, $R^6$, $R^7$ and $R^{11}$ are Me, and $R^3$, $R^4$, $R^5$, $R^8$, $R^9$ and $R^{10}$ are all H;compound 434: n is 0, X is S, Y is O, $R^2$, $R^6$, $R^7$, $R^8$ and $R^{11}$ are Me, and $R^3$, $R^4$, $R^5$, $R^9$ and $R^{10}$ are all H;compound 435: n is 0, X is S, Y is O, $R^2$, $R^6$, $R^7$, $R^9$ and $R^{11}$ are Me, and $R^3$, $R^4$, $R^5$, $R^8$ and $R^{10}$ are all H;compound 436: n is 0, X is S, Y is O, $R^2$ and $R^6$ are Me, $R^7$ and $R^9$ are both $CF_3$, and $R^3$, $R^4$, $R^5$, $R^8$, $R^{10}$ and $R^{11}$ are all H;compound 437: n is 0, X is S, Y is O, $R^2$ and $R^6$ are Me, $R^8$ and $R^{10}$ are both $CF_3$, and $R^3$, $R^4$, $R^5$, $R^7$, $R^9$ and $R^{11}$ are all H;compound 438: n is 0, X is S, Y is O, $R^2$ and $R^6$ are Me, $R^3$, $R^4$ and $R^5$ are all H, and $R^7$, $R^8$, $R^9$, $R^{10}$ and $R^{11}$ are all F;compound 439: n is 0, X is S, Y is O, $R^2$ and $R^6$ are Me, $R^7$ is $CH_3CH_2$, $R^3$, $R^4$, $R^5$, $R^8$, $R^9$, $R^{10}$ and $R^{11}$ are all H;compound 440: n is 0, X is S, Y is O, $R^2$ and $R^6$ are Me, $R^8$ is $CH_3CH_2$, $R^3$, $R^4$, $R^5$, $R^7$, $R^9$, $R^{10}$ and $R^{11}$ are all H;compound 441: n is 0, X is S, Y is O, $R^2$ and $R^6$ are Me, $R^9$ is $CH_3CH_2$, $R^3$, $R^4$, $R^5$, $R^7$, $R^8$, $R^{10}$ and $R^{11}$ are all H;compound 442: n is 0, X is S, Y is O, $R^2$ and $R^6$ are Me, $R^7$ is $CH(CH_3)_2$, and $R^3$, $R^4$, $R^5$, $R^8$, $R^9$, $R^{10}$ and $R^{11}$ are all H;compound 443: n is 0, X is S, Y is O, $R^2$ and $R^6$ are Me, $R^8$ is $CH(CH_3)_2$, and $R^3$, $R^4$, $R^5$, $R^7$, $R^9$, $R^{10}$ and $R^{11}$ are all H;compound 444: n is 0, X is S, Y is O, $R^2$ and $R^6$ are Me, $R^9$ is $CH(CH_3)_2$, and $R^3$, $R^4$, $R^5$, $R^7$, $R^8$, $R^{10}$ and $R^{11}$ are all H;compound 445: n is 0, X is S, Y is O, $R^2$ and $R^6$ are Me, $R^7$ is $CH_3(CH_2)_2$, and $R^2$, $R^3$, $R^4$, $R^5$, $R^8$, $R^9$, $R^{10}$ and $R^{11}$ are all H;Compound 446: n is 0, X is S, Y is O, $R^2$ and $R^6$ are Me, $R^7$ is $C(CH_3)_3$, and $R^3$, $R^4$, $R^5$, $R^8$, $R^9$, $R^{10}$ and $R^{11}$ are all H;compound 447: n is 0, X is S, Y is O, $R^2$ and $R^6$ are Me, $R^7$ is

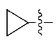,

$R^3$, $R^4$, $R^5$, $R^8$, $R^9$, $R^{10}$ and $R^{11}$ are all H;compound 448: n is 0, X is S, Y is O, $R^2$ and $R^6$ are Me, $R^7$ is

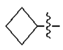,

$R^3$, $R^4$, $R^5$, $R^8$, $R^9$, $R^{10}$ and $R^{11}$ are all H;compound 449: n is 0, X is S, Y is O, $R^2$ and $R^6$ are Me, $R^7$ is $CO_2CH_3$, $R^3$, $R^4$, $R^5$, $R^8$, $R^9$, $R^{10}$ and $R^{11}$ are all H;compound 450: n is 0, X is S, Y is O, $R^2$ and $R^6$ are Me, $R^7$ and $R^8$ are cyclized to form

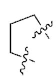,

and $R^3$, $R^4$, $R^5$, $R^9$, $R^{10}$ and $R^{11}$ are all H;compound 451: n is 0, X is S, Y is O, $R^2$ and $R^6$ are Me, $R^8$ and $R^9$ are cyclized to form

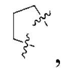,

and $R^3$, $R^4$, $R^5$, $R^7$, $R^{10}$ and $R^{11}$ are all H;compound 452: n is 0, X is S, Y is O, $R^2$ and $R^6$ are Me, $R^8$ and $R^9$ are cyclized to form

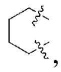,

and $R^3$, $R^4$, $R^5$, $R^7$, $R^{10}$ and $R^{11}$ are all H;compound 453: n is 0, X is S, Y is O, $R^2$ and $R^6$ are Me, $R^7$ and $R^8$ are cyclized to form

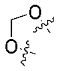, ;

and $R^3$, $R^4$, $R^5$, $R^9$, $R^{10}$ and $R^{11}$ are all H;compound 454: n is 0, X is S, Y is O, $R^2$ and $R^6$ are Me, $R^8$ and $R^9$ are cyclized to form

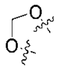,

and $R^3$, $R^4$, $R^5$, $R^7$, $R^{10}$ and $R^{11}$ are all H;compound 455: n is 0, X is S, Y is O, $R^2$ and $R^6$ are Me, $R^8$ and $R^9$ are cyclized to form

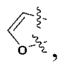,

and $R^3$, $R^4$, $R^5$, $R^7$, $R^{10}$ and $R^{11}$ are all H;compound 456: n is 0, X is S, Y is O, $R^2$ and $R^6$ are Me, $R^7$ and $R^8$ are cyclized to form

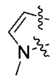,

and $R^3$, $R^4$, $R^5$, $R^9$, $R^{10}$ and $R^{11}$ are all H;compound 457: n is 0, X is S, Y is O, $R^2$ and $R^6$ are Me, $R^7$ and $R^8$ are cyclized to form

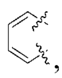,

and $R^3$, $R^4$, $R^5$, $R^9$, $R^{10}$ and $R^{11}$ are all H;compound 458: n is 0, X is S, Y is O, $R^2$ and $R^6$ are Me, $R^7$ and $R^8$ are cyclized to form

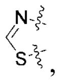,

and $R^3$, $R^4$, $R^5$, $R^9$, $R^{10}$ and $R^{11}$ are all H;compound 459: n is 0, X is S, Y is O, $R^2$ and $R^6$ are Me, $R^7$ and $R^8$ are cyclized to form

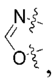,

and $R^3$, $R^4$, $R^5$, $R^9$, $R^{10}$ and $R^{11}$ are all H;compound 460: n is 0, X is S, Y is O, $R^2$ and $R^6$ are Me, $R^8$ and $R^9$ are cyclized to form

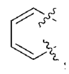

, and $R^3$, $R^4$, $R^5$, $R^7$, $R^{10}$ and $R^{11}$ are all H;compound 461: n is 0, X is S, Y is O, $R^2$ is $C_2H_5$, $R^6$ is Me, and $R^3$, $R^4$, $R^5$, $R^7$, $R^8$, $R^9$, $R^{10}$ and $R^{11}$ are all H;compound 462: n is 0, X is S, Y is O, $R^2$ is $C_2H_5$, $R^6$ is Me, $R^7$ is F, and $R^3$, $R^4$, $R^5$, $R^8$, $R^9$, $R^{10}$ and $R^{11}$ are all H;compound 463: n is 0, X is S, Y is O, $R^2$ is $C_2H_5$, $R^6$ is Me, $R^8$ is F, and $R^3$, $R^4$, $R^5$, $R^7$, $R^9$, $R^{10}$ and $R^{11}$ are all H;compound 464: n is 0, X is S, Y is O, $R^2$ is $C_2H_5$, $R^6$ is Me, $R^9$ is F, and $R^3$, $R^4$, $R^5$, $R^7$, $R^8$, $R^{10}$ and $R^{11}$ are all H;compound 465: n is 0, X is S, Y is O, $R^2$ is $C_2H_5$, $R^6$ is Me, $R^7$ is Cl, and $R^3$, $R^4$, $R^5$, $R^8$, $R^9$, $R^{10}$ and $R^{11}$ are all H;compound 466: n is 0, X is S, Y is O, $R^2$ is $C_2H_5$, $R^6$ is Me, $R^8$ is Cl, and $R^3$, $R^4$, $R^5$, $R^7$, $R^9$, $R^{10}$ and $R^{11}$ are all H;compound 467: n is 0, X is S, Y is O, $R^2$ is $C_2H_5$, $R^6$ is Me, $R^9$ is Cl, and $R^3$, $R^4$, $R^5$, $R^7$, $R^8$, $R^{10}$ and $R^{11}$ are all H;compound 468: n is 0, X is S, Y is O, $R^2$ is $C_2H_5$, $R^6$ is Me, $R^7$ is Br, $R^3$, $R^4$, $R^5$, $R^8$, $R^9$, $R^{10}$ and $R^{11}$ are all H;compound 469: n is 0, X is S, Y is O, $R^2$ is $C_2H_5$, $R^6$ is Me, $R^8$ is Br, $R^3$, $R^4$, $R^5$, $R^7$, $R^9$, $R^{10}$ and $R^{11}$ are all H;compound 470: n is 0, X is S, Y is O, $R^2$ is $C_2H_5$, $R^6$ is Me, $R^9$ is Br, $R^3$, $R^4$, $R^5$, $R^7$, $R^8$, $R^{10}$ and $R^{11}$ are all H;compound 471: n is 0, X is S, Y is O, $R^2$ is $C_2H_5$, $R^6$ is Me, $R^7$ is I, and $R^3$, $R^4$, $R^5$, $R^8$, $R^9$, $R^{10}$ and $R^{11}$ are all H;compound 472: n is 0, X is S, Y is O, $R^2$ is $C_2H_5$, $R^6$ is Me, $R^8$ is I, and $R^3$, $R^4$, $R^5$, $R^7$, $R^9$, $R^{10}$ and $R^{11}$ are all H;compound 473: n is 0, X is S, Y is O, $R^2$ is $C_2H_5$, $R^6$ is Me, $R^9$ is I, and $R^3$, $R^4$, $R^5$, $R^7$, $R^8$, $R^{10}$ and $R^{11}$ are all H;compound 474: n is 0, X is S, Y is O, $R^2$ is $C_2H_5$, $R^6$ is Me, $R^7$ is $NO_2$, $R^3$, $R^4$, $R^5$, $R^8$, $R^9$, $R^{10}$ and $R^{11}$ are all H;compound 475: n is 0, X is S, Y is O, $R^2$ is $C_2H_5$, $R^6$ is Me, $R^8$ is $NO_2$, $R^3$, $R^4$, $R^5$, $R^7$, $R^9$, $R^{10}$ and $R^{11}$ are all H;compound 476: n is 0, X is S, Y is O, $R^2$ is $C_2H_5$, $R^6$ is Me, $R^9$ is $NO_2$, $R^3$, $R^4$, $R^5$, $R^7$, $R^8$, $R^{10}$ and $R^{11}$ are all H;compound 477: n is 0, X is S, Y is O, $R^2$ is $C_2H_5$, $R^6$ is Me, $R^7$ is $CF_3$, $R^3$, $R^4$, $R^5$, $R^8$, $R^9$, $R^{10}$ and $R^{11}$ are all H;compound 478: n is 0, X is S, Y is O, $R^2$ is $C_2H_5$, $R^6$ is Me, $R^8$ is $CF_3$, $R^3$, $R^4$, $R^5$, $R^7$, $R^9$, $R^{10}$ and $R^{11}$ are all H;compound 479: n is 0, X is S, Y is O, $R^2$ is $C_2H_5$, $R^6$ is Me, $R^9$ is $CF_3$, $R^3$, $R^4$, $R^5$, $R^7$, $R^8$, $R^{10}$ and $R^{11}$ are all H;compound 480: n is 0, X is S, Y is O, $R^2$ is $C_2H_5$, $R^6$ is Me, $R^7$ is $OCF_3$, $R^3$, $R^4$, $R^5$, $R^8$, $R^9$, $R^{10}$ and $R^{11}$ are all H;compound 481: n is 0, X is S, Y is O, $R^2$ is $C_2H_5$, $R^6$ is Me, $R^8$ is $OCF_3$, $R^3$, $R^4$, $R^5$, $R^7$, $R^9$, $R^{10}$ and $R^{11}$ are all H;compound 482: n is 0, X is S, Y is O, $R^2$ is $C_2H_5$, $R^6$ is Me, $R^9$ is $OCF_3$, $R^3$, $R^4$, $R^5$, $R^7$, $R^8$, $R^{10}$ and $R^{11}$ are all H;compound 483: n is 0, X is S, Y is O, $R^2$ is $C_2H_5$, $R^6$ is Me, $R^7$ is $OCHF_2$, $R^3$, $R^4$, $R^5$, $R^8$, $R^9$, $R^{10}$ and $R^{11}$ are all H;compound 484: n is 0, X is S, Y is O, $R^2$ is $C_2H_5$, $R^6$ is Me, $R^8$ is $OCHF_2$, $R^3$, $R^4$, $R^5$, $R^7$, $R^9$, $R^{10}$ and $R^{11}$ are all H;compound 485: n is 0, X is S, Y is O, $R^2$ is $C_2H_5$, $R^6$ is Me, $R^9$ is $OCHF_2$, $R^3$, $R^4$, $R^5$, $R^7$, $R^8$, $R^{10}$ and $R^{11}$ are all H;compound 486: n is 0, X is S, Y is O, $R^2$ is $C_2H_5$, $R^6$ is Me, $R^9$ is $SCF_3$, $R^3$, $R^4$, $R^5$, $R^7$, $R^8$, $R^{10}$ and $R^{11}$ are all H;compound 487: n is 0, X is S, Y is O, $R^2$ is $C_2H_5$, $R^6$ is Me, $R^7$ is $OCH_3$, $R^3$, $R^4$, $R^5$, $R^8$, $R^9$, $R^{10}$ and $R^{11}$ are all H;compound 488: n is 0, X is S, Y is O, $R^2$ is $C_2H_5$, $R^6$ is Me, $R^8$ is $OCH_3$, $R^3$, $R^4$, $R^5$, $R^7$, $R^9$, $R^{10}$ and $R^{11}$ are all H;compound 489: n is 0, X is S, Y is O, $R^2$ is $C_2H_5$, $R^6$ is Me, $R^9$ is $OCH_3$, $R^3$, $R^4$, $R^5$, $R^7$, $R^8$, $R^{10}$ and $R^{11}$ are all H;compound 490: n is 0, X is S, Y is O, $R^2$ is $C_2H_5$, $R^6$ is Me, $R^9$ is $OC_2H_5$, $R^3$, $R^4$, $R^5$, $R^7$, $R^8$, $R^{10}$ and $R^{11}$ are all H;compound 491: n is 0, X is S, Y is O, $R^2$ is $C_2H_5$, $R^6$ is Me, $R^7$ is CN, $R^3$, $R^4$, $R^5$, $R^8$, $R^9$, $R^{10}$ and $R^{11}$ are all H;compound 492: n is 0, X is S, Y is O, $R^2$ is $C_2H_5$, $R^6$ is Me, $R^8$ is CN, $R^3$, $R^4$, $R^5$, $R^7$, $R^9$, $R^{10}$ and $R^{11}$ are all H;compound 493: n is 0, X is S, Y is O, $R^2$ is $C_2H_5$, $R^6$ is Me, $R^9$ is CN, $R^3$, $R^4$, $R^5$, $R^7$, $R^8$, $R^{10}$ and $R^{11}$ are all H;compound 494: n is 0, X is S, Y is O, $R^2$ is $C_2H_5$, $R^6$ is Me, $R^7$ is $CH_3$, $R^3$, $R^4$, $R^5$, $R^8$, $R^9$, $R^{10}$ and $R^{11}$ are all H;compound 495: n is 0, X is S, Y is O, $R^2$ is $C_2H_5$, $R^6$ is Me, $R^8$ is $CH_3$, $R^3$, $R^4$, $R^5$, $R^7$, $R^9$, $R^{10}$ and $R^{11}$ are all H;compound 496: n is 0, X is S, Y is O, $R^2$ is $C_2H_5$, $R^6$ is Me, $R^9$ is $CH_3$, $R^3$, $R^4$, $R^5$, $R^7$, $R^8$, $R^{10}$ and $R^{11}$ are all H;compound 497: n is 0, X is S, Y is O, $R^2$ is $C_2H_5$, $R^6$ is Me, $R^7$ is $CH_3SO_2$, $R^3$, $R^4$, $R^5$, $R^8$, $R^9$, $R^{10}$ and $R^{11}$ are all H;compound 498: n is 0, X is S, Y is O, $R^2$ is $C_2H_5$, $R^6$ is Me, $R^8$ is $CH_3SO_2$, $R^3$, $R^4$, $R^5$, $R^7$, $R^9$, $R^{10}$ and $R^{11}$ are all H;compound 499: n is 0, X is S, Y is O, $R^2$ is $C_2H_5$, $R^6$ is Me, $R^9$ is $CH_3SO_2$, $R^3$, $R^4$, $R^5$, $R^7$, $R^9$, $R^{10}$ and $R^{11}$ are all H;compound 500: n is 0, X is S, Y is O, $R^2$ is $C_2H_5$, $R^6$ is Me, $R^7$ is $C_2H_5SO_2$, $R^3$, $R^4$, $R^5$, $R^8$, $R^9$, $R^{10}$ and $R^{11}$ are all H;compound 501: n is 0, X is S, Y is O, $R^2$ is $C_2H_5$, $R^6$ is Me, $R^7$ and $R^8$ are both Cl, and $R^3$, $R^4$, $R^5$, $R^9$, $R^{10}$ and $R^{11}$ are all H;compound 502: n is 0, X is S, Y is O, $R^2$ is $C_2H_5$, $R^6$ is Me, $R^7$ and $R^9$ are both Cl, and $R^3$, $R^4$, $R^5$, $R^8$, $R^{10}$ and $R^{11}$ are all H;compound 503: n is 0, X is S, Y is O, $R^2$ is $C_2H_5$, $R^6$ is Me, $R^7$ and $R^{10}$ are both Cl, and $R^3$, $R^4$, $R^5$, $R^8$, $R^9$ and $R^{11}$ are all H;compound 504: n is 0, X is S, Y is O, $R^2$ is $C_2H_5$, $R^6$ is Me, $R^7$ and $R^{11}$ are both Cl, and $R^3$, $R^4$, $R^5$, $R^8$, $R^9$ and $R^{10}$ are all H;compound 505: n is 0, X is S, Y is O, $R^2$ is $C_2H_5$, $R^6$ is Me, $R^8$ and $R^9$ are both Cl, and $R^3$, $R^4$, $R^5$, $R^7$, $R^{10}$ and $R^{11}$ are all H;compound 506: n is 0, X is S, Y is O, $R^2$ is $C_2H_5$, $R^6$ and $R^7$ are Me, $R^9$ is Cl, and $R^3$, $R^4$, $R^5$, $R^8$, $R^{10}$ and $R^{11}$ are all H;compound 507: n is 0, X is S, Y is O, $R^2$ is $C_2H_5$, $R^6$, $R^7$ and $R^8$ are Me, and $R^3$, $R^4$, $R^5$, $R^9$, $R^{10}$ and $R^{11}$ are all H;compound 508: n is 0, X is S, Y is O, $R^2$ is $C_2H_5$, $R^6$, $R^7$ and $R^9$ are Me, and $R^3$, $R^4$, $R^5$, $R^8$, $R^{10}$ and $R^{11}$ are all H;compound 509: n is 0, X is S, Y is O, $R^2$ is $C_2H_5$, $R^6$, $R^7$ and $R^{10}$ are Me, and $R^3$, $R^4$, $R^5$, $R^8$, $R^9$ and $R^{11}$ are all H;compound 510: n is 0, X is S, Y is O, $R^2$ is $C_2H_5$, $R^7$ and $R^{11}$ are Me, and $R^3$, $R^4$, $R^5$, $R^8$, $R^9$ and $R^{10}$ are all H;compound 511: n is 0, X is S, Y is O, $R^2$ is $C_2H_5$, $R^6$, $R^7$, $R^8$ and $R^{11}$ are Me, and $R^3$, $R^4$, $R^5$, $R^9$ and $R^{10}$ are all H;compound 512: n is 0, X is S, Y is O, $R^2$ is $C_2H_5$, $R^6$, $R^7$, $R^9$ and $R^{11}$ are Me, and $R^3$, $R^4$, $R^5$, $R^8$ and $R^{10}$ are all H;compound 513: n is 0, X is S, Y is O, $R^2$ is $C_2H_5$, $R^6$ is Me, $R^7$ and $R^9$ are both $CF_3$, and $R^3$, $R^4$, $R^5$, $R^8$, $R^{10}$ and $R^{11}$ are all H;compound 514: n is 0, X is S, Y is O, $R^2$ is $C_2H_5$, $R^6$ is Me, $R^8$ and $R^{10}$ are both $CF_3$, and $R^3$, $R^4$, $R^5$, $R^7$, $R^9$ and $R^{11}$ are all H;compound 515: n is 0, X is S, Y is O, $R^2$ is $C_2H_5$, $R^6$ is Me, $R^3$, $R^4$ and $R^5$ are all H, and $R^7$, $R^8$, $R^9$, $R^{10}$ and $R^{11}$ are all F;compound 516: n is 0, X is S, Y is O, $R^2$ is $C_2H_5$, $R^6$ is Me, $R^7$ is $CH_3CH_2$, $R^3$, $R^4$, $R^5$,

$R^8$, $R^9$, $R^{10}$ and $R^{11}$ are all H;compound 517: n is 0, X is S, Y is O, $R^2$ is $C_2H_5$, $R^6$ is Me, $R^8$ is $CH_3CH_2$, $R^3$, $R^4$, $R^5$, $R^7$, $R^9$, $R^{10}$ and $R^{11}$ are all H;compound 518: n is 0, X is S, Y is O, $R^2$ is $C_2H_5$, $R^6$ is Me, $R^9$ is $CH_3CH_2$, $R^3$, $R^4$, $R^5$, $R^7$, $R^8$, $R^{10}$ and $R^{11}$ are all H;compound 519: n is 0, X is S, Y is O, $R^2$ is $C_2H_5$, $R^6$ is Me, $R^7$ is $CH(CH_3)_2$, $R^3$, $R^4$, $R^5$, $R^8$, $R^9$, $R^{10}$ and $R^{11}$ are all H;compound 520: n is 0, X is S, Y is O, $R^2$ is $C_2H_5$, $R^6$ is Me, $R^8$ is $CH(CH_3)_2$, $R^3$, $R^4$, $R^5$, $R^7$, $R^9$, $R^{10}$ and $R^{11}$ are all H;compound 521: n is 0, X is S, Y is O, $R^2$ is $C_2H_5$, $R^6$ is Me, $R^9$ is $CH(CH_3)_2$, $R^3$, $R^4$, $R^5$, $R^7$, $R^8$, $R^{10}$ and $R^{11}$ are all H;compound 522: n is 0, X is S, Y is O, $R^2$ is $C_2H_5$, $R^6$ is Me, $R^7$ is $CH_3(CH_2)_2$, and $R^2$, $R^3$, $R^4$, $R^5$, $R^8$, $R^9$, $R^{10}$ and $R^{11}$ are all H;compound 523: n is 0, X is S, Y is O, $R^2$ is $C_2H_5$, $R^6$ is Me, $R^7$ is $C(CH_3)_3$, $R^3$, $R^4$, $R^5$, $R^8$, $R^9$, $R^{10}$ and $R^{11}$ are all H;compound 524: n is 0, X is S, Y is O, $R^2$ is $C_2H_5$, $R^6$ is Me, $R^7$ is

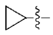,

$R^3$, $R^4$, $R^5$, $R^8$, $R^9$, $R^{10}$ and $R^{11}$ are all H;compound 525: n is 0, X is S, Y is O, $R^2$ is $C_2H_5$, $R^6$ is Me, $R^7$ is

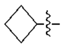,

$R^3$, $R^4$, $R^5$, $R^8$, $R^9$, $R^{10}$ and $R^{11}$ are all H;compound 526: n is 0, X is S, Y is O, $R^2$ is $C_2H_5$, $R^6$ is Me, $R^7$ is $CO_2CH_3$, $R^3$, $R^4$, $R^5$, $R^8$, $R^9$, $R^{10}$ and $R^{11}$ are all H;compound 527: n is 0, X is S, Y is O, $R^2$ is $C_2H_5$, $R^6$ is Me, $R^7$ and $R^8$ are cyclized to form

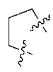

, and $R^3$, $R^4$, $R^5$, $R^9$, $R^{10}$ and $R^{11}$ are all H;compound 528: n is 0, X is S, Y is O, $R^2$ is $C_2H_5$, $R^6$ is Me, $R^8$ and $R^9$ are cyclized to form

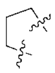,

and $R^3$, $R^4$, $R^5$, $R^7$, $R^{10}$ and $R^{11}$ are all H;compound 529: n is 0, X is S, Y is O, $R^2$ is $C_2H_5$, $R^6$ is Me, $R^8$ and $R^9$ are cyclized to form

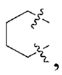,

and $R^3$, $R^4$, $R^5$, $R^7$, $R^{10}$ and $R^{11}$ are all H;compound 530: n is 0, X is S, Y is O, $R^2$ is $C_2H_5$, $R^6$ is Me, $R^7$ and $R^8$ are cyclized to form

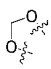,

and $R^3$, $R^4$, $R^5$, $R^9$, $R^{10}$ and $R^{11}$ are all H;compound 531: n is 0, X is S, Y is O, $R^2$ is $C_2H_5$, $R^6$ is Me, $R^8$ and $R^9$ are cyclized to form

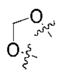,

and $R^3$, $R^4$, $R^5$, $R^7$, $R^{10}$ and $R^{11}$ are all H;compound 532: n is 0, X is S, Y is O, $R^2$ is $C_2H_5$, $R^6$ is Me, $R^8$ and $R^9$ are cyclized to form

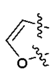,

and $R^3$, $R^4$, $R^5$, $R^7$, $R^{10}$ and $R^{11}$ are all H;compound 533: n is 0, X is S, Y is O, $R^2$ is $C_2H_5$, $R^6$ is Me, $R^7$ and $R^8$ are cyclized to form

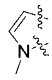,

and $R^3$, $R^4$, $R^5$, $R^9$, $R^{10}$ and $R^{11}$ are all H;compound 534: n is 0, X is S, Y is O, $R^2$ is $C_2H_5$, $R^6$ is Me, $R^7$ and $R^8$ are cyclized to form

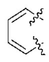,

and $R^3$, $R^4$, $R^5$, $R^9$, $R^{10}$ and $R^{11}$ are all H;compound 535: n is 0, X is S, Y is O, $R^2$ is $C_2H_5$, $R^6$ is Me, $R^7$ and $R^8$ are cyclized to form

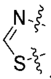, ε

nd $R^3$, $R^4$, $R^5$, $R^9$, $R^{10}$ and $R^{11}$ are all H;compound 536: n is 0, X is S, Y is O, $R^2$ is $C_2H_5$, $R^6$ is Me, $R^7$ and $R^8$ are cyclized to form

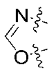,

and $R^3$, $R^4$, $R^5$, $R^9$, $R^{10}$ and $R^{11}$ are all H;compound 537: n is 0, X is S, Y is O, $R^2$ is $C_2H_5$, $R^6$ is Me, $R^8$ and $R^9$ are cyclized to form

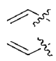,

and $R^3$, $R^4$, $R^5$, $R^7$, $R^{10}$ and $R^{11}$ are all H;compound 796: n is 0, X is S, Y is O, $R^2$ is OMe, $R^6$ is Me, and $R^3$, $R^4$, $R^5$, $R^7$, $R^8$, $R^9$, $R^{10}$ and $R^{11}$ are all H;compound 797: n is 0, X is S, Y is O, $R^2$ is OMe, $R^6$ is Me, $R^7$ is F, and $R^3$, $R^4$, $R^5$, $R^8$, $R^9$, $R^{10}$ and $R^{11}$ are all H;compound 798: n is 0, X is S, Y is O, $R^2$ is OMe, $R^6$ is Me, $R^8$ is F, and $R^3$, $R^4$, $R^5$, $R^7$, $R^9$, $R^{10}$ and $R^{11}$ are all H;compound 799: n is 0, X is S, Y is O, $R^2$ is OMe, $R^6$ is Me, $R^9$ is F, and $R^3$, $R^4$, $R^5$, $R^7$, $R^8$, $R^{10}$ and $R^{11}$ are all H;compound 800: n is 0, X is S, Y is O, $R^2$ is OMe, $R^6$ is Me, $R^7$ is Cl, and $R^3$, $R^4$, $R^5$, $R^8$, $R^9$, $R^{10}$ and $R^{11}$ are all H;compound 801: n is 0, X is S, Y is O, $R^2$ is OMe, $R^6$ is Me, $R^8$ is Cl, and $R^3$, $R^4$, $R^5$, $R^7$, $R^9$, $R^{10}$ and $R^{11}$ are all H;compound 802: n is 0, X is S, Y is O, $R^2$ is OMe, $R^6$ is Me, $R^9$ is Cl, and $R^3$, $R^4$, $R^5$, $R^7$, $R^8$, $R^{10}$ and $R^{11}$ are all H;compound 803: n is 0, X is S, Y is O, $R^2$ is OMe, $R^6$ is Me, $R^7$ is Br, $R^3$, $R^4$, $R^5$, $R^8$, $R^9$, $R^{10}$ and $R^{11}$ are all H;compound 804: n is 0, X is S, Y is O, $R^2$ is OMe, $R^6$ is Me, $R^8$ is Br, $R^3$, $R^4$, $R^5$, $R^7$, $R^9$, $R^{10}$ and $R^{11}$ are all H;compound 805: n is 0, X is S, Y is O, $R^2$ is OMe, $R^6$ is Me, $R^9$ is Br, $R^3$, $R^4$, $R^5$, $R^7$, $R^8$, $R^{10}$ and $R^{11}$ are all H;compound 806: n is 0, X is S, Y is O, $R^2$ is OMe, $R^6$ is Me, $R^7$ is I, and $R^3$, $R^4$, $R^5$, $R^8$, $R^9$, $R^{10}$ and $R^{11}$ are all H;compound 807: n is 0, X is S, Y is O, $R^2$ is OMe, $R^6$ is Me, $R^8$ is I, and $R^3$, $R^4$, $R^5$, $R^7$, $R^9$, $R^{10}$ and $R^{11}$ are all H; compound 808: n is 0, X is S, Y is O, $R^2$ is OMe, $R^6$ is Me, $R^9$ is I, and $R^3$, $R^4$, $R^5$, $R^7$, $R^8$, $R^{10}$ and $R^{11}$ are all H; compound 809: n is 0, X is S, Y is O, $R^2$ is OMe, $R^6$ is Me, $R^7$ is $NO_2$, $R^3$, $R^4$, $R^5$, $R^8$, $R^9$, $R^{10}$ and $R^{11}$ are all H;compound 810: n is 0, X is S, Y is O, $R^2$ is OMe, $R^6$ is Me, $R^8$ is $NO_2$, $R^3$, $R^4$, $R^5$, $R^7$, $R^9$, $R^{10}$ and $R^{11}$ are all H;compound 811: n is 0, X is S, Y is O, $R^2$ is OMe, $R^6$ is Me, $R^9$ is $NO_2$, $R^3$, $R^4$, $R^5$, $R^7$, $R^8$, $R^{10}$ and $R^{11}$ are all H;compound 812: n is 0, X is S, Y is O, $R^2$ is OMe, $R^6$ is Me, $R^7$ is $CF_3$, $R^3$, $R^4$, $R^5$, $R^8$, $R^9$, $R^{10}$ and $R^{11}$ are all H;compound 813: n is 0, X is S, Y is O, $R^2$ is OMe, $R^6$ is Me, $R^8$ is $CF_3$, $R^3$, $R^4$, $R^5$, $R^7$, $R^9$, $R^{10}$ and $R^{11}$ are all H;compound 814: n is 0, X is S, Y is O, $R^2$ is OMe, $R^6$ is Me, $R^9$ is $CF_3$, $R^3$, $R^4$, $R^5$, $R^7$, $R^8$, $R^{10}$ and $R^{11}$ are all H;compound 815: n is 0, X is S, Y is O, $R^2$ is OMe, $R^6$ is Me, $R^7$ is $OCF_3$, $R^3$, $R^4$, $R^5$, $R^8$, $R^9$, $R^{10}$ and $R^{11}$ are all H;compound 816: n is 0, X is S, Y is O, $R^2$ is OMe, $R^6$ is Me, $R^8$ is $OCF_3$, $R^3$, $R^4$, $R^5$, $R^7$, $R^9$, $R^{10}$ and $R^{11}$ are all H;compound 817: n is 0, X is S, Y is O, $R^2$ is OMe, $R^6$ is Me, $R^9$ is $OCF_3$, $R^3$, $R^4$, $R^5$, $R^7$, $R^8$, $R^{10}$ and $R^{11}$ are all H;compound 818: n is 0, X is S, Y is O, $R^2$ is OMe, $R^6$ is Me, $R^7$ is $OCHF_2$, $R^3$, $R^4$, $R^5$, $R^8$, $R^9$, $R^{10}$ and $R^{11}$ are all H;compound 819: n is 0, X is S, Y is O, $R^2$ is OMe, $R^6$ is Me, $R^8$ is $OCHF_2$, $R^3$, $R^4$, $R^5$, $R^7$, $R^9$, $R^{10}$ and $R^{11}$ are all H;compound 820: n is 0, X is S, Y is O, $R^2$ is OMe, $R^6$ is Me, $R^9$ is $OCHF_2$, $R^3$, $R^4$, $R^5$, $R^7$, $R^8$, $R^{10}$ and $R^{11}$ are all H;compound 821: n is 0, X is S, Y is O, $R^2$ is OMe, $R^6$ is Me, $R^9$ is $SCF_3$, $R^3$, $R^4$, $R^5$, $R^7$, $R^8$, $R^{10}$ and $R^{11}$ are all H;compound 822: n is 0, X is S, Y is O, $R^2$ is OMe, $R^6$ is Me, $R^7$ is $OCH_3$, $R^3$, $R^4$, $R^5$, $R^8$, $R^9$, $R^{10}$ and $R^{11}$ are all H;compound 823: n is 0, X is S, Y is O, $R^2$ is OMe, $R^6$ is Me, $R^8$ is $OCH_3$, $R^3$, $R^4$, $R^5$, $R^7$, $R^9$, $R^{10}$ and $R^{11}$ are all H;compound 824: n is 0, X is S, Y is O, $R^2$ is OMe, $R^6$ is Me, $R^9$ is $OCH_3$, $R^3$, $R^4$, $R^5$, $R^7$, $R^8$, $R^{10}$ and $R^{11}$ are all H;compound 825: n is 0, X is S, Y is O, $R^2$ is OMe, $R^6$ is Me, $R^9$ is $OC_2H_5$, $R^3$, $R^4$, $R^5$, $R^7$, $R^8$, $R^{10}$ and $R^{11}$ are all H;compound 826: n is 0, X is S, Y is O, $R^2$ is OMe, $R^6$ is Me, $R^7$ is CN, and $R^3$, $R^4$, $R^5$, $R^8$, $R^9$, $R^{10}$ and $R^{11}$ are all

H;compound 827: n is 0, X is S, Y is O, $R^2$ is OMe, $R^6$ is Me, $R^8$ is CN, and $R^3$, $R^4$, $R^5$, $R^7$, $R^9$, $R^{10}$ and $R^{11}$ are all H;compound 828: n is 0, X is S, Y is O, $R^2$ is OMe, $R^6$ is Me, $R^9$ is CN, and $R^3$, $R^4$, $R^5$, $R^7$, $R^8$, $R^{10}$ and $R^{11}$ are all H;compound 829: n is 0, X is S, Y is O, $R^2$ is OMe, $R^6$ is Me, $R^7$ is $CH_3$, $R^3$, $R^4$, $R^5$, $R^8$, $R^9$, $R^{10}$ and $R^{11}$ are all H;compound 830: n is 0, X is S, Y is O, $R^2$ is OMe, $R^6$ is Me, $R^8$ is $CH_3$, $R^3$, $R^4$, $R^5$, $R^7$, $R^9$, $R^{10}$ and $R^{11}$ are all H;compound 831: n is 0, X is S, Y is O, $R^2$ is OMe, $R^6$ is Me, $R^9$ is $CH_3$, $R^3$, $R^4$, $R^5$, $R^7$, $R^8$, $R^{10}$ and $R^{11}$ are all H;compound 832: n is 0, X is S, Y is O, $R^2$ is OMe, $R^6$ is Me, $R^7$ is $CH_3SO_2$, $R^3$, $R^4$, $R^5$, $R^8$, $R^9$, $R^{10}$ and $R^{11}$ are all H;compound 833: n is 0, X is S, Y is O, $R^2$ is OMe, $R^6$ is Me, $R^8$ is $CH_3SO_2$, $R^3$, $R^4$, $R^5$, $R^7$, $R^9$, $R^{10}$ and $R^{11}$ are all H;compound 834: n is 0, X is S, Y is O, $R^2$ is OMe, $R^6$ is Me, $R^9$ is $CH_3SO_2$, $R^3$, $R^4$, $R^5$, $R^7$, $R^9$, $R^{10}$ and $R^{11}$ are all H;compound 835: n is 0, X is S, Y is O, $R^2$ is OMe, $R^6$ is Me, $R^7$ is $C_2H_5SO_2$, $R^3$, $R^4$, $R^5$, $R^8$, $R^9$, $R^{10}$ and $R^{11}$ are all H;compound 836: n is 0, X is S, Y is O, $R^2$ is OMe, $R^6$ is Me, $R^7$ and $R^8$ are both Cl, and $R^3$, $R^4$, $R^5$, $R^9$, $R^{10}$ and $R^{11}$ are all H;compound 837: n is 0, X is S, Y is O, $R^2$ is OMe, $R^6$ is Me, $R^7$ and $R^9$ are both Cl, and $R^3$, $R^4$, $R^5$, $R^8$, $R^{10}$ and $R^{11}$ are all H;compound 838: n is 0, X is S, Y is O, $R^2$ is OMe, $R^6$ is Me, $R^7$ and $R^{10}$ are both Cl, and $R^3$, $R^4$, $R^5$, $R^8$, $R^9$ and $R^{11}$ are all H;compound 839: n is 0, X is S, Y is O, $R^2$ is OMe, $R^6$ is Me, $R^7$ and $R^{11}$ are both Cl, and $R^3$, $R^4$, $R^5$, $R^8$, $R^9$ and $R^{10}$ are all H;compound 840: n is 0, X is S, Y is O, $R^2$ is OMe, $R^6$ is Me, $R^8$ and $R^9$ are both Cl, and $R^3$, $R^4$, $R^5$, $R^7$, $R^{10}$ and $R^{11}$ are all H;compound 841: n is 0, X is S, Y is O, $R^2$ is OMe, $R^6$ and $R^7$ are Me, $R^9$ is Cl, and $R^3$, $R^4$, $R^5$, $R^8$, $R^{10}$ and $R^{11}$ are all H;compound 842: n is 0, X is S, Y is O, $R^2$ is OMe, $R^6$, $R^7$ and $R^8$ are Me, and $R^3$, $R^4$, $R^5$, $R^9$, $R^{10}$ and $R^{11}$ are all H;compound 843: n is 0, X is S, Y is O, $R^2$ is OMe, $R^6$, $R^7$ and $R^9$ are Me, and $R^3$, $R^4$, $R^5$, $R^8$, $R^{10}$ and $R^{11}$ are all H;compound 844: n is 0, X is S, Y is O, $R^2$ is OMe, $R^6$, $R^7$ and $R^{10}$ are Me, and $R^3$, $R^4$, $R^5$, $R^8$, $R^9$ and $R^{11}$ are all H;compound 845: n is 0, X is S, Y is O, $R^2$ is OMe, $R^7$ and $R^{11}$ are Me, and $R^3$, $R^4$, $R^5$, $R^8$, $R^9$ and $R^{10}$ are all H;compound 846: n is 0, X is S, Y is O, $R^2$ is OMe, $R^6$, $R^7$, $R^8$ and $R^{11}$ are Me, and $R^3$, $R^4$, $R^5$, $R^9$ and $R^{10}$ are all H;compound 847: n is 0, X is S, Y is O, $R^2$ is OMe, $R^6$, $R^7$, $R^9$ and $R^{11}$ are Me, and $R^3$, $R^4$, $R^5$, $R^8$ and $R^{10}$ are all H;compound 848: n is 0, X is S, Y is O, $R^2$ is OMe, $R^6$ is Me, $R^7$ and $R^9$ are both $CF_3$, $R^3$, $R^4$, $R^5$, $R^8$, $R^{10}$ and $R^{11}$ are all H;compound 849: n is 0, X is S, Y is O, $R^2$ is OMe, $R^6$ is Me, $R^8$ and $R^{10}$ are both $CF_3$, $R^3$, $R^4$, $R^5$, $R^7$, $R^9$ and $R^{11}$ are all H;compound 850: n is 0, X is S, Y is O, $R^2$ is OMe, $R^6$ is Me, $R^3$, $R^4$ and $R^5$ are all H, and $R^7$, $R^8$, $R^9$, $R^{10}$ and $R^{11}$ are all F;compound 851: n is 0, X is S, Y is O, $R^2$ is OMe, $R^6$ is Me, $R^7$ is $CH_3CH_2$, $R^3$, $R^4$, $R^5$, $R^8$, $R^9$, $R^{10}$ and $R^{11}$ are all H;compound 852: n is 0, X is S, Y is O, $R^2$ is OMe, $R^6$ is Me, $R^8$ is $CH_3CH_2$, $R^3$, $R^4$, $R^5$, $R^7$, $R^9$, $R^{10}$ and $R^{11}$ are all H;compound 853: n is 0, X is S, Y is O, $R^2$ is OMe, $R^6$ is Me, $R^9$ is $CH_3CH_2$, $R^3$, $R^4$, $R^5$, $R^7$, $R^8$, $R^{10}$ and $R^{11}$ are all H;compound 854: n is 0, X is S, Y is O, $R^2$ is OMe, $R^6$ is Me, $R^7$ is $CH(CH_3)_2$, $R^3$, $R^4$, $R^5$, $R^8$, $R^9$, $R^{10}$ and $R^{11}$ are all H;compound 855: n is 0, X is S, Y is O, $R^2$ is OMe, $R^6$ is Me, $R^8$ is $CH(CH_3)_2$, $R^3$, $R^4$, $R^5$, $R^7$, $R^9$, $R^{10}$ and $R^{11}$ are all H;compound 856: n is 0, X is S, Y is O, $R^2$ is OMe, $R^6$ is Me, $R^9$ is $CH(CH_3)_2$, $R^3$, $R^4$, $R^5$, $R^7$, $R^8$, $R^{10}$ and $R^{11}$ are all H;compound 857: n is 0, X is S, Y is O, $R^2$ is OMe, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;compound 858: n is 0, X is S, Y is O, $R^2$ is OMe, $R^6$ is Me, $R^7$ is $C(CH_3)_3$, and $R^3$, $R^4$, $R^5$, $R^8$, $R^9$, $R^{10}$ and $R^{11}$ are all H;compound 859: n is 0, X is S, Y is O, $R^2$ is OMe, $R^6$ is Me, $R^7$ is

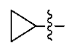

, $R^3$, $R^4$, $R^5$, $R^8$, $R^9$, $R^{10}$ and $R^{11}$ are all H;compound 860: n is 0, X is S, Y is O, $R^2$ is OMe, $R^6$ is Me, $R^7$ is

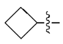,

$R^3$, $R^4$, $R^5$, $R^8$, $R^9$, $R^{10}$ and $R^{11}$ are all H;compound 861: n is 0, X is S, Y is O, $R^2$ is OMe, $R^6$ is Me, $R^7$ is $CO_2CH_3$, $R^3$, $R^4$, $R^5$, $R^8$, $R^9$, $R^{10}$ and $R^{11}$ are all H;compound 862: n is 0, X is S, Y is O, $R^2$ is OMe, $R^6$ is Me, $R^7$ and $R^8$ are cyclized to form

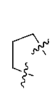,

and $R^3$, $R^4$, $R^5$, $R^9$, $R^{10}$ and $R^{11}$ are all H;compound 863: n is 0, X is S, Y is O, $R^2$ is OMe, $R^6$ is Me, $R^8$ and $R^9$ are cyclized to form

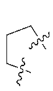,

and $R^3$, $R^4$, $R^5$, $R^7$, $R^{10}$ and $R^{11}$ are all H;compound 864: n is 0, X is S, Y is O, $R^2$ is OMe, $R^6$ is Me, $R^8$ and $R^9$ are cyclized to form

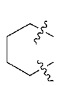,

and $R^3$, $R^4$, $R^5$, $R^7$, $R^{10}$ and $R^{11}$ are all H;compound 865: n is 0, X is S, Y is O, $R^2$ is OMe, $R^6$ is Me, $R^7$ and $R^8$ are cyclized to form

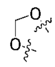,

and $R^3$, $R^4$, $R^5$, $R^9$, $R^{10}$ and $R^{11}$ are all H;compound 866: n is 0, X is S, Y is O, $R^2$ is OMe, $R^6$ is Me, $R^8$ and $R^9$ are cyclized to form

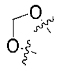,

and $R^3$, $R^4$, $R^5$, $R^7$, $R^{10}$ and $R^{11}$ are all H;compound 867: n is 0, X is S, Y is O, $R^2$ is OMe, $R^6$ is Me, $R^8$ and $R^9$ are cyclized to form

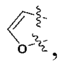, ;

and $R^3$, $R^4$, $R^5$, $R^7$, $R^{10}$ and $R^{11}$ are all H;compound 868: n is 0, X is S, Y is O, $R^2$ is OMe, $R^6$ is Me, $R^7$ and $R^8$ are cyclized to form

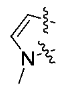,

and $R^3$, $R^4$, $R^5$, $R^9$, $R^{10}$ and $R^{11}$ are all H;compound 869: n is 0, X is S, Y is O, $R^2$ is OMe, $R^6$ is Me, $R^7$ and $R^8$ are cyclized to form

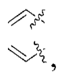,

and $R^3$, $R^4$, $R^5$, $R^9$, $R^{10}$ and $R^{11}$ are all H;compound 870: n is 0, X is S, Y is O, $R^2$ is OMe, $R^6$ is Me, $R^7$ and $R^8$ are cyclized to form

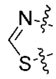,

and $R^3$, $R^4$, $R^5$, $R^9$, $R^{10}$ and $R^{11}$ are all H;compound 871: n is 0, X is S, Y is O, $R^2$ is OMe, $R^6$ is Me, $R^7$ and $R^8$ are cyclized to form

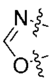, ;

and $R^3$, $R^4$, $R^5$, $R^9$, $R^{10}$ and $R^{11}$ are all H;compound 872: n is 0, X is S, Y is O, $R^2$ is OMe, $R^6$ is Me, $R^8$ and $R^9$ are cyclized to form

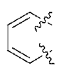,

and $R^3$, $R^4$, $R^5$, $R^7$, $R^{10}$ and $R^{11}$ are all H;compound 538: n is 0, X is SO, Y is O, $R^6$ is Me, and $R^2$, $R^3$, $R^4$, $R^5$, $R^7$, $R^8$, $R^9$, $R^{10}$ and $R^{11}$ are all H;compound 539: n is 0, X is SO, Y is O, $R^6$ is Me, $R^7$ is F, and $R^2$, $R^3$, $R^4$, $R^5$, $R^8$, $R^9$, $R^{10}$ and $R^{11}$ are all H;compound 540: n is 0, X is SO, Y is O, $R^6$ is Me, $R^8$ is F, and $R^2$, $R^3$, $R^4$, $R^5$, $R^7$, $R^9$, $R^{10}$ and $R^{11}$ are all H;compound 541: n is 0, X is SO, Y is O, $R^6$ is Me, $R^9$ is F, and $R^2$, $R^3$, $R^4$, $R^5$, $R^7$, $R^8$, $R^{10}$ and $R^{11}$ are all H;compound 542: n is 0, X is SO, Y is O, $R^6$ is Me, $R^7$ is Cl, and $R^2$, $R^3$, $R^4$, $R^5$, $R^8$, $R^9$, $R^{10}$ and $R^{11}$ are all H;compound 543: n is 0, X is SO, Y is O, $R^6$ is Me, $R^8$ is Cl,

and $R^2$, $R^3$, $R^4$, $R^5$, $R^7$, $R^9$, $R^{10}$ and $R^{11}$ are all H;compound 544: n is 0, X is SO, Y is O, $R^6$ is Me, $R^9$ is Cl, and $R^2$, $R^3$, $R^4$, $R^5$, $R^7$, $R^8$, $R^{10}$ and $R^{11}$ are all H;compound 545: n is 0, X is SO, Y is O, $R^6$ is Me, $R^7$ is Br, and $R^2$, $R^3$, $R^4$, $R^5$, $R^8$, $R^9$, $R^{10}$ and $R^{11}$ are all H;compound 546: n is 0, X is SO, Y is O, $R^6$ is Me, $R^8$ is Br, and $R^2$, $R^3$, $R^4$, $R^5$, $R^7$, $R^9$, $R^{10}$ and $R^{11}$ are all H;compound 547: n is 0, X is SO, Y is O, $R^6$ is Me, $R^9$ is Br, and $R^2$, $R^3$, $R^4$, $R^5$, $R^7$, $R^8$, $R^{10}$ and $R^{11}$ are all H;compound 548: n is 0, X is SO, Y is O, $R^6$ is Me, $R^7$ is I, and $R^2$, $R^3$, $R^4$, $R^5$, $R^8$, $R^9$, $R^{10}$ and $R^{11}$ are all H;compound 549: n is 0, X is SO, Y is O, $R^6$ is Me, $R^8$ is I, and $R^2$, $R^3$, $R^4$, $R^5$, $R^7$, $R^9$, $R^{10}$ and $R^{11}$ are all H;compound 550: n is 0, X is SO, Y is O, $R^6$ is Me, $R^9$ is I, and $R^2$, $R^3$, $R^4$, $R^5$, $R^7$, $R^8$, $R^{10}$ and $R^{11}$ are all H;compound 551: n is 0, X is SO, Y is O, $R^6$ is Me, $R^7$ is $NO_2$, and $R^2$, $R^3$, $R^4$, $R^5$, $R^8$, $R^9$, $R^{10}$ and $R^{11}$ are all H;compound 552: n is 0, X is SO, Y is O, $R^6$ is Me, $R^8$ is $NO_2$, and $R^2$, $R^3$, $R^4$, $R^5$, $R^7$, $R^9$, $R^{10}$ and $R^{11}$ are all H;compound 553: n is 0, X is SO, Y is O, $R^6$ is Me, $R^9$ is $NO_2$, and $R^2$, $R^3$, $R^4$, $R^5$, $R^7$, $R^8$, $R^{10}$ and $R^{11}$ are all H;compound 554: n is 0, X is SO, Y is O, $R^6$ is Me, $R^7$ is $CF_3$, and $R^2$, $R^3$, $R^4$, $R^5$, $R^8$, $R^9$, $R^{10}$ and $R^{11}$ are all H;compound 555: n is 0, X is SO, Y is O, $R^6$ is Me, $R^8$ is $CF_3$, and $R^2$, $R^3$, $R^4$, $R^5$, $R^7$, $R^9$, $R^{10}$ and $R^{11}$ are all H;compound 556: n is 0, X is SO, Y is O, $R^6$ is Me, $R^9$ is $CF_3$, and $R^2$, $R^3$, $R^4$, $R^5$, $R^7$, $R^8$, $R^{10}$ and $R^{11}$ are all H;compound 557: n is 0, X is SO, Y is O, $R^6$ is Me, $R^7$ is $OCF_3$, and $R^2$, $R^3$, $R^4$, $R^5$, $R^8$, $R^9$, $R^{10}$ and $R^{11}$ are all H;compound 558: n is 0, X is SO, Y is O, $R^6$ is Me, $R^8$ is $OCF_3$, and $R^2$, $R^3$, $R^4$, $R^5$, $R^7$, $R^9$, $R^{10}$ and $R^{11}$ are all H;compound 559: n is 0, X is SO, Y is O, $R^6$ is Me, $R^9$ is $OCF_3$, and $R^2$, $R^3$, $R^4$, $R^5$, $R^7$, $R^8$, $R^{10}$ and $R^{11}$ are all H;compound 560: n is 0, X is SO, Y is O, $R^6$ is Me, $R^7$ is $OCHF_2$, and $R^2$, $R^3$, $R^4$, $R^5$, $R^8$, $R^9$, $R^{10}$ and $R^{11}$ are all H;compound 561: n is 0, X is SO, Y is O, $R^6$ is Me, $R^8$ is $OCHF_2$, and $R^2$, $R^3$, $R^4$, $R^5$, $R^7$, $R^9$, $R^{10}$ and $R^{11}$ are all H;compound 562: n is 0, X is SO, Y is O, $R^6$ is Me, $R^9$ is $OCHF_2$, and $R^2$, $R^3$, $R^4$, $R^5$, $R^7$, $R^8$, $R^{10}$ and $R^{11}$ are all H;compound 563: n is 0, X is SO, Y is O, $R^6$ is Me, $R^9$ is $SCF_3$, and $R^2$, $R^3$, $R^4$, $R^5$, $R^7$, $R^8$, $R^{10}$ and $R^{11}$ are all H;compound 564: n is 0, X is SO, Y is O, $R^6$ is Me, $R^7$ is $OCH_3$, and $R^2$, $R^3$, $R^4$, $R^5$, $R^8$, $R^9$, $R^{10}$ and $R^{11}$ are all H;compound 565: n is 0, X is SO, Y is O, $R^6$ is Me, $R^8$ is $OCH_3$, and $R^2$, $R^3$, $R^4$, $R^5$, $R^7$, $R^9$, $R^{10}$ and $R^{11}$ are all H;compound 566: n is 0, X is SO, Y is O, $R^6$ is Me, $R^9$ is $OCH_3$, and $R^2$, $R^3$, $R^4$, $R^5$, $R^7$, $R^8$, $R^{10}$ and $R^{11}$ are all H;compound 567: n is 0, X is SO, Y is O, $R^6$ is Me, $R^9$ is $OC_2H_5$, and $R^2$, $R^3$, $R^4$, $R^5$, $R^7$, $R^8$, $R^{10}$ and $R^{11}$ are all H;compound 568: n is 0, X is SO, Y is O, $R^6$ is Me, $R^7$ is CN, and $R^2$, $R^3$, $R^4$, $R^5$, $R^8$, $R^9$, $R^{10}$ and $R^{11}$ are all H;compound 569: n is 0, X is SO, Y is O, $R^6$ is Me, $R^8$ is CN, and $R^2$, $R^3$, $R^4$, $R^5$, $R^7$, $R^9$, $R^{10}$ and $R^{11}$ are all H;compound 570: n is 0, X is SO, Y is O, $R^6$ is Me, $R^9$ is CN, and $R^2$, $R^3$, $R^4$, $R^5$, $R^7$, $R^8$, $R^{10}$ and $R^{11}$ are all H;compound 571: n is 0, X is SO, Y is O, $R^6$ is Me, $R^7$ is $CH_3$, and $R^2$, $R^3$, $R^4$, $R^5$, $R^8$, $R^9$, $R^{10}$ and $R^{11}$ are all H;compound 572: n is 0, X is SO, Y is O, $R^6$ is Me, $R^8$ is $CH_3$, and $R^2$, $R^3$, $R^4$, $R^5$, $R^7$, $R^9$, $R^{10}$ and $R^{11}$ are all H;compound 573: n is 0, X is SO, Y is O, $R^6$ is Me, $R^9$ is $CH_3$, and $R^2$, $R^3$, $R^4$, $R^5$, $R^7$, $R^8$, $R^{10}$ and $R^{11}$ are all H;compound 574: n is 0, X is SO, Y is O, $R^6$ is Me, $R^7$ is $CH_3SO_2$, and $R^2$, $R^3$, $R^4$, $R^5$, $R^8$, $R^9$, $R^{10}$ and $R^{11}$ are all H;compound 575: n is 0, X is SO, Y is O, $R^6$ is Me, $R^8$ is $CH_3SO_2$, and $R^2$, $R^3$, $R^4$, $R^5$, $R^7$, $R^9$, $R^{10}$ and $R^{11}$ are all H;compound 576: n is 0, X is SO, Y is O, $R^6$ is Me, $R^9$ is $CH_3SO_2$, and $R^2$, $R^3$, $R^4$, $R^5$, $R^7$, $R^8$, $R^{10}$ and $R^{11}$ are all H;compound 577: n is 0, X is SO, Y is O, $R^6$ is Me, $R^7$ is $C_2H_5SO_2$, and $R^2$, $R^3$, $R^4$, $R^5$, $R^8$, $R^9$, $R^{10}$ and $R^{11}$ are all H;compound 578: n is 0, X is SO, Y is O, $R^6$ is Me, $R^7$ and $R^8$ are both Cl, and $R^2$, $R^3$, $R^4$, $R^5$, $R^9$, $R^{10}$ and $R^{11}$ are all H;compound 579: n is 0, X is SO, Y is O, $R^6$ is Me, $R^7$ and $R^9$ are both Cl, and $R^2$, $R^3$, $R^4$, $R^5$, $R^8$, $R^{10}$ and $R^{11}$ are all H;compound 580: n is 0, X is SO, Y is O, $R^6$ is Me, $R^7$ and $R^{10}$ are both Cl, and $R^2$, $R^3$, $R^4$, $R^5$, $R^8$, $R^9$ and $R^{11}$ are all H;compound 581: n is 0, X is SO, Y is O, $R^6$ is Me, $R^7$ and $R^{11}$ are both Cl, and $R^2$, $R^3$, $R^4$, $R^5$, $R^8$, $R^9$ and $R^{10}$ are all H;compound 582: n is 0, X is SO, Y is O, $R^6$ is Me, $R^8$ and $R^9$ are both Cl, and $R^2$, $R^3$, $R^4$, $R^5$, $R^7$, $R^{10}$ and $R^{11}$ are all H;compound 583: n is 0, X is SO, Y is O, $R^6$ and $R^7$ are Me, $R^9$ is Cl, and $R^2$, $R^3$, $R^4$, $R^5$, $R^8$, $R^{10}$ and $R^{11}$ are all H;compound 584: n is 0, X is SO, Y is O, $R^6$, $R^7$ and $R^8$ are Me, and $R^2$, $R^3$, $R^4$, $R^5$, $R^9$, $R^{10}$ and $R^{11}$ are all H;compound 585: n is 0, X is SO, Y is O, $R^6$, $R^7$ and $R^9$ are Me, and $R^2$, $R^3$, $R^4$, $R^5$, $R^8$, $R^{10}$ and $R^{11}$ are all H;compound 586: n is 0, X is SO, Y is O, $R^6$, $R^7$ and $R^{10}$ are Me, and $R^2$, $R^3$, $R^4$, $R^5$, $R^8$, $R^9$ and $R^{11}$ are all H;compound 587: n is 0, X is SO, Y is O, $R^6$, $R^7$ and $R^{11}$ are Me, and $R^2$, $R^3$, $R^4$, $R^5$, $R^8$, $R^9$ and $R^{10}$ are all H;compound 588: n is 0, X is SO, Y is O, $R^6$, $R^7$, $R^8$ and $R^{11}$ are Me, and $R^2$, $R^3$, $R^4$, $R^5$, $R^9$ and $R^{10}$ are all H;compound 589: n is 0, X is SO, Y is O, $R^6$, $R^7$, $R^9$ and $R^{11}$ are Me, and $R^2$, $R^3$, $R^4$, $R^5$, $R^8$ and $R^{10}$ are all H;compound 590: n is 0, X is SO, Y is O, $R^6$ is Me, $R^7$ and $R^9$ are both $CF_3$, and $R^2$, $R^3$, $R^4$, $R^5$, $R^8$, $R^{10}$ and $R^{11}$ are all H;compound 591: n is 0, X is SO, Y is O, $R^6$ is Me, $R^8$ and $R^{10}$ are both $CF_3$, and $R^2$, $R^3$, $R^4$, $R^5$, $R^7$, $R^9$ and $R^{11}$ are all H;compound 592: n is 0, X is SO, Y is O, $R^6$ is Me, $R^2$, $R^3$, $R^4$ and $R^5$ are all H, and $R^7$, $R^8$, $R^9$, $R^{10}$ and $R^{11}$ are all F;compound 593: n is 0, X is SO, Y is O, $R^6$ is Me, $R^7$ is $CH_3CH_2$, and $R^2$, $R^3$, $R^4$, $R^5$, $R^8$, $R^9$, $R^{10}$ and $R^{11}$ are all H;compound 594: n is 0, X is SO, Y is O, $R^6$ is Me, $R^8$ is $CH_3CH_2$, and $R^2$, $R^3$, $R^4$, $R^5$, $R^7$, $R^9$, $R^{10}$ and $R^{11}$ are all H;compound 595: n is 0, X is SO, Y is O, $R^6$ is Me, $R^9$ is $CH_3CH_2$, and $R^2$, $R^3$, $R^4$, $R^5$, $R^7$, $R^8$, $R^{10}$ and $R^{11}$ are all H;compound 596: n is 0, X is SO, Y is O, $R^6$ is Me, $R^7$ is $CH(CH_3)_2$, and $R^2$, $R^3$, $R^4$, $R^5$, $R^8$, $R^9$, $R^{10}$ and $R^{11}$ are all H;compound 597: n is 0, X is SO, Y is O, $R^6$ is Me, $R^8$ is $CH(CH_3)_2$, and $R^2$, $R^3$, $R^4$, $R^5$, $R^7$, $R^9$, $R^{10}$ and $R^{11}$ are all H;compound 598: n is 0, X is SO, Y is O, $R^6$ is Me, $R^9$ is $CH(CH_3)_2$, and $R^2$, $R^3$, $R^4$, $R^5$, $R^7$, $R^8$, $R^{10}$ and $R^{11}$ are all H;compound 599: n is 0, X is SO, Y is O, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;compound 600: n is 0, X is SO, Y is O, $R^6$ is Me, $R^7$ is $C(CH_3)_3$, and $R^2$, $R^3$, $R^4$, $R^5$, $R^8$, $R^9$, $R^{10}$ and $R^{11}$ are all H;compound 601: n is 0, X is SO, Y is O, $R^6$ is Me, $R^7$ is

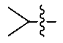

, and $R^2$, $R^3$, $R^4$, $R^5$, $R^8$, $R^9$, $R^{10}$ and $R^{11}$ are all H;compound 602: n is 0, X is SO, Y is O, $R^6$ is Me, $R^7$ is

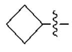,

and $R^2$, $R^3$, $R^4$, $R^5$, $R^8$, $R^9$, $R^{10}$ and $R^{11}$ are all H;compound 603: n is 0, X is SO, Y is O, $R^6$ is Me, $R^7$ is $CO_2CH_3$, and $R^2$, $R^3$, $R^4$, $R^5$, $R^8$, $R^9$, $R^{10}$ and $R^{11}$ are all H;compound 604: n is 0, X is SO, Y is O, $R^6$ is Me, $R^7$ and $R^8$ are cyclized to form

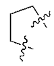

, and $R^2$, $R^3$, $R^4$, $R^5$, $R^9$, $R^{10}$ and $R^{11}$ are all H;compound 605: n is 0, X is SO, Y is O, $R^6$ is Me, $R^8$ and $R^9$ are cyclized to form

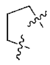,

and $R^2$, $R^3$, $R^4$, $R^5$, $R^7$, $R^{10}$ and $R^{11}$ are all H;compound 606: n is 0, X is SO, Y is O, $R^6$ is Me, $R^8$ and $R^9$ are cyclized to form

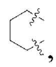,

and $R^2$, $R^3$, $R^4$, $R^5$, $R^7$, $R^{10}$ and $R^{11}$ are all H;compound 607: n is 0, X is SO, Y is O, $R^6$ is Me, $R^7$ and $R^8$ are cyclized to form

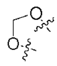,

and $R^2$, $R^3$, $R^4$, $R^5$, $R^9$, $R^{10}$ and $R^{11}$ are all H;compound 608: n is 0, X is SO, Y is O, $R^6$ is Me, $R^8$ and $R^9$ are cyclized to form

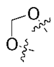,

and $R^2$, $R^3$, $R^4$, $R^5$, $R^7$, $R^{10}$ and $R^{11}$ are all H;compound 609: n is 0, X is SO, Y is O, $R^6$ is Me, $R^7$ and $R^8$ are cyclized to form

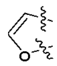,

and $R^2$, $R^3$, $R^4$, $R^5$, $R^9$, $R^{10}$ and $R^{11}$ are all H;compound 610: n is 0, X is SO, Y is O, $R^6$ is Me, $R^7$ and $R^8$ are cyclized to form

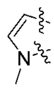,

and $R^2$, $R^3$, $R^4$, $R^5$, $R^9$, $R^{10}$ and $R^{11}$ are all H;compound 611: n is 0, X is SO, Y is O, $R^6$ is Me, $R^7$ and $R^8$ are cyclized to form

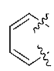,

and $R^2$, $R^3$, $R^4$, $R^5$, $R^9$, $R^{10}$ and $R^{11}$ are all H;compound 612: n is 0, X is SO, Y is O, $R^6$ is Me, $R^7$ and $R^8$ are cyclized to form

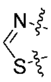,

and $R^2$, $R^3$, $R^4$, $R^5$, $R^9$, $R^{10}$ and $R^{11}$ are all H;compound 613: n is 0, X is SO, Y is O, $R^6$ is Me, $R^7$ and $R^8$ are cyclized to form

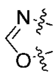

, and $R^2$, $R^3$, $R^4$, $R^5$, $R^9$, $R^{10}$ and $R^{11}$ are all H;compound 614: n is 0, X is SO, Y is O, $R^6$ is Me, $R^8$ and $R^9$ are cyclized to form

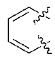,

and $R^2$, $R^3$, $R^4$, $R^5$, $R^7$, $R^{10}$ and $R^{11}$ are all H;compound 615: n is 0, X is $SO_2$, Y is O, $R^6$ is Me, and $R^2$, $R^3$, $R^4$, $R^5$, $R^7$, $R^8$, $R^9$, $R^{10}$ and $R^{11}$ are all H;compound 616: n is 0, X is $SO_2$, Y is O, $R^6$ is Me, $R^7$ is F, and $R^2$, $R^3$, $R^4$, $R^5$, $R^8$, $R^9$, $R^{10}$ and $R^{11}$ are all H;compound 617: n is 0, X is $SO_2$, Y is O, $R^6$ is Me, $R^8$ is F, and $R^2$, $R^3$, $R^4$, $R^5$, $R^7$, $R^9$, $R^{10}$ and $R^{11}$ are all H;compound 618: n is 0, X is $SO_2$, Y is O, $R^6$ is Me, $R^9$ is F, and $R^2$, $R^3$, $R^4$, $R^5$, $R^7$, $R^8$, $R^{10}$ and $R^{11}$ are all H;compound 619: n is 0, X is $SO_2$, Y is O, $R^6$ is Me, $R^7$ is Cl, and $R^2$, $R^3$, $R^4$, $R^5$, $R^8$, $R^9$, $R^{10}$ and $R^{11}$ are all H;compound 620: n is 0, X is $SO_2$, Y is O, $R^6$ is Me, $R^8$ is Cl, and $R^2$, $R^3$, $R^4$, $R^5$, $R^7$, $R^9$, $R^{10}$ and $R^{11}$ are all H;compound 621: n is 0, X is $SO_2$, Y is O, $R^6$ is Me, $R^9$ is Cl, and $R^2$, $R^3$, $R^4$, $R^5$, $R^7$, $R^8$, $R^{10}$ and $R^{11}$ are all H;compound 622: n is 0, X is $SO_2$, Y is O, $R^6$ is Me, $R^7$ is Br, and $R^2$, $R^3$, $R^4$, $R^5$, $R^8$, $R^9$, $R^{10}$ and $R^{11}$ are all H;compound 623: n is 0, X is $SO_2$, Y is O, $R^6$ is Me, $R^8$ is Br, and $R^2$, $R^3$, $R^4$, $R^5$, $R^7$, $R^9$, $R^{10}$ and $R^{11}$ are all H;compound 624: n is 0, X is $SO_2$, Y is O, $R^6$ is Me, $R^9$ is Br, and $R^2$, $R^3$, $R^4$, $R^5$, $R^7$, $R^8$, $R^{10}$ and $R^{11}$ are all H;compound 625: n is 0, X is $SO_2$, Y is O, $R^6$ is Me, $R^7$ is I, and $R^2$, $R^3$, $R^4$, $R^5$, $R^8$, $R^9$, $R^{10}$ and $R^{11}$ are all H;compound 626: n is 0, X is $SO_2$, Y is O, $R^6$ is Me, $R^8$ is I, and $R^2$, $R^3$, $R^4$, $R^5$, $R^7$, $R^9$, $R^{10}$ and $R^{11}$ are all H;compound 627: n is 0, X is $SO_2$, Y is O, $R^6$ is Me, $R^9$ is I, and $R^2$, $R^3$, $R^4$, $R^5$, $R^7$, $R^8$, $R^{10}$ and $R^{11}$ are all H;compound 628: n is 0, X is $SO_2$, Y is O, $R^6$ is Me, $R^7$ is $NO_2$, and $R^2$, $R^3$, $R^4$, $R^5$, $R^8$, $R^9$, $R^{10}$ and $R^{11}$ are all H;compound 629: n is 0, X is $SO_2$, Y is O, $R^6$ is Me, $R^8$ is $NO_2$, and $R^2$, $R^3$, $R^4$, $R^5$, $R^7$, $R^9$, $R^{10}$ and $R^{11}$ are all H;compound 630: n is 0, X is $SO_2$, Y is O, $R^6$ is Me, $R^9$ is $NO_2$, and $R^2$, $R^3$, $R^4$, $R^5$, $R^7$, $R^8$, $R^{10}$ and $R^{11}$ are all H;compound 631: n is 0, X is $SO_2$, Y is O, $R^6$ is Me, $R^7$ is $CF_3$, and $R^2$, $R^3$, $R^4$, $R^5$, $R^8$, $R^9$, $R^{10}$ and $R^{11}$ are all H;compound 632: n is 0, X is $SO_2$, Y is O, $R^6$ is Me, $R^8$ is $CF_3$, and $R^2$, $R^3$, $R^4$, $R^5$, $R^7$, $R^9$, $R^{10}$ and $R^{11}$ are all H;compound 633: n is 0, X is $SO_2$, Y is O, $R^6$ is Me, $R^9$ is $CF_3$, and $R^2$, $R^3$, $R^4$, $R^5$, $R^7$, $R^8$, $R^{10}$ and $R^{11}$ are all H;compound 634: n is 0, X is $SO_2$, Y is O, $R^6$ is Me, $R^7$ is $OCF_3$, and $R^2$, $R^3$, $R^4$, $R^5$, $R^8$, $R^9$, $R^{10}$ and $R^{11}$ are all H;compound 635: n is 0, X is $SO_2$, Y is O, $R^6$ is Me, $R^8$ is $OCF_3$, and $R^2$, $R^3$, $R^4$, $R^5$, $R^7$, $R^9$, $R^{10}$ and $R^{11}$ are all H;compound 636: n is 0, X is $SO_2$, Y is O, $R^6$ is Me, $R^9$ is $OCF_3$, and $R^2$, $R^3$, $R^4$, $R^5$, $R^7$, $R^8$, $R^{10}$ and $R^{11}$ are all H;compound 637: n is 0, X is $SO_2$, Y is O, $R^6$ is Me, $R^7$ is $OCHF_2$, and $R^2$, $R^3$, $R^4$, $R^5$, $R^8$, $R^9$, $R^{10}$ and $R^{11}$ are all H;compound 638: n is 0, X is $SO_2$, Y is O, $R^6$ is Me, $R^8$ is $OCHF_2$, and $R^2$, $R^3$, $R^4$, $R^5$, $R^7$, $R^9$, $R^{10}$ and $R^{11}$ are all H;compound 639: n is 0, X is $SO_2$, Y is O, $R^6$ is Me, $R^9$ is $OCHF_2$, and $R^2$, $R^3$, $R^4$, $R^5$, $R^7$, $R^8$, $R^{10}$ and $R^{11}$ are all H;compound 640: n is 0, X is $SO_2$, Y is O, $R^6$ is Me, $R^9$ is $SCF_3$, and $R^2$, $R^3$, $R^4$, $R^5$, $R^7$, $R^8$, $R^{10}$ and $R^{11}$ are all H;compound 641: n is 0, X is $SO_2$, Y is O, $R^6$ is Me, $R^7$ is $OCH_3$, and $R^2$, $R^3$, $R^4$, $R^5$, $R^8$, $R^9$, $R^{10}$ and $R^{11}$ are all H;compound 642: n is 0, X is $SO_2$, Y is O, $R^6$ is Me, $R^8$ is $OCH_3$, and $R^2$, $R^3$, $R^4$, $R^5$, $R^7$, $R^9$, $R^{10}$ and $R^{11}$ are all H;compound 643: n is 0, X is $SO_2$, Y is O, $R^6$ is Me, $R^9$ is $OCH_3$, and $R^2$, $R^3$, $R^4$, $R^5$, $R^7$, $R^8$, $R^{10}$ and $R^{11}$ are all H;compound 644: n is 0, X is $SO_2$, Y is O, $R^6$ is Me, $R^9$ is $OC_2H_5$, $R^2$, $R^3$, $R^4$, $R^5$, $R^7$, $R^8$, $R^{10}$ and $R^{11}$ are all H;compound 645: n is 0, X is $SO_2$, Y is O, $R^6$ is Me, $R^7$ is CN, and $R^2$, $R^3$, $R^4$, $R^5$, $R^8$, $R^9$, $R^{10}$ and $R^{11}$ are all H;compound 646: n is 0, X is $SO_2$, Y is O, $R^6$ is Me, $R^8$ is CN, and $R^2$, $R^3$, $R^4$, $R^5$, $R^7$, $R^9$, $R^{10}$ and $R^{11}$ are all H;compound 647: n is 0, X is $SO_2$, Y is O, $R^6$ is Me, $R^9$ is CN, and $R^2$, $R^3$, $R^4$, $R^5$, $R^7$, $R^8$, $R^{10}$ and $R^{11}$ are all H;compound 648: n is 0, X is $SO_2$, Y is O, $R^6$ is Me, $R^7$ is $CH_3$, and $R^2$, $R^3$, $R^4$, $R^5$, $R^8$, $R^9$, $R^{10}$ and $R^{11}$ are all H;compound 649: n is 0, X is $SO_2$, Y is O, $R^6$ is Me, $R^8$ is $CH_3$, and $R^2$, $R^3$, $R^4$, $R^5$, $R^7$, $R^9$, $R^{10}$ and $R^{11}$ are all H;compound 650: n is 0, X is $SO_2$, Y is O, $R^6$ is Me, $R^9$ is $CH_3$, and $R^2$, $R^3$, $R^4$, $R^5$, $R^7$, $R^8$, $R^{10}$ and $R^{11}$ are all H;compound 651: n is 0, X is $SO_2$, Y is O, $R^6$ is Me, $R^7$ is $CH_3SO_2$, $R^2$, $R^3$, $R^4$, $R^5$, $R^8$, $R^9$, $R^{10}$ and $R^{11}$ are all H;compound 652: n is 0, X is $SO_2$, Y is O, $R^6$ is Me, $R^8$ is $CH_3SO_2$, $R^2$, $R^3$, $R^4$, $R^5$, $R^7$, $R^9$, $R^{10}$ and $R^{11}$ are all H;compound 653: n is 0, X is $SO_2$, Y is O, $R^6$ is Me, $R^9$ is $CH_3SO_2$, $R^2$, $R^3$, $R^4$, $R^5$, $R^7$, $R^8$, $R^{10}$ and $R^{11}$ are all H;compound 654: n is 0, X is $SO_2$, Y is O, $R^6$ is Me, $R^7$ is $C_2H_5SO_2$, $R^2$, $R^3$, $R^4$, $R^5$, $R^8$, $R^9$, $R^{10}$ and $R^{11}$ are all H;compound 655: n is 0, X is $SO_2$, Y is O, $R^6$ is Me, $R^7$ and $R^8$ are both Cl, and $R^2$, $R^3$, $R^4$, $R^5$, $R^9$, $R^{10}$ and $R^{11}$ are all H;compound 656: n is 0, X is $SO_2$, Y is O, $R^6$ is Me, $R^7$ and $R^9$ are both Cl, and $R^2$, $R^3$, $R^4$, $R^5$, $R^8$, $R^{10}$ and $R^{11}$ are all H;compound 657: n is 0, X is $SO_2$, Y is O, $R^6$ is Me, $R^7$ and $R^{10}$ are both Cl, and $R^2$, $R^3$, $R^4$, $R^5$, $R^8$, $R^9$ and $R^{11}$ are all H;compound 658: n is 0, X is $SO_2$, Y is O, $R^6$ is Me, $R^7$ and $R^{11}$ are both Cl, and $R^2$, $R^3$, $R^4$, $R^5$, $R^8$, $R^9$ and $R^{10}$ are all H;compound 659: n is 0, X is $SO_2$, Y is O, $R^6$ is Me, $R^8$ and $R^9$ are both Cl, and $R^2$, $R^3$, $R^4$, $R^5$, $R^7$, $R^{10}$ and $R^{11}$ are all H;compound 660: n is 0, X is $SO_2$, Y is O, $R^6$ and $R^7$ are Me, $R^9$ is Cl, and $R^2$, $R^3$, $R^4$, $R^5$, $R^8$, $R^{10}$ and $R^{11}$ are all H;compound 661: n is 0, X is $SO_2$, Y is O, $R^6$, $R^7$ and $R^8$ are Me, and $R^2$, $R^3$, $R^4$, $R^5$, $R^9$, $R^{10}$ and $R^{11}$ are all H;compound 662: n is 0, X is $SO_2$, Y is O, $R^6$, $R^7$ and $R^9$ are Me, and $R^2$, $R^3$, $R^4$, $R^5$, $R^8$, $R^{10}$ and $R^{11}$ are all H;compound 663: n is 0, X is $SO_2$, Y is O, $R^6$, $R^7$ and $R^{10}$ are Me, and $R^2$, $R^3$, $R^4$, $R^5$, $R^8$, $R^9$ and $R^{11}$ are all H;compound 664: n is 0, X is $SO_2$, Y is O, $R^6$, $R^7$ and $R^{11}$ are Me, and $R^2$, $R^3$, $R^4$, $R^5$, $R^8$, $R^9$ and $R^{10}$ are all H;compound 665: n is 0, X is $SO_2$, Y is O, $R^6$, $R^7$, $R^8$ and $R^{11}$ are Me, and $R^2$, $R^3$, $R^4$, $R^5$, $R^9$ and $R^{10}$ are all H;compound 666: n is 0, X is $SO_2$, Y is O, $R^6$, $R^7$, $R^9$ and $R^{11}$ are Me, and $R^2$, $R^3$, $R^4$, $R^5$, $R^8$ and $R^{10}$ are all H;compound 667: n is 0, X is $SO_2$, Y is O, $R^6$ is Me, $R^7$ and $R^9$ are both $CF_3$, $R^2$, $R^3$, $R^4$, $R^5$, $R^8$, $R^{10}$ and $R^{11}$ are all H;compound 668: n is 0, X is $SO_2$, Y is O, $R^6$ is Me, $R^8$ and $R^{10}$ are both $CF_3$, $R^2$, $R^3$, $R^4$, $R^5$, $R^7$, $R^9$ and $R^{11}$ are all H;compound 669: n is 0, X is $SO_2$, Y is O, $R^6$ is Me, $R^2$, $R^3$, $R^4$ and $R^5$ are all H, and $R^7$, $R^8$, $R^9$, $R^{10}$ and $R^{11}$ are all F;compound 670: n is 0, X is $SO_2$, Y is O, $R^6$ is Me, $R^7$ is $CH_3CH_2$, $R^2$, $R^3$, $R^4$, $R^5$, $R^8$, $R^9$, $R^{10}$ and $R^{11}$ are all H;compound 671: n is 0, X is $SO_2$, Y is O, $R^6$ is Me, $R^8$ is $CH_3CH_2$, $R^2$, $R^3$, $R^4$, $R^5$, $R^7$, $R^9$, $R^{10}$ and $R^{11}$ are all H;compound 672: n is 0, X is $SO_2$, Y is O, $R^6$ is Me, $R^9$ is $CH_3CH_2$, $R^2$, $R^3$, $R^4$, $R^5$, $R^7$, $R^8$, $R^{10}$ and $R^{11}$ are all H;compound 673: n is 0, X is $SO_2$, Y is O, $R^6$ is Me, $R^7$ is $CH(CH_3)_2$, $R^2$, $R^3$, $R^4$, $R^5$, $R^8$, $R^9$, $R^{10}$ and $R^{11}$ are all H;compound 674: n is 0, X is $SO_2$, Y is O, $R^6$ is Me, $R^8$ is

$CH(CH_3)_2$, $R^2$, $R^3$, $R^4$, $R^5$, $R^7$, $R^9$, $R^{10}$ and $R^{11}$ are all H;compound 675: n is 0, X is $SO_2$, Y is O, $R^6$ is Me, $R^9$ is $CH(CH_3)_2$, $R^2$, $R^3$, $R^4$, $R^5$, $R^7$, $R^8$, $R^{10}$ and $R^{11}$ are all H;Compound 676: n is 0, X is $SO_2$, Y is O, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;compound 677: n is 0, X is $SO_2$, Y is O, $R^6$ is Me, $R^7$ is $C(CH_3)_3$, $R^2$, $R^3$, $R^4$, $R^5$, $R^8$, $R^9$, $R^{10}$ and $R^{11}$ are all H;compound 678: n is 0, X is $SO_2$, Y is O, $R^6$ is Me, $R^7$ is

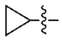

, $R^2$, $R^3$, $R^4$, $R^5$, $R^8$, $R^9$, $R^{10}$ and $R^{11}$ are all H;compound 679: n is 0, X is $SO_2$, Y is O, $R^6$ is Me, $R^7$ is

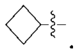

,

$R^2$, $R^3$, $R^4$, $R^5$, $R^8$, $R^9$, $R^{10}$ and $R^{11}$ are all H;compound 680: n is 0, X is $SO_2$, Y is O, $R^6$ is Me, $R^7$ is $CO_2CH_3$, $R^2$, $R^3$, $R^4$, $R^5$, $R^8$, $R^9$, $R^{10}$ and $R^{11}$ are all H;compound 681: n is 0, X is $SO_2$, Y is O, $R^6$ is Me, $R^7$ and $R^8$ are cyclized to form

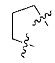

,

and $R^2$, $R^3$, $R^4$, $R^5$, $R^9$, $R^{10}$ and $R^{11}$ are all H;compound 682: n is 0, X is $SO_2$, Y is O, $R^6$ is Me, $R^8$ and $R^9$ are cyclized to form

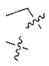

,

and $R^2$, $R^3$, $R^4$, $R^5$, $R^7$, $R^{10}$ and $R^{11}$ are all H;compound 683: n is 0, X is $SO_2$, Y is O, $R^6$ is Me, $R^8$ and $R^9$ are cyclized to form

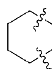

,

and $R^2$, $R^3$, $R^4$, $R^5$, $R^7$, $R^{10}$ and $R^{11}$ are all H;compound 684: n is 0, X is $SO_2$, Y is O, $R^6$ is Me, $R^7$ and $R^8$ are cyclized to form

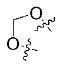

,

and $R^2$, $R^3$, $R^4$, $R^5$, $R^9$, $R^{10}$ and $R^{11}$ are all H;compound 685: n is 0, X is $SO_2$, Y is O, $R^6$ is Me, $R^8$ and $R^9$ are cyclized to form

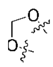

,

and $R^2$, $R^3$, $R^4$, $R^5$, $R^7$, $R^{10}$ and $R^{11}$ are all H;compound 686: n is 0, X is $SO_2$, Y is O, $R^6$ is Me, $R^7$ and $R^8$ are cyclized to form

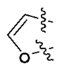

,

and $R^2$, $R^3$, $R^4$, $R^5$, $R^9$, $R^{10}$ and $R^{11}$ are all H;compound 687: n is 0, X is $SO_2$, Y is O, $R^6$ is Me, $R^7$ and $R^8$ are cyclized to form

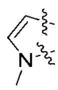

,

and $R^2$, $R^3$, $R^4$, $R^5$, $R^9$, $R^{10}$ and $R^{11}$ are all H;compound 688: n is 0, X is $SO_2$, Y is O, $R^6$ is Me, $R^7$ and $R^8$ are cyclized to form

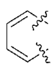

,

and $R^2$, $R^3$, $R^4$, $R^5$, $R^9$, $R^{10}$ and $R^{11}$ are all H;compound 689: n is 0, X is $SO_2$, Y is O, $R^6$ is Me, $R^7$ and $R^8$ are cyclized to form

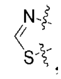,

and $R^2$, $R^3$, $R^4$, $R^5$, $R^9$, $R^{10}$ and $R^{11}$ are all H;compound 690: n is 0, X is $SO_2$, Y is O, $R^6$ is Me, $R^7$ and $R^8$ are cyclized to form

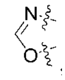

, and $R^2$, $R^3$, $R^4$, $R^5$, $R^9$, $R^{10}$ and $R^{11}$ are all H;compound 691: n is 0, X is $SO_2$, Y is O, $R^6$ is Me, $R^8$ and $R^9$ are cyclized to form

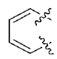,

and $R^2$, $R^3$, $R^4$, $R^5$, $R^7$, $R^{10}$ and $R^{11}$ are all H;compound 692: n is 1, X is S, Y is O, $R^6$ is Me, $R^2$, $R^3$, $R^4$, $R^5$, $R^7$, $R^8$, $R^9$, $R^{10}$ and $R^{11}$ are all H;compound 693: n is 2, X is S, Y is O, $R^6$ is Me, $R^2$, $R^3$, $R^4$, $R^5$, $R^7$, $R^8$, $R^9$, $R^{10}$ and $R^{11}$ are all H;compound 697: n is 0, X is S, Y is O, $R^2$ is

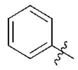,

$R^3$, $R^4$, $R^5$, $R^7$, $R^8$, $R^9$, $R^{10}$ and $R^{11}$ are all H, and $R^6$ is $CH_3$.

According to another preferred embodiment, in formula (I), $R^1$ is selected from the group consisting of $C_{1-6}$ alkyl, -$(CH_2)_z$-phenyl, an unsaturated five-membered ring group containing a heteroatom selected from class B, a saturated four-membered ring group containing a heteroatom selected from class B, an unsaturated six-membered ring group containing a heteroatom selected from class B, a saturated cycloalkyl group of $C_{4-8}$, benzothiazolyl, benzoxazolyl, benzopyrazolyl, phenylpropylthiophenyl, N-methyl-benzopyrolyl,

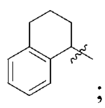;

z is an integer from 1 to 6; each of said class B heteroatoms is each independently at least one selected from O, S, N; and optionally, the class B heteroatom contains $C_{1-3}$ alkyl as a substituent; $R^2$ is selected from H, n-butyl, -$CH_2$-phenyl; $R^3$, $R^4$ and $R^5$ are all H; $R^6$ is methyl; n is 0; X is S; Y is O. More preferably, wherein, in formula (I), the substitution of n, X, Y, $R^1$, $R^2$, $R^3$, $R^4$, $R^5$ and $R^6$ is as follows:

compound 694: n is 0, X is S, Y is O, $R^1$ is

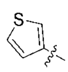,

$R^2$, $R^3$, $R^4$ and $R^5$ are all H, and $R^6$ is $CH_3$;compound 695: n is 0, X is S, Y is O, $R^1$ is

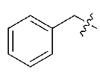,

$R^2$ is H, $R^3$, $R^4$ and $R^5$ are all H, and $R^6$ is $CH_3$;compound 696: n is 0, X is S, Y is O, $R^1$ is

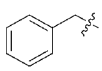,

$R^2$ is

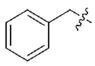,

$R^3$, $R^4$ and $R^5$ are all H, $R^6$ is $CH_3$;compound 698: n is 0, X is S, Y is O, $R^1$ is $CH_3$, $R^2$ is $CH_3$, $R^3$, $R^4$ and $R^5$ are all H, and $R^6$ is $CH_3$;compound 699: n is 0, X is S, Y is O, $R^1$ is $CH_3CH_2$, $R^2$ is $CH_3$, $R^3$, $R^4$ and $R^5$ are all H, and $R^6$ is $CH_3$;compound 700: n is 0, X is S, Y is O, $R^1$ is $CH_3CH_2$, $R^2$ is $CH_3CH_2$, $R^3$, $R^4$ and $R^5$ are all H, and $R^6$ is $CH_3$;compound 701: n is 0, X is S, Y is O, $R^1$ is $(CH_3)_2CH_2$, $R^2$ is $(CH_3)_2CH_2$, $R^3$, $R^4$ and $R^5$ are all H, and $R^6$ is $CH_3$; compound 702: n is 0, X is S, Y is O, $R^1$ is $(CH_3)_2CH_2$, $R^2$ is $CH_3CH_2$, $R^3$, $R^4$ and $R^5$ are all H, and $R^6$ is $CH_3$;compound 703: n is 0, X is S, Y is O, $R^1$ is $CH_3CH_3CH_2$, $R^2$ is $CH_3CH_3CH_2$, $R^3$, $R^4$ and $R^5$ are all H, and $R^6$ is $CH_3$;compound 704: n is 0, X is S, Y is O, $R^1$ is

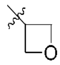,

$R^2$, $R^3$, $R^4$ and $R^5$ are all H, and $R^6$ is $CH_3$;compound 705: n is 0, X is S, Y is O, $R^1$ is

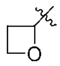,

$R^2$, $R^3$, $R^4$ and $R^5$ are all H, and $R^6$ is $CH_3$;compound 706: n is 0, X is S, Y is O, $R^1$ is

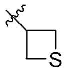,

$R^2$, $R^3$, $R^4$ and $R^5$ are all H, and $R^6$ is $CH_3$;compound 707: n is 0, X is S, Y is O, $R^1$ is

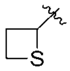,

$R^2$, $R^3$, $R^4$ and $R^5$ are all H, and $R^6$ is $CH_3$;compound 708: n is 0, X is S, Y is O, $R^1$ is

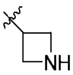,

$R^2$, $R^3$, $R^4$ and $R^5$ are all H, and $R^6$ is $CH_3$;compound 709: n is 0, X is S, Y is O, $R^1$ is

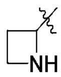,

$R^2$, $R^3$, $R^4$ and $R^5$ are all H, and $R^6$ is $CH_3$;compound 710: n is 0, X is S, Y is O, $R^1$ is

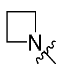,

$R^2$, $R^3$, $R^4$ and $R^5$ are all H, and $R^6$ is $CH_3$;compound 711: n is 0, X is S, Y is O, $R^1$ is

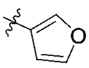,

$R^2$, $R^3$, $R^4$ and $R^5$ are all H, and $R^6$ is $CH_3$;compound 712: n is 0, X is S, Y is O, $R^1$ is

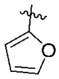,

$R^2$, $R^3$, $R^4$ and $R^5$ are all H, and $R^6$ is $CH_3$;compound 713: n is 0, X is S, Y is O, $R^1$ is

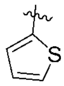,

$R^2$, $R^3$, $R^4$ and $R^5$ are all H, and $R^6$ is $CH_3$;compound 714: n is 0, X is S, Y is O, $R^1$ is

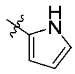,

$R^2$, $R^3$, $R^4$ and $R^5$ are all H, and $R^6$ is $CH_3$;compound 715: n is 0, X is S, Y is O, $R^1$ is

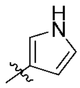,

$R^2$, $R^3$, $R^4$ and $R^5$ are all H, and $R^6$ is $CH_3$;compound 716: n is 0, X is S, Y is O, $R^1$ is

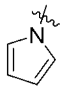,

, $R^2$, $R^3$, $R^4$ and $R^5$ are all H, and $R^6$ is $CH_3$;compound 717: n is 0, X is S, Y is O, $R^1$ is

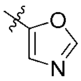,

$R^2$, $R^3$, $R^4$ and $R^5$ are all H, and $R^6$ is $CH_3$;compound 718: n is 0, X is S, Y is O, $R^1$ is

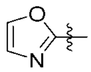,

$R^2$, $R^3$, $R^4$ and $R^5$ are all H, and $R^6$ is $CH_3$;compound 719: n is 0, X is S, Y is O, $R^1$ is

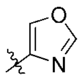,

$R^2$, $R^3$, $R^4$ and $R^5$ are all H, and $R^6$ is $CH_3$;compound 720: n is 0, X is S, Y is O, $R^1$ is

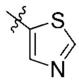,

$R^2$, $R^3$, $R^4$ and $R^5$ are all H, and $R^6$ is $CH_3$;compound 721: n is 0, X is S, Y is O, $R^1$ is

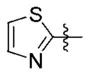,

$R^2$, $R^3$, $R^4$ and $R^5$ are all H, and $R^6$ is $CH_3$;compound 722: n is 0, X is S, Y is O, $R^1$ is

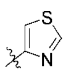,

$R^2$, $R^3$, $R^4$ and $R^5$ are all H, and $R^6$ is $CH_3$;compound 723: n is 0, X is S, Y is O, $R^1$ is

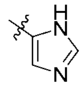,

$R^2$, $R^3$, $R^4$ and $R^5$ are all H, and $R^6$ is $CH_3$;compound 724: n is 0, X is S, Y is O, $R^1$ is

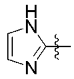,

$R^2$, $R^3$, $R^4$ and $R^5$ are all H, and $R^6$ is $CH_3$;compound 725: n is 0, X is S, Y is O, $R^1$ is

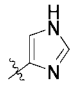,

$R^2$, $R^3$, $R^4$ and $R^5$ are all H, and $R^6$ is $CH_3$;compound 726: n is 0, X is S, Y is O, $R^1$ is

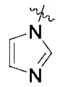,

$R^2$, $R^3$, $R^4$ and $R^5$ are all H, and $R^6$ is $CH_3$;compound 727: n is 0, X is S, Y is O, $R^1$ is

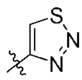,

$R^2$, $R^3$, $R^4$ and $R^5$ are all H, and $R^6$ is $CH_3$;compound 728: n is 0, X is S, Y is O, $R^1$ is

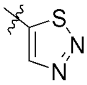,

$R^2$, $R^3$, $R^4$ and $R^5$ are all H, and $R^6$ is $CH_3$;compound 729: n is 0, X is S, Y is O, $R^1$ is

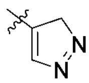,

$R^2$, $R^3$, $R^4$ and $R^5$ are all H, and $R^6$ is $CH_3$;compound 730: n is 0, X is S, Y is O, $R^1$ is

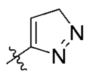,

$R^2$, $R^3$, $R^4$ and $R^5$ are all H, and $R^6$ is $CH_3$;compound 731: n is 0, X is S, Y is O, $R^1$ is

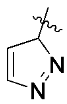,

$R^2$, $R^3$, $R^4$ and $R^5$ are all H, and $R^6$ is $CH_3$;compound 732: n is 0, X is S, Y is O, $R^1$ is

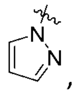

,

$R^2$, $R^3$, $R^4$ and $R^5$ are all H, and $R^6$ is $CH_3$;compound 733: n is 0, X is S, Y is O, $R^1$ is

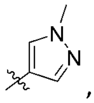

,

$R^2$, $R^3$, $R^4$ and $R^5$ are all H, and $R^6$ is $CH_3$;compound 734: n is 0, X is S, Y is O, $R^1$ is

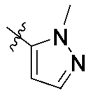

,

$R^2$, $R^3$, $R^4$ and $R^5$ are all H, and $R^6$ is $CH_3$;compound 735: n is 0, X is S, Y is O, $R^1$ is

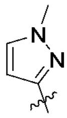

,

$R^2$, $R^3$, $R^4$ and $R^5$ are all H, and $R^6$ is $CH_3$;compound 736: n is 0, X is S, Y is O, $R^1$ is

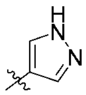

,

$R^2$, $R^3$, $R^4$ and $R^5$ are all H, and $R^6$ is $CH_3$;compound 737: n is 0, X is S, Y is O, $R^1$ is

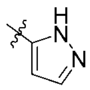

,

$R^2$, $R^3$, $R^4$ and $R^5$ are all H, and $R^6$ is $CH_3$;compound 738: n is 0, X is S, Y is O, $R^1$ is

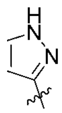

,

$R^2$, $R^3$, $R^4$ and $R^5$ are all H, and $R^6$ is $CH_3$;compound 739: n is 0, X is S, Y is O, $R^1$ is

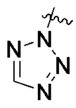

,

$R^2$, $R^3$, $R^4$ and $R^5$ are all H, and $R^6$ is $CH_3$;compound 740: n is 0, X is S, Y is O, $R^1$ is

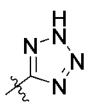

,

$R^2$, $R^3$, $R^4$ and $R^5$ are all H, and $R^6$ is $CH_3$;compound 741: n is 0, X is S, Y is O, $R^1$ is

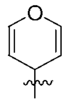,

$R^2$, $R^3$, $R^4$ and $R^5$ are all H, and $R^6$ is $CH_3$;compound 742: n is 0, X is S, Y is O, $R^1$ is

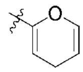,

$R^2$, $R^3$, $R^4$ and $R^5$ are all H, and $R^6$ is $CH_3$;compound 743: n is 0, X is S, Y is O, $R^1$ is

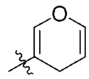,

$R^2$, $R^3$, $R^4$ and $R^5$ are all H, and $R^6$ is $CH_3$;compound 744: n is 0, X is S, Y is O, $R^1$ is

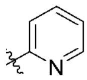

, $R^2$, $R^3$, $R^4$ and $R^5$ are all H, and $R^6$ is $CH_3$;compound 745: n is 0, X is S, Y is O, $R^1$ is

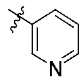,

$R^2$, $R^3$, $R^4$ and $R^5$ are all H, and $R^6$ is $CH_3$;compound 746: n is 0, X is S, Y is O, $R^1$ is

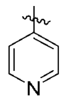,

$R^2$, $R^3$, $R^4$ and $R^5$ are all H, and $R^6$ is $CH_3$;compound 747: n is 0, X is S, Y is O, $R^1$ is

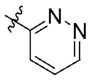,

$R^2$, $R^3$, $R^4$ and $R^5$ are all H, and $R^6$ is $CH_3$;compound 748: n is 0, X is S, Y is O, $R^1$ is

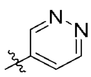,

$R^2$, $R^3$, $R^4$ and $R^5$ are all H, and $R^6$ is $CH_3$;compound 749: n is 0, X is S, Y is O, $R^1$ is

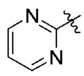

, $R^2$, $R^3$, $R^4$ and $R^5$ are all H, and $R^6$ is $CH_3$;compound 750: n is 0, X is S, Y is O, $R^1$ is

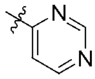,

$R^2$, $R^3$, $R^4$ and $R^5$ are all H, and $R^6$ is $CH_3$;compound 751: n is 0, X is S, Y is O, $R^1$ is

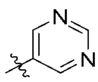,

$R^2$, $R^3$, $R^4$ and $R^5$ are all H, and $R^6$ is $CH_3$;compound 752: n is 0, X is S, Y is O, $R^1$ is

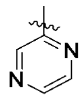,

$R^2$, $R^3$, $R^4$ and $R^5$ are all H, and $R^6$ is $CH_3$;compound 753: n is 0, X is S, Y is O, $R^1$ is

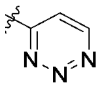,

$R^2$, $R^3$, $R^4$ and $R^5$ are all H, and $R^6$ is $CH_3$;compound 754: n is 0, X is S, Y is O, $R^1$ is

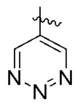,

$R^2$, $R^3$, $R^4$ and $R^5$ are all H, and $R^6$ is $CH_3$;compound 755: n is 0, X is S, Y is O, $R^1$ is

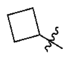,

$R^2$, $R^3$, $R^4$ and $R^5$ are all H, and $R^6$ is $CH_3$;compound 756: n is 0, X is S, Y is O, $R^1$ is

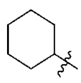,

$R^2$, $R^3$, $R^4$ and $R^5$ are all H, and $R^6$ is $CH_3$; compound 757: n is 0, X is S, Y is O, $R^1$ is

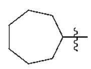,

$R^2$, $R^3$, $R^4$ and $R^5$ are all H, and $R^6$ is $CH_3$;compound 758: n is 0, X is S, Y is O, $R^1$ is

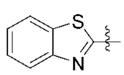,

$R^2$, $R^3$, $R^4$ and $R^5$ are all H, and $R^6$ is $CH_3$;compound 759: n is 0, X is S, Y is O, $R^1$ is

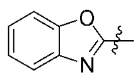,

$R^2$, $R^3$, $R^4$ and $R^5$ are all H, and $R^6$ is $CH_3$;compound 760: n is 0, X is S, Y is O, $R^1$ is

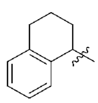,

$R^2$, $R^3$, $R^4$ and $R^5$ are all H, and $R^6$ is $CH_3$;compound 761: n is 0, X is S, Y is O, $R^1$ is

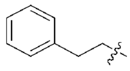,

$R^2$, $R^3$, $R^4$ and $R^5$ are all H, and $R^6$ is $CH_3$;compound 762: n is 0, X is S, Y is O, $R^1$ is

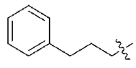,

, $R^2$, $R^3$, $R^4$ and $R^5$ are all H, and $R^6$ is $CH_3$;compound 763: n is 0, X is S, Y is O, $R^1$ is

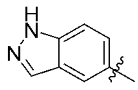,

$R^2$, $R^3$, $R^4$ and $R^5$ are all H, and $R^6$ is $CH_3$;compound 764: n is 0, X is S, Y is O, $R^1$ is

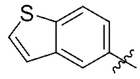,

$R^2$, $R^3$, $R^4$ and $R^5$ are all H, and $R^6$ is $CH_3$;compound 765: n is 0, X is S, Y is O, $R^1$ is

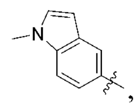,

$R^2$, $R^3$, $R^4$ and $R^5$ are all H, and $R^6$ is $CH_3$;compound 766: n is 0, X is S, Y is O, $R^1$ is

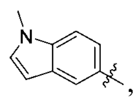,

$R^2$, $R^3$, $R^4$ and $R^5$ are all H, and $R^6$ is $CH_3$;compound 767: n is 0, X is S, Y is O, $R^1$ is $CH_3(CH_2)_3$, $R^2$ is $CH_3(CH_2)_3$, $R^3$, $R^4$ and $R^5$ are all H, and $R^6$ is $CH_3$.

Preferably, in formula (1), X and Y are both O. More preferably, the compound of formula (I) has the structure of a compound of formula (I-A):

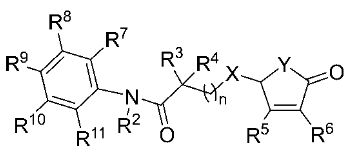 formula (I-A),

wherein, in formula (I-A), the substitution of n, X, Y, $R^2$, $R^3$, $R^4$, $R^5$, $R^6$, $R^7$, $R^8$, $R^9$, $R^{10}$ and $R^{11}$ is as follows:

compound 768: n is 0, X is O, Y is O, $R^6$ is Me, $R^2$, $R^3$, $R^4$, $R^5$, $R^7$, $R^8$, $R^9$, $R^{10}$ and $R^{11}$ are all H;compound 769: n is 0, X is O, Y is O, $R^6$ is Me, $R^7$ is F, and $R^2$, $R^3$, $R^4$, $R^5$, $R^7$, $R^9$, $R^{10}$ and $R^{11}$ are all H;compound 770: n is 0, X is O, Y is O, $R^6$ is Me, $R^8$ is F, and $R^2$, $R^3$, $R^4$, $R^5$, $R^7$, $R^9$, $R^{10}$ and $R^{11}$ are all H;compound 771: n is 0, X is O, Y is O, $R^6$ is Me, $R^9$ is F, and $R^2$, $R^3$, $R^4$, $R^5$, $R^7$, $R^8$, $R^{10}$ and $R^{11}$ are all H;compound 772: n is 0, X is O, Y is O, $R^6$ is Me, $R^7$ is Cl, and $R^2$, $R^3$, $R^4$, $R^5$, $R^8$, $R^9$, $R^{10}$ and $R^{11}$ are all H;compound 773: n is 0, X is O, Y is O, $R^6$ is Me, $R^8$ is Cl, and $R^2$, $R^3$, $R^4$, $R^5$, $R^7$, $R^9$, $R^{10}$ and $R^{11}$ are all H;compound 774: n is 0, X is O, Y is O, $R^6$ is Me, $R^9$ is Cl, and $R^2$, $R^3$, $R^4$, $R^5$, $R^7$, $R^8$, $R^{10}$ and $R^{11}$ are all H;compound 775: n is 0, X is O, Y is O, $R^6$ is Me, $R^7$ is Br, and $R^2$, $R^3$, $R^4$, $R^5$, $R^8$, $R^9$, $R^{10}$ and $R^{11}$ are all H;compound 776: n is 0, X is O, Y is O, $R^6$ is Me, $R^8$ is Br, and $R^2$, $R^3$, $R^4$, $R^5$, $R^7$, $R^9$, $R^{10}$ and $R^{11}$ are all H;compound 777: n is 0, X is O, Y is O, $R^6$ is Me, $R^9$ is Br, and $R^2$, $R^3$, $R^4$, $R^5$, $R^7$, $R^8$, $R^{10}$ and $R^{11}$ are all H;compound 778: n is 0, X is O, Y is O, $R^6$ is Me, $R^7$ is $NO_2$, and $R^2$, $R^3$, $R^4$, $R^5$, $R^8$, $R^9$, $R^{10}$ and $R^{11}$ are all H;compound 779: n is 0, X is O, Y is O, $R^6$ is Me, $R^8$ is $NO_2$, and $R^2$, $R^3$, $R^4$, $R^5$, $R^7$, $R^9$, $R^{10}$ and $R^{11}$ are all H;compound 780: n is 0, X is O, Y is O, $R^6$ is Me, $R^9$ is $NO_2$, and $R^2$, $R^3$, $R^4$, $R^5$, $R^7$, $R^8$, $R^{10}$ and $R^{11}$ are all H;compound 781: n is 0, X is O, Y is O, $R^6$ is Me, $R^7$ is $CF_3$, and $R^2$, $R^3$, $R^4$, $R^5$, $R^8$, $R^9$, $R^{10}$ and $R^{11}$ are all H;compound 782: n is 0, X is O, Y is O,

$R^6$ is Me, $R^8$ is $CF_3$, and $R^2$, $R^3$, $R^4$, $R^5$, $R^7$, $R^9$, $R^{10}$ and $R^{11}$ are all H;compound 783: n is 0, X is O, Y is O, $R^6$ is Me, $R^9$ is $CF_3$, and $R^2$, $R^3$, $R^4$, $R^5$, $R^7$, $R^8$, $R^{10}$ and $R^{11}$ are all H;compound 784: n is 0, X is O, Y is O, $R^6$ is Me, $R^7$ is $OCH_3$, and $R^2$, $R^3$, $R^4$, $R^5$, $R^8$, $R^9$, $R^{10}$ and $R^{11}$ are all H;compound 785: n is 0, X is O, Y is O, $R^6$ is Me, $R^8$ is $OCH_3$, and $R^2$, $R^3$, $R^4$, $R^5$, $R^7$, $R^9$, $R^{10}$ and $R^{11}$ are all H;compound 786: n is 0, X is O, Y is O, $R^6$ is Me, $R^9$ is $OCH_3$, and $R^2$, $R^3$, $R^4$, $R^5$, $R^7$, $R^8$, $R^{10}$ and $R^{11}$ are all H;compound 787: n is 0, X is O, Y is O, $R^6$ and $R^7$ are Me, and $R^2$, $R^3$, $R^4$, $R^5$, $R^8$, $R^9$, $R^{10}$ and $R^{11}$ are all H;compound 788: n is 0, X is O, Y is O, $R^6$ and $R^8$ are Me, and $R^2$, $R^3$, $R^4$, $R^5$, $R^7$, $R^9$, $R^{10}$ and $R^{11}$ are all H;compound 789: n is 0, X is O, Y is O, $R^6$ and $R^9$ are Me, and $R^2$, $R^3$, $R^4$, $R^5$, $R^7$, $R^8$, $R^{10}$ and $R^{11}$ are all H;compound 790: n is 0, X is O, Y is O, $R^5$ is Me, $R^7$ is $CH_3CH_2$, and $R^2$, $R^3$, $R^4$, $R^6$, $R^8$, $R^9$, $R^{10}$ and $R^{11}$ are all H;compound 791: n is 0, X is O, Y is O, $R^5$ is Me, $R^8$ is $CH_3CH_2$, and $R^2$, $R^3$, $R^4$, $R^6$, $R^7$, $R^9$, $R^{10}$ and $R^{11}$ are all H;compound 792: n is 0, X is O, Y is O, $R^5$ is Me, $R^9$ is $CH_3CH_2$, and $R^2$, $R^3$, $R^4$, $R^6$, $R^7$, $R^8$, $R^{10}$ and $R^{11}$ are all H;compound 793: n is 0, X is O, Y is O, $R^5$ is Me, $R^7$ is $CH(CH_3)_2$, and $R^2$, $R^3$, $R^4$, $R^6$, $R^8$, $R^9$, $R^{10}$ and $R^{11}$ are all H;compound 794: n is 0, X is O, Y is O, $R^5$ is Me, $R^8$ is $CH(CH_3)_2$, and $R^2$, $R^3$, $R^4$, $R^6$, $R^7$, $R^9$, $R^{10}$ and $R^{11}$ are all H;compound 795: n is 0, X is O, Y is O, $R^5$ is Me, $R^9$ is $CH(CH_3)_2$, and $R^2$, $R^3$, $R^4$, $R^6$, $R^7$, $R^8$, $R^{10}$ and $R^{11}$ are all H.

The compound of formula (I) may exist in different stereoisomers or optical isomers or tautomers, and the present invention includes all stereoisomers or optical isomers or tautomers and mixtures thereof in various ratios.

The present invention is not particularly limited to a specific method for preparing the amide compound described in the first aspect, and those skilled in the art can obtain the aforementioned compound of the present invention according to a specific structural formula provided by the present invention in combination with a known process route in the field of organic synthesis, and the following description of the present invention exemplifies several examples to illustrate the preparation method of the compound of the present invention, and those skilled in the art can also obtain a specific preparation method of all the remaining compounds by substituting the kind of raw materials according to the preparation method of the following compound of the present invention. The present invention is not further described in detail with respect to the preparation of all compounds, and the person skilled in the art is not to be understood as a limitation of the invention.

Preferably, in order to obtain the compound having the structure represented by formula (I) according to the present invention in a higher yield, the present invention provides the method described in the second aspect for preparing the amide compound described in the first aspect. the method comprising: carrying out contact reaction on the compound shown in a formula (V-1) and the compound shown in a formula (V-2);

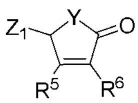 formula (V-1), 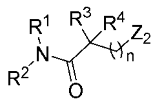 formula (V-2),

wherein the substitution of the substituents in the formulae (V-1) and (V-2) corresponds to the same substitution in the amide compound described in the first aspect, and one of $Z_1$ and $Z_2$ is -Br and the other is -XH, and X is as defined as corresponding to the definition in the amide compound described in the first aspect.

The present invention does not specifically require the specific operating conditions of the contact reaction, and those skilled in the art can determine reasonable ranges or values according to the knowledge known in the art of organic synthesis in combination with the preparation methods provided as examples in the following of the present invention. The present invention is not described in detail herein, and those skilled in the art should not be construed as limited thereto.

Preferably, in order to obtain the compound having the structure shown in formula (I) in higher yield, the invention also provides a method for preparing the amide compound described in the first aspect, which comprises: carrying out coupling reaction on a compound shown in a formula (II) and a compound shown in a formula (III);

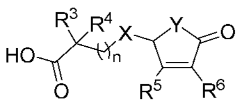 formula (II) 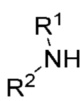 formula (III);

wherein the substitution of the substituents in the formulae (III) and (II) is the same as the substitution in the amide compound described in the first aspect.

The present invention has no particular requirements on the molar ratio of the compound represented by the formula (III) to the compound represented by the formula (II) and the reaction conditions, and those skilled in the art can determine the appropriate ratio of the amounts and the reaction conditions according to the structural formula characteristics and the knowledge of organic synthesis known in the art.

The present invention does not require particular operating conditions for the coupling reaction, and those skilled in the art can determine reasonable ranges or values according to the knowledge known in the art of organic synthesis in combination with the preparation methods provided by way of example hereinafter in the present invention. The present invention is not described in detail herein, and those skilled in the art should not be construed as limited thereto.

More preferably, the preparation method of the compound having the structure of formula (II) comprises: removing tert-butoxy in the compound shown in the formula (IV) in the presence of an acidic substance and a solvent;

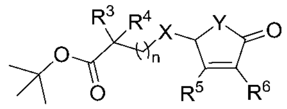 formula (IV);

wherein, in formula (IV),
n, X, Y, $R^3$, $R^4$, $R^5$, $R^6$ have the same definition as correspondingly described in the foregoing description of the present invention.

The reaction conditions for removing the tert-butoxy group from the compound represented by the formula (IV) are not particularly required in the present invention, and those skilled in the art can determine suitable reaction conditions according to the structural formula characteristics in combination with the knowledge of organic synthesis known in the art.

It will be understood by those skilled in the art that the method of the present invention may further include a step of purifying the obtained product, and there is no particular requirement for the purification method, and various purification methods conventionally used by those skilled in the art may be employed, for example, filtration, removal of a solvent, extraction with an extraction agent, drying with a drying agent, and removal of impurities by column chromatography or the like may be employed.

The fourth aspect of the invention provides an amide compound of the first aspect for use in agriculture.

Preferably, the agricultural application is selected from at least one application of increasing crop yield, managing abiotic stress on plants, regulating plant growth, enhancing disease resistance of plants, promoting seed germination.

Preferably, the seeds are selected from at least one of wheat, soybean, barley, corn, rice, sorghum, solanaceous plants, barnyard grass, leguminous plants such as peanut, crabgrass, and foxtail.

The amide compound in the present invention may be present alone or as an active ingredient in a powder, a solution, etc. in the application of the second aspect, and those skilled in the art should not be construed as limiting the present invention.

In a fifth aspect, the present invention provides a biostimulant, which comprises a plant growth regulating effective amount of at least one of the amide compounds described in the first aspect, and optionally an adjuvant.

Preferably, the biostimulant is applied to a plant to increase the biomass or yield of the plant by modulating the growth and development process of the plant; the plant is selected from at least one of wheat, peanut, corn, soybean and alfalfa.

The present invention has no particular requirement on the specific type of the adjuvant in the biostimulator, and the skilled person can select the corresponding adjuvant known in the art according to the dosage form to prepare the biostimulator of the present invention, and the present invention is not described in detail herein, and the skilled person should not be understood as limiting the present invention.

Preferably, in the biostimulant, the content of the amide compound is 1-99.99wt%, or the content of the amide compound is 0.001 ppm to 100 ppm.

The present invention also provides a method of application, which comprises applying an effective amount of the amide compound according to the first aspect and/or the modulator according to the third aspect to a plant propagation material-containing and/or a plant growing area.

It should be noted that the amide compounds described in the present invention can be used alone or in combination with plant hormones, such as auxins, gibberellins, cytokinins, ethylene, abscisic acid, and other compounds. Exemplarily, the amide compounds provided in the present invention can be used in combination with brassinosteroids, polyamines and jasmonic acid, pyraclostrobin, metyltetraprole, triclopyricarb, pyrametostrobin, fenaminstrobin, azoxystrobin, fluoxastrobin, trifloxystrobin, coumoxystrobin, pydiflumetofen, pyraclostrobin, boscalid, benzovindiflupyr, isopyrazam, sedaxane, penflufen, fluxapyroxad, and fluopyram, among others. The combined use can achieve a synergistic effect.

The invention also provides a weed control method, which comprises the following steps: applying a germination-promoting amount of the amide compound of the first aspect and/or the regulator of the third aspect to a weed-containing area to cause "space-time mismatch germination" of weeds, and then spraying a herbicide to the weeds after emergence.

In the present invention, the weed-containing region includes agricultural lands having root parasitic weeds, which may include, but are not limited to, paddy fields, wheat fields, sunflower fields, carrot fields, tobacco fields, solanaceae plant fields, corn fields, leguminous plant fields such as peanuts, or sorghum fields; preferably, the root parasitic weeds are striga asiatica and/or broomrape; the Striga is selected from at least one of *Striga asiatica* (L.) o. Kuntze, *Striga masuria* (Ham. ex Benth.) Benth, and *Striga densiflora* Benth; the broomrape is selected from at least one of *Orobanche minor* Sm and *Orobanche ramosa* L.

The present invention is not particularly limited to the specific type of herbicide, and a person skilled in the art can

select a herbicide known in the art, and the present invention will not be described in detail herein, and the person skilled in the art will not be construed as limiting the present invention.

The present invention will be described in detail below by way of examples. Unless otherwise specified, the room temperature or the ambient temperature referred to in the following examples means 25 ± 3 °C.

The various starting materials used in the examples below are, unless otherwise specified, commercially available and the resulting products are prepared and characterized by nuclear magnetic data.

The yields in the present invention are all calculated based on the main raw materials in the last step of chemical reaction to obtain the compound, unless otherwise specified.

CK represents a blank control unless otherwise specified.

Preparation example 1: preparation of compound I-1

This preparation is intended to illustrate the synthesis of compound I-1.

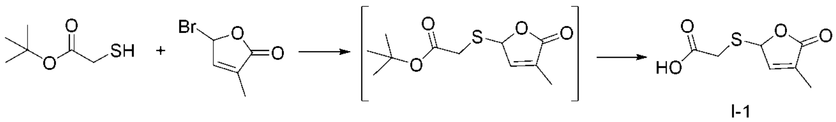

I-1

Adding 20 mmol of potassium carbonate and 20 mmol of tert-butyl 2-mercaptoacetate into a reaction bottle, adding 50 mL of acetone under stirring, reacting at room temperature for 15 min, adding 20 mmol of 5-bromo-3-methylfuran-2 (5H) -one, continuously stirring for reacting for 3h, removing insoluble substances by diatomite assisted suction filtration, removing acetone in the solution under reduced pressure, and purifying the obtained intermediate by column chromatography to obtain a compound IV-1 with the yield of 65%; nuclear magnetic data were $^1$H NMR (400 MHz, $CDCl_3$) $\delta$ 7.00 (s, 1H), 6.15 (s, 1H), 3.43 (d, $J$ = 15.6 Hz, 1H), 3.19 (d, $J$ = 15.6 Hz, 1H), 1.96 (s, 3H), 1.47 (s, 9H);

Dissolving 10 mmol of the compound IV-1 in 20 mL of dichloromethane solution, adding 5 mL of trifluoroacetic acid, reacting for 2 hours at room temperature, and purifying by column chromatography to obtain a compound I-1 with the yield of 90%; the nuclear magnetic data were $^1$H NMR (400 MHz, DMSO-$d_6$) $\delta$ 12.76 (s, 1H), 7.36 (s, 1H), 6.37 (s, 1H), 3.40 (q, $J$ = 15.6 Hz, 2H), 1.84 (s, 3H).

Preparation example 2: preparation of compound I-2

This preparation is illustrative of the synthesis of compound I-2, and the following synthetic method is provided as an example in the present invention to give said compound I-2.

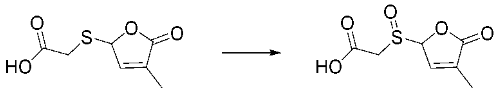

I-1 I-2

Dissolving 30 mol of compound I-1 synthesized in preparation example 1 in 60 mL of dichloromethane, adding 30 mmol of mechloroperbenzoic acid under an ice bath condition, reacting for 4 hours, adding saturated sodium bicarbonate solution to quench the reaction, extracting with water, washing an organic phase with saturated saline solution, drying with anhydrous sodium sulfate, concentrating in vacuum, and purifying by column chromatography to obtain compound I-2 with the yield of 60%; the nuclear magnetic data were $^1$H NMR (400 MHz, DMSO-$d_6$) $\delta$ 13.48 (s, 1H), 7.53 (s, 1H), 6.24 (s, 1H), 3.81 (dd, $J$ = 17.6, 15.2 Hz, 2H), 1.90 (s, 3H).

Preparation example 3: preparation of Compound I-3

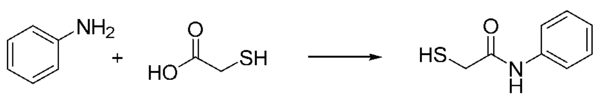

I-3

21 mmol of aniline and 21 mmol of mercaptoacetic acid are dissolved in 20 mL of anhydrous toluene and heated to 110 °C for 12 hours. Cooling the reaction solution to room temperature, and then carrying out vacuum concentration to

obtain an oily substance, and carrying out column chromatography to obtain a white solid I-3 with the yield of 80%; the nuclear magnetic data are: $^1$H NMR (400 MHz, DMSO-$d_6$) $\delta$ 10.07 (d, $J$ = 10.3 Hz, 1H), 7.57 (t, $J$ = 6.6 Hz, 2H), 7.29 (dt, $J$ = 13.3, 7.8 Hz, 2H), 7.05 (q, $J$ = 6.7, 6.0 Hz, 1H), 3.39 - 3.20 (m, 2H), 2.95 (s, 1H).

Preparation example 4: preparation of compound I-4

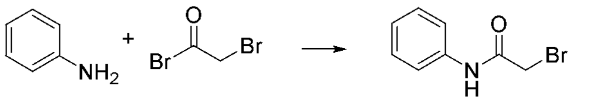

I-4

Dissolving 20 mmol of aniline in 20 mL of dichloromethane, adding 21 mmol of bromoacetyl bromide and 25 mmol of potassium carbonate, reacting for 2 hours at room temperature, filtering out solids after complete reaction, and concentrating a compound I-4 obtained by subjecting an organic phase to column chromatography, wherein the yield is 85%; the nuclear magnetic data were $^1$H NMR (400 MHz, $CDCl_3$)$\delta$ 9.90 (s, 1H), 7.75 - 7.49 (m, 2H), 7.40 - 7.24 (m, 2H), 7.07 (tt, $J$ = 7.2, 2.0 Hz, 1H), 4.24 (s, 2H).

Preparation example 5: preparation of compound I-5

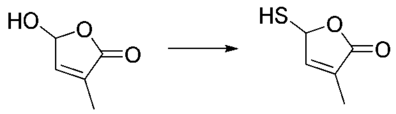

I-5

10 mmol of 5-hydroxy-3-methylfuran-2 (5H) -one was dissolved in 10 mL of toluene solution, then 5 mmol of Lawson's reagent was added and heated to reflux for 12 hours, after the reaction was complete, it was quenched with ice water, extracted with ethyl acetate, the organic phase was washed with saturated brine, dried over anhydrous sodium sulfate, concentrated in vacuo and purified by column chromatography to give compound I-5 in 36% yield with nuclear magnetic data of $^1$H NMR (400 MHz, DMSO-$d_6$) $\delta$ 7.41 (dd, $J$ = 6.4, 1.2 Hz, 1H), 6.07 (dd, $J$ = 6.4, 1.2 Hz, 1H), 2.02(s,3H) 1.40 (s, 1H).

Preparation example 6: preparation of Compound 1

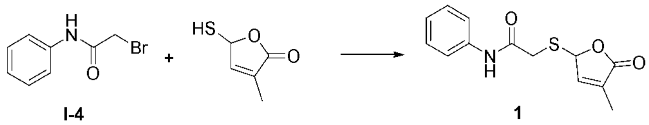

I-4 1

Compound I-4 (3 mmol) and I-5 (2.8 mmol) were dissolved in 5mL of N, N-dimethylformamide, followed by addition of potassium carbonate (4.5 mmol), and reacted at room temperature for 3 hours. After the reaction is completed, removing solids by suction filtration, extracting by using ethyl acetate and water, collecting an organic phase, washing by using saturated saline solution, drying by using anhydrous sodium sulfate, and carrying out vacuum concentration to obtain an oily substance, wherein the yield of a compound 1 of column chromatography is 25%; the nuclear magnetic data were $^1$H NMR (400 MHz, DMSO-$d_6$) $\delta$ 10.15 (s, 1H), 7.56 (d, $J$ = 8.0 Hz, 2H), 7.40 - 7.25 (m, 3H), 7.06 (t, $J$ = 7.2 Hz, 1H), 6.43 (s, 1H), 3.56 - 3.43 (m, 2H), 1.82 (s, 3H).

Preparation example 7: preparation of compound 1

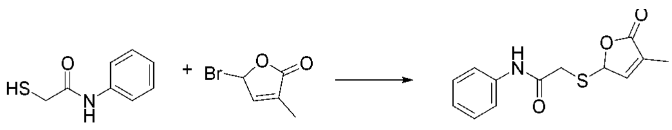

I-3

1

Compound I-3(13.3 mmol) and 13.3 mmol 5-bromo-3-methylfuran-2 (5H) -one) were dissolved in 200mL dichloromethane under ice-cooling condition, followed by addition of 15 mmol triethylamine, transfer to room temperature and stirring for 1 hour, TLC showed complete reaction, the mixture was concentrated in vacuo to give an oil, which was purified by column chromatography to afford compound 1 in 67%, $^1H$ NMR (400 MHz, DMSO-$d_6$) $\delta$ 10.15 (s, 1H), 7.56 (d, $J$ = 8.0 Hz, 2H), 7.40 - 7.25 (m, 3H), 7.06 (t, $J$ = 7.2 Hz, 1H), 6.43 (s, 1H), 3.56 - 3.43 (m, 2H), 1.82 (s, 3H),

Preparation example 8: preparation of compound 1

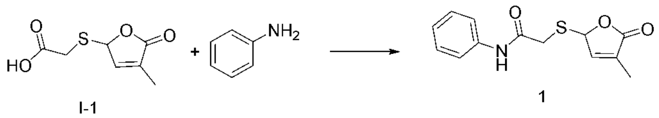

I-1

1

Dissolving 1 mmol of compound I-1 synthesized in preparation example 1 in 5mL of dichloromethane solution, adding 1.5 mmol of (7-azobenzotriazole) -N, N, N ', N' -tetramethylurea Hexafluorophosphate (HATU) and 2 mmol of triethylamine, stirring at room temperature for 15 minutes, adding 1.1 mmol of aniline into the reaction solution, reacting at room temperature for 6 hours, adding water into the reaction solution for extraction, washing the organic phase with 1mol/L hydrochloric acid and saturated saline water respectively, drying with anhydrous sodium sulfate, vacuum concentrating, and purifying by column chromatography to obtain the compound 1 with the yield of 50%; the nuclear magnetic data were $^1H$ NMR (400 MHz, DMSO-$d_6$) $\delta$ 10.15 (s, 1H), 7.56 (d, $J$ = 8.0 Hz, 2H), 7.40 - 7.25 (m, 3H), 7.06 (t, $J$ = 7.2 Hz, 1H), 6.43 (s, 1H), 3.56 - 3.43 (m, 2H), 1.82 (s, 3H).

Preparation example 9: preparation of compound 4

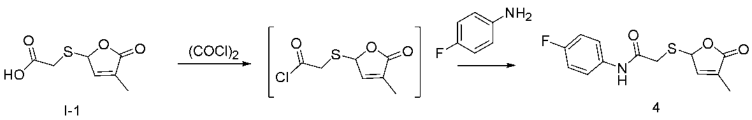


I-1

4

Dissolving 1 mmol of compound I-1 synthesized in preparation example 1 in 5mL of dichloromethane solution under ice bath condition, adding 1.1 mmol of oxalyl chloride and 0.1 mmol of N, N-dimethylformamide, reacting at room temperature for 2 hours to obtain an oily substance, dissolving the oily substance in 5mL of dichloromethane solution, adding 1 mmol of para-fluoroaniline and 1 mmol of triethylamine, reacting at room temperature for 2 hours, and purifying by column chromatography to obtain a compound 4 with the yield of 63%; the nuclear magnetic data were $^1H$ NMR (400 MHz, $CDCl_3$) $\delta$ 8.29 (s, 1H), 7.57 - 7.50 (m, 2H), 7.11 - 6.98 (m, 3H), 6.15 - 6.03 (m, 1H), 3.62 - 3.41 (m, 2H), 1.99 (t, $J$ = 2.0 Hz, 3H).

Preparation example 10: preparation of compound 5

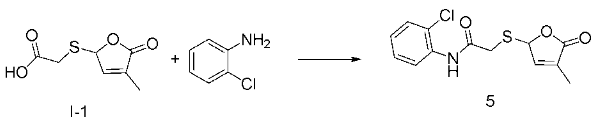

I-1

5

Dissolving 1 mmol of compound I-1 synthesized in preparation example 1 in 5mL of dichloromethane solution, adding 1.5 mmol of 1- (3-dimethylaminopropyl) -3-ethylcarbodiimide hydrochloride (EDCI) and 0.1 mmol of 4-Dimethylaminopyridine (DMAP), adding 1.1 mmol of o-chloroaniline into the reaction solution, reacting for 6 hours at room

temperature under the protection of argon, adding water into the reaction solution for extraction, washing an organic phase with saturated saline solution, drying with anhydrous sodium sulfate, concentrating in vacuum, and purifying by column chromatography to obtain a compound 5, wherein the yield is 75%; the nuclear magnetic data were $^1$H NMR (400 MHz, $CDCl_3$) $\delta$ 8.80 (s, 1H), 8.32 (d, $J$ = 8.0 Hz, 1H), 7.39 (d, $J$ = 9.2 Hz, 1H), 7.29 (t, $J$ = 8.0 Hz, 1H), 7.08 (t, $J$ = 8.4 Hz, 1H), 7.02 (s, 1H), 6.09 (s, 1H), 3.62 (d, $J$ = 16.8 Hz, 1H), 3.46 (d, $J$ = 16.8 Hz, 1H), 1.96 (s, 3H).

Preparation example 11: preparation of compound 9

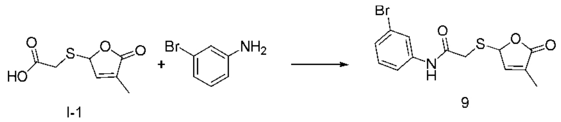

I-1

9

Dissolving 1 mmol of compound I-1 synthesized in preparation example 1 in 5mL of dichloromethane solution, adding 1.5 mmol of (7-azobenzotriazole) -N, N, N ', N' -tetramethylurea Hexafluorophosphate (HATU) and 2 mmol of N-ethyldiisopropylamine (DIPEA), stirring at room temperature for 15 minutes, adding 1.1 mmol of m-bromoaniline into the reaction solution, reacting at room temperature for 3 hours, adding water into the reaction solution for extraction, washing the organic phase with 1mol/L of hydrochloric acid and saturated salt solution in turn, drying with sodium sulfate, vacuum concentrating, and purifying by column chromatography to obtain compound 9 with yield of 71%; the nuclear magnetic data were $^1$H NMR (400 MHz, $CDCl_3$) $\delta$ 8.34 (s, 1H), 7.85 (s, 1H), 7.45 (d, $J$ = 8.4 Hz, 1H), 7.31 - 7.26 (m, 1H), 7.21 (t, $J$ = 8.0 Hz, 1H), 7.02 (s, 1H), 6.09 (s, 1H), 3.51 (q, $J$ = 16.8 Hz, 2H), 1.99 (s, 3H).

Preparation example 12: preparation of compound 19

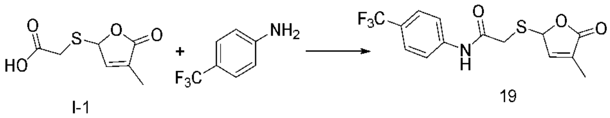

I-1

19

Dissolving 1 mmol of compound I-1 synthesized in preparation example 1 in 5mL of dichloromethane solution, adding 1.5 mmol of benzotriazole-N, N, N ', N' -tetramethyluronium Hexafluorophosphate (HBTU) and 2 mmol of N-ethyldiisopropylamine (DIPEA), stirring at room temperature for 15 minutes, adding 1.1 mmol of 4-trifluoromethylaniline into the reaction solution, continuing to react for 3 hours, adding water into the reaction solution for extraction, washing the organic phase with 1mol/L hydrochloric acid and saturated saline in sequence, drying with sodium sulfate, vacuum concentrating, and purifying by column chromatography to obtain compound 19 with a yield of 68%; the nuclear magnetic data were $^1$H NMR (400 MHz, DMSO-$d_6$) $\delta$ 10.52 (s, 1H), 7.77 (d, $J$ = 8.4 Hz, 2H), 7.68 (d, $J$ = 8.4 Hz, 2H), 7.36 (s, 1H), 6.42 (s, 1H), 3.62 - 3.46 (m, 2H), 1.82 (s, 3H).

Preparation example 13: preparation of compound 22

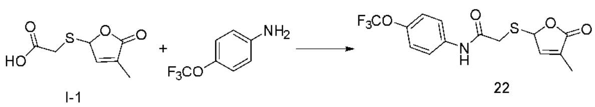

I-1

22

Dissolving 1 mmol of compound I-1 synthesized in preparation example 1 in 5mL of dichloromethane solution, adding 2 mmol of 1- (3-dimethylaminopropyl) -3-ethylcarbodiimide hydrochloride (EDCI) and 2 mmol of 1-Hydroxybenzotriazole (HOBT), adding 1.1 mmol of 4-trifluoromethoxyaniline into the reaction solution, reacting for 5 hours at room temperature, adding water into the reaction solution for extraction, washing an organic phase with saturated saline, drying the organic phase with anhydrous sodium sulfate, concentrating in vacuum, and purifying by column chromatography to obtain compound 22, wherein the yield is 62%; the nuclear magnetic data were $^1$H NMR (400 MHz, $CDCl_3$) $\delta$ 8.43 (s, 1H), 7.60 (d, $J$ = 8.8 Hz, 2H), 7.20 (d, $J$ = 8.4 Hz, 2H), 7.03 (s, 1H), 6.10 (s, 1H), 3.61 - 3.43 (m, 2H), 1.99 (s, 3H).

Preparation example 14: preparation of compound 768

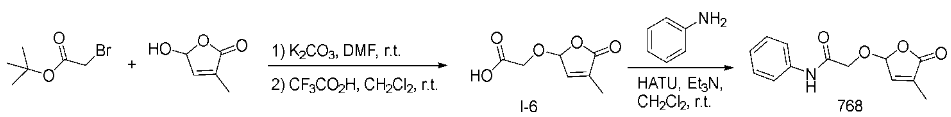


I-6

768

5 mmol of 5-hydroxy-3-methyl-2 (5H) -furanone are dissolved in 10 mL of N, N-dimethylformamide, and 6 mmol of potassium carbonate and 6 mmol of tert-butyl bromoacetate are added. After heating to 60 °C for reaction for 3 hours, extraction with water and ethyl acetate, washing of the organic phase with saturated brine, drying over anhydrous sodium sulfate, concentration in vacuo and purification by column chromatography of the resulting pale yellow solid (yield 62%; nuclear magnetic data were $^1$H NMR (400 MHz, $CDCl_3$) $\delta$ 6.97 - 6.86 (m, 1H), 5.98 - 5.90 (m, 1H), 4.22 (s, 2H), 1.95 (s, 3H), 1.49 (s, 9H); the resulting solid was dissolved in 10 mL of dichloromethane, 2 mL of trifluoroacetic acid was added, reaction was carried out at room temperature for 2 hours, and the oily substance concentrated in vacuo was subjected to column chromatography to give Compound I-6 in 92% yield and nuclear magnetic data $^1$H NMR (400 MHz, DMSO-$d_6$) $\delta$ 12.94 (s, 1H), 7.32 - 6.97 (m, 1H), 6.07 (m, 1H), 4.27 (d, $J$ = 3.8 Hz, 2H), 1.85 (s, 3H);
Dissolving 2 mmol of compound I-6 in 5mL of dichloromethane, adding 3 mmol (7-azobenzotriazole) -N, N, N ', N' -tetramethylurea Hexafluorophosphate (HATU) and 5 mmol of triethylamine, stirring for 15 minutes at room temperature, adding 2.5 mmol of aniline into the reaction solution, reacting for 6 hours at room temperature, adding water into the reaction solution for extraction, washing the organic phase with 1mol/L hydrochloric acid and saturated salt solution respectively, drying with anhydrous sodium sulfate, concentrating in vacuum, and purifying by column chromatography to obtain a compound 768 with the yield of 58%; the nuclear magnetic data were $^1$H NMR (400 MHz, $CDCl_3$) $\delta$ 8.07 (s, 1H), 7.54 (d, $J$ = 7.6 Hz, 2H), 7.34 (t, $J$ = 8.0 Hz, 2H), 7.15 (t, $J$ = 7.6 Hz, 1H), 6.94 - 6.87 (m, 1H), 5.94 (s, 1H), 4.34 (dd, $J$ = 35.6, 14.8 Hz, 2H), 2.17 (s, 3H).

Preparation example 15: preparation of compound 782

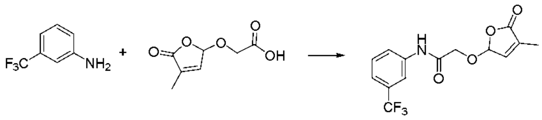

Dissolving 2 mmol of compound I-6 in 5mL of dichloromethane, adding 3 mmol of (7-azobenzotriazole) -N, N, N ', N' -tetramethylurea Hexafluorophosphate (HATU) and 5 mmol of triethylamine, stirring for 15 minutes at room temperature, adding 2.5 mmol of 3-trifluoromethylaniline into the reaction solution, reacting for 5 hours at room temperature, adding water into the reaction solution for extraction, washing the organic phase with 1mol/L hydrochloric acid and saturated salt water respectively, drying with anhydrous sodium sulfate, concentrating in vacuum, and purifying by column chromatography to obtain compound 782, wherein the yield is 65%, and the nuclear magnetic data is as follows: $^1$H NMR (400 MHz, $CDCl_3$) $\delta$ 8.20 (s, 1H), 7.83 (s, 1H), 7.78 (d, $J$ = 8.0 Hz, 1H), 7.46 (t, $J$ = 8.0 Hz, 1H), 7.40 (d, $J$ = 7.6 Hz, 1H), 6.97 - 6.91 (m, 1H), 5.96 (s, 1H), 4.37 (dd, $J$ = 36.4, 15.2 Hz, 2H), 2.02 (s, 3H).

Preparation example 16: preparation of Compound 789

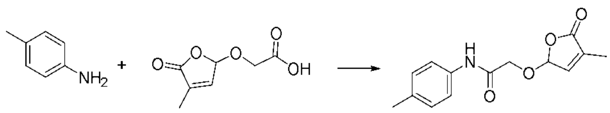

Dissolving 2 mmol of compound I-6 in 5mL of dichloromethane, adding 3 mmol of (7-azobenzotriazole) -N, N, N', N' -tetramethylurea Hexafluorophosphate (HATU) and 5 mmol of triethylamine, stirring for 15 minutes at room temperature, adding 2.5 mmol of 4-methylaniline into the reaction solution, reacting for 5 hours at room temperature, adding water into the reaction solution for extraction, washing the organic phase with 1mol/L hydrochloric acid and saturated salt water respectively, drying with anhydrous sodium sulfate, concentrating in vacuum, and purifying by column chromatography to

obtain compound 782, wherein the yield is 60%, and the nuclear magnetic data is as follows: 1H NMR (400 MHz, CDCl3) δ 8.01 (s, 1H), 7.42 (d, J = 7.6 Hz, 2H), 7.14 (d, J = 7.6 Hz, 2H), 6.91 (s, 1H), 5.93 (s, 1H), 4.33 (dd, J = 36.8, 14.9 Hz, 2H), 2.32 (s, 3H), 2.00 (s, 3H).

The characterization data for some of the compounds of the invention are listed in table 1.

TABLE 1

| Compound number | Nuclear Magnetism (NMR) |
|---|---|
| 1 | $^1$H NMR (400 MHz, DMSO-$d_6$) δ 10.15 (s, 1H), 7.56 (d, *J* = 8.0 Hz, 2H), 7.40 - 7.25 (m, 3H), 7.06 (t, *J* = 7.2 Hz, 1H), 6.43 (s, 1H), 3.56 - 3.43 (m, 2H), 1.82 (s, 3H). |
| 2 | $^1$H NMR (400 MHz, $CDCl_3$) δ 8.41 (s, 1H), 8.28 - 8.20 (m, 1H), 7.18 - 7.07 (m, 3H), 7.02 (t, *J* = 1.6 Hz, 1H), 6.10 (t, *J* = 2.0 Hz, 1H), 3.69 - 3.39 (m, 2H), 1.97 (s, 3H). |
| 3 | $^1$H NMR (400 MHz, $CDCl_3$) δ 8.38 (s, 1H), 7.54 (dt, *J* = 10.7, 2.4 Hz, 1H), 7.32 - 7.28 (m, 1H), 7.22 - 7.19 (m, 1H), 7.03 (t, *J* = 1.6 Hz, 1H), 6.84 (td, *J* = 8.0, 2.4 Hz, 1H), 6.09 (s, 1H), 3.51 (q, *J* = 16.4 Hz, 2H), 1.99 (s, 3H). |
| 4 | $^1$H NMR (400 MHz, $CDCl_3$) δ 8.29 (s, 1H), 7.57 - 7.50 (m, 2H), 7.11 - 6.98 (m, 3H), 6.15 - 6.03 (m, 1H), 3.62 - 3.41 (m, 2H), 1.99 (t, *J* = 2.0 Hz, 3H). |
| 5 | $^1$H NMR (400 MHz, $CDCl_3$) δ 8.80 (s, 1H), 8.32 (d, *J* = 8.0 Hz, 1H), 7.39 (d, *J* = 9.2 Hz, 1H), 7.29 (t, *J* = 8.0 Hz, 1H), 7.08 (t, *J* = 8.4 Hz, 1H), 7.02 (s, 1H), 6.09 (s, 1H), 3.62 (d, *J* = 16.8 Hz, 1H), 3.46 (d, *J* = 16.8 Hz, 1H), 1.96 (s, 3H). |
| 9 | $^1$H NMR (400 MHz, $CDCl_3$) δ 8.34 (s, 1H), 7.85 (s, 1H), 7.45 (d, *J* = 8.4 Hz, 1H), 7.31 - 7.26 (m, 1H), 7.21 (t, *J* = 8.0 Hz, 1H), 7.02 (s, 1H), 6.09 (s, 1H), 3.51 (q, *J* = 16.4 Hz, 2H), 1.99 (s, 3H). |
| 15 | $^1$H NMR (400 MHz, $CDCl_3$) δ 8.72 (s, 1H), 8.50 (s, 1H), 8.03 - 7.90 (m, 2H), 7.51 (t, *J* = 8.0 Hz, 1H), 7.08 - 7.03 (m, 1H), 6.13 (t, *J* = 2.0 Hz, 1H), 3.66 - 3.50 (m, 2H), 2.00 (s, 3H). |
| 16 | $^1$H NMR (400 MHz, $CDCl_3$) δ 8.73 (s, 1H), 8.24 (d, *J* = 9.2 Hz, 2H), 7.78 (d, *J* = 9.2 Hz, 2H), 7.04 (d, *J* = 1.6 Hz, 1H), 6.10 (t, *J* = 2.0 Hz, 1H), 3.56 (d, *J* = 3.2 Hz, 2H), 2.01 (s, 3H). |
| 17 | $^1$H NMR (400 MHz, $CDCl_3$) δ 8.51 (s, 1H), 7.92 (s, 1H), 7.73 (d, *J* = 7.6 Hz, 1H), 7.54 - 7.33 (m, 2H), 7.04 (s, 1H), 6.11 (s, 1H), 3.62 - 3.45 (m, 2H), 2.00 (s, 3H). |
| 18 | $^1$H NMR (400 MHz, $CDCl_3$) δ 8.71 (s, 1H), 8.20 (d, *J* = 8.0 Hz, 1H), 7.73 - 7.54 (m, 2H), 7.29 (d, *J* = 7.6 Hz, 1H), 7.01 (s, 1H), 6.05 (s, 1H), 3.74 - 3.32 (m, 2H), 1.99 (s, 3H). |
| 19 | $^1$H NMR (400 MHz, DMSO-$d_6$) δ 10.52 (s, 1H), 7.77 (d, *J* = 8.4 Hz, 2H), 7.68 (d, *J* = 8.4 Hz, 2H), 7.36 (s, 1H), 6.42 (s, 1H), 3.62 - 3.46 (m, 2H), 1.82 (s, 3H). |
| 22 | $^1$H NMR (400 MHz, $CDCl_3$) δ 8.43 (s, 1H), 7.60 (d, *J* = 8.8 Hz, 2H), 7.20 (d, *J* = 8.4 Hz, 2H), 7.03 (s, 1H), 6.10 (s, 1H), 3.61 - 3.43 (m, 2H), 1.99 (s, 3H). |
| 26 | $^1$H NMR (400 MHz, $CDCl_3$) δ 8.62 (s, 1H), 7.74 - 7.58 (m, 4H), 7.04 (s, 1H), 6.12 (s, 1H), 3.54 (q, *J* = 16.4 Hz, 2H), 1.99 (s, 3H). |
| 27 | $^1$H NMR (400 MHz, $CDCl_3$) δ 8.65 (s, 1H), 8.31 (dd, *J* = 8.0, 1.6Hz, 1H), 7.08 (td, *J* = 7.6, 1.6 Hz, 1H), 7.03 - 7.00 (m, 1H), 6.97 (td, *J* = 7.8, 1.6 Hz, 1H), 6.90 (dd, *J* = 8.0, 1.6 Hz, 1H), 6.11 (t, *J* = 2.0 Hz, 1H), 3.92 (s, 3H), 3.61 (d, *J* = 16.4 Hz, 1H), 3.43 (d, *J* = 16.4 Hz, 1H), 1.95 (s, 3H). |
| 28 | $^1$H NMR (400 MHz, $CDCl_3$) δ 8.27 (s, 1H), 7.33 - 7.25 (m, 2H), 7.23 (d, *J* = 8.0 Hz, 1H), 7.06 (d, *J* = 8.0 Hz, 1H), 7.02 (t, *J* = 1.6 Hz, 1H), 6.19 - 5.99 (m, 1H), 3.82 (s, 3H), 3.64 - 3.37 (m, 2H), 1.98 (t, *J* = 1.6 Hz, 3H). |
| 29 | $^1$H NMR (400 MHz, $CDCl_3$) δ 8.22 (s, 1H), 7.45 (d, *J* = 9.2 Hz, 2H), 7.02 (t, *J* = 1.6 Hz, 1H), 6.92 - 6.83 (m, 2H), 6.10 (t, *J* = 2.0 Hz, 1H), 3.79 (s, 3H), 3.61 - 3.39 (m, 2H), 1.98 (s, 3H). |
| 30 | $^1$H NMR (400 MHz, $CDCl_3$) δ 8.58 (s, 1H), 8.03 (d, *J* = 8.4 Hz, 2H), 7.65 (d, *J* = 8.4 Hz, 2H), 7.03 (s, 1H), 6.11 (s, 1H), 4.36 (q, *J* = 7.2 Hz, 2H), 3.53 (q, *J* = 16.4 Hz, 2H), 1.98 (s, 3H), 1.38 (t, *J* = 7.2 Hz, 3H). |

(continued)

| Compound number | Nuclear Magnetism (NMR) |
|---|---|
| 31 | $^1$H NMR (400 MHz, $CDCl_3$) δ 8.96 (s, 1H), 8.35 (d, *J* = 8.8 Hz, 1H), 7.67 - 7.56 (m, 2H), 7.25 (d, *J* = 9.6 Hz, 1H), 7.13 - 7.05 (m, 1H), 6.09 (t, *J* = 2.0 Hz, 1H), 3.87 - 3.31 (m, 2H), 1.99 (s, 3H). |
| 32 | $^1$H NMR (400 MHz, $CDCl_3$) δ 8.63 (s, 1H), 8.04 - 8.01 (m, 1H), 7.78 - 7.70 (m, 1H), 7.49 - 7.40 (m, 2H), 7.09 - 7.00 (m, 1H), 6.12 (t, *J* = 2.0 Hz, 1H), 3.63 - 3.47 (m, 2H), 1.99 (s, 3H). |
| 34 | $^1$H NMR (400 MHz, $CDCl_3$) δ 8.19 (s, 1H), 7.85 (d, *J* = 8.0 Hz, 1H), 7.22 (dd, *J* = 12.4, 7.2 Hz, 2H), 7.10 (t, *J* = 7.6 Hz, 1H), 7.02 (s, 1H), 6.09 (s, 1H), 3.65 - 3.39 (m, 2H), 2.32 (s, 3H), 1.99 (s, 3H). |
| 35 | $^1$H NMR (400 MHz, $CDCl_3$) δ 8.24 (s, 1H), 7.40 (s, 1H), 7.34 (d, *J* = 8.0 Hz, 1H), 7.22 (t, *J* = 7.6 Hz, 1H), 7.02 (s, 1H), 6.96 (d, *J* = 7.6 Hz, 1H), 6.10 (s, 1H), 3.62 - 3.38 (m, 2H), 2.35 (s, 3H), 1.98 (s, 3H). |
| 36 | $^1$H NMR (400 MHz, $CDCl_3$) δ 8.21 (s, 1H), 7.45 (d, *J* = 8.0 Hz, 2H), 7.17 (d, *J* = 8.0 Hz, 2H), 7.04 (t, *J* = 1.6 Hz, 1H), 6.12 (t, *J* = 2.0 Hz, 1H), 3.63 - 3.40 (m, 2H), 2.35 (s, 3H), 2.00 (t, *J* = 1.6 Hz, 3H). |
| 39 | $^1$H NMR (400 MHz, $CDCl_3$) δ 8.71 (s, 1H), 7.91 (d, *J* = 8.4 Hz, 2H), 7.79 (d, *J* = 8.8Hz, 2H), 7.04 (s, 1H), 6.11 (s, 1H), 3.66 - 3.48 (m, 2H), 3.05 (s, 3H), 2.00 (s, 3H). |
| 45 | $^1$H NMR (400 MHz, $CDCl_3$) δ 8.37 (s, 1H), 7.84 (s, 1H), 7.39 (s, 2H), 7.03 (s, 1H), 6.08 (s, 1H), 3.58 - 3.44 (m, 2H), 2.00 (s, 3H). |
| 46 | $^1$H NMR (400 MHz, DMSO-$d_6$) δ 9.54 (s, 1H), 7.47 (d, *J* = 8.4 Hz, 1H), 7.39 (s, 1H), 7.31 (s,1H), 7.23 (d, *J* = 10.8 Hz, 1H), 6.44 (s, 1H), 3.54 (s, 2H), 2.20 (s, 3H), 1.84 (s, 3H). |
| 58 | $^1$H NMR (400 MHz, $CDCl_3$) δ 8.18 (s, 1H), 7.46 (d, *J* = 8.4 Hz, 2H), 7.18 (d, *J* = 8.4 Hz, 2H), 7.02 (s, 1H), 6.09 (s, 1H), 3.60 - 3.38 (m, 2H), 2.62 (q, *J* = 7.6 Hz, 2H), 1.98 (s, 3H), 1.22 (t, *J* = 7.6 Hz, 3H). |
| 59 | 1H NMR (400 MHz, CDCl3) δ 8.21 (s, 1H), 7.77 - 7.64 (m, 1H), 7.35 - 7.27 (m, 1H), 7.25 - 7.18 (m, 2H), 7.01 (t, J = 1.8 Hz, 1H), 6.10 (t, J = 1.9 Hz, 1H), 3.63 - 3.43 (m, 2H), 3.10 (p, J = 6.9 Hz, 1H), 2.00 (t, J = 1.8 Hz, 3H), 1.27 (dd, J = 6.8, 2.3 Hz, 6H). |
| 60 | 1H NMR (600 MHz, CD3CN) δ 8.73 (s, 1H), 7.51 - 7.38 (m, 2H), 7.26 (t, J = 7.9 Hz, 1H), 7.13 (s, 1H), 7.03 (d, J = 7.7 Hz, 1H), 6.23 (s, 1H), 3.57 - 3.35 (m, 2H), 2.88 (dq, J = 14.0, 7.0 Hz, 1H), 1.89 (s, 3H), 1.24 (d, J = 7.2 Hz, 6H). |
| 61 | 1H NMR (400 MHz, CDCl3) δ 8.25 (s, 1H), 7.46 (d, J = 8.4 Hz, 2H), 7.20 (d, J = 8.4 Hz, 2H), 7.08 - 6.99 (m, 1H), 6.15 - 6.06 (m, 1H), 3.59 - 3.37 (m, 2H), 2.88 (p, J = 6.9 Hz, 1H), 1.98 (s, 3H), 1.23 (d, J = 6.9 Hz, 6H). |
| 62 | 1H NMR (400 MHz, CDCl3) δ 8.23 (s, 1H), 7.86 (dd, J = 8.0, 1.3 Hz, 1H), 7.25 - 7.16 (m, 2H), 7.16 - 7.10 (m, 1H), 7.01 (t, J = 1.7 Hz, 1H), 6.09 (t, J = 1.9 Hz, 1H), 3.66 - 3.40 (m, 2H), 2.61 (dd, J = 8.7, 6.7 Hz, 2H), 1.99 (s, 3H), 1.74 - 1.59 (m, 2H), 0.99 (t, J = 7.3 Hz, 3H). |
| 65 | 1H NMR (400 MHz, CDCl3) δ 11.66 (s, 1H), 8.68 (dd, J = 8.5, 1.2 Hz, 1H), 8.06 (dd, J = 8.0, 1.7 Hz, 1H), 7.57 (ddd, J = 8.8, 7.3, 1.7 Hz, 1H), 7.14 (td, J = 7.7, 1.2 Hz, 1H), 7.07 (t, J = 1.7 Hz, 1H), 6.23 - 6.10 (m, 1H), 3.95 (s, 3H), 3.68 (d, J = 16.3 Hz, 1H), 3.45 (d, J = 16.3 Hz, 1H), 1.96 (t, J = 1.8 Hz, 3H). |
| 67 | $^1$H NMR (400 MHz, $CDCl_3$) δ 8.22 (s, 1H), 7.47 (s, 1H), 7.22 (dd, *J* = 8.0, 2.0 Hz, 1H), 7.17 (d, *J* = 8.0 Hz, 1H), 7.01 (t, *J* = 1.6 Hz, 1H), 6.10 (t, *J* = 2.0 Hz, 1H), 3.59 - 3.34 (m, 2H), 2.88 (dt, *J* = 12.8, 7.6 Hz, 4H), 2.07 (p, *J* = 7.6 Hz, 2H), 1.97 (s, 3H). |
| 68 | $^1$H NMR (400 MHz, $CDCl_3$) δ 8.12 (s, 1H), 7.27 (d, *J* = 2.4 Hz, 1H), 7.22 (dd, *J* = 8.0, 2.4 Hz, 1H), 7.05 - 6.99 (m, 2H), 6.09 (t, *J* = 2.0 Hz, 1H), 3.59 - 3.37 (m, 2H), 2.74 (d, *J* = 13.6 Hz, 4H), 1.97 (t, *J* = 1.6 Hz, 3H), 1.77 (t, *J* = 3.2 Hz, 4H). |
| 70 | $^1$H NMR (400 MHz, $CDCl_3$) δ 8.21 (s, 1H), 7.24 (d, *J* = 2.0 Hz, 1H), 7.03 - 7.01 (m, 1H), 6.86 (dd, *J* = 8.4, 2.1 Hz, 1H), 6.76 (d, *J* = 8.4 Hz, 1H), 6.12 - 6.07 (m, 1H), 5.96 (s, 2H), 3.59 - 3.31 (m, 2H), 1.98 (t, *J* = 2.0 Hz, 3H). |

(continued)

| Compound number | Nuclear Magnetism (NMR) |
|---|---|
| 71 | $^1$H NMR (400 MHz, $CDCl_3$) $\delta$ 8.36 (s, 1H), 7.93 (d, $J$ = 2.0 Hz, 1H), 7.62 (d, $J$ = 2.0 Hz, 1H), 7.46 (d, $J$ = 8.8 Hz, 1H), 7.33 (dd, $J$ = 8.8, 2.0 Hz, 1H), 7.03 (s, 1H), 6.75 (t, $J$ = 1.6 Hz, 1H), 6.12 (s, 1H), 3.62 - 3.43 (m, 2H), 1.99 (s, 3H). |
| 74 | $^1$H NMR (400 MHz, $CDCl_3$) $\delta$ 9.07 (s, 1H), 8.61 (s, 1H), 8.42 (s, 1H), 7.92 (d, $J$ = 8.8 Hz, 1H), 7.71 (d, $J$ = 8.4 Hz, 1H), 7.05 (s, 1H), 6.15 (s, 1H), 3.58 (q, $J$ = 16.4 Hz, 2H), 2.00 (s, 3H). |
| 75 | $^1$H NMR (400 MHz, $CDCl_3$) $\delta$ 8.93 (s, 1H), 8.20 (s, 1H), 8.06 (s, 1H), 7.67 (d, $J$ = 8.4 Hz, 1H), 7.29 (d, $J$ = 10.4 Hz, 1H), 7.03 (s, 1H), 6.16 (s, 1H), 3.73 - 3.42 (m, 2H), 1.96 (s, 3H). |
| 76 | $^1$H NMR (400 MHz, $CDCl_3$) $\delta$ 8.61 (s, 1H), 8.23 (s, 1H), 7.78 (d, $J$ = 8.4 Hz, 3H), 7.52 (d, $J$ = 10.4 Hz, 1H), 7.43 (dt, $J$ = 16.0, 7.2 Hz, 2H), 7.02 (s, 1H), 6.13 (s, 1H), 3.67 - 3.47 (m, 2H), 1.96 (s, 3H). |
| 77 | $^1$H NMR (400 MHz, $CDCl_3$) $\delta$ 8.28 (s, 1H), 7.55 (d, $J$ = 8.0 Hz, 2H), 7.34 (t, $J$ = 8.0 Hz, 2H), 7.14 (t, $J$ = 7.6 Hz, 1H), 6.02 (s, 2H), 3.56 - 3.39 (m, 2H), 2.19 (s, 3H). |
| 153 | $^1$H NMR (400 MHz, $CDCl_3$) $\delta$ 8.30 (s, 1H), 7.56 (d, $J$ = 7.6 Hz, 2H), 7.35 (t, $J$ = 8.0 Hz, 2H), 7.14 (t, $J$ = 7.6 Hz, 1H), 5.91 (s, 1H), 3.44 (s, 2H), 2.07 (s, 3H), 1.88 (s, 3H). |
| 230 | $^1$H NMR (400 MHz, $CDCl_3$) $\delta$ 8.40 (s, 1H), 7.53 (d, $J$ = 8.0 Hz, 2H), 7.35 (t, $J$ = 8.0 Hz, 2H), 7.21 (s, 1H), 7.15 (t, $J$ = 7.6 Hz, 1H), 4.06 (dt, $J$ = 4.0, 2.0 Hz, 1H), 3.40 (d, $J$ = 3.2 Hz, 2H), 2.91 (dd, $J$ = 19.2, 6.4 Hz, 1H), 2.41 (dd, $J$ = 19.2, 1.6 Hz, 1H), 1.78 (s, 3H). |
| 384 | $^1$H NMR (400 MHz, $CDCl_3$) $\delta$ 7.45 (t, $J$ = 7.6 Hz, 2H), 7.39 (d, $J$ = 7.2 Hz, 1H), 7.22 (d, $J$ = 7.6 Hz, 2H), 7.00 (s, 1H), 6.28 (s, 1H), 3.38 (d, $J$ = 15.2 Hz, 1H), 3.30 (s, 3H), 3.20 (d, $J$ = 15.2 Hz, 1H), 1.93 (s, 3H). |
| 461 | $^1$H NMR (400 MHz, $CDCl_3$) $\delta$ 7.44 (t, $J$ = 7.2 Hz, 2H), 7.39 (d, $J$ = 7.2 Hz, 1H), 7.20 - 7.16 (m, 2H), 7.00 (s, 1H), 6.25 (s, 1H), 3.76 (q, $J$ = 7.2 Hz, 2H), 3.32 (d, $J$ = 15.2 Hz, 1H), 3.13 (d, $J$ = 15.2 Hz, 1H), 1.92 (s, 3H), 1.13 (t, $J$ = 7.2 Hz, 3H). |
| 538 | $^1$H NMR (400 MHz, DMSO-$d_6$) $\delta$ 10.48 (d, $J$ = 20.4 Hz, 1H), 7.67 - 7.50 (m, 3H), 7.34 (ddd, $J$ = 8.6, 7.2, 1.6 Hz, 2H), 7.10 (td, $J$ = 7.2, 1.6 Hz, 1H), 6.35 (dt, $J$ = 38.4, 2.0 Hz, 1H), 4.30 - 3.82 (m, 2H), 1.93 (dt, $J$ = 13.2, 1.6 Hz, 3H). |
| 615 | $^1$H NMR (400 MHz, DMSO-$d_6$) $\delta$ 10.55 (s, 1H), 7.59 (d, $J$ = 8.0 Hz, 3H), 7.36 (t, $J$ = 8.0 Hz, 2H), 7.14 (t, $J$ = 7.6 Hz, 1H), 6.63 (s, 1H), 4.66 - 4.25 (m, 2H), 1.95 (s, 3H). |
| 692 | $^1$H NMR (400 MHz, $CDCl_3$) $\delta$ 7.55 (d, $J$ = 8.0 Hz, 2H), 7.49 (s, 1H), 7.32 (t, $J$ = 7.6 Hz, 2H), 7.11 (t, $J$ = 7.2 Hz, 1H), 7.02 - 6.98 (m, 1H), 6.07 - 6.03 (m, 1H), 3.15 - 3.03 (m, 2H), 2.87 - 2.69 (m, 2H), 1.97 (t, $J$ = 2.0 Hz, 3H). |
| 693 | $^1$H NMR (400 MHz, $CDCl_3$) $\delta$ 7.55 (d, $J$ = 8.0 Hz, 2H), 7.40 (s, 1H), 7.32 (t, $J$ = 7.6 Hz, 2H), 7.10 (t, $J$ = 7.2 Hz, 1H), 7.00 - 6.96 (m, 1H), 6.01 - 5.95 (m, 1H), 2.82 (t, $J$ = 6.8 Hz, 2H), 2.63 - 2.42 (m, 2H), 2.10 (t, $J$ = 6.8 Hz, 2H), 1.96 (t, $J$ = 2.0 Hz, 3H). |
| 694 | $^1$H NMR (400 MHz, $CDCl_3$) $\delta$ 8.93 (s, 1H), 8.56 (s, 1H), 7.62 (d, $J$ = 29.6 Hz, 1H), 7.10 (dd, $J$ = 12.7, 4.8 Hz, 1H), 7.02 (s, 1H), 6.10 (s, 1H), 3.59 - 3.41 (m, 2H), 1.98 (s, 3H). |
| 695 | $^1$H NMR (400 MHz, DMSO-$d_6$) $\delta$ 8.58 (s, 1H), 7.33 (t, $J$ = 7.6 Hz, 3H), 7.29 - 7.22 (m, 3H), 6.39 (s, 1H), 4.27 (d, $J$ = 5.6 Hz, 2H), 3.34 (d, $J$ = 12.4 Hz, 2H), 1.81 (s, 3H). |
| 696 | $^1$H NMR (400 MHz, $CDCl_3$) $\delta$ 7.42 - 7.29 (m, 6H), 7.28 - 7.22 (m, 2H), 7.15 (dd, $J$ = 7.6, 3.3 Hz, 2H), 7.03 (dd, $J$ = 3.2, 1.6 Hz,1H), 6.26 (d, $J$ = 2.8 Hz, 1H), 4.64 (qd, $J$ = 14.8, 3.5 Hz, 2H), 4.49 (d, $J$ = 3.6 Hz, 2H), 3.69 (dd, $J$ = 14.8, 3.4 Hz, 1H), 3.55 (dd, $J$ = 14.8, 3.5 Hz, 1H), 1.95 (dt, $J$ = 3.6, 1.6 Hz,3H). |
| 697 | $^1$H NMR (400 MHz, $CDCl_3$) $\delta$ 7.28 (d, $J$ = 18.8 Hz, 10H), 7.03 (t, $J$ = 1.6 Hz, 1H), 6.34 (t, $J$ = 2.0 Hz, 1H), 3.56 (d, $J$ = 15.2 Hz, 1H), 3.35 (d, $J$ = 15.2 Hz, 1H), 1.94 (t, $J$ = 2.0 Hz, 3H). |
| 703 | $^1$H NMR (400 MHz, $CDCl_3$) $\delta$ 7.06 - 6.99 (m, 1H), 6.26 (t, $J$ = 2.0 Hz, 1H), 3.72 - 3.44 (m, 2H), 3.36 - 3.15 (m, 4H), 2.00 - 1.93 (m, 3H), 1.59 (ddd, $J$ = 15.6, 8.0, 3.2 Hz, 4H), 0.92 (ddd, $J$ = 15.2, 7.6, 3.6 Hz, 6H). |

(continued)

| Compound number | Nuclear Magnetism (NMR) |
|---|---|
| 733 | $^1$H NMR (400 MHz, $CDCl_3$) δ 8.59 (s, 1H), 7.85 (s, 1H), 7.46 (s, 1H), 7.01 (s, 1H), 6.11 (s, 1H), 3.85 (s, 3H), 3.63 - 3.30 (m, 2H), 1.94 (s, 3H). |
| 752 | $^1$H NMR (400 MHz, $CDCl_3$) δ 9.54 (s, 1H), 8.73 (s, 1H), 8.43 (s, 1H), 8.34 (s, 1H), 7.05 (t, *J* = 1.6 Hz, 1H), 6.18 - 6.07 (m, 1H), 3.70 - 3.34 (m, 2H), 2.01 (t, *J* = 2.0 Hz, 3H). |
| 755 | $^1$H NMR (400 MHz, $CDCl_3$) δ 6.99 (t, *J* = 1.6 Hz, 1H), 6.62 (s, 1H), 6.13 - 5.98 (m, 1H), 4.36 (p, *J* = 8.0 Hz, 1H), 3.43 - 3.16 (m, 2H), 2.34 (qd, *J* = 7.2, 2.9 Hz, 2H), 1.95 - 1.82 (m, 5H), 1.73 (tdd, *J* = 10.4, 8.0, 2.8 Hz, 3H). |
| 756 | $^1$H NMR (400 MHz, $CDCl_3$) δ 6.98 (s, 1H), 6.36 (s, 1H), 6.02 (t, *J* = 2.0 Hz, 1H), 3.82 - 3.70 (m, 1H), 3.46 - 3.21 (m, 2H), 1.97 (d, *J* = 1.6 Hz, 3H), 1.93 (m, 2H), 1.73 (d, *J* = 13.2 Hz,2H), 1.62 (d, *J* = 13.2 Hz, 1H), 1.37 (q, *J* = 12.4 Hz, 2H), 1.19 (q, *J* = 12.0 Hz, 3H). |
| 757 | $^1$H NMR (400 MHz, $CDCl_3$) δ 7.03 - 6.95 (m, 1H), 6.41 (d, *J* = 8.0 Hz, 1H), 6.02 (p, *J* = 2.0 Hz, 1H), 3.97 - 3.90 (m, 1H), 3.43 - 3.17 (m, 2H), 1.97 (t, *J* = 2.0 Hz, 3H), 1.92 (d, *J* = 11.4 Hz, 2H), 1.75 - 1.42 (m, 10H). |
| 758 | $^1$H NMR (400 MHz, $CDCl_3$) δ 7.82 (dd, *J* = 17.2, 8.0 Hz, 2H), 7.48 (ddd, *J* = 8.0, 7.2, 1.2 Hz, 1H), 7.41 - 7.32 (m, 1H), 6.98 (t, *J* = 1.6 Hz, 1H), 6.10 (t, *J* = 2.0 Hz, 1H), 3.70 (d, *J* = 16.4 Hz, 1H), 3.54 (d, *J* = 16.4 Hz, 1H), 1.95 (s, 3H). |
| 759 | $^1$H NMR (400 MHz, $CDCl_3$) δ 7.55 (d, *J* = 7.6 Hz, 1H), 7.50 - 7.43 (m, 1H), 7.37 - 7.28 (m, 2H), 7.03 (t, *J* = 1.6 Hz, 1H), 6.22 (t, *J* = 2.0 Hz, 1H), 3.84 (s, 2H), 1.96 (t, *J* = 2.0 Hz, 3H). |
| 760 | $^1$H NMR (400 MHz, $CDCl_3$) δ 7.33 - 7.28 (m, 1H), 7.20 (dt, *J* = 7.2, 3.6 Hz, 1H), 7.14 - 7.07 (m, 1H), 6.95 (dt, *J* = 10.4, 1.6 Hz, 1H), 6.64 (d, *J* = 9.6 Hz, 1H), 6.03 (t, *J* = 2.0 Hz, 1H), 5.18 (t, *J* = 7.2 Hz, 1H), 3.55 - 3.24 (m, 2H), 2.90 - 2.69 (m, 2H), 2.15 - 2.02 (m, 1H), 1.97 (dt, *J* = 5.6, 1.8 Hz, 2H), 1.86 (s, 3H), 1.61 (s,2H). |
| 761 | $^1$H NMR (400 MHz, $CDCl_3$) δ 7.37 - 7.19 (m, 5H), 6.87 - 6.83 (m, 1H), 6.45 (s, 1H), 5.93 - 5.88 (m, 1H), 3.57 (td, *J* = 7.2, 5.6 Hz, 2H), 3.43 - 3.15 (m, 2H), 2.87 (t, *J* = 7.2 Hz, 2H), 1.95 (t, *J* = 2.0 Hz, 3H). |
| 762 | $^1$H NMR (400 MHz, $CDCl_3$) δ 7.33 - 7.25 (m, 2H), 7.22 - 7.15 (m, 3H), 7.00 - 6.95 (m, 1H), 6.49 (s, 1H), 6.02 (t, *J* = 2.0 Hz, 1H), 3.42 - 3.15 (m,4H), 2.68 (t, *J* = 7.6 Hz, 2H), 1.97 (t, *J* = 2.0 Hz, 3H), 1.93 - 1.83 (m, 2H). |
| 763 | $^1$H NMR (400 MHz, DMSO-$d_6$) δ 13.00 (s, 1H), 10.16 (s, 1H), 8.12 - 7.94 (m, 2H), 7.49 (d, *J* = 8.8 Hz, 1H), 7.41 - 7.32 (m, 1H), 6.48 - 6.38 (m, 1H), 3.61 - 3.43 (m, 2H), 1.82 (s, 3H). |
| 764 | $^1$H NMR (400 MHz, $CDCl_3$) δ 8.43 (s, 1H), 8.19 (d, *J* = 2.0 Hz, 1H), 7.81 (d, *J* = 8.4 Hz, 1H), 7.46 (d, *J* = 5.2 Hz, 1H), 7.40 (dd, *J* = 8.4, 2.1 Hz, 1H), 7.30 (d, *J* = 5.2 Hz, 1H), 7.05 - 7.01 (m, 1H), 6.12 (t, *J* = 2.0 Hz, 1H), 3.68 - 3.43 (m, 2H), 1.98 (s, 3H). |
| 765 | $^1$H NMR (400 MHz, $CDCl_3$) δ 8.50 (s, 1H), 7.77 (d, *J* = 7.6 Hz, 1H), 7.22 (t, *J* = 8.0 Hz, 1H), 7.16 (d, *J* = 8.4 Hz, 1H), 7.08 (d, *J* = 3.2 Hz, 1H), 7.02 (t, *J* = 1.6 Hz, 1H), 6.53 (d, *J* = 3.2 Hz, 1H), 6.15 - 6.11 (m, 1H), 3.80 (s, 3H), 3.67 - 3.44 (m, 2H), 1.96 (s, 3H). |
| 766 | $^1$H NMR (400 MHz, $CDCl_3$) δ 8.26 (s, 1H), 7.85 (d, *J* = 1.6 Hz, 1H), 7.27 (dd, *J* = 7.2, 3.2 Hz, 2H), 7.05 (d, *J* = 2.8 Hz, 1H), 7.04 - 7.01 (m, 1H), 6.45 (d, *J* = 3.2 Hz, 1H), 6.12 (t, *J* = 2.0 Hz, 1H), 3.77 (s, 3H), 3.62 - 3.41 (m, 2H), 1.97 (s, 3H). |
| 767 | $^1$H NMR (400 MHz, $CDCl_3$) δ 7.04 (s, 1H), 6.26 (s, 1H), 3.69 - 3.44 (m, 2H), 3.38 - 3.18 (m, 4H), 1.95 (t, *J* = 2.0 Hz, 3H), 1.54 (dt, *J* = 15.2, 7.6 Hz, 4H), 1.32 (dq, *J* = 9.6, 7.6 Hz, 4H), 0.94 (dt, *J* = 12.4, 7.2 Hz, 6H). |
| 768 | $^1$H NMR (400 MHz, $CDCl_3$) δ 8.07 (s, 1H), 7.54 (d, *J* = 7.6 Hz, 2H), 7.34 (t, *J* = 8.0 Hz, 2H), 7.15 (t, *J* = 7.6 Hz, 1H), 6.94 - 6.87 (m, 1H), 5.94 (s, 1H), 4.34 (dd, *J* = 35.6, 14.8 Hz, 2H), 2.17 (s, 3H)。 |
| 782 | $^1$H NMR (400 MHz, $CDCl_3$) δ 8.20 (s, 1H), 7.83 (s, 1H), 7.78 (d, *J* = 8.0 Hz, 1H), 7.46 (t, *J* = 8.0 Hz, 1H), 7.40 (d, *J* = 7.6 Hz, 1H), 6.97 - 6.91 (m, 1H), 5.96 (s, 1H), 4.37 (dd, *J* = 36.4, 15.2 Hz, 2H), 2.02 (s, 3H). |

(continued)

| Compound number | Nuclear Magnetism (NMR) |
|---|---|
| 786 | $^1H$ NMR (400 MHz, $CDCl_3$) $\delta$ ppm: 8.00 (s, 1H), 7.44 (d, *J* = 9.2 Hz, 2H), 6.93 - 6.90 (m, 1H), 6.87 (d, *J* = 9.2 Hz, 2H), 5.93 (s, 1H), 4.33 (dd, *J* = 35.2, 14.8 Hz, 2H), 3.79 (s, 3H), 2.00 (s, 3H). |
| 789 | $^1H$ NMR (400 MHz, $CDCl_3$) δ 8.01 (s, 1H), 7.42 (d, *J* = 7.6 Hz, 2H), 7.14 (d, *J* = 7.6 Hz, 2H), 6.91 (s, 1H), 5.93 (s, 1H), 4.33 (dd, *J* = 36.8, 14.9 Hz, 2H), 2.32 (s, 3H), 2.00 (s, 3H). |
| 873 | $^1H$ NMR (400 MHz, DMSO) δ 10.09 (s, 1H), 8.76 (s, 1H), 7.56 (d, *J* = 7.7 Hz, 2H), 7.30 (t, *J* = 7.9 Hz, 2H), 7.05 (t, *J* = 7.4 Hz, 1H), 6.77 (s, 1H), 5.43 (s, 1H), 3.43 - 3.28 (m, 2H), 1.72 (s, 3H). |
| 874 | $^1H$ NMR (400 MHz, $CDCl_3$) δ 11.66 (s, 1H), 8.68 (dd, *J* = 8.5, 1.2 Hz, 1H), 8.06 (dd, *J* = 8.0, 1.7 Hz, 1H), 7.57 (ddd, *J* = 8.8, 7.3, 1.7 Hz, 1H), 7.14 (td, *J* = 7.7, 1.2 Hz, 1H), 7.07 (t, *J* = 1.7 Hz, 1H), 6.23 - 6.10 (m, 1H), 3.95 (s, 3H), 3.68 (d, *J* = 16.3 Hz, 1H), 3.45 (d, *J* = 16.3 Hz, 1H), 1.96 (t, *J* = 1.8 Hz, 3H). |
| 875 | $^1H$ NMR (400 MHz, $CDCl_3$) δ 8.53 (s, 1H), 8.13 (t, *J* = 1.9 Hz, 1H), 7.88 (ddd, *J* = 8.1, 2.3, 1.1 Hz, 1H), 7.81 (dt, *J* = 7.9, 1.3 Hz, 1H), 7.42 (t, *J* = 7.9 Hz, 1H), 7.03 (t, *J* = 1.6 Hz, 1H), 6.12 (t, *J* = 1.9 Hz, 1H), 3.91 (s, 3H), 3.65 - 3.43 (m, 2H), 1.98 (t, *J* = 1.8 Hz, 3H). |
| 876 | $^1H$ NMR (600 MHz, DMSO) δ 10.49 (s, 1H), 7.81 (dd, *J* = 127.5, 9.6 Hz, 4H), 7.34 (s, 1H), 6.41 (s, 1H), 3.81 (s, 3H), 3.70 - 3.40 (m, 2H), 1.83 (d, *J* = 26.2 Hz, 3H). |
| 877 | $^1H$ NMR (600 MHz, DMSO) δ 9.50 (s, 1H), 7.60 (d, *J* = 7.9 Hz, 1H), 7.48 - 7.18 (m, 9H), 6.27 (s, 1H), 3.47 - 3.29 (m, 2H), 1.84 (s, 3H). |
| 878 | $^1H$ NMR (600 MHz, $CD_3CN$) δ 8.82 (s, 1H), 7.84 (s, 1H), 7.58 (dd, *J* = 18.0, 7.7 Hz, 3H), 7.46 - 7.31 (m, 5H), 7.09 (s, 1H), 6.20 (s, 1H), 3.56 - 3.35 (m, 2H), 1.85 (s, 3H). |
| 879 | $^1H$ NMR (600 MHz, DMSO) δ 10.28 (s, 1H), 7.66 (dd, *J* = 30.3, 8.3 Hz, 5H), 7.43 (d, *J* = 8.4 Hz, 2H), 7.34 (d, *J* = 22.8 Hz, 2H), 6.44 (s, 1H), 3.53 (q, *J* = 14.8 Hz, 2H), 1.83 (s, 3H). |
| 880 | $^1H$ NMR (600 MHz, $CDCl_3$) δ 8.05 (s, 1H), 7.86 (d, *J* = 8.1 Hz, 1H), 7.29 (q, *J* = 6.9 Hz, 3H), 7.24 - 7.10 (m, 5H), 6.69 (s, 1H), 5.84 (s, 1H), 4.04 (s, 2H), 3.31 (dd, *J* = 92.3, 16.5 Hz, 2H), 1.92 (s, 3H). |
| 881 | $^1H$ NMR (600 MHz, $CDCl_3$) δ 8.44 (s, 1H), 7.39 (s, 2H), 7.30 - 7.10 (m, 6H), 7.00 - 6.91 (m, 2H), 6.07 (s, 1H), 3.93 (s, 2H), 3.56 - 3.30 (m, 2H), 1.92 (s, 3H). |
| 882 | $^1H$ NMR (400 MHz, $CDCl_3$) δ 8.24 (s, 1H), 7.51 - 7.43 (m, 2H), 7.27 (d, *J* = 7.3 Hz, 3H), 7.23 - 7.12 (m, 5H), 7.01 (t, *J* = 1.7 Hz, 1H), 6.08 (p, *J* = 2.0 Hz, 1H), 3.95 (s, 2H), 3.61 - 3.39 (m, 2H), 1.97 (t, *J* = 1.8 Hz, 3H). |
| 883 | $^1H$ NMR (500 MHz, Chloroform-*d*) δ 9.29 (s, 1H), 7.60 - 7.54 (m, 2H), 7.47 - 7.40 (m, 1H), 7.14 (tt, *J* = 6.9, 1.1 Hz, 1H), 6.74 (dq, *J* = 4.3, 1.4 Hz, 1H), 5.19 (dq, *J* = 4.4, 1.1 Hz, 1H), 3.63 (d, *J* = 16.1 Hz, 1H), 3.51 (d, *J* = 16.1 Hz, 1H), 1.91 (d, *J* = 2.5 Hz, 3H). |

Example 1

Germination promoting activity test of *Phelipanche aegyptiaca* seeds

Step 1: weighing a compound, dissolving the compound in a dimethyl sulfoxide solution to prepare a mother solution with the concentration of 10 mM, and diluting the mother solution with water to the required concentration when in use; and

*Phelipanche aegyptiaca* seeds were first immersed in a 1 wt% sodium hypochlorite solution for 1 min, then immersed in 75 vol% ethanol for 1 min, washed 3 times with sterile water, and air-dried in a clean bench for later use;

Step 2: taking a culture dish with the diameter of 9 cm, placing 2 layers of filter paper at the bottom of the culture dish, adding 5mL of sterile water to moisten the culture dish, uniformly spreading glass fiber filter paper (Whatman GF/A) with the diameter of 9 mm on the filter paper, and uniformly spraying the disinfected seed of the oroxylum indicum on the glass fiber filter paper, wherein each piece of filter paper has about 40 grains; sealing the culture dish by a Parafilm sealing film and placing the culture dish in an incubator at 30 °C for pre-culture for 7 days;

Step 3: taking out the glass fiber filter paper with the seed of the *Phelipanche aegyptiaca*, sucking the surface

moisture of the seed with the filter paper, placing the seed in a 24-hole plate, adding 100μL of compound solution containing 1 vol% dimethyl sulfoxide and having the concentration of $10^{-7}$mol/L into the hole, sealing the hole with a Parafilm sealing film, placing the hole in an incubator at 30 °C for 3 days, and then counting the germination rate; the blank was 1 vol% aqueous dimethyl sulfoxide. Each group of experiments was repeated 3 times, the final data was the average of 3 experiments, and the germination promotion rate was calculated according to the following formula:

$$\text{germination rate} = \text{number of germinated seeds/total seeds} * 100\%$$

The germination promotion rates of the compounds were graded by the following grading method:
A : 90-100%; B : 80-89%; C : 70-79%; D: 60-69; E : 50-59 %; F : 30-49 %; G : 29% or less.

The data of the results are shown in Table 2.

TABLE 2

| Compound number | Percentage of germination promotion of *Phelipanche aegyptiaca* seeds /% | Compound number | Percentage of germination promotion of *Phelipanche aegyptiaca* seeds /% | Compound number | Percentage of germination promotion of *Phelipanche aegyptiaca* seeds /% |
|---|---|---|---|---|---|
| 1 | C | 45 | C | 696 | B |
| 2 | A | 46 | B | 697 | A |
| 3 | E | 58 | D | 703 | B |
| 4 | A | 67 | B | 733 | E |
| 5 | D | 68 | E | 745 | E |
| 9 | B | 70 | E | 752 | E |
| 15 | E | 71 | B | 755 | E |
| 16 | E | 72 | E | 756 | A |
| 17 | C | 74 | B | 757 | C |
| 18 | E | 75 | C | 758 | D |
| 19 | C | 786 | F | 759 | E |
| 22 | E | 76 | E | 760 | A |
| 26 | E | 77 | E | 761 | D |
| 27 | C | 153 | E | 762 | D |
| 28 | B | 230 | E | 763 | E |
| 29 | B | 384 | B | 764 | B |
| 30 | E | 461 | B | 765 | E |
| 31 | E | 538 | C | 766 | E |
| 32 | E | 615 | B | 767 | B |
| 34 | A | 692 | E | 768 | F |
| 35 | B | 693 | E | 789 | F |
| 36 | A | 694 | B | CK | G |
| 39 | D | 695 | B | 59 | A |
| 60 | B | 61 | C | 62 | A |
| 65 | C | 873 | C | 874 | C |
| 875 | F | 876 | F | 877 | B |
| 878 | E | 879 | E | 880 | A |

(continued)

| Compound number | Percentage of germination promotion of *Phelipanche aegyptiaca* seeds /% | Compound number | Percentage of germination promotion of *Phelipanche aegyptiaca* seeds /% | Compound number | Percentage of germination promotion of *Phelipanche aegyptiaca* seeds /% |
|---|---|---|---|---|---|
| 881 | D | 882 | D | 883 | C |

As can be seen from the data in the table 2, the compound disclosed by the invention has a good promotion effect on germination of the seed of the *Phelipanche aegyptiaca* seeds, and the compound disclosed by the invention can effectively promote the germination of the seed of the root parasitic weed and can be well applied to the field control of the root parasitic weed.

The invention exemplarily provides seed germination diagrams of the blank control and the *Phelipanche aegyptiaca* seeds treated by the compound 1, as shown in FIG. 1 and FIG. 2, it is obvious from the diagrams that the germination rate of the *Phelipanche aegyptiaca* seeds treated by the compound provided by the invention is obviously better than that of the blank control group.

Example 2

Wheat seed germination experiment

Step 1: weighing a compound, dissolving the compound in a dimethyl sulfoxide solution to prepare a mother solution with the concentration of 1000ppm, and diluting the mother solution with water to the required concentration when in use; and

soaking wheat seeds in a 1 wt% sodium hypochlorite solution for 2 min, soaking the wheat seeds in 75 vol % ethanol for 2 min, washing the wheat seeds with sterile water for 3 times, and airing the wheat seeds in an ultra-clean bench for later use;

Step 2: taking a culture dish with the diameter of 9 cm, placing 2 layers of filter paper at the bottom of the culture dish, the sterilized wheat seeds were evenly sprinkled on the filter paper, with about 30 seeds per filter paper. 5 ml of an aqueous solution containing 20 vol% PEG6000 and a compound solution with a concentration of 1000 ppm in 0.1 vol% dimethyl sulfoxide was added. sealing the culture dish by a Parafilm sealing film, placing the culture dish in an incubator at the temperature of 25 °C, carrying out constant temperature culture on the culture dish, counting the germination rate of the wheat seeds at the fifth day, and taking the length of the seeds when the length of buds reaches the length of the seeds or the length of roots reaches the length of the seeds as a germination standard; the blank control was an aqueous solution of 0.1 vol% dimethyl sulfoxide. Each experiment was repeated three times, and the final data was the average of the three experiments. The germination promotion rate was calculated according to the following formula:

$$\text{germination rate} = \text{number of germinated seeds/total seeds } 100\%$$

The data of the results are shown in Table 3.

TABLE 3

| Compound number | Germination rate of wheat seeds% | Compound number | Germination rate of wheat seeds% |
|---|---|---|---|
| CK | 35.6 | 230 | 41.1 |
| 1 | 48.9 | 538 | 46.7 |
| 2 | 41.1 | 615 | 44.4 |
| 27 | 63.3 | 696 | 58.9 |
| 74 | 43.3 | 768 | 63.3 |

As can be seen from the data in Table 3, the compound of the invention has a very good promoting effect on the germination of wheat seeds, which indicates that the compound of the invention can effectively promote the germination of

wheat seeds.

The present invention exemplarily provides seed germination graphs of wheat seeds treated by the blank control (FIG. 3), the compound 1 (FIG. 4) and the compound 768 (FIG. 5), and it is obvious from the graphs that the germination rate of the wheat seeds treated by the compound provided by the present invention is obviously better than that of the blank control group.

Example 3

Yield test for crops

Step 1: weighing the compound of the invention, Brassinolide, Pydiflumetofen and Pyraclostrobin in dimethyl sulfoxide solution, dissolving and preparing into mother solution with the concentration of 10 mM, and diluting with water to the required concentration when in use;

Step 2: when peanuts in a field grow to a flowering stage, corns grow to trumpet, rice grows to a booting stage, and when wheat grows to the heading stage, a compound solution containing 1 vol % dimethyl sulfoxide and having a concentration of $1 \times 10^{-5}$mol/L, a compound solution containing 1 vol % dimethyl sulfoxide and having a concentration of $10 \times 10^{-5}$mol/L, a compound solution containing 1 vol % dimethyl sulfoxide and having a concentration of $100 \times 10^{-5}$mol/L, a compound containing $1 \times 10^{-5}$mol/L of dimethyl sulfoxide and having a concentration of $7 \times 10^{-7}$mol/L of Brassinolide, a Pyraclostrobin solution containing $20 \times 10^{-5}$mol/L of dimethyl sulfoxide and having a volume ratio of 1:0.1:5, a Brassinolide solution containing $7 \times 10^{-7}$mol/L of dimethyl sulfoxide and a Pyraclostrobin solution containing $20 \times 10^{-5}$mol/L of dimethyl sulfoxide (the volume ratio is 0.1:5) (see in particular table 3). The control group was sprayed with a solution containing 1 vol% dimethyl sulfoxide. Each field plot area was 25 $m^2$. In order to reduce errors during harvesting, the crops in each cell are harvested manually. After the grains are collected and dried in the sun, the weight of the grains in each processing cell is weighed. The grain yield increase rate of each district is calculated according to the following formula:

yield increase (%) = [ (weight of grain harvested from treatment group-weight of grain harvested from control group)/ weight of grain harvested from control group ] × 100%;

The data of the results are shown in Table 4.

TABLE 4

| Compound number | Yield increase% | | | |
|---|---|---|---|---|
| | wheat | Peanut | Corn | Rice |
| compound 1 | 6.6-11.4 | 7.6-14.3 | 9.1-12.6 | 6.8-9.7 |
| compound 1($1\times10^{-5}$mol/L) | 6.6 | 7.6 | 9.1 | 6.8 |
| compound 1($10\times10^{-5}$mol/L) | 8.3 | 10.4 | 10.2 | 8.0 |
| compound 1($100\times10^{-5}$mol/L) | 11.4 | 14.3 | 12.6 | 9.7 |
| 1+ Brassinolide +Pyraclostrobin | 18.3 | 20.1 | 16.8 | 15.4 |
| Brassinolide +Pyraclostrobin | 15.2 | 17.3 | 13.4 | 12.8 |
| compound 768 | 5.9 | 6.3 | 7.1 | 5.7 |

As can be seen from the data in Table 4, the compound of the invention has excellent yield-increasing effect on wheat, peanut, corn and rice; when the compound is compounded with other compounds for use, the yield is increased better than that of single application; the compound of the invention can effectively promote the yield increase of crops.

The present invention exemplarily provides graphs of FIG. 6 and FIG. 7 as wheat blank control and drought resistance effect of wheat after applying compound 1 of $1\times10^{-6}$ mol/L. Specifically, FIG. 6 is a diagram of drought-resistant effect of wheat treated by $1\times10^{-6}$ mol/L of compound 1; FIG. 7 is a diagram of drought-resistant effect of wheat treated by a blank Control (CK) according to the present invention. As can be seen from the comparison between the FIG. 6 and the FIG. 7, the compound provided by the invention can obviously improve the drought tolerance effect of wheat, and further proves that the compound provided by the invention is beneficial to abiotic stress management of plants.

In addition, the invention exemplarily provides a blank control group of a corn field and a crop yield increase map of applying $4\times10^{-5}$mol/L of the compound 1, as shown in FIG. 8, it is obvious that the corn crops in the corn field after being treated by the compound 1 provided by the invention grow more exuberantly, while the control group shows a wilting state

of drooping. The compound of the invention can effectively promote the yield increase of crops.

Furthermore, under greenhouse experimental conditions, the compounds provided in the present invention can achieve a yield increase rate of up to 40% or more in model plants such as *Arabidopsis thaliana* and rice. Therefore, it can be predicted that the compounds provided in the present invention all possess good yield-increasing effects.

Furthermore, the data provided in Table 4 of the present invention are results obtained from field trials. Influenced by factors such as pests, diseases, and climate, the yield increase rates provided in Table 4 are below 40%. However, the compounds provided in the present invention exhibit a relatively significant yield-increasing effect on crops and possess notable commercial development potential.

Example 4

Root growth promotion experiment of *Arabidopsis thaliana*

Plant material: *Arabidopsis thaliana* (Columbia wild type, Col),
sterile culture of *Arabidopsis thaliana* in Petri dishes

(1) Sterilization of seeds of uniform size were picked and filled into 1.5ml EP tubes and sterilized by adding 1ml of 75 vol% ethanol upside down for 10 minutes. After centrifugation for 30 seconds in a rapid centrifuge, the ethanol was aspirated out with a pipette. Then, 1ml of 10% sodium hypochlorite solution was added to the EP tube, and after being turned upside down for 1 minute, the mixture was centrifuged by a quick centrifuge for 30 seconds, and then the sodium hypochlorite solution was aspirated by a pipette. The seeds were washed 10 times 30s each time with sterile deionized water in a sterile super clean bench.

(2) 1/2 preparation of MS solid culture medium, weighing MS powder, 4-morpholine ethanesulfonic acid and sucrose according to the proportion in the following table 5, adding deionized water to a constant volume of 1L, measuring pH with a pH meter, and adjusting pH to about 5.8 with concentrated sodium hydroxide. Then, the mixture is fully dissolved by a stirrer and then is subpackaged into a 250ml conical flask, and 0.8 % agar powder is added. Then, the mixture was sterilized in an autoclave at 120 °C for 20 minutes.

TABLE 5

| Reagent | Amount of addition |
|---|---|
| MS powder | 2.165g |
| Sucrose | 10g |
| 4-morpholine ethanesulfonic acid | 1g |
| Agar powder | 8g |
| Deionized water | 1L |

(3) Sterile culture of *Arabidopsis thaliana* in petri dishes: in a sterile super-clean bench, the test compound is prepared to be 1mM by DMSO, the temperature of the culture medium after sterilization is reduced to about 60 °C, and 0.1 vol % DMSO (blank control) or 0.1 vol% DMSO containing 1 μM of the compound is respectively added; after mixing, the mixture was poured into a petri dish, after it was cooled and solidified, the sterilized seeds were sucked up with a 200ml pipette and spotted one by one on the surface of the solid medium in the petri dish, which was then covered with a petri dish lid and sealed with a sealing film (Parafilm). The sealed petri dish was placed upside down in a refrigerator and vernalized at 4 °C in the dark for three days. After that, the culture dish was vertically placed in a phytotron, and cultured under 22 °C and 120 phototons μmol $m^2$s-1 illumination conditions. The photoperiod and dark period were 16 hours and 8 hours, respectively. Ten days after the culture, photographs were taken and the length of the main root was measured using Image J.

$$\text{Root length growth rate (\%)} = [\ (\text{treated root length-control root length})/\text{control root length}\ ]\ 100\%$$

The data of the results are shown in Table 6

TABLE 6

| Compound | Root length increase compared to blank control (%) |
|---|---|
| Compound 1 (1μM) | 37 |

(continued)

| Compound | Root length increase compared to blank control (%) |
|---|---|
| Compound 768 (1μM) | 33 |
| Compound 880 (1μM) | 312 |

It can be seen from the data in Table 6 that the compounds of the present invention have a significant promoting effect on the root length of *Arabidopsis thaliana*.

The present invention exemplarily provides images of *Arabidopsis thaliana* root growth after treatment with a blank control (Fig. 9) and Compound 880 (Fig. 10). It can be clearly seen from the figures that the root length of *Arabidopsis thaliana* treated with the compound provided by the present invention is significantly better than that of the blank control group.

Example 5

Experiment for influence of compound on soil microbial population and soil fertility

The same field is selected and divided into two areas, wherein the area I is an experimental group: dissolving the compound 1 in 5 vol% DMSO aqueous solution at a dose of 10 g/mu, spraying the compound on the ground surface once every two months, and spraying the compound 3 times in total; zone two is control group: a 5 vol% DMSO aqueous solution was sprayed onto the surface once every two months for a total of 3 times. After the first spraying, respectively taking about 200 g of soil samples from plots of an experimental group and a control group after one year, placing the soil samples in sterile sealed sample bags, and storing the samples in a refrigerator at 4 °C; placing another 10g of the mixture in a sterile test tube, and quickly freezing the mixture by liquid nitrogen; taken back to the laboratory and put into a -80 °C refrigerator for standby.

Soil fertility determination: total Nitrogen (TN), Ammoniacal nitrogen (NH 4+ -N), Nitrate nitrogen (-N), Nitrite nitrogen (2-N), Total Phosphorus (TP), Rapidly Available Phosphorus (AP), Total Carbon (TC), and Total Organic Carbon (TOC) and the like were measured according to "soil agrochemical analysis"(《土壤农化分析》).

The results are shown in Table 7

TABLE 7

| | Control group | Experimental group |
|---|---|---|
| Total nitrogen | 1.35±0.15 g/kg | 1.63±0.34 g/kg |
| Ammoniacal nitrogen | 15.32±1.82 mg/kg | 12.67±2.86 mg/kg |
| Nitrate nitrogen | 3.51±0.72 mg/kg | 3.2±0.8 mg/kg |
| Nitrite nitrogen | 0.34±0.14 mg/kg | 0.28±0.13 mg/kg |
| Total phosphorus | 0.76±0.26 g/kg | 0.98±0.37 g/kg |
| Rapidly Available Phosphorus | 0.6±0.07 g/kg | 0.67±0.12 g/kg |
| Total carbon | 12.83±2.11 g/kg | 14.92±1.14 g/kg |
| Total organic carbon | 6.14±1.21 g/kg | 7.58±1.26 g/kg |

Soil microorganism detection: soil leachable microorganisms were stained by DAPI and counted using a flow cytometer (FACS verse, BD) at 360 nm excitation wavelength and 460 nm emission wavelength.

And analyzing the community composition of the soil microorganism sample by adopting a high-throughput sequencing technology (Illumina Hiseq), and constructing a PCR amplification library after DNA extraction. The sequenced sequences were QIIME screened and aligned with the SILVA132 database with 97% as the lowest support threshold. The analysis of the microbial community was performed based on a Biolog-ECO test plate (ECO MicroPlate, USA).

The results are shown in Table 8

TABLE 8

| | Blank group | Experimental group |
|---|---|---|
| Proteobacteria | 22.35% | 31.05% |
| Acidobacteria | 21.66% | 24.57% |
| Chloroflexi | 19.35% | 11.25% |
| Actinobacteria | 15.11% | 8.85% |
| Firmicutes | 2.14% | 4.34% |
| Bacteroidetes | 2.98% | 3.21% |
| Nitrospirae | 3.22% | 4.22% |
| Rokubacteria | 2.79% | 3.83% |
| Gemmatimonadetes | 2.83% | 2.75% |
| Latescibacteria | 2.52% | 1.68% |
| Cyanobacteria | 1.12% | 0.97% |
| Nitrospinae | 0.72% | 0.80% |
| Patescibacteria | 0.82% | 0.40% |
| Spirochaetes | 1.38% | 0.98% |
| norank-Bacteria | 1.01% | 1.10% |

It can be seen from tables 7 and 8 that the microbial flora composition in the application group is changed compared with that in the blank control soil, and the contents of total carbon, total nitrogen, total phosphorus and total organic carbon are all improved to a certain extent. The compound of the invention is shown to be capable of changing microbial populations and improving soil fertility.

Example 6

Growth promotion experiment of wheat

Test method

Wheat (west nong 529), dry seeds are soaked in test liquid with the concentration of 1ppm (blank group is 5% DMSO water solution) for 13h, then washed with clear water for 3 times, and then washed for 3 times, and then spread out in a room to be dried to remove water. Adding a proper amount of vermiculite into a 180ml disposable paper cup, pouring a proper amount of clear water on the vermiculite, sowing, and then starting to culture, wherein each group is repeated for 10 times. After 16DAT is cultured, cleaning is carried out to remove impurities on the root of the wheat plant, water on the root is absorbed by absorbent paper, the plant height (the length from the stem base to the tail end of the longest leaf), the root length (the length from the stem base to the tail end of the longest fibrous root), the total fresh weight of the plant, the fresh weight of the root, the fresh weight of the overground part and other indexes are investigated, and DPS19.5 software is adopted to carry out variance analysis (complete random design single factor test statistical analysis, DUNCAN's new multiple comparison method).

The results of the experiment are shown in Table 9

TABLE 9

| | Plant height(mm) | Root length(mm) | Fresh weight on ground(g) | Fresh weight underground(g) | Total fresh weight (g) |
|---|---|---|---|---|---|
| Blank | 151.7±2.8 | 260.3±3.3 | 1.574±0.064 | 1.697±0.097 | 3.271±0.084 |
| Compound 1 | 166.0±1.7 | 283.9±2.5 | 1.837±0.088 | 2.683±0.054 | 4.520±0.065 |

As can be seen from the data in Table 9, the compounds of the present invention have a significant effect on the growth of wheat.

The invention exemplarily provides a wheat growth chart after blank control (FIG. 11) and compound 1 (FIG. 12), and as is obvious from the figure, the wheat growth vigor after the compound treatment provided by the invention is obviously better than that of the blank control group.

The preferred embodiments of the present invention have been described above in detail, but the present invention is not limited thereto. Within the scope of the technical idea of the invention, many simple modifications can be made to the technical solution of the invention, including various technical features being combined in any other suitable way, and these simple modifications and combinations should also be regarded as the disclosure of the invention, and all fall within the scope of the invention.

## Claims

1. An amide compound, which is **characterized by** having a structure shown in a formula (I),

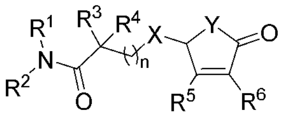 formula (I),

wherein, in formula (I),

$R^1$ and $R^2$ are each independently selected from H, substituted or unsubstituted $C_{1-12}$ alkyl, substituted or unsubstituted $C_{3-12}$ cycloalkyl,

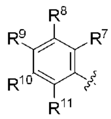 ,

, substituted or unsubstituted $C_{1-8}$ heterocycle containing at least one heteroatom selected from N, O and S, wherein $R^7$, $R^8$, $R^9$, $R^{10}$ and $R^{11}$ are each independently selected from H, halogen, nitro, cyano, phenyl, benzyl, $C_{1-6}$ alkyl, $C_{3-6}$ cycloalkyl, $C_{1-6}$ alkyl substituted by 1-9 halogens, $C_{1-6}$ alkoxy, $C_{1-6}$ alkoxy substituted by 1-9 halogens, $C_{1-6}$ alkylthio, $C_{1-6}$ alkylthio substituted by 1-9 halogens, -COO-$C_{1-6}$ alkyl and -$S(O)_2$-$C_{1-6}$ alkyl, or any two adjacent of $R^7$, $R^8$, $R^9$, $R^{10}$ and $R^{11}$ are cyclized together to form a 3-8 membered ring structure;and the substituents optionally present on $R^1$ and $R^2$ are each independently selected from the group consisting of $C_{1-12}$ alkyl, phenyl;

$R^3$, $R^4$, $R^5$ and $R^6$ are each independently selected from H, $C_{1-12}$ alkyl and $C_{3-12}$ cycloalkyl;

n is 0, 1, 2, 3 or 4;

X is O, S, -S(O)- or -$S(O)_2$-; Y is O, NH, S, $CH_2$ or -$N(CH_3)$-.

2. The compound of claim 1, wherein, in formula (I), X and Y are not both O.

3. The compound according to claim 2, wherein, in formula (I),

$R^1$ and $R^2$ are each independently selected from H,

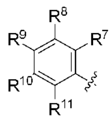 ,

$C_{1-6}$ alkyl, -$(CH_2)_z$-phenyl, an unsaturated five-membered ring group containing a heteroatom selected from class B, a saturated four-membered ring group containing a heteroatom of class B, an unsaturated six-membered ring group containing a heteroatom of class B, a saturated $C_{4-8}$ cycloalkyl, benzothiazolyl, benzoxazolyl, benzopyrazolyl, phenylpropylthiophenyl, N-methyl-benzopyrolyl,

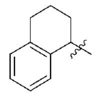

;

z is an integer from 1 to 6; each of said heteroatom selected from class B is each independently at least one selected from O, S, N; and optionally, the heteroatom selected from class B contains $C_{1-3}$ alkyl as a substituent; wherein $R^7$, $R^8$, $R^9$, $R^{10}$ and $R^{11}$ are independently selected from H, halogen, nitro, cyano, phenyl, benzyl, $C_{1-6}$ alkyl, $C_{3-6}$ cycloalkyl, $C_{1-6}$ alkyl substituted by 1-6 halogens, $C1_{-6}$ alkoxy, $C_{1-6}$ alkoxy substituted by 1-6 halogens, $C_{1-6}$ alkylthio, $C_{1-6}$ alkylthio substituted by 1-3 halogens, -COO-$C_{1-6}$ alkyl and -$S(O)_2$-$C_{1-6}$ alkyl, or any two adjacent of $R^7$, $R^8$ and $R^9$ are cyclized together to form a $C_{3-6}$ saturated cycloalkane structure with or without O atom; or any two adjacent of $R^7$, $R^8$ and $R^9$ are cyclized together to form an unsaturated naphthenic structure of $C_{3-6}$ containing or not containing class A heteroatoms, wherein the class A heteroatoms are selected from at least one of O, S, N, and optionally, $C_{1-3}$ alkyl is contained on the class A heteroatoms as a substituent;
$R^3$, $R^4$, $R^5$ and $R^6$ are each independently selected from H, $C_{1-6}$ alkyl and $C_{3-6}$ cycloalkyl;
n is 0, 1, 2 or 3;
X is S, -S(O)- or -$S(O)_2$-;
Y is O, NH, -$CH_2$- or -$N(CH_3)$-.

**4.** A compound according to claim 2 or 3, wherein, in formula (I),

$R^1$ is

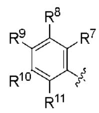

,

wherein, $R^7$, $R^3$, $R^9$, $R^{10}$ and $R^{11}$ are each independently selected from H, halogen, nitro, cyano, phenyl, benzyl, $C_{1-6}$ alkyl, $C_{3-6}$ cycloalkyl, $C_{1-6}$ alkyl substituted by 1-6 halogens, $C_{1-6}$ alkoxy, $C_{1-6}$ alkoxy substituted by 1-6 halogens, $C_{1-6}$ alkylthio, $C_{1-6}$ alkylthio substituted by 1-3 halogens, -COO-$C_{1-6}$ alkyl and-$S(O)_2$-$C_{1-6}$ alkyl, or any two adjacent of $R^7$, $R^8$ and $R^9$ are cyclized together to form a $C_{3-6}$ saturated cycloalkane structure with or without O atom; or any two adjacent of $R^7$, $R^8$ and $R^9$ are cyclized together to form an unsaturated naphthenic structure of $C_{3-6}$ containing or not containing class A heteroatoms, wherein the class A heteroatoms are selected from at least one of O, S, N, and optionally, $C_{1-3}$ alkyl is contained on the class A heteroatoms as a substituent;
$R^2$, $R^5$ and $R^6$ are each independently selected from H, methyl, ethyl, n-propyl, isopropyl, tert-butyl; or $R^2$ is phenyl;
$R^3$ and $R^4$ are both H;
n is 0, 1 or 2;
X is S, -S(O)- or -$S(O)_2$-;
Y is O, NH, -$CH_2$- or -$N(CH_3)$-.

**5.** The compound of claim 4, wherein the compound of formula (I) has the structure of the compound of formula (I-A):

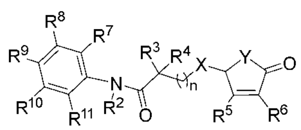

formula (I-A),

wherein, in formula (I-A), the substitution of n, X, Y, $R^2$, $R^3$, $R^4$, $R^5$, $R^6$, $R^7$, $R^3$, $R^9$, $R^{10}$ and $R^{11}$ is as follows:

compound 1: n is 0, X is S, Y is O, $R^6$ is Me, and $R^2$, $R^3$, $R^4$, $R^5$, $R^7$, $R^8$, $R^9$, $R^{10}$ and $R^{11}$ are all H;
compound 2: n is 0, X is S, Y is O, $R^6$ is Me, $R^7$ is F, and $R^2$, $R^3$, $R^4$, $R^5$, $R^8$, $R^9$, $R^{10}$ and $R^{11}$ are all H;
compound 3: n is 0, X is S, Y is O, $R^6$ is Me, $R^8$ is F, and $R^2$, $R^3$, $R^4$, $R^5$, $R^7$, $R^9$, $R^{10}$ and $R^{11}$ are all H;
compound 4: n is 0, X is S, Y is O, $R^6$ is Me, $R^9$ is F, and $R^2$, $R^3$, $R^4$, $R^5$, $R^7$, $R^8$, $R^{10}$ and $R^{11}$ are all H;
compound 5: n is 0, X is S, Y is O, $R^6$ is Me, $R^7$ is Cl, and $R^2$, $R^3$, $R^4$, $R^5$, $R^8$, $R^9$, $R^{10}$ and $R^{11}$ are all H;
compound 6: n is 0, X is S, Y is O, $R^6$ is Me, $R^8$ is Cl, and $R^2$, $R^3$, $R^4$, $R^5$, $R^7$, $R^9$, $R^{10}$ and $R^{11}$ are all H;
compound 7: n is 0, X is S, Y is O, $R^6$ is Me, $R^9$ is Cl, and $R^2$, $R^3$, $R^4$, $R^5$, $R^7$, $R^8$, $R^{10}$ and $R^{11}$ are all H;

compound 8: n is 0, X is S, Y is O, $R^6$ is Me, $R^7$ is Br, and $R^2$, $R^3$, $R^4$, $R^5$, $R^A$, $R^9$, $R^{10}$ and $R^{11}$ are all H;
compound 9: n is 0, X is S, Y is O, $R^6$ is Me, $R^8$ is Br, and $R^2$, $R^3$, $R^4$, $R^5$, $R^7$, $R^9$, $R^{10}$ and $R^{11}$ are all H;
compound 10: n is 0, X is S, Y is O, $R^6$ is Me, $R^9$ is Br, and $R^2$, $R^3$, $R^4$, $R^5$, $R^7$, $R^8$, $R^{10}$ and $R^{11}$ are all H;
compound 11: n is 0, X is S, Y is O, $R^6$ is Me, $R^7$ is I, and $R^2$, $R^3$, $R^4$, $R^5$, $R^8$, $R^9$, $R^{10}$ and $R^{11}$ are all H;
compound 12: n is 0, X is S, Y is O, $R^6$ is Me, $R^8$ is I, and $R^2$, $R^3$, $R^4$, $R^5$, $R^7$, $R^9$, $R^{10}$ and $R^{11}$ are all H;
compound 13: n is 0, X is S, Y is O, $R^6$ is Me, $R^9$ is I, and $R^2$, $R^3$, $R^4$, $R^5$, $R^7$, $R^8$, $R^{10}$ and $R^{11}$ are all H;
compound 14: n is 0, X is S, Y is O, $R^6$ is Me, $R^7$ is $NO_2$, and $R^2$, $R^3$, $R^4$, $R^5$, $R^8$, $R^9$, $R^{10}$ and $R^{11}$ are all H;
compound 15: n is 0, X is S, Y is O, $R^6$ is Me, $R^8$ is $NO_2$, and $R^2$, $R^3$, $R^4$, $R^5$, $R^7$, $R^9$, $R^{10}$ and $R^{11}$ are all H;
compound 16: n is 0, X is S, Y is O, $R^6$ is Me, $R^9$ is $NO_2$, and $R^2$, $R^3$, $R^4$, $R^5$, $R^7$, $R^8$, $R^{10}$ and $R^{11}$ are all H;
compound 17: n is 0, X is S, Y is O, $R^6$ is Me, $R^7$ is $CF_3$, and $R^2$, $R^3$, $R^4$, $R^5$, $R^8$, $R^9$, $R^{10}$ and $R^{11}$ are all H;
compound 18: n is 0, X is S, Y is O, $R^6$ is Me, $R^8$ is $CF_3$, and $R^2$, $R^3$, $R^4$, $R^5$, $R^7$, $R^9$, $R^{10}$ and $R^{11}$ are all H;
compound 19: n is 0, X is S, Y is O, $R^6$ is Me, $R^9$ is $CF_3$, and $R^2$, $R^3$, $R^4$, $R^5$, $R^7$, $R^8$, $R^{10}$ and $R^{11}$ are all H;
compound 20: n is 0, X is S, Y is O, $R^6$ is Me, $R^7$ is $OCF_3$, and $R^2$, $R^3$, $R^4$, $R^5$, $R^8$, $R^9$, $R^{10}$ and $R^{11}$ are all H;
compound 21: n is 0, X is S, Y is O, $R^6$ is Me, $R^8$ is $OCF_3$, and $R^2$, $R^3$, $R^4$, $R^5$, $R^7$, $R^9$, $R^{10}$ and $R^{11}$ are all H;
compound 22: n is 0, X is S, Y is O, $R^6$ is Me, $R^9$ is $OCF_3$, and $R^2$, $R^3$, $R^4$, $R^5$, $R^7$, $R^8$, $R^{10}$ and $R^{11}$ are all H;
compound 23: n is 0, X is S, Y is O, $R^6$ is Me, $R^7$ is $OCHF_2$, and $R^2$, $R^3$, $R^4$, $R^5$, $R^8$, $R^9$, $R^{10}$ and $R^{11}$ are all H;
compound 24: n is 0, X is S, Y is O, $R^6$ is Me, $R^8$ is $OCHF_2$, and $R^2$, $R^3$, $R^4$, $R^5$, $R^7$, $R^9$, $R^{10}$ and $R^{11}$ are all H;
compound 25: n is 0, X is S, Y is O, $R^6$ is Me, $R^9$ is $OCHF_2$, and $R^2$, $R^3$, $R^4$, $R^5$, $R^7$, $R^8$, $R^{10}$ and $R^{11}$ are all H;
compound 26: n is 0, X is S, Y is O, $R^6$ is Me, $R^9$ is $SCF_3$, and $R^2$, $R^3$, $R^4$, $R^5$, $R^7$, $R^8$, $R^{10}$ and $R^{11}$ are all H;
compound 27: n is 0, X is S, Y is O, $R^6$ is Me, $R^7$ is $OCH_3$, and $R^2$, $R^3$, $R^4$, $R^5$, $R^8$, $R^9$, $R^{10}$ and $R^{11}$ are all H;
compound 28: n is 0, X is S, Y is O, $R^6$ is Me, $R^8$ is $OCH_3$, and $R^2$, $R^3$, $R^4$, $R^5$, $R^7$, $R^9$, $R^{10}$ and $R^{11}$ are all H;
compound 29: n is 0, X is S, Y is O, $R^6$ is Me, $R^9$ is $OCH_3$, and $R^2$, $R^3$, $R^4$, $R^5$, $R^7$, $R^8$, $R^{10}$ and $R^{11}$ are all H;
compound 30: n is 0, X is S, Y is O, $R^6$ is Me, $R^9$ is $COOC_2H_5$, and $R^2$, $R^3$, $R^4$, $R^5$, $R^7$, $R^8$, $R^{10}$ and $R^{11}$ are all H;
compound 31: n is 0, X is S, Y is O, $R^6$ is Me, $R^7$ is CN, and $R^2$, $R^3$, $R^4$, $R^5$, $R^8$, $R^9$, $R^{10}$ and $R^{11}$ are all H;
compound 32: n is 0, X is S, Y is O, $R^6$ is Me, $R^8$ is CN, and $R^2$, $R^3$, $R^4$, $R^5$, $R^7$, $R^9$, $R^{10}$ and $R^{11}$ are all H;
compound 33: n is 0, X is S, Y is O, $R^6$ is Me, $R^9$ is CN, and $R^2$, $R^3$, $R^4$, $R^5$, $R^7$, $R^8$, $R^{10}$ and $R^{11}$ are all H;
compound 34: n is 0, X is S, Y is O, $R^6$ and $R^7$ are Me, and $R^2$, $R^3$, $R^4$, $R^5$, $R^8$, $R^9$, $R^{10}$ and $R^{11}$ are all H;
compound 35: n is 0, X is S, Y is O, $R^6$ and $R^8$ are Me, and $R^2$, $R^3$, $R^4$, $R^5$, $R^7$, $R^9$, $R^{10}$ and $R^{11}$ are all H;
compound 36: n is 0, X is S, Y is O, $R^6$ and $R^9$ are Me, and $R^2$, $R^3$, $R^4$, $R^5$, $R^7$, $R^8$, $R^{10}$ and $R^{11}$ are all H;
compound 37: n is 0, X is S, Y is O, $R^6$ is Me, $R^7$ is $CH_3SO_2$, and $R^2$, $R^3$, $R^4$, $R^5$, $R^8$, $R^9$, $R^{10}$ and $R^{11}$ are all H;
compound 38: n is 0, X is S, Y is O, $R^6$ is Me, $R^8$ is $CH_3SO_2$, and $R^2$, $R^3$, $R^4$, $R^5$, $R^7$, $R^9$, $R^{10}$ and $R^{11}$ are all H;
compound 39: n is 0, X is S, Y is O, $R^6$ is Me, $R^9$ is $CH_3SO_2$, and $R^2$, $R^3$, $R^4$, $R^5$, $R^7$, $R^8$, $R^{10}$ and $R^{11}$ are all H;
compound 40: n is 0, X is S, Y is O, $R^6$ is Me, $R^7$ is $C_2H_5SO_2$, and $R^2$, $R^3$, $R^4$, $R^5$, $R^8$, $R^9$, $R^{10}$ and $R^{11}$ are all H;
compound 874: n is 0, X is S, Y is O, $R^6$ is Me, $R^7$ is $CO_2CH_3$, and $R^2$, $R^3$, $R^4$, $R^5$, $R^8$, $R^9$, $R^{10}$ and $R^{11}$ are all H;
compound 875: n is 0, X is S, Y is O, $R^6$ is Me, $R^8$ is $CO_2CH_3$, and $R^2$, $R^3$, $R^4$, $R^5$, $R^7$, $R^9$, $R^{10}$ and $R^{11}$ are all H;
compound 876: n is 0, X is S, Y is O, $R^6$ is Me, $R^9$ is $CO_2CH_3$, and $R^2$, $R^3$, $R^4$, $R^5$, $R^7$, $R^8$, $R^{10}$ and $R^{11}$ are all H;
compound 877: n is 0, X is S, Y is O, $R^6$ is Me, $R^7$ is Ph, and $R^2$, $R^3$, $R^4$, $R^5$, $R^8$, $R^9$, $R^{10}$ and $R^{11}$ are all H;
compound 878: n is 0, X is S, Y is O, $R^6$ is Me, $R^8$ is Ph, and $R^2$, $R^3$, $R^4$, $R^5$, $R^7$, $R^9$, $R^{10}$ and $R^{11}$ are all H;
compound 879: n is 0, X is S, Y is O, $R^6$ is Me, $R^9$ is Ph, and $R^2$, $R^3$, $R^4$, $R^5$, $R^7$, $R^8$, $R^{10}$ and $R^{11}$ are all H;
compound 880: n is 0, X is S, Y is O, $R^6$ is Me, $R^7$ is Bn, and $R^2$, $R^3$, $R^4$, $R^5$, $R^8$, $R^9$, $R^{10}$ and $R^{11}$ are all H;
compound 881: n is 0, X is S, Y is O, $R^6$ is Me, $R^8$ is Bn, and $R^2$, $R^3$, $R^4$, $R^5$, $R^7$, $R^9$, $R^{10}$ and $R^{11}$ are all H;
compound 882: n is 0, X is S, Y is O, $R^6$ is Me, $R^9$ is Bn, and $R^2$, $R^3$, $R^4$, $R^5$, $R^7$, $R^8$, $R^{10}$ and $R^{11}$ are all H;
compound 41: n is 0, X is S, Y is O, $R^6$ is Me, $R^7$ and $R^8$ are both Cl, and $R^2$, $R^3$, $R^4$, $R^5$, $R^9$, $R^{10}$ and $R^{11}$ are all H;
compound 42: n is 0, X is S, Y is O, $R^6$ is Me, $R^7$ and $R^9$ are both Cl, and $R^2$, $R^3$, $R^4$, $R^5$, $R^8$, $R^{10}$ and $R^{11}$ are all H;
compound 43: n is 0, X is S, Y is O, $R^6$ is Me, $R^7$ and $R^{10}$ are both Cl, and $R^2$, $R^3$, $R^4$, $R^5$, $R^8$, $R^9$ and $R^{11}$ are all H;
compound 44: n is 0, X is S, Y is O, $R^6$ is Me, $R^7$ and $R^{11}$ are both Cl, and $R^2$, $R^3$, $R^4$, $R^5$, $R^8$, $R^9$ and $R^{10}$ are all H;
compound 45: n is 0, X is S, Y is O, $R^6$ is Me, $R^8$ and $R^9$ are both Cl, and $R^2$, $R^3$, $R^4$, $R^5$, $R^7$, $R^{10}$ and $R^{11}$ are all H;
compound 46: n is 0, X is S, Y is O, $R^6$ and $R^7$ are Me, $R^9$ is Cl, and $R^2$, $R^3$, $R^4$, $R^3$, $R^8$, $R^{10}$ and $R^{11}$ are all H;
compound 47: n is 0, X is S, Y is O, $R^6$, $R^7$ and $R^8$ are all Me, and $R^2$, $R^3$, $R^4$, $R^5$, $R^9$, $R^{10}$ and $R^{11}$ are all H;
compound 48: n is 0, X is S, Y is O, $R^6$, $R^7$ and $R^9$ are all Me, and $R^2$, $R^3$, $R^4$, $R^5$, $R^8$, $R^{10}$ and $R^{11}$ are all H;
compound 49: n is 0, X is S, Y is O, $R^6$, $R^7$ and $R^{10}$ are all Me, and $R^2$, $R^3$, $R^4$, $R^5$, $R^8$, $R^9$ and $R^{11}$ are all H;
compound 50: n is 0, X is S, Y is O, $R^6$, $R^7$ and $R^{11}$ are all Me, and $R^2$, $R^3$, $R^4$, $R^5$, $R^8$, $R^9$ and $R^{10}$ are all H;
compound 51: n is 0, X is S, Y is O, $R^6$, $R^7$, $R^8$ and $R^{11}$ are all Me, and $R^2$, $R^3$, $R^4$, $R^5$, $R^9$ and $R^{10}$ are all H;
compound 52: n is 0, X is S, Y is O, $R^6$, $R^7$, $R^9$ and $R^{11}$ are all Me, and $R^2$, $R^3$, $R^4$, $R^5$, $R^8$ and $R^{10}$ are all H;
compound 53: n is 0, X is S, Y is O, $R^6$ is Me, $R^7$ and $R^9$ are both $CF_3$, and $R^2$, $R^3$, $R^4$, $R^5$, $R^8$, $R^{10}$ and $R^{11}$ are all H;
compound 54: n is 0, X is S, Y is O, $R^6$ is Me, $R^8$ and $R^{10}$ are both $CF_3$, and $R^2$, $R^3$, $R^4$, $R^5$, $R^7$, $R^9$ and $R^{11}$ are all H;
compound 55: n is 0, X is S, Y is O, $R^6$ is Me, $R^2$, $R^3$, $R^4$ and $R^5$ are all H, and $R^7$, $R^8$, $R^9$, $R^{10}$ and $R^{11}$ are all F;
compound 56: n is 0, X is S, Y is O, $R^6$ is Me, $R^7$ is $CH_3CH_2$, and $R^2$, $R^3$, $R^4$, $R^5$, $R^8$, $R^9$, $R^{10}$ and $R^{11}$ are all H;

compound 57: n is 0, X is S, Y is O, $R^6$ is Me, $R^8$ is $CH_3CH_2$, and $R^2$, $R^3$, $R^4$, $R^5$, $R^7$, $R^9$, $R^{10}$ and $R^{11}$ are all H;
compound 58: n is 0, X is S, Y is O, $R^6$ is Me, $R^9$ is $CH_3CH_2$, and $R^2$, $R^3$, $R^4$, $R^5$, $R^7$, $R^8$, $R^{10}$ and $R^{11}$ are all H;
compound 59: n is 0, X is S, Y is O, $R^6$ is Me, $R^7$ is $CH(CH_3)_2$, and $R^2$, $R^3$, $R^4$, $R^5$, $R^8$, $R^9$, $R^{10}$ and $R^{11}$ are all H;
compound 60: n is 0, X is S, Y is O, $R^6$ is Me, $R^8$ is $CH(CH_3)_2$, and $R^2$, $R^3$, $R^4$, $R^5$, $R^7$, $R^9$, $R^{10}$ and $R^{11}$ are all H;
compound 61: n is 0, X is S, Y is O, $R^6$ is Me, $R^9$ is $CH(CH_3)_2$, and $R^2$, $R^3$, $R^4$, $R^5$, $R^7$, $R^8$, $R^{10}$ and $R^{11}$ are all H;
compound 62: n is 0, X is S, Y is O, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;
compound 63: n is 0, X is S, Y is O, $R^6$ is Me, $R^7$ is

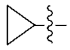

, and $R^2$, $R^3$, $R^4$, $R^5$, $R^8$, $R^9$, $R^{10}$ and $R^{11}$ are all H;
compound 64: n is 0, X is S, Y is O, $R^6$ is Me, $R^7$ is

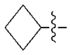
,

and $R^2$, $R^3$, $R^4$, $R^5$, $R^8$, $R^9$, $R^{10}$ and $R^{11}$ are all H;
compound 65: n is 0, X is S, Y is O, $R^6$ is Me, $R^7$ is $CO_2CH_3$, and $R^2$, $R^3$, $R^4$, $R^5$, $R^8$, $R^9$, $R^{10}$ and $R^{11}$ are all H;
compound 66: n is 0, X is S, Y is O, $R^6$ is Me, $R^7$ and $R^8$ are cyclized to form

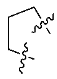
,

and $R^2$, $R^3$, $R^4$, $R^5$, $R^9$, $R^{10}$ and $R^{11}$ are all H;
compound 67: n is 0, X is S, Y is O, $R^6$ is Me, $R^8$ and $R^9$ are cyclized to form

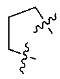
,

and $R^2$, $R^3$, $R^4$, $R^5$, $R^7$, $R^{10}$ and $R^{11}$ are all H;
compound 68: n is 0, X is S, Y is O, $R^6$ is Me, $R^8$ and $R^9$ are cyclized to form

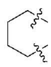
,

and $R^2$, $R^3$, $R^4$, $R^5$, $R^7$, $R^{10}$ and $R^{11}$ are all H;
compound 69: n is 0, X is S, Y is O, $R^6$ is Me, $R^7$ and $R^8$ are cyclized to form

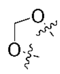
,

and $R^2$, $R^3$, $R^4$, $R^5$, $R^9$, $R^{10}$ and $R^{11}$ are all H;
compound 70: n is 0, X is S, Y is O, $R^6$ is Me, $R^8$ and $R^9$ are cyclized to form

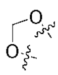
,

and $R^2$, $R^3$, $R^4$, $R^5$, $R^7$, $R^{10}$ and $R^{11}$ are all H;
compound 71: n is 0, X is S, Y is O, $R^6$ is Me, $R^8$ and $R^9$ are cyclized to form

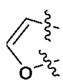
,

and $R^2$, $R^3$, $R^4$, $R^5$, $R^7$, $R^{10}$ and $R^{11}$ are H;

compound 72: n is 0, X is S, Y is O, $R^6$ is Me, $R^7$ and $R^8$ are cyclized to form

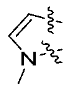,

and $R^2$, $R^3$, $R^4$, $R^5$, $R^9$, $R^{10}$ and $R^{11}$ are all H;

compound 73: n is 0, X is S, Y is O, $R^6$ is Me, $R^7$ and $R^8$ are cyclized to form

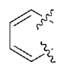,

and $R^2$, $R^3$, $R^4$, $R^5$, $R^9$, $R^{10}$ and $R^{11}$ are all H;

compound 74: n is 0, X is S, Y is O, $R^6$ is Me, $R^8$ and $R^9$ are cyclized to form

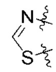,

and $R^2$, $R^3$, $R^4$, $R^5$, $R^7$, $R^{10}$ and $R^{11}$ are all H;

compound 75: n is 0, X is S, Y is O, $R^6$ is Me, $R^8$ and $R^9$ are cyclized to form

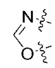,

and $R^2$, $R^3$, $R^4$, $R^5$, $R^7$, $R^{10}$ and $R^{11}$ are all H;

compound 76: n is 0, X is S, Y is O, $R^6$ is Me, $R^8$ and $R^9$ are cyclized to form

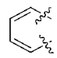,

and $R^2$, $R^3$, $R^4$, $R^5$, $R^7$, $R^{10}$ and $R^{11}$ are all H;

compound 77: n is 0, X is S, Y is O, $R^5$ is Me, and $R^2$, $R^3$, $R^4$, $R^6$, $R^7$, $R^8$, $R^9$, $R^{10}$ and $R^{11}$ are all H;

compound 78: n is 0, X is S, Y is O, $R^5$ is Me, $R^7$ is F, and $R^2$, $R^3$, $R^4$, $R^6$, $R^8$, $R^9$, $R^{10}$ and $R^{11}$ are all H;

compound 79: n is 0, X is S, Y is O, $R^5$ is Me, $R^8$ is F, and $R^2$, $R^3$, $R^4$, $R^6$, $R^7$, $R^9$, $R^{10}$ and $R^{11}$ are all H;

compound 80: n is 0, X is S, Y is O, $R^5$ is Me, $R^9$ is F, and $R^2$, $R^3$, $R^4$, $R^6$, $R^7$, $R^8$, $R^{10}$ and $R^{11}$ are all H;

compound 81: n is 0, X is S, Y is O, $R^5$ is Me, $R^7$ is Cl, and $R^2$, $R^3$, $R^4$, $R^6$, $R^8$, $R^9$, $R^{10}$ and $R^{11}$ are all H;

compound 82: n is 0, X is S, Y is O, $R^5$ is Me, $R^8$ is Cl, and $R^2$, $R^3$, $R^4$, $R^6$, $R^7$, $R^9$, $R^{10}$ and $R^{11}$ are all H;

compound 83: n is 0, X is S, Y is O, $R^5$ is Me, $R^9$ is Cl, and $R^2$, $R^3$, $R^4$, $R^6$, $R^7$, $R^8$, $R^{10}$ and $R^{11}$ are all H;

compound 84: n is 0, X is S, Y is O, $R^5$ is Me, $R^7$ is Br, and $R^2$, $R^3$, $R^4$, $R^6$, $R^8$, $R^9$, $R^{10}$ and $R^{11}$ are all H;

compound 85: n is 0, X is S, Y is O, $R^5$ is Me, $R^8$ is Br, and $R^2$, $R^3$, $R^4$, $R^6$, $R^7$, $R^9$, $R^{10}$ and $R^{11}$ are all H;

compound 86: n is 0, X is S, Y is O, $R^5$ is Me, $R^9$ is Br, and $R^2$, $R^3$, $R^4$, $R^6$, $R^7$, $R^8$, $R^{10}$ and $R^{11}$ are all H;

compound 87: n is 0, X is S, Y is O, $R^5$ is Me, $R^7$ is I, and $R^2$, $R^3$, $R^4$, $R^6$, $R^8$, $R^9$, $R^{10}$ and $R^{11}$ are all H;

compound 88: n is 0, X is S, Y is O, $R^5$ is Me, $R^8$ is I, and $R^2$, $R^3$, $R^4$, $R^6$, $R^7$, $R^9$, $R^{10}$ and $R^{11}$ are all H;

compound 89: n is 0, X is S, Y is O, $R^5$ is Me, $R^9$ is I, and $R^2$, $R^3$, $R^4$, $R^6$, $R^7$, $R^8$, $R^{10}$ and $R^{11}$ are all H;

compound 90: n is 0, X is S, Y is O, $R^5$ is Me, $R^7$ is $NO_2$, and $R^2$, $R^3$, $R^4$, $R^6$, $R^8$, $R^9$, $R^{10}$ and $R^{11}$ are all H;

compound 91: n is 0, X is S, Y is O, $R^5$ is Me, $R^8$ is $NO_2$, and $R^2$, $R^3$, $R^4$, $R^6$, $R^7$, $R^9$, $R^{10}$ and $R^{11}$ are all H;

compound 92: n is 0, X is S, Y is O, $R^5$ is Me, $R^9$ is $NO_2$, and $R^2$, $R^3$, $R^4$, $R^6$, $R^7$, $R^8$, $R^{10}$ and $R^{11}$ are all H;

compound 93: n is 0, X is S, Y is O, $R^5$ is Me, $R^7$ is $CF_3$, and $R^2$, $R^3$, $R^4$, $R^6$, $R^8$, $R^9$, $R^{10}$ and $R^{11}$ are all H;

compound 94: n is 0, X is S, Y is O, $R^5$ is Me, $R^8$ is $CF_3$, and $R^2$, $R^3$, $R^4$, $R^6$, $R^7$, $R^9$, $R^{10}$ and $R^{11}$ are all H;

compound 95: n is 0, X is S, Y is O, $R^5$ is Me, $R^9$ is $CF_3$, and $R^2$, $R^3$, $R^4$, $R^6$, $R^7$, $R^8$, $R^{10}$ and $R^{11}$ are all H;

compound 96: n is 0, X is S, Y is O, $R^5$ is Me, $R^7$ is $OCF_3$, and $R^2$, $R^3$, $R^4$, $R^6$, $R^8$, $R^9$, $R^{10}$ and $R^{11}$ are all H;

compound 97: n is 0, X is S, Y is O, $R^5$ is Me, $R^8$ is $OCF_3$, and $R^2$, $R^3$, $R^4$, $R^6$, $R^7$, $R^9$, $R^{10}$ and $R^{11}$ are all H;

compound 98: n is 0, X is S, Y is O, $R^5$ is Me, $R^9$ is $OCF_3$, and $R^2$, $R^3$, $R^4$, $R^6$, $R^7$, $R^8$, $R^{10}$ and $R^{11}$ are all H;

compound 99: n is 0, X is S, Y is O, $R^5$ is Me, $R^7$ is $OCHF_2$, and $R^2$, $R^3$, $R^4$, $R^6$, $R^8$, $R^9$, $R^{10}$ and $R^{11}$ are all H;

compound 100: n is 0, X is S, Y is O, $R^5$ is Me, $R^8$ is $OCHF_2$, and $R^2$, $R^3$, $R^4$, $R^6$, $R^7$, $R^9$, $R^{10}$ and $R^{11}$ are all H;

compound 101: n is 0, X is S, Y is O, $R^5$ is Me, $R^9$ is $OCHF_2$, and $R^2$, $R^3$, $R^4$, $R^6$, $R^7$, $R^8$, $R^{10}$ and $R^{11}$ are all H;
compound 102: n is 0, X is S, Y is O, $R^5$ is Me, $R^9$ is $SCF_3$, and $R^2$, $R^3$, $R^4$, $R^6$, $R^7$, $R^8$, $R^{10}$ and $R^{11}$ are all H;
compound 103: n is 0, X is S, Y is O, $R^5$ is Me, $R^7$ is $OCH_3$, and $R^2$, $R^3$, $R^4$, $R^6$, $R^8$, $R^9$, $R^{10}$ and $R^{11}$ are all H;
compound 104: n is 0, X is S, Y is O, $R^5$ is Me, $R^8$ is $OCH_3$, and $R^2$, $R^3$, $R^4$, $R^6$, $R^7$, $R^9$, $R^{10}$ and $R^{11}$ are all H;
compound 105: n is 0, X is S, Y is O, $R^5$ is Me, $R^9$ is $OCH_3$, and $R^2$, $R^3$, $R^4$, $R^6$, $R^7$, $R^8$, $R^{10}$ and $R^{11}$ are all H;
compound 106: n is 0, X is S, Y is O, $R^5$ is Me, $R^9$ is $OC_2H_5$, and $R^2$, $R^3$, $R^4$, $R^6$, $R^8$, $R^7$, $R^{10}$ and $R^{11}$ are all H;
compound 107: n is 0, X is S, Y is O, $R^5$ is Me, $R^7$ is CN, and $R^2$, $R^3$, $R^4$, $R^6$, $R^8$, $R^9$, $R^{10}$ and $R^{11}$ are all H;
compound 108: n is 0, X is S, Y is O, $R^5$ is Me, $R^8$ is CN, and $R^2$, $R^3$, $R^4$, $R^6$, $R^7$, $R^9$, $R^{10}$ and $R^{11}$ are all H;
compound 109: n is 0, X is S, Y is O, $R^5$ is Me, $R^9$ is CN, and $R^2$, $R^3$, $R^4$, $R^6$, $R^7$, $R^8$, $R^{10}$ and $R^{11}$ are all H;
compound 110: n is 0, X is S, Y is O, $R^5$ and $R^7$ are Me, and $R^2$, $R^3$, $R^4$, $R^6$, $R^8$, $R^9$, $R^{10}$ and $R^{11}$ are all H;
compound 111: n is 0, X is S, Y is O, $R^5$ and $R^8$ are Me, and $R^2$, $R^3$, $R^4$, $R^6$, $R^7$, $R^9$, $R^{10}$ and $R^{11}$ are all H;
compound 112: n is 0, X is S, Y is O, $R^5$ and $R^9$ are Me, and $R^2$, $R^3$, $R^4$, $R^6$, $R^7$, $R^8$, $R^{10}$ and $R^{11}$ are all H;
compound 113: n is 0, X is S, Y is O, $R^5$ is Me, $R^7$ is $CH_3SO_2$, and $R^2$, $R^3$, $R^4$, $R^6$, $R^8$, $R^9$, $R^{10}$ and $R^{11}$ are all H;
compound 114: n is 0, X is S, Y is O, $R^5$ is Me, $R^8$ is $CH_3SO_2$, and $R^2$, $R^3$, $R^4$, $R^6$, $R^7$, $R^9$, $R^{10}$ and $R^{11}$ are all H;
compound 115: n is 0, X is S, Y is O, $R^5$ is Me, $R^9$ is $CH_3SO_2$, and $R^2$, $R^3$, $R^4$, $R^6$, $R^7$, $R^8$, $R^{10}$ and $R^{11}$ are all H;
compound 116: n is 0, X is S, Y is O, $R^5$ is Me, $R^7$ is $C_2H_5SO_2$, and $R^2$, $R^3$, $R^4$, $R^6$, $R^8$, $R^9$, $R^{10}$ and $R^{11}$ are all H;
compound 117: n is 0, X is S, Y is O, $R^5$ is Me, $R^7$ and $R^8$ are both Cl, and $R^2$, $R^3$, $R^4$, $R^6$, $R^9$, $R^{10}$ and $R^{11}$ are all H;
compound 118: n is 0, X is S, Y is O, $R^5$ is Me, $R^7$ and $R^9$ are both Cl, and $R^2$, $R^3$, $R^4$, $R^6$, $R^8$, $R^{10}$ and $R^{11}$ are all H;
compound 119: n is 0, X is S, Y is O, $R^5$ is Me, $R^7$ and $R^{10}$ are both Cl, and $R^2$, $R^3$, $R^4$, $R^6$, $R^8$, $R^9$ and $R^{11}$ are all H;
compound 120: n is 0, X is S, Y is O, $R^5$ is Me, Rand $R^{11}$ are both Cl, and $R^2$, $R^3$, $R^4$, $R^6$, $R^8$, $R^9$ and $R^{10}$ are all H;
compound 121: n is 0, X is S, Y is O, $R^5$ is Me, $R^8$ and $R^9$ are both Cl, and $R^2$, $R^3$, $R^4$, $R^6$, $R^7$, $R^{10}$ and $R^{11}$ are all H;
compound 122: n is 0, X is S, Y is O, $R^5$ and $R^7$ are Me, $R^9$ is Cl, and $R^2$, $R^3$, $R^4$, $R^6$, $R^8$, $R^{10}$ and $R^{11}$ are all H;
compound 123: n is 0, X is S, Y is O, $R^5$, $R^7$ and $R^8$ are all Me, and $R^2$, $R^3$, $R^4$, $R^6$, $R^9$, $R^{10}$ and $R^{11}$ are all H;
compound 124: n is 0, X is S, Y is O, $R^5$, $R^7$ and $R^9$ are all Me, and $R^2$, $R^3$, $R^4$, $R^6$, $R^8$, $R^{10}$ and $R^{11}$ are all H;
compound 125: n is 0, X is S, Y is O, $R^5$, $R^7$ and $R^{10}$ are all Me, and $R^2$, $R^3$, $R^4$, $R^6$, $R^8$, $R^9$ and $R^{11}$ are all H;
compound 126: n is 0, X is S, Y is O, $R^5$, $R^7$ and $R^{11}$ are all Me, and $R^2$, $R^3$, $R^4$, $R^6$, $R^8$, $R^9$ and $R^{10}$ are all H;
compound 127: n is 0, X is S, Y is O, $R^5$, $R^7$, $R^8$ and $R^{11}$ are all Me, and $R^2$, $R^3$, $R^4$, $R^6$, $R^9$ and $R^{10}$ are all H;
compound 128: n is 0, X is S, Y is O, $R^5$, $R^7$, $R^9$ and $R^{11}$ are all Me, and $R^2$, $R^3$, $R^4$, $R^6$, $R^8$ and $R^{10}$ are all H;
compound 129: n is 0, X is S, Y is O, $R^5$ is Me, $R^7$ and $R^9$ are both $CF_3$, and $R^2$, $R^3$, $R^4$, $R^6$, $R^8$, $R^{10}$ and $R^{11}$ are all H;
compound 130: n is 0, X is S, Y is O, $R^5$ is Me, $R^8$ and $R^{10}$ are both $CF_3$, and $R^2$, $R^3$, $R^4$, $R^6$, $R^7$, $R^9$ and $R^{11}$ are all H;
compound 131: n is 0, X is S, Y is O, $R^5$ is Me, $R^2$, $R^3$, $R^4$ and $R^6$ are all H, and $R^7$, $R^8$, $R^9$, $R^{10}$ and $R^{11}$ are all F;
compound 132: n is 0, X is S, Y is O, $R^5$ is Me, $R^7$ is $CH_3CH_2$, and $R^2$, $R^3$, $R^4$, $R^6$, $R^8$, $R^9$, $R^{10}$ and $R^{11}$ are all H;
compound 133: n is 0, X is S, Y is O, $R^5$ is Me, $R^8$ is $CH_3CH_2$, and $R^2$, $R^3$, $R^4$, $R^6$, $R^7$, $R^9$, $R^{10}$ and $R^{11}$ are all H;
compound 134: n is 0, X is S, Y is O, $R^5$ is Me, $R^9$ is $CH_3CH_2$, and $R^2$, $R^3$, $R^4$, $R^6$, $R^7$, $R^8$, $R^{10}$ and $R^{11}$ are all H;
compound 135: n is 0, X is S, Y is O, $R^5$ is Me, $R^7$ is $CH(CH_3)_2$, and $R^2$, $R^3$, $R^4$, $R^6$, $R^8$, $R^9$, $R^{10}$ and $R^{11}$ are all H;
compound 136: n is 0, X is S, Y is O, $R^5$ is Me, $R^8$ is $CH(CH_3)_2$, and $R^2$, $R^3$, $R^4$, $R^6$, $R^7$, $R^9$, $R^{10}$ and $R^{11}$ are all H;
compound 137: n is 0, X is S, Y is O, $R^5$ is Me, $R^9$ is $CH(CH_3)_2$, and $R^2$, $R^3$, $R^4$, $R^6$, $R^7$, $R^8$, $R^{10}$ and $R^{11}$ are all H;
compound 138: n is 0, X is S, Y is O, $R^5$ is Me, $R^7$ is $CH_3(CH_2)_2$, $R^2$, $R^3$, $R^4$, $R^6$, $R^3$, $R^9$, $R^{10}$ and $R^{11}$ are all H;
compound 139: n is 0, X is S, Y is O, $R^5$ is Me, $R^7$ is

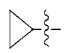

, and $R^2$, $R^3$, $R^4$, $R^6$, $R^8$, $R^9$, $R^{10}$ and $R^{11}$ are all H;
compound 140: n is 0, X is S, Y is O, $R^5$ is Me, $R^7$ is

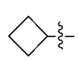

, and $R^2$, $R^3$, $R^4$, $R^6$, $R^8$, $R^9$, $R^{10}$ and $R^{11}$ are all H;
compound 141: n is 0, X is S, Y is O, $R^5$ is Me, $R^7$ is $CO_2CH_3$, and $R^2$, $R^3$, $R^4$, $R^6$, $R^8$, $R^9$, $R^{10}$ and $R^{11}$ are all H;
compound 142: n is 0, X is S, Y is O, $R^5$ is Me, $R^7$ and $R^8$ are cyclized to form

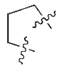

,

and $R^2$, $R^3$, $R^4$, $R^6$, $R^9$, $R^{10}$ and $R^{11}$ are all H;
compound 143: n is 0, X is S, Y is O, $R^3$ is Me, $R^8$ and $R^9$ are cyclized to form

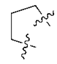

and $R^2$, $R^3$, $R^4$, $R^6$, $R^7$, $R^{10}$ and $R^{11}$ are all H;
compound 144: n is 0, X is S, Y is O, $R^3$ is Me, $R^8$ and $R^9$ are cyclized to form

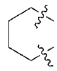

and $R^2$, $R^3$, $R^4$, $R^6$, $R^7$, $R^{10}$ and $R^{11}$ are all H;
compound 145: n is 0, X is S, Y is O, $R^5$ is Me, $R^7$ and $R^8$ are cyclized to form

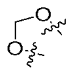

and $R^2$, $R^3$, $R^4$, $R^6$, $R^9$, $R^{10}$ and $R^{11}$ are all H;
compound 146: n is 0, X is S, Y is O, $R^5$ is Me, $R^8$ and $R^9$ are cyclized to form

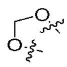

and $R^2$, $R^3$, $R^4$, $R^6$, $R^7$, $R^{10}$ and $R^{11}$ are all H;
compound 147: n is 0, X is S, Y is O, $R^3$ is Me, $R^7$ and $R^8$ are cyclized to form

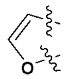

and $R^2$, $R^3$, $R^4$, $R^6$, $R^9$, $R^{10}$ and $R^{11}$ are all H;
compound 148: n is 0, X is S, Y is O, $R^3$ is Me, $R^7$ and $R^8$ are cyclized to

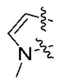

and $R^2$, $R^3$, $R^4$, $R^6$, $R^9$, $R^{10}$ and $R^{11}$ are all H;
compound 149: n is 0, X is S, Y is O, $R^3$ is Me, $R^7$ and $R^8$ are cyclized to form

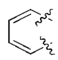

and $R^2$, $R^3$, $R^4$, $R^6$, $R^9$, $R^{10}$ and $R^{11}$ are all H;
compound 150: n is 0, X is S, Y is O, $R^5$ is Me, $R^7$ and $R^8$ are cyclized to form

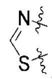

and $R^2$, $R^3$, $R^4$, $R^6$, $R^9$, $R^{10}$ and $R^{11}$ are all H;
compound 151: n is 0, X is S, Y is O, $R^5$ is Me, $R^7$ and $R^8$ are cyclized to form

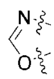

and $R^2$, $R^3$, $R^4$, $R^6$, $R^9$, $R^{10}$ and $R^{11}$ are all H;

compound 152: n is 0, X is S, Y is O, $R^5$ is Me, $R^8$ and $R^9$ are cyclized to form

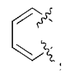

, and $R^2$, $R^3$, $R^4$, $R^6$, $R^7$, $R^{10}$ and $R^{11}$ are all H;
compound 153: n is 0, X is S, Y is O, $R^5$ and $R^6$ are Me, and $R^2$, $R^3$, $R^4$, $R^7$, $R^8$, $R^9$, $R^{10}$ and $R^{11}$ are H;
compound 154: n is 0, X is S, Y is O, $R^5$ and $R^6$ are both Me, $R^7$ is F, and $R^2$, $R^3$, $R^4$, $R^8$, $R^9$, $R^{10}$ and $R^{11}$ are all H;
compound 155: n is 0, X is S, Y is O, $R^5$ and $R^6$ are both Me, $R^8$ is F, and $R^2$, $R^3$, $R^4$, $R^7$, $R^9$, $R^{10}$ and $R^{11}$ are all H;
compound 156: n is 0, X is S, Y is O, $R^5$ and $R^6$ are both Me, $R^9$ is F, and $R^2$, $R^3$, $R^4$, $R^7$, $R^8$, $R^{10}$ and $R^{11}$ are all H;
compound 157: n is 0, X is S, Y is O, $R^5$ and $R^6$ are both Me, $R^7$ is Cl, and $R^2$, $R^3$, $R^4$, $R^8$, $R^9$, $R^{10}$ and $R^{11}$ are all H;
compound 158: n is 0, X is S, Y is O, $R^5$ and $R^6$ are both Me, $R^8$ is Cl, and $R^2$, $R^3$, $R^4$, $R^7$, $R^9$, $R^{10}$ and $R^{11}$ are all H;
compound 159: n is 0, X is S, Y is O, $R^5$ and $R^6$ are both Me, $R^9$ is Cl, and $R^2$, $R^3$, $R^4$, $R^7$, $R^8$, $R^{10}$ and $R^{11}$ are all H;
compound 160: n is 0, X is S, Y is O, $R^5$ and $R^6$ are both Me, $R^7$ is Br, $R^2$, $R^3$, $R^4$, $R^8$, $R^9$, $R^{10}$ and $R^{11}$ are all H;
compound 161: n is 0, X is S, Y is O, $R^5$ and $R^6$ are both Me, $R^8$ is Br, $R^2$, $R^3$, $R^4$, $R^7$, $R^9$, $R^{10}$ and $R^{11}$ are all H;
compound 162: n is 0, X is S, Y is O, $R^5$ and $R^6$ are both Me, $R^9$ is Br, $R^2$, $R^3$, $R^4$, $R^7$, $R^8$, $R^{10}$ and $R^{11}$ are all H;
compound 163: n is 0, X is S, Y is O, $R^5$ and $R^6$ are both Me, $R^7$ is I, and $R^2$, $R^3$, $R^4$, $R^8$, $R^9$, $R^{10}$ and $R^{11}$ are all H;
compound 164: n is 0, X is S, Y is O, $R^5$ and $R^6$ are both Me, $R^8$ is I, and $R^2$, $R^3$, $R^4$, $R^7$, $R^9$, $R^{10}$ and $R^{11}$ are all H;
compound 165: n is 0, X is S, Y is O, $R^5$ and $R^6$ are both Me, $R^9$ is I, and $R^2$, $R^3$, $R^4$, $R^7$, $R^8$, $R^{10}$ and $R^{11}$ are all H;
compound 166: n is 0, X is S, Y is O, $R^5$ and $R^6$ are both Me, $R^7$ is $NO_2$, $R^2$, $R^3$, $R^4$, $R^8$, $R^9$, $R^{10}$ and $R^{11}$ are all H;
compound 167: n is 0, X is S, Y is O, $R^5$ and $R^6$ are both Me, $R^8$ is $NO_2$, $R^2$, $R^3$, $R^4$, $R^7$, $R^9$, $R^{10}$ and $R^{11}$ are all H;
compound 168: n is 0, X is S, Y is O, $R^5$ and $R^6$ are both Me, $R^9$ is $NO_2$, $R^2$, $R^3$, $R^4$, $R^7$, $R^8$, $R^{10}$ and $R^{11}$ are all H;
compound 169: n is 0, X is S, Y is O, $R^5$ and $R^6$ are both Me, $R^7$ is $CF_3$, $R^2$, $R^3$, $R^4$, $R^8$, $R^9$, $R^{10}$ and $R^{11}$ are all H;
compound 170: n is 0, X is S, Y is O, $R^5$ and $R^6$ are both Me, $R^8$ is $CF_3$, $R^2$, $R^3$, $R^4$, $R^7$, $R^9$, $R^{10}$ and $R^{11}$ are all H;
compound 171: n is 0, X is S, Y is O, $R^5$ and $R^6$ are both Me, $R^9$ is $CF_3$, $R^2$, $R^3$, $R^4$, $R^7$, $R^8$, $R^{10}$ and $R^{11}$ are all H;
compound 172: n is 0, X is S, Y is O, $R^5$ and $R^6$ are both Me, $R^7$ is $OCF_3$, $R^2$, $R^3$, $R^4$, $R^8$, $R^9$, $R^{10}$ and $R^{11}$ are all H;
compound 173: n is 0, X is S, Y is O, $R^5$ and $R^6$ are both Me, $R^8$ is $OCF_3$, $R^2$, $R^3$, $R^4$, $R^7$, $R^9$, $R^{10}$ and $R^{11}$ are all H;
compound 174: n is 0, X is S, Y is O, $R^5$ and $R^6$ are both Me, $R^9$ is $OCF_3$, $R^2$, $R^3$, $R^4$, $R^7$, $R^8$, $R^{10}$ and $R^{11}$ are all H;
compound 175: n is 0, X is S, Y is O, $R^5$ and $R^6$ are both Me, $R^7$ is $OCHF_2$, $R^2$, $R^3$, $R^4$, $R^8$, $R^9$, $R^{10}$ and $R^{11}$ are all H;
compound 176: n is 0, X is S, Y is O, $R^5$ and $R^6$ are both Me, $R^8$ is $OCHF_2$, $R^2$, $R^3$, $R^4$, $R^7$, $R^9$, $R^{10}$ and $R^{11}$ are all H;
compound 177: n is 0, X is S, Y is O, $R^5$ and $R^6$ are both Me, $R^9$ is $OCHF_2$, $R^2$, $R^3$, $R^4$, $R^7$, $R^8$, $R^{10}$ and $R^{11}$ are all H;
compound 178: n is 0, X is S, Y is O, $R^5$ and $R^6$ are both Me, $R^9$ is $SCF_3$, $R^2$, $R^3$, $R^4$, $R^7$, $R^8$, $R^{10}$ and $R^{11}$ are all H;
compound 179: n is 0, X is S, Y is O, $R^5$ and $R^6$ are both Me, $R^7$ is $OCH_3$, $R^2$, $R^3$, $R^4$, $R^8$, $R^9$, $R^{10}$ and $R^{11}$ are all H;
compound 180: n is 0, X is S, Y is O, $R^5$ and $R^6$ are both Me, $R^8$ is $OCH_3$, $R^2$, $R^3$, $R^4$, $R^7$, $R^9$, $R^{10}$ and $R^{11}$ are all H;
compound 181: n is 0, X is S, Y is O, $R^5$ and $R^6$ are both Me, $R^9$ is $OCH_3$, $R^2$, $R^3$, $R^4$, $R^7$, $R^8$, $R^{10}$ and $R^{11}$ are all H;
compound 182: n is 0, X is S, Y is O, $R^5$ and $R^6$ are both Me, $R^9$ is $OC_2H_5$, $R^2$, $R^3$, $R^4$, $R^8$, $R^7$, $R^{10}$ and $R^{11}$ are all H;
compound 183: n is 0, X is S, Y is O, $R^5$ and $R^6$ are both Me, $R^7$ is CN, and $R^2$, $R^3$, $R^4$, $R^8$, $R^9$, $R^{10}$ and $R^{11}$ are all H;
compound 184: n is 0, X is S, Y is O, $R^5$ and $R^6$ are both Me, $R^8$ is CN, and $R^2$, $R^3$, $R^4$, $R^7$, $R^9$, $R^{10}$ and $R^{11}$ are all H;
compound 185: n is 0, X is S, Y is O, $R^5$ and $R^6$ are both Me, $R^9$ is CN, and $R^2$, $R^3$, $R^4$, $R^7$, $R^8$, $R^{10}$ and $R^{11}$ are all H;
compound 186: n is 0, X is S, Y is O, $R^5$, $R^6$ and $R^7$ are Me, and $R^2$, $R^3$, $R^4$, $R^8$, $R^9$, $R^{10}$ and $R^{11}$ are all H;
compound 187: n is 0, X is S, Y is O, $R^5$, $R^6$ and $R^8$ are Me, and $R^2$, $R^3$, $R^4$, $R^7$, $R^9$, $R^{10}$ and $R^{11}$ are all H;
compound 188: n is 0, X is S, Y is O, $R^5$, $R^6$ and $R^9$ are Me, and $R^2$, $R^3$, $R^4$, $R^7$, $R^8$, $R^{10}$ and $R^{11}$ are all H;
compound 189: n is 0, X is S, Y is O, $R^5$ and $R^6$ are both Me, $R^7$ is $CH_3SO_2$, $R^2$, $R^3$, $R^4$, $R^8$, $R^9$, $R^{10}$ and $R^{11}$ are all H;
compound 190: n is 0, X is S, Y is O, $R^5$ and $R^6$ are both Me, $R^8$ is $CH_3SO_2$, $R^2$, $R^3$, $R^4$, $R^7$, $R^9$, $R^{10}$ and $R^{11}$ are all H;
compound 191: n is 0, X is S, Y is O, $R^5$ and $R^6$ are both Me, $R^9$ is $CH_3SO_2$, $R^2$, $R^3$, $R^4$, $R^7$, $R^8$, $R^{10}$ and $R^{11}$ are all H;
compound 192: n is 0, X is S, Y is O, $R^5$ and $R^6$ are both Me, $R^7$ is $C_2H_5SO_2$, $R^2$, $R^3$, $R^4$, $R^8$, $R^9$, $R^{10}$ and $R^{11}$ are all H;
compound 193: n is 0, X is S, Y is O, $R^5$ and $R^6$ are both Me, $R^7$ and $R^8$ are both Cl, and $R^2$, $R^3$, $R^4$, $R^9$, $R^{10}$ and $R^{11}$ are all H;
compound 194: n is 0, X is S, Y is O, $R^5$ and $R^6$ are both Me, $R^7$ and $R^9$ are both Cl, and $R^2$, $R^3$, $R^4$, $R^8$, $R^{10}$ and $R^{11}$ are all H;
compound 195: n is 0, X is S, Y is O, $R^5$ and $R^6$ are both Me, $R^7$ and $R^{10}$ are both Cl, and $R^2$, $R^3$, $R^4$, $R^8$, $R^9$ and $R^{11}$ are all H;
compound 196: n is 0, X is S, Y is O, $R^5$ and $R^6$ are both Me, $R^7$ and $R^{11}$ are both Cl, and $R^2$, $R^3$, $R^4$, $R^8$, $R^9$ and $R^{10}$ are all H;
compound 197: n is 0, X is S, Y is O, $R^5$ and $R^6$ are both Me, $R^8$ and $R^9$ are both Cl, and $R^2$, $R^3$, $R^4$, $R^7$, $R^{10}$ and $R^{11}$ are all H;

compound 198: n is 0, X is S, Y is O, $R^5$, $R^6$ and $R^7$ are Me, $R^9$ is Cl, and $R^2$, $R^3$, $R^4$, $R^8$, $R^{10}$ and $R^{11}$ are all H;
compound 199: n is 0, X is S, Y is O, $R^5$, $R^6$, $R^7$ and $R^8$ are all Me, and $R^2$, $R^3$, $R^4$, $R^9$, $R^{10}$ and $R^{11}$ are all H;
compound 200: n is 0, X is S, Y is O, $R^5$, $R^6$, $R^7$ and $R^9$ are all Me, and $R^2$, $R^3$, $R^4$, $R^A$, $R^{10}$ and $R^{11}$ are all H;
compound 201: n is 0, X is S, Y is O, $R^5$, $R^6$, $R^7$ and $R^{10}$ are all Me, and $R^2$, $R^3$, $R^4$, $R^8$, $R^9$ and $R^{11}$ are all H;
compound 202: n is 0, X is S, Y is O, $R^5$, $R^6$, $R^7$ and $R^{11}$ are all Me, and $R^2$, $R^3$, $R^4$, $R^8$, $R^9$ and $R^{10}$ are all H;
compound 203: n is 0, X is S, Y is O, $R^5$, $R^6$, $R^7$, $R^8$ and $R^{11}$ are all Me, and $R^2$, $R^3$, $R^4$, $R^9$ and $R^{10}$ are all H;
compound 204: n is 0, X is S, Y is O, $R^5$, $R^6$, $R^7$, $R^9$ and $R^{11}$ are all Me, and $R^2$, $R^3$, $R^4$, $R^8$ and $R^{10}$ are all H;
compound 205: n is 0, X is S, Y is O, $R^5$ and $R^6$ are both Me, $R^7$ and $R^9$ are both $CF_3$, and $R^2$, $R^3$, $R^4$, $R^8$, $R^{10}$ and $R^{11}$ are all H;
compound 206: n is 0, X is S, Y is O, $R^5$ and $R^6$ are both Me, $R^8$ and $R^{10}$ are both $CF_3$, and $R^2$, $R^3$, $R^4$, $R^7$, $R^9$ and $R^{11}$ are all H;
compound 207: n is 0, X is S, Y is O, $R^5$ and $R^6$ are Me, $R^2$, $R^3$ and $R^4$ are H, and $R^7$, $R^8$, $R^9$, $R^{10}$ and $R^{11}$ are F;
compound 208: n is 0, X is S, Y is O, $R^5$ and $R^6$ are both Me, $R^7$ is $CH_3CH_2$, $R^2$, $R^3$, $R^4$, $R^8$, $R^9$, $R^{10}$ and $R^{11}$ are all H;
compound 209: n is 0, X is S, Y is O, $R^5$ and $R^6$ are both Me, $R^8$ is $CH_3CH_2$, $R^2$, $R^3$, $R^4$, $R^7$, $R^9$, $R^{10}$ and $R^{11}$ are all H;
compound 210: n is 0, X is S, Y is O, $R^5$ and $R^6$ are both Me, $R^9$ is $CH_3CH_2$, $R^2$, $R^3$, $R^4$, $R^7$, $R^8$, $R^{10}$ and $R^{11}$ are all H;
compound 211: n is 0, X is S, Y is O, $R^5$ and $R^6$ are both Me, $R^7$ is $CH(CH_3)_2$, $R^2$, $R^3$, $R^4$, $R^8$, $R^9$, $R^{10}$ and $R^{11}$ are all H;
compound 212: n is 0, X is S, Y is O, $R^5$ and $R^6$ are both Me, $R^8$ is $CH(CH_3)_2$, $R^2$, $R^3$, $R^4$, $R^7$, $R^9$, $R^{10}$ and $R^{11}$ are all H;
compound 213: n is 0, X is S, Y is O, $R^5$ and $R^6$ are both Me, $R^9$ is $CH(CH_3)_2$, $R^2$, $R^3$, $R^4$, $R^7$, $R^8$, $R^{10}$ and $R^{11}$ are all H;
compound 214: n is 0, X is S, Y is O, $R^5$ and $R^6$ are both Me, $R^7$ is $CH_3(CH_2)_2$, $R^2$, $R^3$, $R^4$, $R^8$, $R^9$, $R^{10}$ and $R^{11}$ are all H;
compound 215: n is 0, X is S, Y is O, $R^5$ and $R^6$ are both Me, $R^7$ is $C(CH_3)_3$, $R^2$, $R^3$, $R^4$, $R^8$, $R^9$, $R^{10}$ and $R^{11}$ are all H;
compound 216: n is 0, X is S, Y is O, $R^5$ and $R^6$ are both Me, $R^7$ is

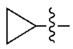,

$R^2$, $R^3$, $R^4$, $R^8$, $R^9$, $R^{10}$ and $R^{11}$ are all H;
compound 217: n is 0, X is S, Y is O, $R^5$ and $R^6$ are both Me, $R^7$ is

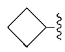

$R^2$, $R^3$, $R^4$, $R^8$, $R^9$, $R^{10}$ and $R^{11}$ are all H;
compound 218: n is 0, X is S, Y is O, $R^5$ and $R^6$ are both Me, $R^7$ is $CO_2CH_3$, $R^2$, $R^3$, $R^4$, $R^8$, $R^9$, $R^{10}$ and $R^{11}$ are all H;
compound 219: n is 0, X is S, Y is O, $R^5$ and $R^6$ are both Me, $R^7$ and $R^8$ are cyclized to form

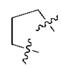

and $R^2$, $R^3$, $R^4$, $R^9$, $R^{10}$ and $R^{11}$ are all H;
compound 220: n is 0, X is S, Y is O, $R^5$ and $R^6$ are both Me, $R^8$ and $R^9$ are cyclized to form

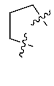,

and $R^2$, $R^3$, $R^4$, $R^7$, $R^{10}$ and $R^{11}$ are all H;
compound 221: n is 0, X is S, Y is O, $R^5$ and $R^6$ are both Me, $R^8$ and $R^9$ are cyclized to form

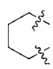,

, and $R^2$, $R^3$, $R^4$, $R^7$, $R^{10}$ and $R^{11}$ are all H;
compound 222: n is 0, X is S, Y is O, $R^5$ and $R^6$ are both Me, $R^7$ and $R^8$ are cyclized to form

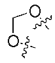,

and $R^2$, $R^3$, $R^4$, $R^9$, $R^{10}$ and $R^{11}$ are all H;
compound 223: n is 0, X is S, Y is O, $R^5$ and $R^6$ are both Me, $R^8$ and $R^9$ are cyclized to form

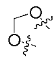,

and $R^2$, $R^3$, $R^4$, $R^7$, $R^{10}$ and $R^{11}$ are all H;
compound 224: n is 0, X is S, Y is O, $R^5$ and $R^6$ are both Me, $R^7$ and $R^8$ are cyclized to form

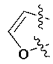,

and $R^2$, $R^3$, $R^4$, $R^9$, $R^{10}$ and $R^{11}$ are all H;
compound 225: n is 0, X is S, Y is O, $R^5$ and $R^6$ are both Me, $R^7$ and $R^8$ are cyclized to form

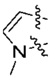,

and $R^2$, $R^3$, $R^4$, $R^9$, $R^{10}$ and $R^{11}$ are all H;
compound 226: n is 0, X is S, Y is O, $R^5$ and $R^6$ are both Me, $R^7$ and $R^8$ are cyclized to form

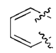,

and $R^2$, $R^3$, $R^4$, $R^9$, $R^{10}$ and $R^{11}$ are all H;
compound 227: n is 0, X is S, Y is O, $R^5$ and $R^6$ are both Me, $R^7$ and $R^8$ are cyclized to form

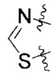,

and $R^2$, $R^3$, $R^4$, $R^9$, $R^{10}$ and $R^{11}$ are all H;
compound 228: n is 0, X is S, Y is O, $R^5$ and $R^6$ are both Me, $R^7$ and $R^8$ are cyclized to form

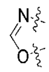,

and $R^2$, $R^3$, $R^4$, $R^9$, $R^{10}$ and $R^{11}$ are all H;
compound 229: n is 0, X is S, Y is O, $R^5$ and $R^6$ are both Me, $R^8$ and $R^9$ are cyclized to form

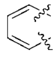,

and $R^2$, $R^3$, $R^4$, $R^7$, $R^{10}$ and $R^{11}$ are all H;
compound 230: n is 0, X is S, Y is $CH_2$, $R^6$ is Me, and $R^2$, $R^3$, $R^4$, $R^5$, $R^7$, $R^8$, $R^9$, $R^{10}$ and $R^{11}$ are all H;
compound 231: n is 0, X is S, Y is $CH_2$, $R^6$ is Me, $R^7$ is F, and $R^2$, $R^3$, $R^4$, $R^5$, $R^8$, $R^9$, $R^{10}$ and $R^{11}$ are all H;
compound 232: n is 0, X is S, Y is $CH_2$, $R^6$ is Me, $R^8$ is F, and $R^2$, $R^3$, $R^4$, $R^5$, $R^7$, $R^9$, $R^{10}$ and $R^{11}$ are all H;
compound 233: n is 0, X is S, Y is $CH_2$, $R^6$ is Me, $R^9$ is F, and $R^2$, $R^3$, $R^4$, $R^5$, $R^7$, $R^8$, $R^{10}$ and $R^{11}$ are all H;
compound 234: n is 0, X is S, Y is $CH_2$, $R^6$ is Me, $R^7$ is Cl, and $R^2$, $R^3$, $R^4$, $R^5$, $R^8$, $R^9$, $R^{10}$ and $R^{11}$ are all H;
compound 235: n is 0, X is S, Y is $CH_2$, $R^6$ is Me, $R^8$ is Cl, and $R^2$, $R^3$, $R^4$, $R^5$, $R^7$, $R^9$, $R^{10}$ and $R^{11}$ are all H;
compound 236: n is 0, X is S, Y is $CH_2$, $R^6$ is Me, $R^9$ is Cl, and $R^2$, $R^3$, $R^4$, $R^5$, $R^7$, $R^8$, $R^{10}$ and $R^{11}$ are all H;
compound 237: n is 0, X is S, Y is $CH_2$, $R^6$ is Me, $R^7$ is Br, and $R^2$, $R^3$, $R^4$, $R^5$, $R^8$, $R^9$, $R^{10}$ and $R^{11}$ are all H;

compound 238: n is 0, X is S, Y is $CH_2$, $R^6$ is Me, $R^8$ is Br, and $R^2$, $R^3$, $R^4$, $R^5$, $R^7$, $R^9$, $R^{10}$ and $R^{11}$ are all H;
compound 239: n is 0, X is S, Y is $CH_2$, $R^6$ is Me, $R^9$ is Br, and $R^2$, $R^3$, $R^4$, $R^5$, $R^7$, $R^8$, $R^{10}$ and $R^{11}$ are all H;
compound 240: n is 0, X is S, Y is $CH_2$, $R^6$ is Me, $R^7$ is I, and $R^2$, $R^3$, $R^4$, $R^5$, $R^8$, $R^9$, $R^{10}$ and $R^{11}$ are all H;
compound 241: n is 0, X is S, Y is $CH_2$, $R^6$ is Me, $R^8$ is I, and $R^2$, $R^3$, $R^4$, $R^5$, $R^7$, $R^9$, $R^{10}$ and $R^{11}$ are all H;
compound 242: n is 0, X is S, Y is $CH_2$, $R^6$ is Me, $R^9$ is I, and $R^2$, $R^3$, $R^4$, $R^5$, $R^7$, $R^8$, $R^{10}$ and $R^{11}$ are all H;
compound 243: n is 0, X is S, Y is $CH_2$, $R^6$ is Me, $R^7$ is $NO_2$, and $R^2$, $R^3$, $R^4$, $R^5$, $R^8$, $R^9$, $R^{10}$ and $R^{11}$ are all H;
compound 244: n is 0, X is S, Y is $CH_2$, $R^6$ is Me, $R^8$ is $NO_2$, and $R^2$, $R^3$, $R^4$, $R^5$, $R^7$, $R^9$, $R^{10}$ and $R^{11}$ are all H;
compound 245: n is 0, X is S, Y is $CH_2$, $R^6$ is Me, $R^9$ is $NO_2$, and $R^2$, $R^3$, $R^4$, $R^5$, $R^7$, $R^8$, $R^{10}$ and $R^{11}$ are all H;
compound 246: n is 0, X is S, Y is $CH_2$, $R^6$ is Me, $R^7$ is $CF_3$, and $R^2$, $R^3$, $R^4$, $R^5$, $R^8$, $R^9$, $R^{10}$ and $R^{11}$ are all H;
compound 247: n is 0, X is S, Y is $CH_2$, $R^6$ is Me, $R^8$ is $CF_3$, and $R^2$, $R^3$, $R^4$, $R^5$, $R^7$, $R^9$, $R^{10}$ and $R^{11}$ are all H;
compound 248: n is 0, X is S, Y is $CH_2$, $R^6$ is Me, $R^9$ is $CF_3$, and $R^2$, $R^3$, $R^4$, $R^5$, $R^7$, $R^8$, $R^{10}$ and $R^{11}$ are all H;
compound 249: n is 0, X is S, Y is $CH_2$, $R^6$ is Me, $R^7$ is $OCF_3$, and $R^2$, $R^3$, $R^4$, $R^5$, $R^8$, $R^9$, $R^{10}$ and $R^{11}$ are all H;
compound 250: n is 0, X is S, Y is $CH_2$, $R^6$ is Me, $R^8$ is $OCF_3$, and $R^2$, $R^3$, $R^4$, $R^5$, $R^7$, $R^9$, $R^{10}$ and $R^{11}$ are all H;
compound 251: n is 0, X is S, Y is $CH_2$, $R^6$ is Me, $R^9$ is $OCF_3$, and $R^2$, $R^3$, $R^4$, $R^5$, $R^7$, $R^8$, $R^{10}$ and $R^{11}$ are all H;
compound 252: n is 0, X is S, Y is $CH_2$, $R^6$ is Me, $R^7$ is $OCHF_2$, and $R^2$, $R^3$, $R^4$, $R^5$, $R^8$, $R^9$, $R^{10}$ and $R^{11}$ are all H;
compound 253: n is 0, X is S, Y is $CH_2$, $R^6$ is Me, $R^8$ is $OCHF_2$, and $R^2$, $R^3$, $R^4$, $R^5$, $R^7$, $R^9$, $R^{10}$ and $R^{11}$ are all H;
compound 254: n is 0, X is S, Y is $CH_2$, $R^6$ is Me, $R^9$ is $OCHF_2$, and $R^2$, $R^3$, $R^4$, $R^5$, $R^7$, $R^8$, $R^{10}$ and $R^{11}$ are all H;
compound 255: n is 0, X is S, Y is $CH_2$, $R^6$ is Me, $R^9$ is $SCF_3$, and $R^2$, $R^3$, $R^4$, $R^5$, $R^7$, $R^8$, $R^{10}$ and $R^{11}$ are all H;
compound 256: n is 0, X is S, Y is $CH_2$, $R^6$ is Me, $R^7$ is $OCH_3$, and $R^2$, $R^3$, $R^4$, $R^5$, $R^8$, $R^9$, $R^{10}$ and $R^{11}$ are all H;
compound 257: n is 0, X is S, Y is $CH_2$, $R^6$ is Me, $R^8$ is $OCH_3$, and $R^2$, $R^3$, $R^4$, $R^5$, $R^7$, $R^9$, $R^{10}$ and $R^{11}$ are all H;
compound 258: n is 0, X is S, Y is $CH_2$, $R^6$ is Me, $R^9$ is $OCH_3$, and $R^2$, $R^3$, $R^4$, $R^5$, $R^7$, $R^8$, $R^{10}$ and $R^{11}$ are all H;
compound 259: n is 0, X is S, Y is $CH_2$, $R^6$ is Me, $R^9$ is $OC_2H_5$, $R^2$, $R^3$, $R^4$, $R^5$, $R^7$, $R^8$, $R^{10}$ and $R^{11}$ are all H;
compound 260: n is 0, X is S, Y is $CH_2$, $R^6$ is Me, $R^7$ is CN, and $R^2$, $R^3$, $R^4$, $R^5$, $R^8$, $R^9$, $R^{10}$ and $R^{11}$ are all H;
compound 261: n is 0, X is S, Y is $CH_2$, $R^6$ is Me, $R^8$ is CN, and $R^2$, $R^3$, $R^4$, $R^5$, $R^7$, $R^9$, $R^{10}$ and $R^{11}$ are all H;
compound 262: n is 0, X is S, Y is $CH_2$, $R^6$ is Me, $R^9$ is CN, and $R^2$, $R^3$, $R^4$, $R^5$, $R^7$, $R^8$, $R^{10}$ and $R^{11}$ are all H;
compound 263: n is 0, X is S, Y is $CH_2$, $R^6$ and $R^7$ are Me, and $R^2$, $R^3$, $R^4$, $R^5$, $R^8$, $R^9$, $R^{10}$ and $R^{11}$ are all H;
compound 264: n is 0, X is S, Y is $CH_2$, $R^6$ and $R^8$ are Me, and $R^2$, $R^3$, $R^4$, $R^5$, $R^7$, $R^9$, $R^{10}$ and $R^{11}$ are all H;
compound 265: n is 0, X is S, Y is $CH_2$, $R^6$ and $R^9$ are Me, and $R^2$, $R^3$, $R^4$, $R^5$, $R^7$, $R^8$, $R^{10}$ and $R^{11}$ are all H;
compound 266: n is 0, X is S, Y is $CH_2$, $R^6$ is Me, $R^7$ is $CH_3SO_2$, $R^2$, $R^3$, $R^4$, $R^5$, $R^8$, $R^9$, $R^{10}$ and $R^{11}$ are all H;
compound 267: n is 0, X is S, Y is $CH_2$, $R^6$ is Me, $R^8$ is $CH_3SO_2$, $R^2$, $R^3$, $R^4$, $R^5$, $R^7$, $R^9$, $R^{10}$ and $R^{11}$ are all H;
compound 268: n is 0, X is S, Y is $CH_2$, $R^6$ is Me, $R^9$ is $CH_3SO_2$, $R^2$, $R^3$, $R^4$, $R^5$, $R^7$, $R^8$, $R^{10}$ and $R^{11}$ are all H;
compound 269: n is 0, X is S, Y is $CH_2$, $R^6$ is Me, $R^7$ is $C_2H_5SO_2$, $R^2$, $R^3$, $R^4$, $R^5$, $R^8$, $R^9$, $R^{10}$ and $R^{11}$ are all H;
compound 270: n is 0, X is S, Y is $CH_2$, $R^6$ is Me, $R^7$ and $R^8$ are both Cl, and $R^2$, $R^3$, $R^4$, $R^5$, $R^9$, $R^{10}$ and $R^{11}$ are all H;
compound 271: n is 0, X is S, Y is $CH_2$, $R^6$ is Me, $R^7$ and $R^9$ are both Cl, and $R^2$, $R^3$, $R^4$, $R^5$, $R^8$, $R^{10}$ and $R^{11}$ are all H;
compound 272: n is 0, X is S, Y is $CH_2$, $R^6$ is Me, $R^7$ and $R^{10}$ are both Cl, and $R^2$, $R^3$, $R^4$, $R^5$, $R^8$, $R^9$ and $R^{11}$ are all H;
compound 273: n is 0, X is S, Y is $CH_2$, $R^6$ is Me, $R^7$ and $R^{11}$ are both Cl, and $R^2$, $R^3$, $R^4$, $R^5$, $R^8$, $R^9$ and $R^{10}$ are all H;
compound 274: n is 0, X is S, Y is $CH_2$, $R^6$ is Me, $R^8$ and $R^9$ are both Cl, and $R^2$, $R^3$, $R^4$, $R^5$, $R^7$, $R^{10}$ and $R^{11}$ are all H;
compound 275: n is 0, X is S, Y is $CH_2$, $R^6$ and $R^7$ are Me, $R^9$ is Cl, and $R^2$, $R^3$, $R^4$, $R^5$, $R^8$, $R^{10}$ and $R^{11}$ are all H;
compound 276: n is 0, X is S, Y is $CH_2$, $R^6$, $R^7$ and $R^8$ are all Me, and $R^2$, $R^3$, $R^4$, $R^5$, $R^9$, $R^{10}$ and $R^{11}$ are all H;
compound 277: n is 0, X is S, Y is $CH_2$, $R^6$, $R^7$ and $R^9$ are all Me, and $R^2$, $R^3$, $R^4$, $R^5$, $R^8$, $R^{10}$ and $R^{11}$ are all H;
compound 278: n is 0, X is S, Y is $CH_2$, $R^6$, $R^7$ and $R^{10}$ are all Me, and $R^2$, $R^3$, $R^4$, $R^5$, $R^8$, $R^9$ and $R^{11}$ are all H;
compound 279: n is 0, X is S, Y is $CH_2$, $R^6$, $R^7$ and $R^{11}$ are all Me, and $R^2$, $R^3$, $R^4$, $R^5$, $R^8$, $R^9$ and $R^{10}$ are all H;
compound 280: n is 0, X is S, Y is $CH_2$, $R^6$, $R^7$, $R^8$ and $R^{11}$ are all Me, and $R^2$, $R^3$, $R^4$, $R^5$, $R^9$ and $R^{10}$ are all H;
compound 281: n is 0, X is S, Y is $CH_2$, $R^6$, $R^7$, $R^9$ and $R^{11}$ are all Me, and $R^2$, $R^3$, $R^4$, $R^5$, $R^8$ and $R^{10}$ are all H;
compound 282: n is 0, X is S, Y is $CH_2$, $R^6$ is Me, $R^7$ and $R^9$ are both $CF_3$, and $R^2$, $R^3$, $R^4$, $R^5$, $R^8$, $R^{10}$ and $R^{11}$ are all H;
compound 283: n is 0, X is S, Y is $CH_2$, $R^6$ is Me, $R^8$ and $R^{10}$ are both $CF_3$, and $R^2$, $R^3$, $R^4$, $R^5$, $R^7$, $R^9$ and $R^{11}$ are all H;
compound 284: n is 0, X is S, Y is $CH_2$, $R^6$ is Me, $R^2$, $R^3$, $R^4$ and $R^5$ are all H, and $R^7$, $R^8$, $R^9$, $R^{10}$ and $R^{11}$ are all F;
compound 285: n is 0, X is S, Y is $CH_2$, $R^6$ is Me, $R^7$ is $CH_3CH_2$, $R^2$, $R^3$, $R^4$, $R^5$, $R^8$, $R^9$, $R^{10}$ and $R^{11}$ are all H;
compound 286: n is 0, X is S, Y is $CH_2$, $R^6$ is Me, $R^8$ is $CH_3CH_2$, $R^2$, $R^3$, $R^4$, $R^5$, $R^7$, $R^9$, $R^{10}$ and $R^{11}$ are all H;
compound 287: n is 0, X is S, Y is $CH_2$, $R^6$ is Me, $R^9$ is $CH_3CH_2$, $R^2$, $R^3$, $R^4$, $R^5$, $R^7$, $R^8$, $R^{10}$ and $R^{11}$ are all H;
compound 288: n is 0, X is S, Y is $CH_2$, $R^6$ is Me, $R^7$ is $CH(CH_3)_2$, $R^2$, $R^3$, $R^4$, $R^5$, $R^8$, $R^9$, $R^{10}$ and $R^{11}$ are all H;
compound 289: n is 0, X is S, Y is $CH_2$, $R^6$ is Me, $R^8$ is $CH(CH_3)_2$, $R^2$, $R^3$, $R^4$, $R^5$, $R^7$, $R^9$, $R^{10}$ and $R^{11}$ are all H;
compound 290: n is 0, X is S, Y is $CH_2$, $R^6$ is Me, $R^9$ is $CH(CH_3)_2$, $R^2$, $R^3$, $R^4$, $R^5$, $R^7$, $R^8$, $R^{10}$ and $R^{11}$ are all H;
compound 291: n is 0, X is S, Y is $CH_2$, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;
Compound 292: n is 0, X is S, Y is $CH_2$, $R^6$ is Me, $R^7$ is $C(CH_3)_3$, $R^2$, $R^3$, $R^4$, $R^5$, $R^8$, $R^9$, $R^{10}$ and $R^{11}$ are all H;
compound 293: n is 0, X is S, Y is $CH_2$, $R^6$ is Me, $R^7$ is

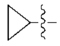

, $R^2$, $R^3$, $R^4$, $R^5$, $R^8$, $R^9$, $R^{10}$ and $R^{11}$ are all H;
compound 294: n is 0, X is S, Y is $CH_2$, $R^6$ is Me, $R^7$ is

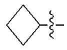

$R^2$, $R^3$, $R^4$, $R^5$, $R^8$, $R^9$, $R^{10}$ and $R^{11}$ are all H;
compound 295: n is 0, X is S, Y is $CH_2$, $R^6$ is Me, $R^7$ is $CO_2CH_3$, $R^2$, $R^3$, $R^4$, $R^5$, $R^8$, $R^9$, $R^{10}$ and $R^{11}$ are all H;
compound 296: n is 0, X is S, Y is $CH_2$, $R^6$ is Me, $R^7$ and $R^8$ are cyclized to form

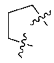,

and $R^2$, $R^3$, $R^4$, $R^5$, $R^9$, $R^{10}$ and $R^{11}$ are all H;
compound 297: n is 0, X is S, Y is $CH_2$, $R^6$ is Me, $R^8$ and $R^9$ are cyclized to form

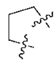,

and $R^2$, $R^3$, $R^4$, $R^5$, $R^7$, $R^{10}$ and $R^{11}$ are all H;
compound 298: n is 0, X is S, Y is $CH_2$, $R^6$ is Me, $R^8$ and $R^9$ are cyclized to form

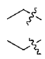, ;

and $R^2$, $R^3$, $R^4$, $R^5$, $R^7$, $R^{10}$ and $R^{11}$ are all H;
compound 299: n is 0, X is S, Y is $CH_2$, $R^6$ is Me, $R^7$ and $R^8$ are cyclized to form

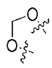, ;

and $R^2$, $R^3$, $R^4$, $R^5$, $R^9$, $R^{10}$ and $R^{11}$ are all H;
compound 300: n is 0, X is S, Y is $CH_2$, $R^6$ is Me, $R^8$ and $R^9$ are cyclized to form

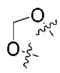,

and $R^2$, $R^3$, $R^4$, $R^5$, $R^7$, $R^{10}$ and $R^{11}$ are all H;
compound 301: n is 0, X is S, Y is $CH_2$, $R^6$ is Me, $R^7$ and $R^8$ are cyclized to form

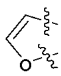,

and $R^2$, $R^3$, $R^4$, $R^5$, $R^9$, $R^{10}$ and $R^{11}$ are all H;
compound 302: n is 0, X is S, Y is $CH_2$, $R^6$ is Me, $R^7$ and $R^8$ are cyclized to form

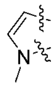,

and $R^2$, $R^3$, $R^4$, $R^5$, $R^9$, $R^{10}$ and $R^{11}$ are all H;
compound 303: n is 0, X is S, Y is $CH_2$, $R^6$ is Me, $R^7$ and $R^8$ are cyclized to form

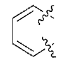,

and $R^2$, $R^3$, $R^4$, $R^5$, $R^9$, $R^{10}$ and $R^{11}$ are all H;
compound 304: n is 0, X is S, Y is $CH_2$, $R^6$ is Me, $R^7$ and $R^8$ are cyclized to form

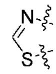,

and $R^2$, $R^3$, $R^4$, $R^5$, $R^9$, $R^{10}$ and $R^{11}$ are all H;
compound 305: n is 0, X is S, Y is $CH_2$, $R^6$ is Me, $R^7$ and $R^8$ are cyclized to form

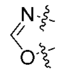,

and $R^2$, $R^3$, $R^4$, $R^3$, $R^9$, $R^{10}$ and $R^{11}$ are all H;
compound 306: n is 0, X is S, Y is $CH_2$, $R^6$ is Me, $R^8$ and $R^9$ are cyclized to form

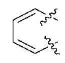,

and $R^2$, $R^3$, $R^4$, $R^5$, $R^7$, $R^{10}$ and $R^{11}$ are all H;
compound 307: n is 0, X is S, Y is NMe, $R^6$ is Me, $R^2$, $R^3$, $R^4$, $R^5$, $R^7$, $R^8$, $R^9$, $R^{10}$ and $R^{11}$ are all H;
compound 308: n is 0, X is S, Y is NMe, $R^6$ is Me, $R^7$ is F, and $R^2$, $R^3$, $R^4$, $R^5$, $R^8$, $R^9$, $R^{10}$ and $R^{11}$ are all H;
compound 309: n is 0, X is S, Y is NMe, $R^6$ is Me, $R^8$ is F, and $R^2$, $R^3$, $R^4$, $R^5$, $R^7$, $R^9$, $R^{10}$ and $R^{11}$ are all H;
compound 310: n is 0, X is S, Y is NMe, $R^6$ is Me, $R^9$ is F, and $R^2$, $R^3$, $R^4$, $R^5$, $R^7$, $R^8$, $R^{10}$ and $R^{11}$ are all H;
compound 311: n is 0, X is S, Y is NMe, $R^6$ is Me, $R^7$ is Cl, and $R^2$, $R^3$, $R^4$, $R^5$, $R^8$, $R^9$, $R^{10}$ and $R^{11}$ are all H;
compound 312: n is 0, X is S, Y is NMe, $R^6$ is Me, $R^8$ is Cl, and $R^2$, $R^3$, $R^4$, $R^5$, $R^7$, $R^9$, $R^{10}$ and $R^{11}$ are all H;
compound 313: n is 0, X is S, Y is NMe, $R^6$ is Me, $R^9$ is Cl, and $R^2$, $R^3$, $R^4$, $R^5$, $R^7$, $R^8$, $R^{10}$ and $R^{11}$ are all H;
compound 314: n is 0, X is S, Y is NMe, $R^6$ is Me, $R^7$ is Br, and $R^2$, $R^3$, $R^4$, $R^5$, $R^8$, $R^9$, $R^{10}$ and $R^{11}$ are all H;
compound 315: n is 0, X is S, Y is NMe, $R^6$ is Me, $R^8$ is Br, and $R^2$, $R^3$, $R^4$, $R^5$, $R^7$, $R^9$, $R^{10}$ and $R^{11}$ are all H;
compound 316: n is 0, X is S, Y is NMe, $R^6$ is Me, $R^9$ is Br, and $R^2$, $R^3$, $R^4$, $R^5$, $R^7$, $R^8$, $R^{10}$ and $R^{11}$ are all H;
compound 317: n is 0, X is S, Y is NMe, $R^6$ is Me, $R^7$ is I, and $R^2$, $R^3$, $R^4$, $R^5$, $R^8$, $R^9$, $R^{10}$ and $R^{11}$ are all H;
compound 318: n is 0, X is S, Y is NMe, $R^6$ is Me, $R^8$ is I, and $R^2$, $R^3$, $R^4$, $R^5$, $R^7$, $R^9$, $R^{10}$ and $R^{11}$ are all H;
compound 319: n is 0, X is S, Y is NMe, $R^6$ is Me, $R^9$ is I, and $R^2$, $R^3$, $R^4$, $R^5$, $R^7$, $R^8$, $R^{10}$ and $R^{11}$ are all H;
compound 320: n is 0, X is S, Y is NMe, $R^6$ is Me, $R^7$ is $NO_2$, and $R^2$, $R^3$, $R^4$, $R^5$, $R^8$, $R^9$, $R^{10}$ and $R^{11}$ are all H;
compound 321: n is 0, X is S, Y is NMe, $R^6$ is Me, $R^8$ is $NO_2$, and $R^2$, $R^3$, $R^4$, $R^5$, $R^7$, $R^9$, $R^{10}$ and $R^{11}$ are all H;
compound 322: n is 0, X is S, Y is NMe, $R^6$ is Me, $R^9$ is $NO_2$, and $R^2$, $R^3$, $R^4$, $R^5$, $R^7$, $R^8$, $R^{10}$ and $R^{11}$ are all H;
compound 323: n is 0, X is S, Y is NMe, $R^6$ is Me, $R^7$ is $CF_3$, and $R^2$, $R^3$, $R^4$, $R^5$, $R^8$, $R^9$, $R^{10}$ and $R^{11}$ are all H;
compound 324: n is 0, X is S, Y is NMe, $R^6$ is Me, $R^8$ is $CF_3$, and $R^2$, $R^3$, $R^4$, $R^5$, $R^7$, $R^9$, $R^{10}$ and $R^{11}$ are all H;
compound 325: n is 0, X is S, Y is NMe, $R^6$ is Me, $R^9$ is $CF_3$, and $R^2$, $R^3$, $R^4$, $R^5$, $R^7$, $R^8$, $R^{10}$ and $R^{11}$ are all H;
compound 326: n is 0, X is S, Y is NMe, $R^6$ is Me, $R^7$ is $OCF_3$, and $R^2$, $R^3$, $R^4$, $R^5$, $R^8$, $R^9$, $R^{10}$ and $R^{11}$ are all H;
compound 327: n is 0, X is S, Y is NMe, $R^6$ is Me, $R^8$ is $OCF_3$, and $R^2$, $R^3$, $R^4$, $R^5$, $R^7$, $R^9$, $R^{10}$ and $R^{11}$ are all H;
compound 328: n is 0, X is S, Y is NMe, $R^6$ is Me, $R^9$ is $OCF_3$, and $R^2$, $R^3$, $R^4$, $R^5$, $R^7$, $R^8$, $R^{10}$ and $R^{11}$ are all H;
compound 329: n is 0, X is S, Y is NMe, $R^6$ is Me, $R^7$ is $OCHF_2$, and $R^2$, $R^3$, $R^4$, $R^5$, $R^8$, $R^9$, $R^{10}$ and $R^{11}$ are all H;
compound 330: n is 0, X is S, Y is NMe, $R^6$ is Me, $R^8$ is $OCHF_2$, and $R^2$, $R^3$, $R^4$, $R^5$, $R^7$, $R^9$, $R^{10}$ and $R^{11}$ are all H;
compound 331: n is 0, X is S, Y is NMe, $R^6$ is Me, $R^9$ is $OCHF_2$, and $R^2$, $R^3$, $R^4$, $R^5$, $R^7$, $R^8$, $R^{10}$ and $R^{11}$ are all H;
compound 332: n is 0, X is S, Y is NMe, $R^6$ is Me, $R^9$ is $SCF_3$, and $R^2$, $R^3$, $R^4$, $R^5$, $R^7$, $R^8$, $R^{10}$ and $R^{11}$ are all H;
compound 333: n is 0, X is S, Y is NMe, $R^6$ is Me, $R^7$ is $OCH_3$, and $R^2$, $R^3$, $R^4$, $R^5$, $R^8$, $R^9$, $R^{10}$ and $R^{11}$ are all H;
compound 334: n is 0, X is S, Y is NMe, $R^6$ is Me, $R^8$ is $OCH_3$, and $R^2$, $R^3$, $R^4$, $R^5$, $R^7$, $R^9$, $R^{10}$ and $R^{11}$ are all H;
compound 335: n is 0, X is S, Y is NMe, $R^6$ is Me, $R^9$ is $OCH_3$, and $R^2$, $R^3$, $R^4$, $R^5$, $R^7$, $R^8$, $R^{10}$ and $R^{11}$ are all H;
compound 336: n is 0, X is S, Y is NMe, $R^6$ is Me, $R^9$ is $OC_2H_5$, and $R^2$, $R^3$, $R^4$, $R^5$, $R^7$, $R^8$, $R^{10}$ and $R^{11}$ are all H;
compound 337: n is 0, X is S, Y is NMe, $R^6$ is Me, $R^7$ is CN, and $R^2$, $R^3$, $R^4$, $R^5$, $R^8$, $R^9$, $R^{10}$ and $R^{11}$ are all H;
compound 338: n is 0, X is S, Y is NMe, $R^6$ is Me, $R^8$ is CN, and $R^2$, $R^3$, $R^4$, $R^s$, $R^7$, $R^9$, $R^{10}$ and $R^{11}$ are all H;
compound 339: n is 0, X is S, Y is NMe, $R^6$ is Me, $R^9$ is CN, and $R^2$, $R^3$, $R^4$, $R^s$, $R^7$, $R^8$, $R^{10}$ and $R^{11}$ are all H;
compound 340: n is 0, X is S, Y is NMe, $R^6$ is Me, $R^7$ is $CH_3$, and $R^2$, $R^3$, $R^4$, $R^5$, $R^8$, $R^9$, $R^{10}$ and $R^{11}$ are all H;

compound 341: n is 0, X is S, Y is NMe, $R^6$ is Me, $R^8$ is $CH_3$, and $R^2$, $R^3$, $R^4$, $R^5$, $R^7$, $R^9$, $R^{10}$ and $R^{11}$ are all H;
compound 342: n is 0, X is S, Y is NMe, $R^6$ is Me, $R^9$ is $CH_3$, and $R^2$, $R^3$, $R^4$, $R^5$, $R^7$, $R^8$, $R^{10}$ and $R^{11}$ are all H;
compound 343: n is 0, X is S, Y is NMe, $R^6$ is Me, $R^7$ is $CH_3SO_2$, and $R^2$, $R^3$, $R^4$, $R^5$, $R^8$, $R^9$, $R^{10}$ and $R^{11}$ are all H;
compound 344: n is 0, X is S, Y is NMe, $R^6$ is Me, $R^8$ is $CH_3SO_2$, and $R^2$, $R^3$, $R^4$, $R^5$, $R^7$, $R^9$, $R^{10}$ and $R^{11}$ are all H;
compound 345: n is 0, X is S, Y is NMe, $R^6$ is Me, $R^9$ is $CH_3SO_2$, and $R^2$, $R^3$, $R^4$, $R^5$, $R^7$, $R^8$, $R^{10}$ and $R^{11}$ are all H;
compound 346: n is 0, X is S, Y is NMe, $R^6$ is Me, $R^7$ is $C_2H_5SO_2$, $R^2$, $R^3$, $R^4$, $R^5$, $R^8$, $R^9$, $R^{10}$ and $R^{11}$ are all H;
compound 347: n is 0, X is S, Y is NMe, $R^6$ is Me, $R^7$ and $R^8$ are both Cl, and $R^2$, $R^3$, $R^4$, $R^5$, $R^9$, $R^{10}$ and $R^{11}$ are all H;
compound 348: n is 0, X is S, Y is NMe, $R^6$ is Me, $R^7$ and $R^9$ are both Cl, and $R^2$, $R^3$, $R^4$, $R^5$, $R^8$, $R^{10}$ and $R^{11}$ are all H;
compound 349: n is 0, X is S, Y is NMe, $R^6$ is Me, $R^7$ and $R^{10}$ are both Cl, and $R^2$, $R^3$, $R^4$, $R^5$, $R^8$, $R^9$ and $R^{11}$ are all H;
compound 350: n is 0, X is S, Y is NMe, $R^6$ is Me, $R^7$ and $R^{11}$ are both Cl, and $R^2$, $R^3$, $R^4$, $R^5$, $R^8$, $R^9$ and $R^{10}$ are all H;
compound 351: n is 0, X is S, Y is NMe, $R^6$ is Me, $R^8$ and $R^9$ are both Cl, and $R^2$, $R^3$, $R^4$, $R^5$, $R^7$, $R^{10}$ and $R^{11}$ are all H;
compound 352: n is 0, X is S, Y is NMe, $R^6$ and $R^7$ are Me, $R^9$ is Cl, and $R^2$, $R^3$, $R^4$, $R^5$, $R^8$, $R^{10}$ and $R^{11}$ are all H;
compound 353: n is 0, X is S, Y is NMe, $R^6$, $R^7$ and $R^8$ are Me, and $R^2$, $R^3$, $R^4$, $R^5$, $R^9$, $R^{10}$ and $R^{11}$ are all H;
compound 354: n is 0, X is S, Y is NMe, $R^6$, $R^7$ and $R^9$ are Me, and $R^2$, $R^3$, $R^4$, $R^5$, $R^8$, $R^{10}$ and $R^{11}$ are all H;
compound 355: n is 0, X is S, Y is NMe, $R^6$, $R^7$ and $R^{10}$ are Me, and $R^2$, $R^3$, $R^4$, $R^5$, $R^8$, $R^9$ and $R^{11}$ are all H;
compound 356: n is 0, X is S, Y is NMe, $R^6$, $R^7$ and $R^{11}$ are Me, and $R^2$, $R^3$, $R^4$, $R^5$, $R^8$, $R^9$ and $R^{10}$ are all H;
compound 357: n is 0, X is S, Y is NMe, $R^6$, $R^7$, $R^8$ and $R^{11}$ are Me, and $R^2$, $R^3$, $R^4$, $R^5$, $R^9$ and $R^{10}$ are all H;
compound 358: n is 0, X is S, Y is NMe, $R^6$, $R^7$, $R^9$ and $R^{11}$ are Me, and $R^2$, $R^3$, $R^4$, $R^5$, $R^8$ and $R^{10}$ are all H;
compound 359: n is 0, X is S, Y is NMe, $R^6$ is Me, $R^7$ and $R^9$ are both $CF_3$, $R^2$, $R^3$, $R^4$, $R^5$, $R^8$, $R^{10}$ and $R^{11}$ are all H;
compound 360: n is 0, X is S, Y is NMe, $R^6$ is Me, $R^8$ and $R^{10}$ are both $CF_3$, $R^2$, $R^3$, $R^4$, $R^5$, $R^7$, $R^9$ and $R^{11}$ are all H;
compound 361: n is 0, X is S, Y is NMe, $R^6$ is Me, $R^2$, $R^3$, $R^4$ and $R^5$ are all H, and $R^7$, $R^8$, $R^9$, $R^{10}$ and $R^{11}$ are all F;
compound 362: n is 0, X is S, Y is NMe, $R^6$ is Me, $R^7$ is $CH_3CH_2$, and $R^2$, $R^3$, $R^4$, $R^5$, $R^8$, $R^9$, $R^{10}$ and $R^{11}$ are all H;
compound 363: n is 0, X is S, Y is NMe, $R^6$ is Me, $R^8$ is $CH_3CH_2$, and $R^2$, $R^3$, $R^4$, $R^s$, $R^7$, $R^9$, $R^{10}$ and $R^{11}$ are all H;
compound 364: n is 0, X is S, Y is NMe, $R^6$ is Me, $R^9$ is $CH_3CH_2$, and $R^2$, $R^3$, $R^4$, $R^s$, $R^7$, $R^8$, $R^{10}$ and $R^{11}$ are all H;
compound 365: n is 0, X is S, Y is NMe, $R^6$ is Me, $R^7$ is $CH(CH_3)_2$, $R^2$, $R^3$, $R^4$, $R^5$, $R^8$, $R^9$, $R^{10}$ and $R^{11}$ are all H;
compound 366: n is 0, X is S, Y is NMe, $R^6$ is Me, $R^8$ is $CH(CH_3)_2$, $R^2$, $R^3$, $R^4$, $R^5$, $R^7$, $R^9$, $R^{10}$ and $R^{11}$ are all H;
compound 367: n is 0, X is S, Y is NMe, $R^6$ is Me, $R^9$ is $CH(CH_3)_2$, $R^2$, $R^3$, $R^4$, $R^5$, $R^7$, $R^8$, $R^{10}$ and $R^{11}$ are all H;
compound 368: n is 0, X is S, Y is NMe, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;
compound 369: n is 0, X is S, Y is NMe, $R^6$ is Me, $R^7$ is $C(CH_3)_3$, $R^2$, $R^3$, $R^4$, $R^5$, $R^8$, $R^9$, $R^{10}$ and $R^{11}$ are all H;
compound 370: n is 0, X is S, Y is NMe, $R^6$ is Me, $R^7$ is

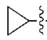,

and $R^2$, $R^3$, $R^4$, $R^5$, $R^8$, $R^9$, $R^{10}$ and $R^{11}$ are all H;
compound 371: n is 0, X is S, Y is NMe, $R^6$ is Me, $R^7$ is

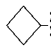,

and $R^2$, $R^3$, $R^4$, $R^5$, $R^8$, $R^9$, $R^{10}$ and $R^{11}$ are all H;
compound 372: n is 0, X is S, Y is NMe, $R^6$ is Me, $R^7$ is $CO_2CH_3$, and $R^2$, $R^3$, $R^4$, $R^5$, $R^8$, $R^9$, $R^{10}$ and $R^{11}$ are all H;
compound 373: n is 0, X is S, Y is NMe, $R^6$ is Me, $R^7$ and $R^8$ are cyclized to form

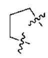,

and $R^2$, $R^3$, $R^4$, $R^5$, $R^9$, $R^{10}$ and $R^{11}$ are all H;
compound 374: n is 0, X is S, Y is NMe, $R^6$ is Me, $R^8$ and $R^9$ are cyclized to form

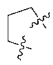,

and $R^2$, $R^3$, $R^4$, $R^5$, $R^7$, $R^{10}$ and $R^{11}$ are all H;
compound 375: n is 0, X is S, Y is NMe, $R^6$ is Me, $R^8$ and $R^9$ are cyclized to form

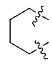,

and $R^2$, $R^3$, $R^4$, $R^5$, $R^7$, $R^{10}$ and $R^{11}$ are all H;
compound 376: n is 0, X is S, Y is NMe, $R^6$ is Me, $R^7$ and $R^8$ are cyclized to form

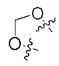,

and $R^2$, $R^3$, $R^4$, $R^5$, $R^9$, $R^{10}$ and $R^{11}$ are all H;
compound 377: n is 0, X is S, Y is NMe, $R^6$ is Me, $R^8$ and $R^9$ are cyclized to form

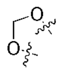,

and $R^2$, $R^3$, $R^4$, $R^5$, $R^7$, $R^{10}$ and $R^{11}$ are all H;
compound 378: n is 0, X is S, Y is NMe, $R^6$ is Me, $R^7$ and $R^8$ are cyclized to form

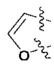,

and $R^2$, $R^3$, $R^4$, $R^5$, $R^9$, $R^{10}$ and $R^{11}$ are all H;
compound 379: n is 0, X is S, Y is NMe, $R^6$ is Me, $R^7$ and $R^8$ are cyclized to form

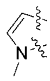,

and $R^2$, $R^3$, $R^4$, $R^5$, $R^9$, $R^{10}$ and $R^{11}$ are all H;
compound 380: n is 0, X is S, Y is NMe, $R^6$ is Me, $R^7$ and $R^8$ are cyclized to form

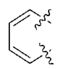,

and $R^2$, $R^3$, $R^4$, $R^5$, $R^9$, $R^{10}$ and $R^{11}$ are all H;
compound 381: n is 0, X is S, Y is NMe, $R^6$ is Me, $R^7$ and $R^8$ are cyclized to form

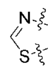,

and $R^2$, $R^3$, $R^4$, $R^5$, $R^9$, $R^{10}$ and $R^{11}$ are all H;
compound 382: n is 0, X is S, Y is NMe, $R^6$ is Me, $R^7$ and $R^8$ are cyclized to form

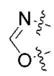

, and $R^2$, $R^3$, $R^4$, $R^5$, $R^9$, $R^{10}$ and $R^{11}$ are all H;
compound 383: n is 0, X is S, Y is NMe, $R^6$ is Me, $R^8$ and $R^9$ are cyclized to form

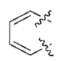,

and $R^2$, $R^3$, $R^4$, $R^5$, $R^7$, $R^{10}$ and $R^{11}$ are all H;

compound 873: n is 0, X is S, Y is NH, $R^6$ is Me, and $R^2$, $R^3$, $R^4$, $R^5$, $R^7$, $R^8$, $R^9$, $R^{10}$ and $R^{11}$ are all H;

compound 883: n is 0, X is S, Y is S, $R^6$ is Me, $R^2$, $R^3$, $R^4$, $R^5$, $R^7$, $R^8$, $R^9$, $R^{10}$ and $R^{11}$ are all H;

compound 384: n is 0, X is S, Y is O, $R^2$ and $R^6$ are Me, and $R^3$, $R^4$, $R^5$, $R^7$, $R^8$, $R^9$, $R^{10}$ and $R^{11}$ are all H;

compound 385: n is 0, X is S, Y is O, $R^2$ and $R^6$ are Me, $R^7$ is F, and $R^3$, $R^4$, $R^5$, $R^8$, $R^9$, $R^{10}$ and $R^{11}$ are all H;

compound 386: n is 0, X is S, Y is O, $R^2$ and $R^6$ are Me, $R^8$ is F, and $R^3$, $R^4$, $R^5$, $R^7$, $R^9$, $R^{10}$ and $R^{11}$ are all H;

compound 387: n is 0, X is S, Y is O, $R^2$ and $R^6$ are Me, $R^9$ is F, and $R^3$, $R^4$, $R^5$, $R^7$, $R^8$, $R^{10}$ and $R^{11}$ are all H;

compound 388: n is 0, X is S, Y is O, $R^2$ and $R^6$ are Me, $R^7$ is Cl, and $R^3$, $R^4$, $R^5$, $R^8$, $R^9$, $R^{10}$ and $R^{11}$ are all H;

compound 389: n is 0, X is S, Y is O, $R^2$ and $R^6$ are Me, $R^8$ is Cl, and $R^3$, $R^4$, $R^5$, $R^7$, $R^9$, $R^{10}$ and $R^{11}$ are all H;

compound 390: n is 0, X is S, Y is O, $R^2$ and $R^6$ are Me, $R^9$ is Cl, and $R^3$, $R^4$, $R^5$, $R^7$, $R^8$, $R^{10}$ and $R^{11}$ are all H;

compound 391: n is 0, X is S, Y is O, $R^2$ and $R^6$ are Me, $R^7$ is Br, and $R^3$, $R^4$, $R^5$, $R^8$, $R^9$, $R^{10}$ and $R^{11}$ are all H;

compound 392: n is 0, X is S, Y is O, $R^2$ and $R^6$ are Me, $R^8$ is Br, and $R^3$, $R^4$, $R^5$, $R^7$, $R^9$, $R^{10}$ and $R^{11}$ are all H;

compound 393: n is 0, X is S, Y is O, $R^2$ and $R^6$ are Me, $R^9$ is Br, and $R^3$, $R^4$, $R^5$, $R^7$, $R^8$, $R^{10}$ and $R^{11}$ are all H;

compound 394: n is 0, X is S, Y is O, $R^2$ and $R^6$ are Me, $R^7$ is I, and $R^3$, $R^4$, $R^5$, $R^8$, $R^9$, $R^{10}$ and $R^{11}$ are all H;

compound 395: n is 0, X is S, Y is O, $R^2$ and $R^6$ are Me, $R^8$ is I, and $R^3$, $R^4$, $R^5$, $R^7$, $R^9$, $R^{10}$ and $R^{11}$ are all H;

compound 396: n is 0, X is S, Y is O, $R^2$ and $R^6$ are Me, $R^9$ is I, and $R^3$, $R^4$, $R^5$, $R^7$, $R^8$, $R^{10}$ and $R^{11}$ are all H;

compound 397: n is 0, X is S, Y is O, $R^2$ and $R^6$ are Me, $R^7$ is $NO_2$, and $R^3$, $R^4$, $R^5$, $R^8$, $R^9$, $R^{10}$ and $R^{11}$ are all H;

compound 398: n is 0, X is S, Y is O, $R^2$ and $R^6$ are Me, $R^8$ is $NO_2$, and $R^3$, $R^4$, $R^5$, $R^7$, $R^9$, $R^{10}$ and $R^{11}$ are all H;

compound 399: n is 0, X is S, Y is O, $R^2$ and $R^6$ are Me, $R^9$ is $NO_2$, and $R^3$, $R^4$, $R^5$, $R^7$, $R^8$, $R^{10}$ and $R^{11}$ are all H;

compound 400: n is 0, X is S, Y is O, $R^2$ and $R^6$ are Me, $R^7$ is $CF_3$, and $R^3$, $R^4$, $R^5$, $R^8$, $R^9$, $R^{10}$ and $R^{11}$ are all H;

compound 401: n is 0, X is S, Y is O, $R^2$ and $R^6$ are Me, $R^8$ is $CF_3$, and $R^3$, $R^4$, $R^5$, $R^7$, $R^9$, $R^{10}$ and $R^{11}$ are all H;

compound 402: n is 0, X is S, Y is O, $R^2$ and $R^6$ are Me, $R^9$ is $CF_3$, and $R^3$, $R^4$, $R^5$, $R^7$, $R^8$, $R^{10}$ and $R^{11}$ are all H;

compound 403: n is 0, X is S, Y is O, $R^2$ and $R^6$ are Me, $R^7$ is $OCF_3$, and $R^3$, $R^4$, $R^5$, $R^8$, $R^9$, $R^{10}$ and $R^{11}$ are all H;

compound 404: n is 0, X is S, Y is O, $R^2$ and $R^6$ are Me, $R^8$ is $OCF_3$, and $R^3$, $R^4$, $R^5$, $R^7$, $R^9$, $R^{10}$ and $R^{11}$ are all H;

compound 405: n is 0, X is S, Y is O, $R^2$ and $R^6$ are Me, $R^9$ is $OCF_3$, and $R^3$, $R^4$, $R^5$, $R^7$, $R^8$, $R^{10}$ and $R^{11}$ are all H;

compound 406: n is 0, X is S, Y is O, $R^2$ and $R^6$ are Me, $R^7$ is $OCHF_2$, and $R^3$, $R^4$, $R^5$, $R^8$, $R^9$, $R^{10}$ and $R^{11}$ are all H;

compound 407: n is 0, X is S, Y is O, $R^2$ and $R^6$ are Me, $R^8$ is $OCHF_2$, and $R^3$, $R^4$, $R^5$, $R^7$, $R^9$, $R^{10}$ and $R^{11}$ are all H;

compound 408: n is 0, X is S, Y is O, $R^2$ and $R^6$ are Me, $R^9$ is $OCHF_2$, and $R^3$, $R^4$, $R^5$, $R^7$, $R^8$, $R^{10}$ and $R^{11}$ are all H;

compound 409: n is 0, X is S, Y is O, $R^2$ and $R^6$ are Me, $R^9$ is $SCF_3$, and $R^3$, $R^4$, $R^5$, $R^7$, $R^8$, $R^{10}$ and $R^{11}$ are all H;

compound 410: n is 0, X is S, Y is O, $R^2$ and $R^6$ are Me, $R^7$ is $OCH_3$, and $R^3$, $R^4$, $R^5$, $R^8$, $R^9$, $R^{10}$ and $R^{11}$ are all H;

compound 411: n is 0, X is S, Y is O, $R^2$ and $R^6$ are Me, $R^8$ is $OCH_3$, and $R^3$, $R^4$, $R^5$, $R^7$, $R^9$, $R^{10}$ and $R^{11}$ are all H;

compound 412: n is 0, X is S, Y is O, $R^2$ and $R^6$ are Me, $R^9$ is $OCH_3$, and $R^3$, $R^4$, $R^5$, $R^7$, $R^8$, $R^{10}$ and $R^{11}$ are all H;

compound 413: n is 0, X is S, Y is O, $R^2$ and $R^6$ are Me, $R^9$ is $OC_2H_5$, and $R^3$, $R^4$, $R^5$, $R^7$, $R^8$, $R^{10}$ and $R^{11}$ are all H;

compound 414: n is 0, X is S, Y is O, $R^2$ and $R^6$ are Me, $R^7$ is CN, and $R^3$, $R^4$, $R^5$, $R^A$, $R^9$, $R^{10}$ and $R^{11}$ are all H;

compound 415: n is 0, X is S, Y is O, $R^2$ and $R^6$ are Me, $R^8$ is CN, and $R^3$, $R^4$, $R^5$, $R^7$, $R^9$, $R^{10}$ and $R^{11}$ are all H;

compound 416: n is 0, X is S, Y is O, $R^2$ and $R^6$ are $M_e$, $R^9$ is CN, and $R^3$, $R^4$, $R^5$, $R^7$, $R^8$, $R^{10}$ and $R^{11}$ are all H;

compound 417: n is 0, X is S, Y is O, $R^2$ and $R^6$ are Me, $R^7$ is $CH_3$, and $R^3$, $R^4$, $R^5$, $R^A$, $R^9$, $R^{10}$ and $R^{11}$ are all H;

compound 418: n is 0, X is S, Y is O, $R^2$ and $R^6$ are Me, $R^8$ is $CH_3$, and $R^3$, $R^4$, $R^5$, $R^7$, $R^9$, $R^{10}$ and $R^{11}$ are all H;

compound 419: n is 0, X is S, Y is O, $R^2$ and $R^6$ are Me, $R^9$ is $CH_3$, and $R^3$, $R^4$, $R^5$, $R^7$, $R^8$, $R^{10}$ and $R^{11}$ are all H;

compound 420: n is 0, X is S, Y is O, $R^2$ and $R^6$ are Me, $R^7$ is $CH_3SO_2$, and $R^3$, $R^4$, $R^5$, $R^8$, $R^9$, $R^{10}$ and $R^{11}$ are all H;

compound 421: n is 0, X is S, Y is O, $R^2$ and $R^6$ are Me, $R^8$ is $CH_3SO_2$, and $R^3$, $R^4$, $R^5$, $R^7$, $R^9$, $R^{10}$ and $R^{11}$ are all H;

compound 422: n is 0, X is S, Y is O, $R^2$ and $R^6$ are Me, $R^9$ is $CH_3SO_2$, and $R^3$, $R^4$, $R^5$, $R^7$, $R^9$, $R^{10}$ and $R^{11}$ are all H;

compound 423: n is 0, X is S, Y is O, $R^2$ and $R^6$ are Me, $R^7$ is $C_2H_5SO_2$, and $R^3$, $R^4$, $R^5$, $R^8$, $R^9$, $R^{10}$ and $R^{11}$ are all H;

compound 424: n is 0, X is S, Y is O, $R^2$ and $R^6$ are Me, $R^7$ and $R^8$ are both Cl, and $R^3$, $R^4$, $R^5$, $R^9$, $R^{10}$ and $R^{11}$ are all H;

compound 425: n is 0, X is S, Y is O, $R^2$ and $R^6$ are Me, $R^7$ and $R^9$ are both Cl, and $R^3$, $R^4$, $R^5$, $R^8$, $R^{10}$ and $R^{11}$ are all H;

compound 426: n is 0, X is S, Y is O, $R^2$ and $R^6$ are Me, $R^7$ and $R^{10}$ are both Cl, and $R^3$, $R^4$, $R^5$, $R^8$, $R^9$ and $R^{11}$ are all H;

compound 427: n is 0, X is S, Y is O, $R^2$ and $R^6$ are Me, $R^7$ and $R^{11}$ are both Cl, and $R^3$, $R^4$, $R^5$, $R^8$, $R^9$ and $R^{10}$ are all H;

compound 428: n is 0, X is S, Y is O, $R^2$ and $R^6$ are Me, $R^8$ and $R^9$ are both Cl, and $R^3$, $R^4$, $R^5$, $R^7$, $R^{10}$ and $R^{11}$ are all H;

compound 429: n is 0, X is S, Y is O, $R^2$, $R^6$ and $R^7$ are Me, $R^9$ is Cl, and $R^3$, $R^4$, $R^5$, $R^3$, $R^{10}$ and $R^{11}$ are all H;

compound 430: n is 0, X is S, Y is O, $R^2$, $R^6$, $R^7$ and $R^8$ are Me, and $R^3$, $R^4$, $R^5$, $R^9$, $R^{10}$ and $R^{11}$ are all H;

compound 431: n is 0, X is S, Y is O, $R^2$, $R^6$, $R^7$ and $R^9$ are Me, and $R^3$, $R^4$, $R^5$, $R^8$, $R^{10}$ and $R^{11}$ are all H;

compound 432: n is 0, X is S, Y is O, $R^2$, $R^6$, $R^7$ and $R^{10}$ are Me, and $R^3$, $R^4$, $R^5$, $R^A$, $R^9$ and $R^{11}$ are all H;

compound 433: n is 0, X is S, Y is O, $R^2$, $R^6$, $R^7$ and $R^{11}$ are Me, and $R^3$, $R^4$, $R^5$, $R^8$, $R^9$ and $R^{10}$ are all H;

compound 434: n is 0, X is S, Y is O, $R^2$, $R^6$, $R^7$, $R^8$ and $R^{11}$ are Me, and $R^3$, $R^4$, $R^5$, $R^9$ and $R^{10}$ are all H;
compound 435: n is 0, X is S, Y is O, $R^2$, $R^6$, $R^7$, $R^9$ and $R^{11}$ are Me, and $R^3$, $R^4$, $R^5$, $R^8$ and $R^{10}$ are all H;
compound 436: n is 0, X is S, Y is O, $R^2$ and $R^6$ are Me, $R^7$ and $R^9$ are both $CF_3$, and $R^3$, $R^4$, $R^5$, $R^8$, $R^{10}$ and $R^{11}$ are all H;
compound 437: n is 0, X is S, Y is O, $R^2$ and $R^6$ are Me, $R^8$ and $R^{10}$ are both $CF_3$, and $R^3$, $R^4$, $R^5$, $R^7$, $R^9$ and $R^{11}$ are all H;
compound 438: n is 0, X is S, Y is O, $R^2$ and $R^6$ are Me, $R^3$, $R^4$ and $R^5$ are all H, and $R^7$, $R^8$, $R^9$, $R^{10}$ and $R^{11}$ are all F;
compound 439: n is 0, X is S, Y is O, $R^2$ and $R^6$ are Me, $R^7$ is $CH_3CH_2$, $R^3$, $R^4$, $R^5$, $R^A$, $R^9$, $R^{10}$ and $R^{11}$ are all H;
compound 440: n is 0, X is S, Y is O, $R^2$ and $R^6$ are Me, $R^8$ is $CH_3CH_2$, $R^3$, $R^4$, $R^5$, $R^7$, $R^9$, $R^{10}$ and $R^{11}$ are all H;
compound 441: n is 0, X is S, Y is O, $R^2$ and $R^6$ are Me, $R^9$ is $CH_3CH_2$, $R^3$, $R^4$, $R^5$, $R^7$, $R^8$, $R^{10}$ and $R^{11}$ are all H;
compound 442: n is 0, X is S, Y is O, $R^2$ and $R^6$ are Me, $R^7$ is $CH(CH_3)_2$, and $R^3$, $R^4$, $R^5$, $R^8$, $R^9$, $R^{10}$ and $R^{11}$ are all H;
compound 443: n is 0, X is S, Y is O, $R^2$ and $R^6$ are Me, $R^8$ is $CH(CH_3)_2$, and $R^3$, $R^4$, $R^5$, $R^7$, $R^9$, $R^{10}$ and $R^{11}$ are all H;
compound 444: n is 0, X is S, Y is O, $R^2$ and $R^6$ are Me, $R^9$ is $CH(CH_3)_2$, and $R^3$, $R^4$, $R^5$, $R^7$, $R^8$, $R^{10}$ and $R^{11}$ are all H;
compound 445: n is 0, X is S, Y is O, $R^2$ and $R^6$ are Me, $R^7$ is $CH_3(CH_2)_2$, and $R^2$, $R^3$, $R^4$, $R^5$, $R^8$, $R^9$, $R^{10}$ and $R^{11}$ are all H;
compound 446: n is 0, X is S, Y is O, $R^2$ and $R^6$ are Me, $R^7$ is $C(CH_3)_3$, and $R^3$, $R^4$, $R^5$, $R^8$, $R^9$, $R^{10}$ and $R^{11}$ are all H;
compound 447: n is 0, X is S, Y is O, $R^2$ and $R^6$ are Me, $R^7$ is

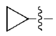

, $R^3$, $R^4$, $R^5$, $R^A$, $R^9$, $R^{10}$ and $R^{11}$ are all H;
compound 448: n is 0, X is S, Y is O, $R^2$ and $R^6$ are Me, $R^7$ is

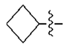,

$R^3$, $R^4$, $R^5$, $R^8$, $R^9$, $R^{10}$ and $R^{11}$ are all H;
compound 449: n is 0, X is S, Y is O, $R^2$ and $R^6$ are Me, $R^7$ is $CO_2CH_3$, $R^3$, $R^4$, $R^5$, $R^8$, $R^9$, $R^{10}$ and $R^{11}$ are all H;
compound 450: n is 0, X is S, Y is O, $R^2$ and $R^6$ are Me, $R^7$ and $R^8$ are cyclized to form

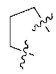,

and $R^3$, $R^4$, $R^5$, $R^9$, $R^{10}$ and $R^{11}$ are all H;
compound 451: n is 0, X is S, Y is O, $R^2$ and $R^6$ are Me, $R^8$ and $R^9$ are cyclized to form

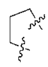,

and $R^3$, $R^4$, $R^5$, $R^7$, $R^{10}$ and $R^{11}$ are all H;
compound 452: n is 0, X is S, Y is O, $R^2$ and $R^6$ are Me, $R^8$ and $R^9$ are cyclized to form

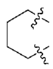, ;

and $R^3$, $R^4$, $R^5$, $R^7$, $R^{10}$ and $R^{11}$ are all H;
compound 453: n is 0, X is S, Y is O, $R^2$ and $R^6$ are Me, $R^7$ and $R^8$ are cyclized to form

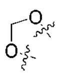,

and $R^3$, $R^4$, $R^5$, $R^9$, $R^{10}$ and $R^{11}$ are all H;
compound 454: n is 0, X is S, Y is O, $R^2$ and $R^6$ are Me, $R^8$ and $R^9$ are cyclized to form

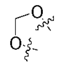,

and $R^3$, $R^4$, $R^5$, $R^7$, $R^{10}$ and $R^{11}$ are all H;
compound 455: n is 0, X is S, Y is O, $R^2$ and $R^6$ are Me, $R^8$ and $R^9$ are cyclized to form

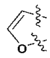,

and $R^3$, $R^4$, $R^3$, $R^7$, $R^{10}$ and $R^{11}$ are all H;
compound 456: n is 0, X is S, Y is O, $R^2$ and $R^6$ are Me, $R^7$ and $R^8$ are cyclized to form

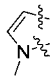,

and $R^3$, $R^4$, $R^5$, $R^9$, $R^{10}$ and $R^{11}$ are all H;
compound 457: n is 0, X is S, Y is O, $R^2$ and $R^6$ are Me, $R^7$ and $R^8$ are cyclized to form

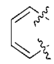,

and $R^3$, $R^4$, $R^5$, $R^9$, $R^{10}$ and $R^{11}$ are all H;
compound 458: n is 0, X is S, Y is O, $R^2$ and $R^6$ are Me, $R^7$ and $R^8$ are cyclized to form

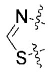,

and $R^3$, $R^4$, $R^5$, $R^9$, $R^{10}$ and $R^{11}$ are all H;
compound 459: n is 0, X is S, Y is O, $R^2$ and $R^6$ are Me, $R^7$ and $R^8$ are cyclized to form

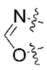,

and $R^3$, $R^4$, $R^5$, $R^9$, $R^{10}$ and $R^{11}$ are all H;
compound 460: n is 0, X is S, Y is O, $R^2$ and $R^6$ are Me, $R^8$ and $R^9$ are cyclized to form

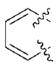, ;

and $R^3$, $R^4$, $R^5$, $R^7$, $R^{10}$ and $R^{11}$ are all H;
compound 461: n is 0, X is S, Y is O, $R^2$ is $C_2H_5$, $R^6$ is Me, and $R^3$, $R^4$, $R^5$, $R^7$, $R^8$, $R^9$, $R^{10}$ and $R^{11}$ are all H;
compound 462: n is 0, X is S, Y is O, $R^2$ is $C_2H_5$, $R^6$ is Me, $R^7$ is F, and $R^3$, $R^4$, $R^5$, $R^8$, $R^9$, $R^{10}$ and $R^{11}$ are all H;
compound 463: n is 0, X is S, Y is O, $R^2$ is $C_2H_5$, $R^6$ is Me, $R^8$ is F, and $R^3$, $R^4$, $R^5$, $R^7$, $R^9$, $R^{10}$ and $R^{11}$ are all H;
compound 464: n is 0, X is S, Y is O, $R^2$ is $C_2H_5$, $R^6$ is Me, $R^9$ is F, and $R^3$, $R^4$, $R^5$, $R^7$, $R^8$, $R^{10}$ and $R^{11}$ are all H;
compound 465: n is 0, X is S, Y is O, $R^2$ is $C_2H_5$, $R^6$ is Me, $R^7$ is Cl, and $R^3$, $R^4$, $R^5$, $R^A$, $R^9$, $R^{10}$ and $R^{11}$ are all H;
compound 466: n is 0, X is S, Y is O, $R^2$ is $C_2H_5$, $R^6$ is Me, $R^8$ is Cl, and $R^3$, $R^4$, $R^5$, $R^7$, $R^9$, $R^{10}$ and $R^{11}$ are all H;
compound 467: n is 0, X is S, Y is O, $R^2$ is $C_2H_5$, $R^6$ is Me, $R^9$ is Cl, and $R^3$, $R^4$, $R^5$, $R^7$, $R^8$, $R^{10}$ and $R^{11}$ are all H;
compound 468: n is 0, X is S, Y is O, $R^2$ is $C_2H_5$, $R^6$ is Me, $R^7$ is Br, $R^3$, $R^4$, $R^5$, $R^A$, $R^9$, $R^{10}$ and $R^{11}$ are all H;
compound 469: n is 0, X is S, Y is O, $R^2$ is $C_2H_5$, $R^6$ is Me, $R^8$ is Br, $R^3$, $R^4$, $R^5$, $R^7$, $R^9$, $R^{10}$ and $R^{11}$ are all H;
compound 470: n is 0, X is S, Y is O, $R^2$ is $C_2H_5$, $R^6$ is Me, $R^9$ is $B_r$, $R^3$, $R^4$, $R^5$, $R^7$, $R^8$, $R^{10}$ and $R^{11}$ are all H;
compound 471: n is 0, X is S, Y is O, $R^2$ is $C_2H_5$, $R^6$ is Me, $R^7$ is I, and $R^3$, $R^4$, $R^5$, $R^8$, $R^9$, $R^{10}$ and $R^{11}$ are all H;
compound 472: n is 0, X is S, Y is O, $R^2$ is $C_2H_5$, $R^6$ is Me, $R^8$ is I, and $R^3$, $R^4$, $R^5$, $R^7$, $R^9$, $R^{10}$ and $R^{11}$ are all H;
compound 473: n is 0, X is S, Y is O, $R^2$ is $C_2H_5$, $R^6$ is Me, $R^9$ is I, and $R^3$, $R^4$, $R^5$, $R^7$, $R^8$, $R^{10}$ and $R^{11}$ are all H;
compound 474: n is 0, X is S, Y is O, $R^2$ is $C_2H_5$, $R^6$ is Me, $R^7$ is $NO_2$, $R^3$, $R^4$, $R^5$, $R^8$, $R^9$, $R^{10}$ and $R^{11}$ are all H;
compound 475: n is 0, X is S, Y is O, $R^2$ is $C_2H_5$, $R^6$ is Me, $R^8$ is $NO_2$, $R^3$, $R^4$, $R^5$, $R^7$, $R^9$, $R^{10}$ and $R^{11}$ are all H;

compound 476: n is 0, X is S, Y is O, $R^2$ is $C_2H_5$, $R^6$ is Me, $R^9$ is $NO_2$, $R^3$, $R^4$, $R^5$, $R^7$, $R^8$, $R^{10}$ and $R^{11}$ are all H;
compound 477: n is 0, X is S, Y is O, $R^2$ is $C_2H_5$, $R^6$ is Me, $R^7$ is $CF_3$, $R^3$, $R^4$, $R^5$, $R^8$, $R^9$, $R^{10}$ and $R^{11}$ are all H;
compound 478: n is 0, X is S, Y is O, $R^2$ is $C_2H_5$, $R^6$ is Me, $R^8$ is $CF_3$, $R^3$, $R^4$, $R^5$, $R^7$, $R^9$, $R^{10}$ and $R^{11}$ are all H;
compound 479: n is 0, X is S, Y is O, $R^2$ is $C_2H_5$, $R^6$ is Me, $R^9$ is $CF_3$, $R^3$, $R^4$, $R^5$, $R^7$, $R^8$, $R^{10}$ and $R^{11}$ are all H;
compound 480: n is 0, X is S, Y is O, $R^2$ is $C_2H_5$, $R^6$ is Me, $R^7$ is $OCF_3$, $R^3$, $R^4$, $R^5$, $R^A$, $R^9$, $R^{10}$ and $R^{11}$ are all H;
compound 481: n is 0, X is S, Y is O, $R^2$ is $C_2H_5$, $R^6$ is Me, $R^8$ is $OCF_3$, $R^3$, $R^4$, $R^5$, $R^7$, $R^9$, $R^{10}$ and $R^{11}$ are all H;
compound 482: n is 0, X is S, Y is O, $R^2$ is $C_2H_5$, $R^6$ is Me, $R^9$ is $OCF_3$, $R^3$, $R^4$, $R^5$, $R^7$, $R^8$, $R^{10}$ and $R^{11}$ are all H;
compound 483: n is 0, X is S, Y is O, $R^2$ is $C_2H_5$, $R^6$ is Me, $R^7$ is $OCHF_2$, $R^3$, $R^4$, $R^5$, $R^8$, $R^9$, $R^{10}$ and $R^{11}$ are all H;
compound 484: n is 0, X is S, Y is O, $R^2$ is $C_2H_5$, $R^6$ is Me, $R^8$ is $OCHF_2$, $R^3$, $R^4$, $R^5$, $R^7$, $R^9$, $R^{10}$ and $R^{11}$ are all H;
compound 485: n is 0, X is S, Y is O, $R^2$ is $C_2H_5$, $R^6$ is Me, $R^9$ is $OCHF_2$, $R^3$, $R^4$, $R^5$, $R^7$, $R^8$, $R^{10}$ and $R^{11}$ are all H;
compound 486: n is 0, X is S, Y is O, $R^2$ is $C_2H_5$, $R^6$ is Me, $R^9$ is $SCF_3$, $R^3$, $R^4$, $R^5$, $R^7$, $R^8$, $R^{10}$ and $R^{11}$ are all H;
compound 487: n is 0, X is S, Y is O, $R^2$ is $C_2H_5$, $R^6$ is Me, $R^7$ is $OCH_3$, $R^3$, $R^4$, $R^5$, $R^8$, $R^9$, $R^{10}$ and $R^{11}$ are all H;
compound 488: n is 0, X is S, Y is O, $R^2$ is $C_2H_5$, $R^6$ is Me, $R^8$ is $OCH_3$, $R^3$, $R^4$, $R^5$, $R^7$, $R^9$, $R^{10}$ and $R^{11}$ are all H;
compound 489: n is 0, X is S, Y is O, $R^2$ is $C_2H_5$, $R^6$ is Me, $R^9$ is $OCH_3$, $R^3$, $R^4$, $R^5$, $R^7$, $R^8$, $R^{10}$ and $R^{11}$ are all H;
compound 490: n is 0, X is S, Y is O, $R^2$ is $C_2H_5$, $R^6$ is Me, $R^9$ is $OC_2H_5$, $R^3$, $R^4$, $R^5$, $R^7$, $R^8$, $R^{10}$ and $R^{11}$ are all H;
compound 491: n is 0, X is S, Y is O, $R^2$ is $C_2H_5$, $R^6$ is Me, $R^7$ is CN, $R^3$, $R^4$, $R^5$, $R^3$, $R^9$, $R^{10}$ and $R^{11}$ are all H;
compound 492: n is 0, X is S, Y is O, $R^2$ is $C_2H_5$, $R^6$ is Me, $R^8$ is CN, $R^3$, $R^4$, $R^5$, $R^7$, $R^9$, $R^{10}$ and $R^{11}$ are all H;
compound 493: n is 0, X is S, Y is O, $R^2$ is $C_2H_5$, $R^6$ is Me, $R^9$ is CN, $R^3$, $R^4$, $R^5$, $R^7$, $R^8$, $R^{10}$ and $R^{11}$ are all H;
compound 494: n is 0, X is S, Y is O, $R^2$ is $C_2H_5$, $R^6$ is $M_e$, $R^7$ is $CH_3$, $R^3$, $R^4$, $R^5$, $R^8$, $R^9$, $R^{10}$ and $R^{11}$ are all H;
compound 495: n is 0, X is S, Y is O, $R^2$ is $C_2H_5$, $R^6$ is Me, $R^8$ is $CH_3$, $R^3$, $R^4$, $R^5$, $R^7$, $R^9$, $R^{10}$ and $R^{11}$ are all H;
compound 496: n is 0, X is S, Y is O, $R^2$ is $C_2H_5$, $R^6$ is Me, $R^9$ is $CH_3$, $R^3$, $R^4$, $R^5$, $R^7$, $R^8$, $R^{10}$ and $R^{11}$ are all H;
compound 497: n is 0, X is S, Y is O, $R^2$ is $C_2H_5$, $R^6$ is Me, $R^7$ is $CH_3SO_2$, $R^3$, $R^4$, $R^5$, $R^8$, $R^9$, $R^{10}$ and $R^{11}$ are all H;
compound 498: n is 0, X is S, Y is O, $R^2$ is $C_2H_5$, $R^6$ is Me, $R^8$ is $CH_3SO_2$, $R^3$, $R^4$, $R^5$, $R^7$, $R^9$, $R^{19}$ and $R^{11}$ are all H;
compound 499: n is 0, X is S, Y is O, $R^2$ is $C_2H_5$, $R^6$ is Me, $R^9$ is $CH_3SO_2$, $R^3$, $R^4$, $R^5$, $R^7$, $R^9$, $R^{10}$ and $R^{11}$ are all H;
compound 500: n is 0, X is S, Y is O, $R^2$ is $C_2H_5$, $R^6$ is Me, $R^7$ is $C_2H_5SO_2$, $R^3$, $R^4$, $R^5$, $R^8$, $R^9$, $R^{10}$ and $R^{11}$ are all H;
compound 501: n is 0, X is S, Y is O, $R^2$ is $C_2H_5$, $R^6$ is Me, $R^7$ and $R^8$ are both Cl, and $R^3$, $R^4$, $R^5$, $R^9$, $R^{10}$ and $R^{11}$ are all H;
compound 502: n is 0, X is S, Y is O, $R^2$ is $C_2H_5$, $R^6$ is Me, $R^7$ and $R^9$ are both Cl, and $R^3$, $R^4$, $R^5$, $R^8$, $R^{10}$ and $R^{11}$ are all H;
compound 503: n is 0, X is S, Y is O, $R^2$ is $C_2H_5$, $R^6$ is Me, $R^7$ and $R^{10}$ are both Cl, and $R^3$, $R^4$, $R^5$, $R^8$, $R^9$ and $R^{11}$ are all H;
compound 504: n is 0, X is S, Y is O, $R^2$ is $C_2H_5$, $R^6$ is Me, $R^7$ and $R^{11}$ are both Cl, and $R^3$, $R^4$, $R^5$, $R^8$, $R^9$ and $R^{10}$ are all H;
compound 505: n is 0, X is S, Y is O, $R^2$ is $C_2H_5$, $R^6$ is Me, $R^8$ and $R^9$ are both Cl, and $R^3$, $R^4$, $R^5$, $R^7$, $R^{10}$ and $R^{11}$ are all H;
compound 506: n is 0, X is S, Y is O, $R^2$ is $C_2H_5$, $R^6$ and $R^7$ are Me, $R^9$ is Cl, and $R^3$, $R^4$, $R^5$, $R^8$, $R^{10}$ and $R^{11}$ are all H;
compound 507: n is 0, X is S, Y is O, $R^2$ is $C_2H_5$, $R^6$, $R^7$ and $R^8$ are Me, and $R^3$, $R^4$, $R^5$, $R^9$, $R^{10}$ and $R^{11}$ are all H;
compound 508: n is 0, X is S, Y is O, $R^2$ is $C_2H_5$, $R^6$, $R^7$ and $R^9$ are Me, and $R^3$, $R^4$, $R^5$, $R^8$, $R^{10}$ and $R^{11}$ are all H;
compound 509: n is 0, X is S, Y is O, $R^2$ is $C_2H_5$, $R^6$, $R^7$ and $R^{10}$ are Me, and $R^3$, $R^4$, $R^5$, $R^8$, $R^9$ and $R^{11}$ are all H;
compound 510: n is 0, X is S, Y is O, $R^2$ is $C_2H_5$, $R^7$ and $R^{11}$ are Me, and $R^3$, $R^4$, $R^5$, $R^8$, $R^9$ and $R^{10}$ are all H;
compound 511: n is 0, X is S, Y is O, $R^2$ is $C_2H_5$, $R^6$, $R^7$, $R^8$ and $R^{11}$ are Me, and $R^3$, $R^4$, $R^5$, $R^9$ and $R^{10}$ are all H;
compound 512: n is 0, X is S, Y is O, $R^2$ is $C_2H_5$, $R^6$, $R^7$, $R^9$ and $R^{11}$ are Me, and $R^3$, $R^4$, $R^5$, $R^8$ and $R^{10}$ are all H;
compound 513: n is 0, X is S, Y is O, $R^2$ is $C_2H_5$, $R^6$ is Me, $R^7$ and $R^9$ are both $CF_3$, and $R^3$, $R^4$, $R^5$, $R^8$, $R^{10}$ and $R^{11}$ are all H;
compound 514: n is 0, X is S, Y is O, $R^2$ is $C_2H_5$, $R^6$ is Me, $R^8$ and $R^{10}$ are both $CF_3$, and $R^3$, $R^4$, $R^5$, $R^7$, $R^9$ and $R^{11}$ are all H;
compound 515: n is 0, X is S, Y is O, $R^2$ is $C_2H_5$, $R^6$ is Me, $R^3$, $R^4$ and $R^5$ are all H, and $R^7$, $R^8$, $R^9$, $R^{10}$ and $R^{11}$ are all F;
compound 516: n is 0, X is S, Y is O, $R^2$ is $C_2H_5$, $R^6$ is Me, $R^7$ is $CH_3CH_2$, $R^3$, $R^4$, $R^5$, $R^8$, $R^9$, $R^{10}$ and $R^{11}$ are all H;
compound 517: n is 0, X is S, Y is O, $R^2$ is $C_2H_5$, $R^6$ is Me, $R^8$ is $CH_3CH_2$, $R^3$, $R^4$, $R^5$, $R^7$, $R^9$, $R^{10}$ and $R^{11}$ are all H;
compound 518: n is 0, X is S, Y is O, $R^2$ is $C_2H_5$, $R^6$ is Me, $R^9$ is $CH_3CH_2$, $R^3$, $R^4$, $R^5$, $R^7$, $R^8$, $R^{10}$ and $R^{11}$ are all H;
compound 519: n is 0, X is S, Y is O, $R^2$ is $C_2H_5$, $R^6$ is Me, $R^7$ is $CH(CH_3)_2$, $R^3$, $R^4$, $R^5$, $R^8$, $R^9$, $R^{10}$ and $R^{11}$ are all H;
compound 520: n is 0, X is S, Y is O, $R^2$ is $C_2H_5$, $R^6$ is Me, $R^8$ is $CH(CH_3)_2$, $R^3$, $R^4$, $R^5$, $R^7$, $R^9$, $R^{10}$ and $R^{11}$ are all H;
compound 521: n is 0, X is S, Y is O, $R^2$ is $C_2H_5$, $R^6$ is Me, $R^9$ is $CH(CH_3)_2$, $R^3$, $R^4$, $R^5$, $R^7$, $R^8$, $R^{10}$ and $R^{11}$ are all H;
compound 522: n is 0, X is S, Y is O, $R^2$ is $C_2H_5$, $R^6$ is Me, $R^7$ is $CH_3(CH_2)_2$, and $R^2$, $R^3$, $R^4$, $R^5$, $R^8$, $R^9$, $R^{10}$ and $R^{11}$ are all H;
compound 523: n is 0, X is S, Y is O, $R^2$ is $C_2H_5$, $R^6$ is Me, $R^7$ is $C(CH_3)_3$, $R^3$, $R^4$, $R^5$, $R^8$, $R^9$, $R^{10}$ and $R^{11}$ are all H;

compound 524: n is 0, X is S, Y is O, $R^2$ is $C_2H_5$, $R^6$ is Me, $R^7$ is

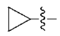

, $R^3$, $R^4$, $R^5$, $R^8$, $R^9$, $R^{10}$ and $R^{11}$ are all H;
compound 525: n is 0, X is S, Y is O, $R^2$ is $C_2H_5$, $R^6$ is Me, $R^7$ is

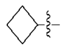,

$R^3$, $R^4$, $R^5$, $R^8$, $R^9$, $R^{10}$ and $R^{11}$ are all H;
compound 526: n is 0, X is S, Y is O, $R^2$ is $C_2H_5$, $R^6$ is Me, $R^7$ is $CO_2CH_3$, $R^3$, $R^4$, $R^5$, $R^8$, $R^9$, $R^{10}$ and $R^{11}$ are all H;
compound 527: n is 0, X is S, Y is O, $R^2$ is $C_2H_5$, $R^6$ is Me, $R^7$ and $R^8$ are cyclized to form

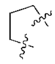,

and $R^3$, $R^4$, $R^5$, $R^9$, $R^{10}$ and $R^{11}$ are all H;
compound 528: n is 0, X is S, Y is O, $R^2$ is $C_2H_5$, $R^6$ is Me, $R^8$ and $R^9$ are cyclized to form

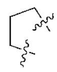,

and $R^3$, $R^4$, $R^5$, $R^7$, $R^{10}$ and $R^{11}$ are all H;
compound 529: n is 0, X is S, Y is O, $R^2$ is $C_2H_5$, $R^6$ is Me, $R^8$ and $R^9$ are cyclized to form

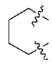,

and $R^3$, $R^4$, $R^5$, $R^7$, $R^{10}$ and $R^{11}$ are all H;
compound 530: n is 0, X is S, Y is O, $R^2$ is $C_2H_5$, $R^6$ is Me, $R^7$ and $R^8$ are cyclized to form

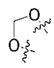,

and $R^3$, $R^4$, $R^5$, $R^9$, $R^{10}$ and $R^{11}$ are all H;
compound 531: n is 0, X is S, Y is O, $R^2$ is $C_2H_5$, $R^6$ is Me, $R^8$ and $R^9$ are cyclized to form

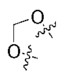,

and $R^3$, $R^4$, $R^5$, $R^7$, $R^{19}$ and $R^{11}$ are all H;
compound 532: n is 0, X is S, Y is O, $R^2$ is $C_2H_5$, $R^6$ is Me, $R^8$ and $R^9$ are cyclized to form

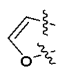,

and $R^3$, $R^4$, $R^5$, $R^7$, $R^{10}$ and $R^{11}$ are all H;
compound 533: n is 0, X is S, Y is O, $R^2$ is $C_2H_5$, $R^6$ is Me, $R^7$ and $R^8$ are cyclized to form

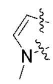,

and $R^3$, $R^4$, $R^5$, $R^9$, $R^{10}$ and $R^{11}$ are all H;

compound 534: n is 0, X is S, Y is O, $R^2$ is $C_2H_5$, $R^6$ is Me, $R^7$ and $R^8$ are cyclized to form

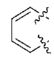,

and $R^3$, $R^4$, $R^5$, $R^9$, $R^{10}$ and $R^{11}$ are all H;
compound 535: n is 0, X is S, Y is O, $R^2$ is $C_2H_5$, $R^6$ is Me, $R^7$ and $R^8$ are cyclized to form

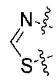,

and $R^3$, $R^4$, $R^5$, $R^9$, $R^{10}$ and $R^{11}$ are all H;
compound 536: n is 0, X is S, Y is O, $R^2$ is $C_2H_5$, $R^6$ is Me, $R^7$ and $R^8$ are cyclized to form

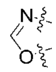,

and $R^3$, $R^4$, $R^5$, $R^9$, $R^{10}$ and $R^{11}$ are all H;
compound 537: n is 0, X is S, Y is O, $R^2$ is $C_2H_5$, $R^6$ is Me, $R^8$ and $R^9$ are cyclized to form

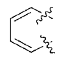,

and $R^3$, $R^4$, $R^5$, $R^7$, $R^{10}$ and $R^{11}$ are all H;
compound 796: n is 0, X is S, Y is O, $R^2$ is OMe, $R^6$ is Me, and $R^3$, $R^4$, $R^5$, $R^7$, $R^8$, $R^9$, $R^{10}$ and $R^{11}$ are all H;
compound 797: n is 0, X is S, Y is O, $R^2$ is OMe, $R^6$ is Me, $R^7$ is F, and $R^3$, $R^4$, $R^5$, $R^8$, $R^9$, $R^{10}$ and $R^{11}$ are all H;
compound 798: n is 0, X is S, Y is O, $R^2$ is OMe, $R^6$ is Me, $R^8$ is F, and $R^3$, $R^4$, $R^5$, $R^7$, $R^9$, $R^{10}$ and $R^{11}$ are all H;
compound 799: n is 0, X is S, Y is O, $R^2$ is OMe, $R^6$ is Me, $R^9$ is F, and $R^3$, $R^4$, $R^5$, $R^7$, $R^8$, $R^{10}$ and $R^{11}$ are all H;
compound 800: n is 0, X is S, Y is O, $R^2$ is OMe, $R^6$ is Me, $R^7$ is Cl, and $R^3$, $R^4$, $R^5$, $R^8$, $R^9$, $R^{10}$ and $R^{11}$ are all H;
compound 801: n is 0, X is S, Y is O, $R^2$ is OMe, $R^6$ is Me, $R^8$ is Cl, and $R^3$, $R^4$, $R^5$, $R^7$, $R^9$, $R^{10}$ and $R^{11}$ are all H;
compound 802: n is 0, X is S, Y is O, $R^2$ is OMe, $R^6$ is Me, $R^9$ is Cl, and $R^3$, $R^4$, $R^5$, $R^7$, $R^8$, $R^{10}$ and $R^{11}$ are all H;
compound 803: n is 0, X is S, Y is O, $R^2$ is OMe, $R^6$ is Me, $R^7$ is Br, $R^3$, $R^4$, $R^5$, $R^8$, $R^9$, $R^{10}$ and $R^{11}$ are all H;
compound 804: n is 0, X is S, Y is O, $R^2$ is OMe, $R^6$ is Me, $R^8$ is Br, $R^3$, $R^4$, $R^5$, $R^7$, $R^9$, $R^{10}$ and $R^{11}$ are all H;
compound 805: n is 0, X is S, Y is O, $R^2$ is OMe, $R^6$ is Me, $R^9$ is Br, $R^3$, $R^4$, $R^5$, $R^7$, $R^8$, $R^{10}$ and $R^{11}$ are all H;
compound 806: n is 0, X is S, Y is O, $R^2$ is OMe, $R^6$ is Me, $R^7$ is I, and $R^3$, $R^4$, $R^5$, $R^8$, $R^9$, $R^{10}$ and $R^{11}$ are all H;
compound 807: n is 0, X is S, Y is O, $R^2$ is OMe, $R^6$ is Me, $R^8$ is I, and $R^3$, $R^4$, $R^5$, $R^7$, $R^9$, $R^{10}$ and $R^{11}$ are all H;
compound 808: n is 0, X is S, Y is O, $R^2$ is OMe, $R^6$ is Me, $R^9$ is I, and $R^3$, $R^4$, $R^5$, $R^7$, $R^8$, $R^{10}$ and $R^{11}$ are all H;
compound 809: n is 0, X is S, Y is O, $R^2$ is OMe, $R^6$ is Me, $R^7$ is $NO_2$, $R^3$, $R^4$, $R^5$, $R^8$, $R^9$, $R^{10}$ and $R^{11}$ are all H;
compound 810: n is 0, X is S, Y is O, $R^2$ is OMe, $R^6$ is Me, $R^8$ is $NO_2$, $R^3$, $R^4$, $R^5$, $R^7$, $R^9$, $R^{10}$ and $R^{11}$ are all H;
compound 811: n is 0, X is S, Y is O, $R^2$ is OMe, $R^6$ is Me, $R^9$ is $NO_2$, $R^3$, $R^4$, $R^5$, $R^7$, $R^8$, $R^{10}$ and $R^{11}$ are all H;
compound 812: n is 0, X is S, Y is O, $R^2$ is OMe, $R^6$ is Me, $R^7$ is $CF_3$, $R^3$, $R^4$, $R^5$, $R^8$, $R^9$, $R^{10}$ and $R^{11}$ are all H;
compound 813: n is 0, X is S, Y is O, $R^2$ is OMe, $R^6$ is Me, $R^8$ is $CF_3$, $R^3$, $R^4$, $R^5$, $R^7$, $R^9$, $R^{10}$ and $R^{11}$ are all H;
compound 814: n is 0, X is S, Y is O, $R^2$ is OMe, $R^6$ is Me, $R^9$ is $CF_3$, $R^3$, $R^4$, $R^5$, $R^7$, $R^8$, $R^{10}$ and $R^{11}$ are all H;
compound 815: n is 0, X is S, Y is O, $R^2$ is OMe, $R^6$ is Me, $R^7$ is $OCF_3$, $R^3$, $R^4$, $R^5$, $R^8$, $R^9$, $R^{10}$ and $R^{11}$ are all H;
compound 816: n is 0, X is S, Y is O, $R^2$ is OMe, $R^6$ is Me, $R^8$ is $OCF_3$, $R^3$, $R^4$, $R^5$, $R^7$, $R^9$, $R^{10}$ and $R^{11}$ are all H;
compound 817: n is 0, X is S, Y is O, $R^2$ is OMe, $R^6$ is Me, $R^9$ is $OCF_3$, $R^3$, $R^4$, $R^5$, $R^7$, $R^8$, $R^{10}$ and $R^{11}$ are all H;
compound 818: n is 0, X is S, Y is O, $R^2$ is OMe, $R^6$ is Me, $R^7$ is $OCHF_2$, $R^3$, $R^4$, $R^5$, $R^8$, $R^9$, $R^{10}$ and $R^{11}$ are all H;
compound 819: n is 0, X is S, Y is O, $R^2$ is OMe, $R^6$ is Me, $R^8$ is $OCHF_2$, $R^3$, $R^4$, $R^5$, $R^7$, $R^9$, $R^{10}$ and $R^{11}$ are all H;
compound 820: n is 0, X is S, Y is O, $R^2$ is OMe, $R^6$ is Me, $R^9$ is $OCHF_2$, $R^3$, $R^4$, $R^5$, $R^7$, $R^8$, $R^{10}$ and $R^{11}$ are all H;
compound 821: n is 0, X is S, Y is O, $R^2$ is OMe, $R^6$ is Me, $R^9$ is $SCF_3$, $R^3$, $R^4$, $R^5$, $R^7$, $R^8$, $R^{10}$ and $R^{11}$ are all H;
compound 822: n is 0, X is S, Y is O, $R^2$ is OMe, $R^6$ is Me, $R^7$ is $OCH_3$, $R^3$, $R^4$, $R^5$, $R^8$, $R^9$, $R^{10}$ and $R^{11}$ are all H;
compound 823: n is 0, X is S, Y is O, $R^2$ is OMe, $R^6$ is Me, $R^8$ is $OCH_3$, $R^3$, $R^4$, $R^5$, $R^7$, $R^9$, $R^{10}$ and $R^{11}$ are all H;
compound 824: n is 0, X is S, Y is O, $R^2$ is OMe, $R^6$ is Me, $R^9$ is $OCH_3$, $R^3$, $R^4$, $R^5$, $R^7$, $R^8$, $R^{10}$ and $R^{11}$ are all H;
compound 825: n is 0, X is S, Y is O, $R^2$ is OMe, $R^6$ is Me, $R^9$ is $OC_2H_5$, $R^3$, $R^4$, $R^5$, $R^7$, $R^8$, $R^{10}$ and $R^{11}$ are all H;
compound 826: n is 0, X is S, Y is O, $R^2$ is OMe, $R^6$ is Me, $R^7$ is CN, and $R^3$, $R^4$, $R^5$, $R^8$, $R^9$, $R^{10}$ and $R^{11}$ are all H;
compound 827: n is 0, X is S, Y is O, $R^2$ is OMe, $R^6$ is Me, $R^8$ is CN, and $R^3$, $R^4$, $R^5$, $R^7$, $R^9$, $R^{10}$ and $R^{11}$ are all H;

compound 828: n is 0, X is S, Y is O, $R^2$ is OMe, $R^6$ is Me, $R^9$ is CN, and $R^3$, $R^4$, $R^5$, $R^7$, $R^8$, $R^{10}$ and $R^{11}$ are all H;
compound 829: n is 0, X is S, Y is O, $R^2$ is OMe, $R^6$ is Me, $R^7$ is $CH_3$, $R^3$, $R^4$, $R^5$, $R^3$, $R^9$, $R^{10}$ and $R^{11}$ are all H;
compound 830: n is 0, X is S, Y is O, $R^2$ is OMe, $R^6$ is Me, $R^8$ is $CH_3$, $R^3$, $R^4$, $R^5$, $R^7$, $R^9$, $R^{10}$ and $R^{11}$ are all H;
compound 831: n is 0, X is S, Y is O, $R^2$ is OMe, $R^6$ is Me, $R^9$ is $CH_3$, $R^3$, $R^4$, $R^5$, $R^7$, $R^8$, $R^{10}$ and $R^{11}$ are all H;
compound 832: n is 0, X is S, Y is O, $R^2$ is OMe, $R^6$ is Me, $R^7$ is $CH_3SO_2$, $R^3$, $R^4$, $R^5$, $R^8$, $R^9$, $R^{10}$ and $R^{11}$ are all H;
compound 833: n is 0, X is S, Y is O, $R^2$ is OMe, $R^6$ is Me, $R^8$ is $CH_3SO_2$, $R^3$, $R^4$, $R^5$, $R^7$, $R^9$, $R^{10}$ and $R^{11}$ are all H;
compound 834: n is 0, X is S, Y is O, $R^2$ is OMe, $R^6$ is Me, $R^9$ is $CH_3SO_2$, $R^3$, $R^4$, $R^5$, $R^7$, $R^9$, $R^{10}$ and $R^{11}$ are all H;
compound 835: n is 0, X is S, Y is O, $R^2$ is OMe, $R^6$ is Me, $R^7$ is $C_2H_5SO_2$, $R^3$, $R^4$, $R^5$, $R^8$, $R^9$, $R^{10}$ and $R^{11}$ are all H;
compound 836: n is 0, X is S, Y is O, $R^2$ is OMe, $R^6$ is Me, $R^7{}_{and}$ $R^8$ are both Cl, and $R^3$, $R^4$, $R^5$, $R^9$, $R^{10}$ and $R^{11}$ are all H;
compound 837: n is 0, X is S, Y is O, $R^2$ is OMe, $R^6$ is Me, $R^7$ and $R^9$ are both Cl, and $R^3$, $R^4$, $R^5$, $R^8$, $R^{10}$ and $R^{11}$ are all H;
compound 838: n is 0, X is S, Y is O, $R^2$ is OMe, $R^6$ is Me, $R^7$ and $R^{10}$ are both Cl, and $R^3$, $R^4$, $R^5$, $R^8$, $R^9$ and $R^{11}$ are all H;
compound 839: n is 0, X is S, Y is O, $R^2$ is OMe, $R^6$ is Me, $R^7$ and $R^{11}$ are both Cl, and $R^3$, $R^4$, $R^5$, $R^8$, $R^9$ and $R^{10}$ are all H;
compound 840: n is 0, X is S, Y is O, $R^2$ is OMe, $R^6$ is Me, $R^8$ and $R^9$ are both Cl, and $R^3$, $R^4$, $R^5$, $R^7$, $R^{10}$ and $R^{11}$ are all H;
compound 841: n is 0, X is S, Y is O, $R^2$ is OMe, $R^6$ and $R^7$ are Me, $R^9$ is Cl, and $R^3$, $R^4$, $R^5$, $R^8$, $R^{10}$ and $R^{11}$ are all H;
compound 842: n is 0, X is S, Y is O, $R^2$ is OMe, $R^6$, $R^7$ and $R^8$ are Me, and $R^3$, $R^4$, $R^5$, $R^9$, $R^{10}$ and $R^{11}$ are al$_l$ H;
compound 843: n is 0, X is S, Y is O, $R^2$ is OMe, $R^6$, $R^7$ and $R^9$ are Me, and $R^3$, $R^4$, $R^5$, $R^8$, $R^{10}$ and $R^{11}$ are all H;
compound 844: n is 0, X is S, Y is O, $R^2$ is OMe, $R^6$, $R^7$ and $R^{10}$ are Me, and $R^3$, $R^4$, $R^5$, $R^8$, $R^9$ and $R^{11}$ are all H;
compound 845: n is 0, X is S, Y is O, $R^2$ is OMe, $R^7$ and $R^{11}$ are Me, and $R^3$, $R^4$, $R^5$, $R^8$, $R^9$ and $R^{10}$ are all H;
compound 846: n is 0, X is S, Y is O, $R^2$ is OMe, $R^6$, $R^7$, $R^8$ and $R^{11}$ are Me, and $R^3$, $R^4$, $R^5$, $R^9$ and $R^{10}$ are all H;
compound 847: n is 0, X is S, Y is O, $R^2$ is OMe, $R^6$, $R^7$, $R^9$ and $R^{11}$ are Me, and $R^3$, $R^4$, $R^5$, $R^8$ and $R^{10}$ are all H;
compound 848: n is 0, X is S, Y is O, $R^2$ is OMe, $R^6$ is Me, $R^7$ and $R^9$ are both $CF_3$, $R^3$, $R^4$, $R^5$, $R^8$, $R^{10}$ and $R^{11}$ are all H;
compound 849: n is 0, X is S, Y is O, $R^2$ is OMe, $R^6$ is Me, $R^8$ and $R^{10}$ are both $CF_3$, $R^3$, $R^4$, $R^5$, $R^7$, $R^9$ and $R^{11}$ are all H;
compound 850: n is 0, X is S, Y is O, $R^2$ is OM$_e$, $R^6$ is Me, $R^3$, $R^4$ and $R^5$ are all H, and $R^7$, $R^8$, $R^9$, $R^{10}$ and $R^{11}$ are all F;
compound 851: n is 0, X is S, Y is O, $R^2$ is OMe, $R^6$ is Me, $R^7$ is $CH_3CH_2$, $R^3$, $R^4$, $R^5$, $R^8$, $R^9$, $R^{10}$ and $R^{11}$ are all H;
compound 852: n is 0, X is S, Y is O, $R^2$ is OMe, $R^6$ is Me, $R^8$ is $CH_3CH_2$, $R^3$, $R^4$, $R^5$, $R^7$, $R^9$, $R^{10}$ and $R^{11}$ are all H;
compound 853: n is 0, X is S, Y is O, $R^2$ is OMe, $R^6$ is Me, $R^9$ is $CH_3CH_2$, $R^3$, $R^4$, $R^5$, $R^7$, $R^8$, $R^{10}$ and $R^{11}$ are all H;
compound 854: n is 0, X is S, Y is O, $R^2$ is OMe, $R^6$ is Me, $R^7$ is $CH(CH_3)_2$, $R^3$, $R^4$, $R^5$, $R^8$, $R^9$, $R^{10}$ and $R^{11}$ are all H;
compound 855: n is 0, X is S, Y is O, $R^2$ is OMe, $R^6$ is Me, $R^8$ is $CH(CH_3)_2$, $R^3$, $R^4$, $R^5$, $R^7$, $R^9$, $R^{10}$ and $R^{11}$ are all H;
compound 856: n is 0, X is S, Y is O, $R^2$ is OMe, $R^6$ is Me, $R^9$ is $CH(CH_3)_2$, $R^3$, $R^4$, $R^5$, $R^7$, $R^8$, $R^{10}$ and $R^{11}$ are all H;
compound 857: n is 0, X is S, Y is O, $R^2$ is OMe, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;
compound 858: n is 0, X is S, Y is O, $R^2$ is OMe, $R^6$ is Me, $R^7$ is $C(CH_3)_3$, and $R^3$, $R^4$, $R^5$, $R^8$, $R^9$, $R^{10}$ and $R^{11}$ are all H;
compound 859: n is 0, X is S, Y is O, $R^2$ is OMe, $R^6$ is Me, $R^7$ is

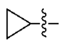,

$R^3$, $R^4$, $R^5$, $R^3$, $R^9$, $R^{10}$ and $R^{11}$ are all H;
compound 860: n is 0, X is S, Y is O, $R^2$ is OMe, $R^6$ is Me, $R^7$ is

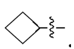,

$R^3$, $R^4$, $R^5$, $R^8$, $R^9$, $R^{10}$ and $R^{11}$ are all H;
compound 861: n is 0, X is S, Y is O, $R^2$ is OMe, $R^6$ is Me, $R^7$ is $CO_2CH_3$, $R^3$, $R^4$, $R^5$, $R^8$, $R^9$, $R^{10}$ and $R^{11}$ are all H;
compound 862: n is 0, X is S, Y is O, $R^2$ is OMe, $R^6$ is Me, $R^7$ and $R^8$ are cyclized to form

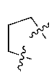,

and $R^3$, $R^4$, $R^5$, $R^9$, $R^{10}$ and $R^{11}$ are all H;
compound 863: n is 0, X is S, Y is O, $R^2$ is OMe, $R^6$ is Me, $R^8$ and $R^9$ are cyclized to form

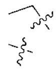,

and $R^3$, $R^4$, $R^5$, $R^7$, $R^{10}$ and $R^{11}$ are all H;
compound 864: n is 0, X is S, Y is O, $R^2$ is OMe, $R^6$ is Me, $R^8$ and $R^9$ are cyclized to form

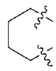,

and $R^3$, $R^4$, $R^5$, $R^7$, $R^{10}$ and $R^{11}$ are all H;
compound 865: n is 0, X is S, Y is O, $R^2$ is OMe, $R^6$ is Me, $R^7$ and $R^8$ are cyclized to form

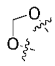,

and $R^3$, $R^4$, $R^5$, $R^9$, $R^{10}$ and $R^{11}$ are all H;
compound 866: n is 0, X is S, Y is O, $R^2$ is OMe, $R^6$ is Me, $R^8$ and $R^9$ are cyclized to form

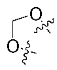,

and $R^3$, $R^4$, $R^5$, $R^7$, $R^{10}$ and $R^{11}$ are all H;
compound 867: n is 0, X is S, Y is O, $R^2$ is OMe, $R^6$ is Me, $R^8$ and $R^9$ are cyclized to form

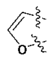,

and $R^3$, $R^4$, $R^5$, $R^7$, $R^{10}$ and $R^{11}$ are all H;
compound 868: n is 0, X is S, Y is O, $R^2$ is OMe, $R^6$ is Me, $R^7$ and $R^8$ are cyclized to form

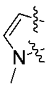,

and $R^3$, $R^4$, $R^5$, $R^9$, $R^{10}$ and $R^{11}$ are all H;
compound 869: n is 0, X is S, Y is O, $R^2$ is OMe, $R^6$ is Me, $R^7$ and $R^8$ are cyclized to form

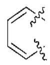,

and $R^3$, $R^4$, $R^5$, $R^9$, $R^{10}$ and $R^{11}$ are all H;
compound 870: n is 0, X is S, Y is O, $R^2$ is OMe, $R^6$ is Me, $R^7$ and $R^8$ are cyclized to form

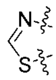,

and $R^3$, $R^4$, $R^5$, $R^9$, $R^{10}$ and $R^{11}$ are all H;
compound 871: n is 0, X is S, Y is O, $R^2$ is OMe, $R^6$ is Me, $R^7$ and $R^8$ are cyclized to form

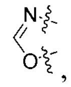

and $R^3$, $R^4$, $R^5$, $R^9$, $R^{10}$ and $R^{11}$ are all H;

compound 872: n is 0, X is S, Y is O, $R^2$ is OMe, $R^6$ is Me, $R^8$ and $R^9$ are cyclized to form

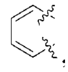

and $R^3$, $R^4$, $R^5$, $R^7$, $R^{10}$ and $R^{11}$ are all H;

compound 538: n is 0, X is SO, Y is O, $R^6$ is Me, and $R^2$, $R^3$, $R^4$, $R^5$, $R^7$, $R^8$, $R^9$, $R^{10}$ and $R^{11}$ are all H;

compound 539: n is 0, X is SO, Y is O, $R^6$ is Me, $R^7$ is F, and $R^2$, $R^3$, $R^4$, $R^5$, $R^8$, $R^9$, $R^{10}$ and $R^{11}$ are all H;

compound 540: n is 0, X is SO, Y is O, $R^6$ is Me, $R^8$ is F, and $R^2$, $R^3$, $R^4$, $R^5$, $R^7$, $R^9$, $R^{10}$ and $R^{11}$ are all H;

compound 541: n is 0, X is SO, Y is O, $R^6$ is Me, $R^9$ is F, and $R^2$, $R^3$, $R^4$, $R^5$, $R^7$, $R^8$, $R^{10}$ and $R^{11}$ are all H;

compound 542: n is 0, X is SO, Y is O, $R^6$ is Me, $R^7$ is Cl, and $R^2$, $R^3$, $R^4$, $R^5$, $R^8$, $R^9$, $R^{10}$ and $R^{11}$ are all H;

compound 543: n is 0, X is SO, Y is O, $R^6$ is Me, $R^8$ is Cl, and $R^2$, $R^3$, $R^4$, $R^5$, $R^7$, $R^9$, $R^{10}$ and $R^{11}$ are all H;

compound 544: n is 0, X is SO, Y is O, $R^6$ is Me, $R^9$ is Cl, and $R^2$, $R^3$, $R^4$, $R^5$, $R^7$, $R^8$, $R^{10}$ and $R^{11}$ are all H;

compound 545: n is 0, X is SO, Y is O, $R^6$ is Me, $R^7$ is Br, and $R^2$, $R^3$, $R^4$, $R^5$, $R^8$, $R^9$, $R^{10}$ and $R^{11}$ are all H;

compound 546: n is 0, X is SO, Y is O, $R^6$ is Me, $R^8$ is Br, and $R^2$, $R^3$, $R^4$, $R^5$, $R^7$, $R^9$, $R^{10}$ and $R^{11}$ are all H;

compound 547: n is 0, X is SO, Y is O, $R^6$ is Me, $R^9$ is Br, and $R^2$, $R^3$, $R^4$, $R^5$, $R^7$, $R^8$, $R^{10}$ and $R^{11}$ are all H;

compound 548: n is 0, X is SO, Y is O, $R^6$ is Me, $R^7$ is I, and $R^2$, $R^3$, $R^4$, $R^5$, $R^8$, $R^9$, $R^{10}$ and $R^{11}$ are all H;

compound 549: n is 0, X is SO, Y is O, $R^6$ is Me, $R^8$ is I, and $R^2$, $R^3$, $R^4$, $R^5$, $R^7$, $R^9$, $R^{10}$ and $R^{11}$ are all H;

compound 550: n is 0, X is SO, Y is O, $R^6$ is Me, $R^9$ is I, and $R^2$, $R^3$, $R^4$, $R^5$, $R^7$, $R^8$, $R^{10}$ and $R^{11}$ are all H;

compound 551: n is 0, X is SO, Y is O, $R^6$ is Me, $R^7$ is $NO_2$, and $R^2$, $R^3$, $R^4$, $R^5$, $R^8$, $R^9$, $R^{10}$ and $R^{11}$ are all H;

compound 552: n is 0, X is SO, Y is O, $R^6$ is Me, $R^8$ is $NO_2$, and $R^2$, $R^3$, $R^4$, $R^5$, $R^7$, $R^9$, $R^{10}$ and $R^{11}$ are all H;

compound 553: n is 0, X is SO, Y is O, $R^6$ is Me, $R^9$ is $NO_2$, and $R^2$, $R^3$, $R^4$, $R^5$, $R^7$, $R^8$, $R^{10}$ and $R^{11}$ are all H;

compound 554: n is 0, X is SO, Y is O, $R^6$ is Me, $R^7$ is $CF_3$, and $R^2$, $R^3$, $R^4$, $R^5$, $R^8$, $R^9$, $R^{10}$ and $R^{11}$ are all H;

compound 555: n is 0, X is SO, Y is O, $R^6$ is Me, $R^8$ is $CF_3$, and $R^2$, $R^3$, $R^4$, $R^5$, $R^7$, $R^9$, $R^{10}$ and $R^{11}$ are all H;

compound 556: n is 0, X is SO, Y is O, $R^6$ is Me, $R^9$ is $CF_3$, and $R^2$, $R^3$, $R^4$, $R^5$, $R^7$, $R^8$, $R^{10}$ and $R^{11}$ are all H;

compound 557: n is 0, X is SO, Y is O, $R^6$ is Me, $R^7$ is $OCF_3$, and $R^2$, $R^3$, $R^4$, $R^3$, $R^8$, $R^9$, $R^{10}$ and $R^{11}$ are all H;

compound 558: n is 0, X is SO, Y is O, $R^6$ is Me, $R^8$ is $OCF_3$, and $R^2$, $R^3$, $R^4$, $R^5$, $R^7$, $R^9$, $R^{10}$ and $R^{11}$ are all H;

compound 559: n is 0, X is SO, Y is O, $R^6$ is Me, $R^9$ is $OCF_3$, and $R^2$, $R^3$, $R^4$, $R^5$, $R^7$, $R^8$, $R^{10}$ and $R^{11}$ are all H;

compound 560: n is 0, X is SO, Y is O, $R^6$ is Me, $R^7$ is $OCHF_2$, and $R^2$, $R^3$, $R^4$, $R^5$, $R^8$, $R^9$, $R^{10}$ and $R^{11}$ are all H;

compound 561: n is 0, X is SO, Y is O, $R^6$ is Me, $R^8$ is $OCHF_2$, and $R^2$, $R^3$, $R^4$, $R^5$, $R^7$, $R^9$, $R^{10}$ and $R^{11}$ are all H;

compound 562: n is 0, X is SO, Y is O, $R^6$ is Me, $R^9$ is $OCHF_2$, and $R^2$, $R^3$, $R^4$, $R^5$, $R^7$, $R^8$, $R^{10}$ and $R^{11}$ are all H;

compound 563: n is 0, X is SO, Y is O, $R^6$ is Me, $R^9$ is $SCF_3$, and $R^2$, $R^3$, $R^4$, $R^5$, $R^7$, $R^8$, $R^{10}$ and $R^{11}$ are all H;

compound 564: n is 0, X is SO, Y is O, $R^6$ is Me, $R^7$ is $OCH_3$, and $R^2$, $R^3$, $R^4$, $R^5$, $R^8$, $R^9$, $R^{10}$ and $R^{11}$ are all H;

compound 565: n is 0, X is SO, Y is O, $R^6$ is Me, $R^8$ is $OCH_3$, and $R^2$, $R^3$, $R^4$, $R^5$, $R^7$, $R^9$, $R^{10}$ and $R^{11}$ are all H;

compound 566: n is 0, X is SO, Y is O, $R^6$ is Me, $R^9$ is $OCH_3$, and $R^2$, $R^3$, $R^4$, $R^5$, $R^7$, $R^8$, $R^{10}$ and $R^{11}$ are all H;

compound 567: n is 0, X is SO, Y is O, $R^6$ is Me, $R^9$ is $OC_2H_5$, and $R^2$, $R^3$, $R^4$, $R^5$, $R^7$, $R^8$, $R^{10}$ and $R^{11}$ are all H;

compound 568: n is 0, X is SO, Y is O, $R^6$ is Me, $R^7$ is CN, and $R^2$, $R^3$, $R^4$, $R^5$, $R^8$, $R^9$, $R^{10}$ and $R^{11}$ are all H;

compound 569: n is 0, X is SO, Y is O, $R^6$ is Me, $R^8$ is CN, and $R^2$, $R^3$, $R^4$, $R^5$, $R^7$, $R^9$, $R^{10}$ and $R^{11}$ are all H;

compound 570: n is 0, X is SO, Y is O, $R^6$ is Me, $R^9$ is CN, and $R^2$, $R^3$, $R^4$, $R^5$, $R^7$, $R^3$, $R^{10}$ and $R^{11}$ are all H;

compound 571: n is 0, X is SO, Y is O, $R^6$ is Me, $R^7$ is $CH_3$, and $R^2$, $R^3$, $R^4$, $R^5$, $R^8$, $R^9$, $R^{10}$ and $R^{11}$ are all H;

compound 572: n is 0, X is SO, Y is O, $R^6$ is Me, $R^8$ is $CH_3$, and $R^2$, $R^3$, $R^4$, $R^5$, $R^7$, $R^9$, $R^{10}$ and $R^{11}$ are all H;

compound 573: n is 0, X is SO, Y is O, $R^6$ is Me, $R^9$ is $CH_3$, and $R^2$, $R^3$, $R^4$, $R^5$, $R^7$, $R^8$, $R^{10}$ and $R^{11}$ are all H;

compound 574: n is 0, X is SO, Y is O, $R^6$ is Me, $R^7$ is $CH_3SO_2$, and $R^2$, $R^3$, $R^4$, $R^5$, $R^8$, $R^9$, $R^{10}$ and $R^{11}$ are all H;

compound 575: n is 0, X is SO, Y is O, $R^6$ is Me, $R^8$ is $CH_3SO_2$, and $R^2$, $R^3$, $R^4$, $R^5$, $R^7$, $R^9$, $R^{10}$ and $R^{11}$ are all H;

compound 576: n is 0, X is SO, Y is O, $R^6$ is Me, $R^9$ is $CH_3SO_2$, and $R^2$, $R^3$, $R^4$, $R^5$, $R^7$, $R^8$, $R^{10}$ and $R^{11}$ are all H;

compound 577: n is 0, X is SO, Y is O, $R^6$ is Me, $R^7$ is $C_2H_5SO_2$, and $R^2$, $R^3$, $R^4$, $R^5$, $R^8$, $R^9$, $R^{10}$ and $R^{11}$ are all H;

compound 578: n is 0, X is SO, Y is O, $R^6$ is Me, $R^7$ and $R^3$ are both Cl, and $R^2$, $R^3$, $R^4$, $R^5$, $R^9$, $R^{10}$ and $R^{11}$ are all H;

compound 579: n is 0, X is SO, Y is O, $R^6$ is Me, $R^7$ and $R^9$ are both Cl, and $R^2$, $R^3$, $R^4$, $R^5$, $R^8$, $R^{10}$ and $R^{11}$ are all H;

compound 580: n is 0, X is SO, Y is O, $R^6$ is Me, $R^7$ and $R^{10}$ are both Cl, and $R^2$, $R^3$, $R^4$, $R^5$, $R^8$, $R^9$ and $R^{11}$ are all H;

compound 581: n is 0, X is SO, Y is O, $R^6$ is Me, $R^7$ and $R^{11}$ are both Cl, and $R^2$, $R^3$, $R^4$, $R^5$, $R^8$, $R^9$ and $R^{10}$ are all H;

compound 582: n is 0, X is SO, Y is O, $R^6$ is Me, $R^8$ and $R^9$ are both Cl, and $R^2$, $R^3$, $R^4$, $R^5$, $R^7$, $R^{10}$ and $R^{11}$ are all H;

compound 583: n is 0, X is SO, Y is O, $R^6$ and $R^7$ are Me, $R^9$ is Cl, and $R^2$, $R^3$, $R^4$, $R^5$, $R^8$, $R^{10}$ and $R^{11}$ are all H;

compound 584: n is 0, X is SO, Y is O, $R^6$, $R^7$ and $R^8$ are Me, and $R^2$, $R^3$, $R^4$, $R^5$, $R^9$, $R^{10}$ and $R^{11}$ are all H;

compound 585: n is 0, X is SO, Y is O, $R^6$, $R^7$ and $R^9$ are Me, and $R^2$, $R^3$, $R^4$, $R^5$, $R^8$, $R^{10}$ and $R^{11}$ are all H;
compound 586: n is 0, X is SO, Y is O, $R^6$, $R^7$ and $R^{10}$ are Me, and $R^2$, $R^3$, $R^4$, $R^5$, $R^8$, $R^9$ and $R^{11}$ are all H;
compound 587: n is 0, X is SO, Y is O, $R^6$, $R^7$ and $R^{11}$ are Me, and $R^2$, $R^3$, $R^4$, $R^5$, $R^8$, $R^9$ and $R^{10}$ are all H;
compound 588: n is 0, X is SO, Y is O, $R^6$, $R^7$, $R^8$ and $R^{11}$ are Me, and $R^2$, $R^3$, $R^4$, $R^5$, $R^9$ and $R^{10}$ are all H;
compound 589: n is 0, X is SO, Y is O, $R^6$, $R^7$, $R^9$ and $R^{11}$ are Me, and $R^2$, $R^3$, $R^4$, $R^5$, $R^8$ and $R^{10}$ are all H;
compound 590: n is 0, X is SO, Y is O, $R^6$ is Me, $R^7$ and $R^9$ are both $CF_3$, and $R^2$, $R^3$, $R^4$, $R^5$, $R^8$, $R^{10}$ and $R^{11}$ are all H;
compound 591: n is 0, X is SO, Y is O, $R^6$ is Me, $R^8$ and $R^{10}$ are both $CF_3$, and $R^2$, $R^3$, $R^4$, $R^5$, $R^7$, $R^9$ and $R^{11}$ are all H;
compound 592: n is 0, X is SO, Y is O, $R^6$ is Me, $R^2$, $R^3$, $R^4$ and $R^5$ are all H, and $R^7$, $R^8$, $R^9$, $R^{10}$ and $R^{11}$ are all F;
compound 593: n is 0, X is SO, Y is O, $R^6$ is Me, $R^7$ is $CH_3CH_2$, and $R^2$, $R^3$, $R^4$, $R^5$, $R^8$, $R^9$, $R^{10}$ and $R^{11}$ are all H;
compound 594: n is 0, X is SO, Y is O, $R^6$ is Me, $R^8$ is $CH_3CH_2$, and $R^2$, $R^3$, $R^4$, $R^5$, $R^7$, $R^9$, $R^{10}$ and $R^{11}$ are all H;
compound 595: n is 0, X is SO, Y is O, $R^6$ is Me, $R^9$ is $CH_3CH_2$, and $R^2$, $R^3$, $R^4$, $R^5$, $R^7$, $R^8$, $R^{10}$ and $R^{11}$ are all H;
compound 596: n is 0, X is SO, Y is O, $R^6$ is Me, $R^7$ is $CH(CH_3)_2$, and $R^2$, $R^3$, $R^4$, $R^5$, $R^8$, $R^9$, $R^{10}$ and $R^{11}$ are all H;
compound 597: n is 0, X is SO, Y is O, $R^6$ is Me, $R^8$ is $CH(CH_3)_2$, and $R^2$, $R^3$, $R^4$, $R^5$, $R^7$, $R^9$, $R^{10}$ and $R^{11}$ are all H;
compound 598: n is 0, X is SO, Y is O, $R^6$ is Me, $R^9$ is $CH(CH_3)_2$, and $R^2$, $R^3$, $R^4$, $R^5$, $R^7$, $R^8$, $R^{10}$ and $R^{11}$ are all H;
compound 599: n is 0, X is SO, Y is O, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;
compound 600: n is 0, X is SO, Y is O, $R^6$ is Me, $R^7$ is $C(CH_3)_3$, and $R^2$, $R^3$, $R^4$, $R^5$, $R^8$, $R^9$, $R^{10}$ and $R^{11}$ are all H;
compound 601: n is 0, X is SO, Y is O, $R^6$ is Me, $R^7$ is

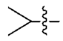 ,

and $R^2$, $R^3$, $R^4$, $R^5$, $R^8$, $R^9$, $R^{10}$ and $R^{11}$ are all H;
compound 602: n is 0, X is SO, Y is O, $R^6$ is Me, $R^7$ is

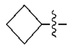,

and $R^2$, $R^3$, $R^4$, $R^5$, $R^8$, $R^9$, $R^{10}$ and $R^{11}$ are all H;
compound 603: n is 0, X is SO, Y is O, $R^6$ is Me, $R^7$ is $CO_2CH_3$, and $R^2$, $R^3$, $R^4$, $R^5$, $R^8$, $R^9$, $R^{10}$ and $R^{11}$ are all H;
compound 604: n is 0, X is SO, Y is O, $R^6$ is Me, $R^7$ and $R^3$ are cyclized to form

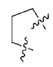,

and $R^2$, $R^3$, $R^4$, $R^5$, $R^9$, $R^{10}$ and $R^{11}$ are all H;
compound 605: n is 0, X is SO, Y is O, $R^6$ is Me, $R^8$ and $R^9$ are cyclized to form

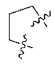,

and $R^2$, $R^3$, $R^4$, $R^5$, $R^7$, $R^{10}$ and $R^{11}$ are all H;
compound 606: n is 0, X is SO, Y is O, $R^6$ is Me, $R^8$ and $R^9$ are cyclized to form

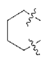,

and $R^2$, $R^3$, $R^4$, $R^5$, $R^7$, $R^{10}$ and $R^{11}$ are all H;
compound 607: n is 0, X is SO, Y is O, $R^6$ is Me, $R^7$ and $R^3$ are cyclized to form

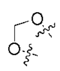,

and $R^2$, $R^3$, $R^4$, $R^5$, $R^9$, $R^{10}$ and $R^{11}$ are all H;
compound 608: n is 0, X is SO, Y is O, $R^6$ is Me, $R^8$ and $R^9$ are cyclized to form

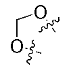,

and $R^2$, $R^3$, $R^4$, $R^5$, $R^7$, $R^{10}$ and $R^{11}$ are all H;
compound 609: n is 0, X is SO, Y is O, $R^6$ is Me, $R^7$ and $R^8$ are cyclized to form

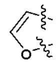,

and $R^2$, $R^3$, $R^4$, $R^5$, $R^9$, $R^{10}$ and $R^{11}$ are all H;
compound 610: n is 0, X is SO, Y is O, $R^6$ is Me, $R^7$ and $R^8$ are cyclized to form

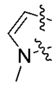,

and $R^2$, $R^3$, $R^4$, $R^5$, $R^9$, $R^{10}$ and $R^{11}$ are all H;
compound 611: n is 0, X is SO, Y is O, $R^6$ is Me, $R^7$ and $R^8$ are cyclized to form

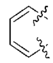,

and $R^2$, $R^3$, $R^4$, $R^5$, $R^9$, $R^{10}$ and $R^{11}$ are all H;
compound 612: n is 0, X is SO, Y is O, $R^6$ is Me, $R^7$ and $R^8$ are cyclized to form

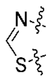,

and $R^2$, $R^3$, $R^4$, $R^5$, $R^9$, $R^{10}$ and $R^{11}$ are all H;
compound 613: n is 0, X is SO, Y is O, $R^6$ is Me, $R^7$ and $R^8$ are cyclized to form

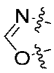,

and $R^2$, $R^3$, $R^4$, $R^5$, $R^9$, $R^{10}$ and $R^{11}$ are all H;
compound 614: n is 0, X is SO, Y is O, $R^6$ is Me, $R^8$ and $R^9$ are cyclized to form

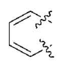,

and $R^2$, $R^3$, $R^4$, $R^5$, $R^7$, $R^{10}$ and $R^{11}$ are all H;
compound 615: n is 0, X is $SO_2$, Y is O, $R^6$ is Me, and $R^2$, $R^3$, $R^4$, $R^5$, $R^7$, $R^8$, $R^9$, $R^{10}$ and $R^{11}$ are all H;
compound 616: n is 0, X is $SO_2$, Y is O, $R^6$ is Me, $R^7$ is F, and $R^2$, $R^3$, $R^4$, $R^5$, $R^8$, $R^9$, $R^{10}$ and $R^{11}$ are all H;
compound 617: n is 0, X is $SO_2$, Y is O, $R^6$ is Me, $R^8$ is F, and $R^2$, $R^3$, $R^4$, $R^5$, $R^7$, $R^9$, $R^{10}$ and $R^{11}$ are all H;
compound 618: n is 0, X is $SO_2$, Y is O, $R^6$ is Me, $R^9$ is F, and $R^2$, $R^3$, $R^4$, $R^5$, $R^7$, $R^8$, $R^{10}$ and $R^{11}$ are all H;
compound 619: n is 0, X is $SO_2$, Y is O, $R^6$ is Me, $R^7$ is Cl, and $R^2$, $R^3$, $R^4$, $R^5$, $R^8$, $R^9$, $R^{10}$ and $R^{11}$ are all H;
compound 620: n is 0, X is $SO_2$, Y is O, $R^6$ is Me, $R^8$ is Cl, and $R^2$, $R^3$, $R^4$, $R^5$, $R^7$, $R^9$, $R^{10}$ and $R^{11}$ are all H;
compound 621: n is 0, X is $SO_2$, Y is O, $R^6$ is Me, $R^9$ is Cl, and $R^2$, $R^3$, $R^4$, $R^5$, $R^7$, $R^8$, $R^{10}$ and $R^{11}$ are all H;
compound 622: n is 0, X is $SO_2$, Y is O, $R^6$ is Me, $R^7$ is Br, and $R^2$, $R^3$, $R^4$, $R^5$, $R^8$, $R^9$, $R^{10}$ and $R^{11}$ are all H;
compound 623: n is 0, X is $SO_2$, Y is O, $R^6$ is Me, $R^8$ is Br, and $R^2$, $R^3$, $R^4$, $R^5$, $R^7$, $R^9$, $R^{10}$ and $R^{11}$ are all H;
compound 624: n is 0, X is $SO_2$, Y is O, $R^6$ is Me, $R^9$ is Br, and $R^2$, $R^3$, $R^4$, $R^5$, $R^7$, $R^8$, $R^{10}$ and $R^{11}$ are all H;
compound 625: n is 0, X is $SO_2$, Y is O, $R^6$ is Me, $R^7$ is I, and $R^2$, $R^3$, $R^4$, $R^5$, $R^8$, $R^9$, $R^{10}$ and $R^{11}$ are all H;
compound 626: n is 0, X is $SO_2$, Y is O, $R^6$ is Me, $R^8$ is I, and $R^2$, $R^3$, $R^4$, $R^5$, $R^7$, $R^9$, $R^{10}$ and $R^{11}$ are all H;
compound 627: n is 0, X is $SO_2$, Y is O, $R^6$ is Me, $R^9$ is I, and $R^2$, $R^3$, $R^4$, $R^5$, $R^7$, $R^8$, $R^{10}$ and $R^{11}$ are all H;
compound 628: n is 0, X is $SO_2$, Y is O, $R^6$ is Me, $R^7$ is $NO_2$, and $R^2$, $R^3$, $R^4$, $R^5$, $R^8$, $R^9$, $R^{10}$ and $R^{11}$ are all H;

compound 629: n is 0, X is $SO_2$, Y is O, $R^6$ is Me, $R^8$ is $NO_2$, and $R^2$, $R^3$, $R^4$, $R^5$, $R^7$, $R^9$, $R^{10}$ and $R^{11}$ are all H;
compound 630: n is 0, X is $SO_2$, Y is O, $R^6$ is Me, $R^9$ is $NO_2$, and $R^2$, $R^3$, $R^4$, $R^5$, $R^7$, $R^8$, $R^{10}$ and $R^{11}$ are all H;
compound 631: n is 0, X is $SO_2$, Y is O, $R^6$ is Me, $R^7$ is $CF_3$, and $R^2$, $R^3$, $R^4$, $R^5$, $R^8$, $R^9$, $R^{10}$ and $R^{11}$ are all H;
compound 632: n is 0, X is $SO_2$, Y is O, $R^6$ is Me, $R^8$ is $CF_3$, and $R^2$, $R^3$, $R^4$, $R^5$, $R^7$, $R^9$, $R^{10}$ and $R^{11}$ are all H;
compound 633: n is 0, X is $SO_2$, Y is O, $R^6$ is Me, $R^9$ is $CF_3$, and $R^2$, $R^3$, $R^4$, $R^5$, $R^7$, $R^8$, $R^{10}$ and $R^{11}$ are all H;
compound 634: n is 0, X is $SO_2$, Y is O, $R^6$ is Me, $R^7$ is $OCF_3$, and $R^2$, $R^3$, $R^4$, $R^5$, $R^8$, $R^9$, $R^{10}$ and $R^{11}$ are all H;
compound 635: n is 0, X is $SO_2$, Y is O, $R^6$ is Me, $R^8$ is $OCF_3$, and $R^2$, $R^3$, $R^4$, $R^5$, $R^7$, $R^9$, $R^{10}$ and $R^{11}$ are all H;
compound 636: n is 0, X is $SO_2$, Y is O, $R^6$ is Me, $R^9$ is $OCF_3$, and $R^2$, $R^3$, $R^4$, $R^5$, $R^7$, $R^8$, $R^{10}$ and $R^{11}$ are all H;
compound 637: n is 0, X is $SO_2$, Y is O, $R^6$ is Me, $R^7$ is $OCHF_2$, and $R^2$, $R^3$, $R^4$, $R^5$, $R^8$, $R^9$, $R^{10}$ and $R^{11}$ are all H;
compound 638: n is 0, X is $SO_2$, Y is O, $R^6$ is Me, $R^8$ is $OCHF_2$, and $R^2$, $R^3$, $R^4$, $R^5$, $R^7$, $R^9$, $R^{10}$ and $R^{11}$ are all H;
compound 639: n is 0, X is $SO_2$, Y is O, $R^6$ is Me, $R^9$ is $OCHF_2$, and $R^2$, $R^3$, $R^4$, $R^5$, $R^7$, $R^8$, $R^{10}$ and $R^{11}$ are all H;
compound 640: n is 0, X is $SO_2$, Y is O, $R^6$ is Me, $R^9$ is $SCF_3$, and $R^2$, $R^3$, $R^4$, $R^5$, $R^7$, $R^8$, $R^{10}$ and $R^{11}$ are all H;
compound 641: n is 0, X is $SO_2$, Y is O, $R^6$ is Me, $R^7$ is $OCH_3$, and $R^2$, $R^3$, $R^4$, $R^5$, $R^8$, $R^9$, $R^{10}$ and $R^{11}$ are all H;
compound 642: n is 0, X is $SO_2$, Y is O, $R^6$ is Me, $R^8$ is $OCH_3$, and $R^2$, $R^3$, $R^4$, $R^5$, $R^7$, $R^9$, $R^{10}$ and $R^{11}$ are all H;
compound 643: n is 0, X is $SO_2$, Y is O, $R^6$ is Me, $R^9$ is $OCH_3$, and $R^2$, $R^3$, $R^4$, $R^5$, $R^7$, $R^8$, $R^{10}$ and $R^{11}$ are all H;
compound 644: n is 0, X is $SO_2$, Y is O, $R^6$ is Me, $R^9$ is $OC_2H_5$, $R^2$, $R^3$, $R^4$, $R^5$, $R^7$, $R^8$, $R^{10}$ and $R^{11}$ are all H;
compound 645: n is 0, X is $SO_2$, Y is O, $R^6$ is Me, $R^7$ is CN, and $R^2$, $R^3$, $R^4$, $R^5$, $R^8$, $R^9$, $R^{10}$ and $R^{11}$ are all H;
compound 646: n is 0, X is $SO_2$, Y is O, $R^6$ is Me, $R^8$ is CN, and $R^2$, $R^3$, $R^4$, $R^5$, $R^7$, $R^9$, $R^{10}$ and $R^{11}$ are all H;
compound 647: n is 0, X is $SO_2$, Y is O, $R^6$ is Me, $R^9$ is CN, and $R^2$, $R^3$, $R^4$, $R^5$, $R^7$, $R^8$, $R^{10}$ and $R^{11}$ are all H;
compound 648: n is 0, X is $SO_2$, Y is O, $R^6$ is Me, $R^7$ is $CH_3$, and $R^2$, $R^3$, $R^4$, $R^5$, $R^8$, $R^9$, $R^{10}$ and $R^{11}$ are all H;
compound 649: n is 0, X is $SO_2$, Y is O, $R^6$ is Me, $R^8$ is $CH_3$, and $R^2$, $R^3$, $R^4$, $R^5$, $R^7$, $R^9$, $R^{10}$ and $R^{11}$ are all H;
compound 650: n is 0, X is $SO_2$, Y is O, $R^6$ is Me, $R^9$ is $CH_3$, and $R^2$, $R^3$, $R^4$, $R^5$, $R^7$, $R^8$, $R^{10}$ and $R^{11}$ are all H;
compound 651: n is 0, X is $SO_2$, Y is O, $R^6$ is Me, $R^7$ is $CH_3SO_2$, $R^2$, $R^3$, $R^4$, $R^5$, $R^8$, $R^9$, $R^{10}$ and $R^{11}$ are all H;
compound 652: n is 0, X is $SO_2$, Y is O, $R^6$ is Me, $R^8$ is $CH_3SO_2$, $R^2$, $R^3$, $R^4$, $R^5$, $R^7$, $R^9$, $R^{10}$ and $R^{11}$ are all H;
compound 653: n is 0, X is $SO_2$, Y is O, $R^6$ is Me, $R^9$ is $CH_3SO_2$, $R^2$, $R^3$, $R^4$, $R^5$, $R^7$, $R^8$, $R^{10}$ and $R^{11}$ are all H;
compound 654: n is 0, X is $SO_2$, Y is O, $R^6$ is Me, $R^7$ is $C_2H_5SO_2$, $R^2$, $R^3$, $R^4$, $R^5$, $R^8$, $R^9$, $R^{10}$ and $R^{11}$ are all H;
compound 655: n is 0, X is $SO_2$, Y is O, $R^6$ is Me, $R^7$ and $R^8$ are both Cl, and $R^2$, $R^3$, $R^4$, $R^5$, $R^9$, $R^{10}$ and $R^{11}$ are all H;
compound 656: n is 0, X is $SO_2$, Y is O, $R^6$ is Me, $R^7$ and $R^9$ are both Cl, and $R^2$, $R^3$, $R^4$, $R^5$, $R^8$, $R^{10}$ and $R^{11}$ are all H;
compound 657: n is 0, X is $SO_2$, Y is O, $R^6$ is Me, $R^7$ and $R^{10}$ are both Cl, and $R^2$, $R^3$, $R^4$, $R^5$, $R^8$, $R^9$ and $R^{11}$ are all H;
compound 658: n is 0, X is $SO_2$, Y is O, $R^6$ is Me, $R^7$ and $R^{11}$ are both Cl, and $R^2$, $R^3$, $R^4$, $R^5$, $R^8$, $R^9$ and $R^{10}$ are all H;
compound 659: n is 0, X is $SO_2$, Y is O, $R^6$ is Me, $R^8$ and $R^9$ are both Cl, and $R^2$, $R^3$, $R^4$, $R^5$, $R^7$, $R^{10}$ and $R^{11}$ are all H;
compound 660: n is 0, X is $SO_2$, Y is O, $R^6$ and $R^7$ are Me, $R^9$ is Cl, and $R^2$, $R^3$, $R^4$, $R^5$, $R^8$, $R^{10}$ and $R^{11}$ are all H;
compound 661: n is 0, X is $SO_2$, Y is O, $R^6$, $R^7$ and $R^8$ are Me, and $R^2$, $R^3$, $R^4$, $R^5$, $R^9$, $R^{10}$ and $R^{11}$ are all H;
compound 662: n is 0, X is $SO_2$, Y is O, $R^6$, $R^7$ and $R^9$ are Me, and $R^2$, $R^3$, $R^4$, $R^5$, $R^8$, $R^{10}$ and $R^{11}$ are all H;
compound 663: n is 0, X is $SO_2$, Y is O, $R^6$, $R^7$ and $R^{10}$ are Me, and $R^2$, $R^3$, $R^4$, $R^5$, $R^8$, $R^9$ and $R^{11}$ are all H;
compound 664: n is 0, X is $SO_2$, Y is O, $R^6$, $R^7$ and $R^{11}$ are Me, and $R^2$, $R^3$, $R^4$, $R^5$, $R^8$, $R^9$ and $R^{10}$ are all H;
compound 665: n is 0, X is $SO_2$, Y is O, $R^6$, $R^7$, $R^A$ and $R^{11}$ are Me, and $R^2$, $R^3$, $R^4$, $R^5$, $R^9$ and $R^{10}$ are all H;
compound 666: n is 0, X is $SO_2$, Y is O, $R^6$, $R^7$, $R^9$ and $R^{11}$ are Me, and $R^2$, $R^3$, $R^4$, $R^5$, $R^8$ and $R^{10}$ are all H;
compound 667: n is 0, X is $SO_2$, Y is O, $R^6$ is Me, $R^7$ and $R^9$ are both $CF_3$, $R^2$, $R^3$, $R^4$, $R^5$, $R^8$, $R^{10}$ and $R^{11}$ are all H;
compound 668: n is 0, X is $SO_2$, Y is O, $R^6$ is Me, $R^8$ and $R^{10}$ are both $CF_3$, $R^2$, $R^3$, $R^4$, $R^5$, $R^7$, $R^9$ and $R^{11}$ are all H;
compound 669: n is 0, X is $SO_2$, Y is O, $R^6$ is Me, $R^2$, $R^3$, $R^4$ and $R^5$ are all H, and $R^7$, $R^8$, $R^9$, $R^{10}$ and $R^{11}$ are all F;
compound 670: n is 0, X is $SO_2$, Y is O, $R^6$ is Me, $R^7$ is $CH_3CH_2$, $R^2$, $R^3$, $R^4$, $R^5$, $R^8$, $R^9$, $R^{10}$ and $R^{11}$ are all H;
compound 671: n is 0, X is $SO_2$, Y is O, $R^6$ is Me, $R^8$ is $CH_3CH_2$, $R^2$, $R^3$, $R^4$, $R^5$, $R^7$, $R^9$, $R^{10}$ and $R^{11}$ are all H;
compound 672: n is 0, X is $SO_2$, Y is O, $R^6$ is Me, $R^9$ is $CH_3CH_2$, $R^2$, $R^3$, $R^4$, $R^5$, $R^7$, $R^8$, $R^{10}$ and $R^{11}$ are all H;
compound 673: n is 0, X is $SO_2$, Y is O, $R^6$ is Me, $R^7$ is $CH(CH_3)_2$, $R^2$, $R^3$, $R^4$, $R^5$, $R^8$, $R^9$, $R^{10}$ and $R^{11}$ are all H;
compound 674: n is 0, X is $SO_2$, Y is O, $R^6$ is Me, $R^8$ is $CH(CH_3)_2$, $R^2$, $R^3$, $R^4$, $R^5$, $R^7$, $R^9$, $R^{10}$ and $R^{11}$ are all H;
compound 675: n is 0, X is $SO_2$, Y is O, $R^6$ is Me, $R^9$ is $CH(CH_3)_2$, $R^2$, $R^3$, $R^4$, $R^5$, $R^7$, $R^8$, $R^{10}$ and $R^{11}$ are all H;
Compound 676: n is 0, X is $SO_2$, Y is O, $R^6$ is Me, $R^7$ is $CH_3(CH_2)_2$, $R^2$, $R^3$, $R^4$, $R^5$, $R^8$, $R^9$, $R^{10}$ and $R^{11}$ are all H;
compound 677: n is 0, X is $SO_2$, Y is O, $R^6$ is Me, $R^7$ is $C(CH_3)_3$, $R^2$, $R^3$, $R^4$, $R^5$, $R^8$, $R^9$, $R^{10}$ and $R^{11}$ are all H;
compound 678: n is 0, X is $SO_2$, Y is O, $R^6$ is Me, $R^7$ is

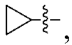,

$R^2$, $R^3$, $R^4$, $R^5$, $R^8$, $R^9$, $R^{10}$ and $R^{11}$ are all H;
compound 679: n is 0, X is $SO_2$, Y is O, $R^6$ is Me, $R^7$ is

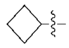,

$R^2$, $R^3$, $R^4$, $R^5$, $R^8$, $R^9$, $R^{10}$ and $R^{11}$ are all H;
compound 680: n is 0, X is $SO_2$, Y is O, $R^6$ is Me, $R^7$ is $CO_2CH_3$, $R^2$, $R^3$, $R^4$, $R^5$, $R^8$, $R^9$, $R^{10}$ and $R^{11}$ are all H;
compound 681: n is 0, X is $SO_2$, Y is O, $R^6$ is Me, $R^7$ and $R^8$ are cyclized to form

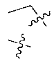,

and $R^2$, $R^3$, $R^4$, $R^5$, $R^9$, $R^{10}$ and $R^{11}$ are all H;
compound 682: n is 0, X is $SO_2$, Y is O, $R^6$ is Me, $R^3$ and $R^9$ are cyclized to form

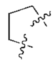,

and $R^2$, $R^3$, $R^4$, $R^5$, $R^7$, $R^{10}$ and $R^{11}$ are all H;
compound 683: n is 0, X is $SO_2$, Y is O, $R^6$ is Me, $R^3$ and $R^9$ are cyclized to form

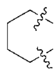,

and $R^2$, $R^3$, $R^4$, $R^5$, $R^7$, $R^{10}$ and $R^{11}$ are all H;
compound 684: n is 0, X is $SO_2$, Y is O, $R^6$ is Me, $R^7$ and $R^8$ are cyclized to form

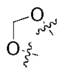,

and $R^2$, $R^3$, $R^4$, $R^5$, $R^9$, $R^{10}$ and $R^{11}$ are all H;
compound 685: n is 0, X is $SO_2$, Y is O, $R^6$ is Me, $R^3$ and $R^9$ are cyclized to form

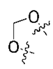,

and $R^2$, $R^3$, $R^4$, $R^5$, $R^7$, $R^{10}$ and $R^{11}$ are all H;
compound 686: n is 0, X is $SO_2$, Y is O, $R^6$ is Me, $R^7$ and $R^8$ are cyclized to form

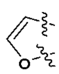,

and $R^2$, $R^3$, $R^4$, $R^5$, $R^9$, $R^{10}$ and $R^{11}$ are all H;
compound 687: n is 0, X is $SO_2$, Y is O, $R^6$ is Me, $R^7$ and $R^8$ are cyclized to form

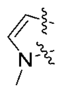,

and $R^2$, $R^3$, $R^4$, $R^5$, $R^9$, $R^{10}$ and $R^{11}$ are all H;
compound 688: n is 0, X is $SO_2$, Y is O, $R^6$ is Me, $R^7$ and $R^8$ are cyclized to form

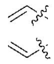,

and $R^2$, $R^3$, $R^4$, $R^5$, $R^9$, $R^{10}$ and $R^{11}$ are all H;
compound 689: n is 0, X is $SO_2$, Y is O, $R^6$ is Me, $R^7$ and $R^8$ are cyclized to form

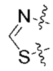,

and $R^2$, $R^3$, $R^4$, $R^3$, $R^9$, $R^{10}$ and $R^{11}$ are all H;
compound 690: n is 0, X is $SO_2$, Y is O, $R^6$ is Me, $R^7$ and $R^8$ are cyclized to form

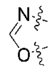,

and $R^2$, $R^3$, $R^4$, $R^5$, $R^9$, $R^{10}$ and $R^{11}$ are all H;
compound 691: n is 0, X is $SO_2$, Y is O, $R^6$ is Me, $R^3$ and $R^9$ are cyclized to form

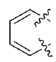,

and $R^2$, $R^3$, $R^4$, $R^5$, $R^7$, $R^{10}$ and $R^{11}$ are all H;
compound 692: n is 1, X is S, Y is O, $R^6$ is Me, $R^2$, $R^3$, $R^4$, $R^5$, $R^7$, $R^3$, $R^9$, $R^{10}$ and $R^{11}$ are all H;
compound 693: n is 2, X is S, Y is O, $R^6$ is Me, $R^2$, $R^3$, $R^4$, $R^5$, $R^7$, $R^3$, $R^9$, $R^{10}$ and $R^{11}$ are all H;
compound 697: n is 0, X is S, Y is O, $R^2$ is

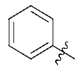 ,

$R^3$, $R^4$, $R^5$, $R^7$, $R^8$, $R^9$, $R^{10}$ and $R^{11}$ are all H, and $R^6$ is $CH_3$.

**6.** A compound according to claim 2 or 3, wherein, in formula (I),

$R^1$ is selected from the group consisting of $C_{1-6}$ alkyl, $-(CH_2)_z$-phenyl, an unsaturated five-membered ring group containing a heteroatom selected from class B, a saturated four-membered ring group containing a heteroatom selected from class B, an unsaturated six-membered ring group containing a heteroatom selected from class B, a saturated cycloalkyl group of $C_{4-8}$, benzothiazolyl, benzoxazolyl, benzopyrazolyl, phenylpropylthiophenyl, N-methyl-benzopyrolyl,

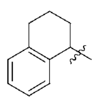 ;

z is an integer from 1 to 6; each of said class B heteroatoms is each independently at least one selected from O, S, N; and optionally, the class B heteroatom contains $C_{1-3}$ alkyl as a substituent;
$R^2$ is selected from H, n-butyl, $-CH_2$-phenyl;
$R^3$, $R^4$ and $R^5$ are all H; $R^6$ is methyl; n is 0; X is S; Y is O.

**7.** The compound according to claim 6, wherein, in formula (I), the substitution of n, X, Y, $R^1$, $R^2$, $R^3$, $R^4$, $R^5$ and $R^6$ is as follows:

compound 694: n is 0, X is S, Y is O, $R^1$ is

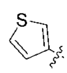

, $R^2$, $R^3$, $R^4$ and $R^5$ are all H, and $R^6$ is $CH_3$;
compound 695: n is 0, X is S, Y is O, $R^1$ is

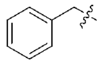,

$R^2$ is H, $R^3$, $R^4$ and $R^5$ are all H, and $R^6$ is $CH_3$;
compound 696: n is 0, X is S, Y is O, $R^1$ is

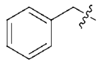,

$R^2$ is

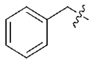,

$R^3$, $R^4$ and $R^5$ are all H, $R^6$ is $CH_3$;
compound 698: n is 0, X is S, Y is O, $R^1$ is $CH_3$, $R^2$ is $CH_3$, $R^3$, $R^4$ and $R^5$ are all H, and $R^6$ is $CH_3$;
compound 699: n is 0, X is S, Y is O, $R^1$ is $CH_3CH_2$, $R^2$ is $CH_3$, $R^3$, $R^4$ and $R^5$ are all H, and $R^6$ is $CH_3$;
compound 700: n is 0, X is S, Y is O, $R^1$ is $CH_3CH_2$, $R^2$ is $CH_3CH_2$, $R^3$, $R^4$ and $R^5$ are all H, and $R^6$ is $CH_3$;
compound 701: n is 0, X is S, Y is O, $R^1$ is $(CH_3)_2CH_2$, $R^2$ is $(CH_3)_2CH_2$, $R^3$, $R^4$ and $R^5$ are all H, and $R^6$ is $CH_3$;
compound 702: n is 0, X is S, Y is O, $R^1$ is $(CH_3)_2CH_2$, $R^2$ is $CH_3CH_2$, $R^3$, $R^4$ and $R^5$ are all H, and $R^6$ is $CH_3$;
compound 703: n is 0, X is S, Y is O, $R^1$ is $CH_3CH_3CH_2$, $R^2$ is $CH_3CH_3CH_2$, $R^3$, $R^4$ and $R^5$ are all H, and $R^6$ is $CH_3$;
compound 704: n is 0, X is S, Y is O, $R^1$ is

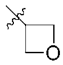,

$R^2$, $R^3$, $R^4$ and $R^5$ are all H, and $R^6$ is $CH_3$;
compound 705: n is 0, X is S, Y is O, $R^1$ is

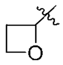,

$R^2$, $R^3$, $R^4$ and $R^5$ are all H, and $R^6$ is $CH_3$;
compound 706: n is 0, X is S, Y is O, $R^1$ is

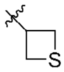,

$R^2$, $R^3$, $R^4$ and $R^5$ are all H, and $R^6$ is $CH_3$;
compound 707: n is 0, X is S, Y is O, $R^1$ is

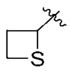,

$R^2$, $R^3$, $R^4$ and $R^5$ are all H, and $R^6$ is $CH_3$;
compound 708: n is 0, X is S, Y is O, $R^1$ is

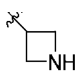,

$R^2$, $R^3$, $R^4$ and $R^5$ are all H, and $R^6$ is $CH_3$;

compound 709: n is 0, X is S, Y is O, $R^1$ is

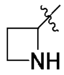,

$R^2$, $R^3$, $R^4$ and $R^5$ are all H, and $R^6$ is $CH_3$;
compound 710: n is 0, X is S, Y is O, $R^1$ is

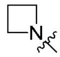

, $R^2$, $R^3$, $R^4$ and $R^5$ are all H, and $R^6$ is $CH_3$;
compound 711: n is 0, X is S, Y is O, $R^1$ is

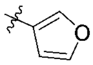,

$R^2$, $R^3$, $R^4$ and $R^5$ are all H, and $R^6$ is $CH_3$;
compound 712: n is 0, X is S, Y is O, $R^1$ is

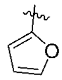,

$R^2$, $R^3$, $R^4$ and $R^5$ are all H, and $R^6$ is $CH_3$;
compound 713: n is 0, X is S, Y is O, $R^1$ is

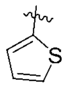,

$R^2$, $R^3$, $R^4$ and $R^5$ are all H, and $R^6$ is $CH_3$;
compound 714: n is 0, X is S, Y is O, $R^1$ is

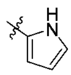,

$R^2$, $R^3$, $R^4$ and $R^5$ are all H, and $R^6$ is $CH_3$;
compound 715: n is 0, X is S, Y is O, $R^1$ is

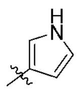,

$R^2$, $R^3$, $R^4$ and $R^5$ are all H, and $R^6$ is $CH_3$;
compound 716: n is 0, X is S, Y is O, $R^1$ is

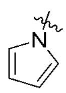,

$R^2$, $R^3$, $R^4$ and $R^5$ are all H, and $R^6$ is $CH_3$;
compound 717: n is 0, X is S, Y is O, $R^1$ is

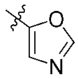,

$R^2$, $R^3$, $R^4$ and $R^5$ are all H, and $R^6$ is $CH_3$;
compound 718: n is 0, X is S, Y is O, $R^1$ is

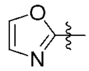,

$R^2$, $R^3$, $R^4$ and $R^5$ are all H, and $R^6$ is $CH_3$;
compound 719: n is 0, X is S, Y is O, $R^1$ is

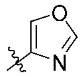,

$R^2$, $R^3$, $R^4$ and $R^5$ are all H, and $R^6$ is $CH_3$;
compound 720: n is 0, X is S, Y is O, $R^1$ is

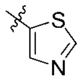,

$R^2$, $R^3$, $R^4$ and $R^5$ are all H, and $R^6$ is $CH_3$;
compound 721: n is 0, X is S, Y is O, $R^1$ is

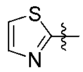,

$R^2$, $R^3$, $R^4$ and $R^5$ are all H, and $R^6$ is $CH_3$;
compound 722: n is 0, X is S, Y is O, $R^1$ is

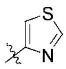,

$R^2$, $R^3$, $R^4$ and $R^5$ are all H, and $R^6$ is $CH_3$;
compound 723: n is 0, X is S, Y is O, $R^1$ is

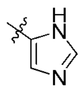,

$R^2$, $R^3$, $R^4$ and $R^5$ are all H, and $R^6$ is $CH_3$;
compound 724: n is 0, X is S, Y is O, $R^1$ is

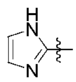,

$R^2$, $R^3$, $R^4$ and $R^5$ are all H, and $R^6$ is $CH_3$;
compound 725: n is 0, X is S, Y is O, $R^1$ is

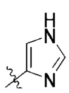,

$R^2$, $R^3$, $R^4$ and $R^5$ are all H, and $R^6$ is $CH_3$;
compound 726: n is 0, X is S, Y is O, $R^1$ is

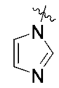,

$R^2$, $R^3$, $R^4$ and $R^5$ are all H, and $R^6$ is $CH_3$;
compound 727: n is 0, X is S, Y is O, $R^1$ is

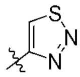,

$R^2$, $R^3$, $R^4$ and $R^5$ are all H, and $R^6$ is $CH_3$;
compound 728: n is 0, X is S, Y is O, $R^1$ is

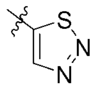,

$R^2$, $R^3$, $R^4$ and $R^5$ are all H, and $R^6$ is $CH_3$;
compound 729: n is 0, X is S, Y is O, $R^1$ is

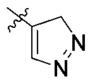.

, $R^2$, $R^3$, $R^4$ and $R^5$ are all H, and $R^6$ is $CH_3$;
compound 730: n is 0, X is S, Y is O, $R^1$ is

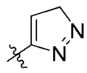,

$R^2$, $R^3$, $R^4$ and $R^5$ are all H, and $R^6$ is $CH_3$;
compound 731: n is 0, X is S, Y is O, $R^1$ is

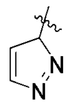,

$R^2$, $R^3$, $R^4$ and $R^5$ are all H, and $R^6$ is $CH_3$;
compound 732: n is 0, X is S, Y is O, $R^1$ is

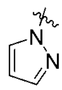,

$R^2$, $R^3$, $R^4$ and $R^5$ are all H, and $R^6$ is $CH_3$;
compound 733: n is 0, X is S, Y is O, $R^1$ is

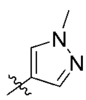,

$R^2$, $R^3$, $R^4$ and $R^5$ are all H, and $R^6$ is $CH_3$;

compound 734: n is 0, X is S, Y is O, $R^1$ is

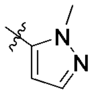,

$R^2$, $R^3$, $R^4$ and $R^5$ are all H, and $R^6$ is $CH_3$;
compound 735: n is 0, X is S, Y is O, $R^1$ is

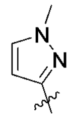,

$R^2$, $R^3$, $R^4$ and $R^5$ are all H, and $R^6$ is $CH_3$;
compound 736: n is 0, X is S, Y is O, $R^1$ is

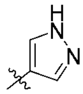,

$R^2$, $R^3$, $R^4$ and $R^5$ are all H, and $R^6$ is $CH_3$;
compound 737: n is 0, X is S, Y is O, $R^1$ is

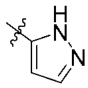,

$R^2$, $R^3$, $R^4$ and $R^5$ are all H, and $R^6$ is $CH_3$;
compound 738: n is 0, X is S, Y is O, $R^1$ is

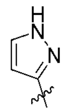,

$R^2$, $R^3$, $R^4$ and $R^5$ are all H, and $R^6$ is $CH_3$;
compound 739: n is 0, X is S, Y is O, $R^1$ is

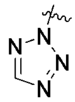,

$R^2$, $R^3$, $R^4$ and $R^5$ are all H, and $R^6$ is $CH_3$;
compound 740: n is 0, X is S, Y is O, $R^1$ is

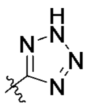,

$R^2$, $R^3$, $R^4$ and $R^5$ are all H, and $R^6$ is $CH_3$;
compound 741: n is 0, X is S, Y is O, $R^1$ is

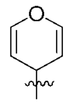
,

$R^2$, $R^3$, $R^4$ and $R^5$ are all H, and $R^6$ is $CH_3$;
compound 742: n is 0, X is S, Y is O, $R^1$ is

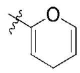
,

$R^2$, $R^3$, $R^4$ and $R^5$ are all H, and $R^6$ is $CH_3$;
compound 743: n is 0, X is S, Y is O, $R^1$ is

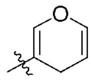
,

$R^2$, $R^3$, $R^4$ and $R^5$ are all H, and $R^6$ is $CH_3$;
compound 744: n is 0, X is S, Y is O, $R^1$ is

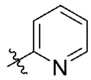

, $R^2$, $R^3$, $R^4$ and $R^5$ are all H, and $R^6$ is $CH_3$;
compound 745: n is 0, X is S, Y is O, $R^1$ is

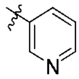
,

$R^2$, $R^3$, $R^4$ and $R^5$ are all H, and $R^6$ is $CH_3$;
compound 746: n is 0, X is S, Y is O, $R^1$ is

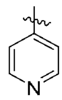
,

$R^2$, $R^3$, $R^4$ and $R^5$ are all H, and $R^6$ is $CH_3$;
compound 747: n is 0, X is S, Y is O, $R^1$ is

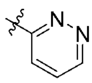
,

$R^2$, $R^3$, $R^4$ and $R^5$ are all H, and $R^6$ is $CH_3$;
compound 748: n is 0, X is S, Y is O, $R^1$ is

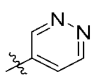
,

$R^2$, $R^3$, $R^4$ and $R^5$ are all H, and $R^6$ is $CH_3$;
compound 749: n is 0, X is S, Y is O, $R^1$ is

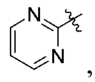

$R^2$, $R^3$, $R^4$ and $R^5$ are all H, and $R^6$ is $CH_3$; compound 750: n is 0, X is S, Y is O, $R^1$ is

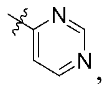

$R^2$, $R^3$, $R^4$ and $R^5$ are all H, and $R^6$ is $CH_3$; compound 751: n is 0, X is S, Y is O, $R^1$ is

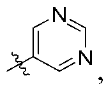

$R^2$, $R^3$, $R^4$ and $R^5$ are all H, and $R^6$ is $CH_3$; compound 752: n is 0, X is S, Y is O, $R^1$ is

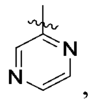

$R^2$, $R^3$, $R^4$ and $R^5$ are all H, and $R^6$ is $CH_3$; compound 753: n is 0, X is S, Y is O, $R^1$ is

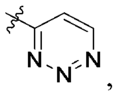

$R^2$, $R^3$, $R^4$ and $R^5$ are all H, and $R^6$ is $CH_3$; compound 754: n is 0, X is S, Y is O, $R^1$ is

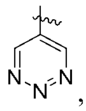

$R^2$, $R^3$, $R^4$ and $R^5$ are all H, and $R^6$ is $CH_3$; compound 755: n is 0, X is S, Y is O, $R^1$ is

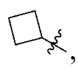

$R^2$, $R^3$, $R^4$ and $R^5$ are all H, and $R^6$ is $CH_3$; compound 756: n is 0, X is S, Y is O, $R^1$ is

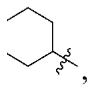

$R^2$, $R^3$, $R^4$ and $R^5$ are all H, and $R^6$ is $CH_3$; compound 757: n is 0, X is S, Y is O, $R^1$ is

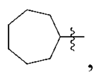

$R^2$, $R^3$, $R^4$ and $R^5$ are all H, and $R^6$ is $CH_3$;
compound 758: n is 0, X is S, Y is O, $R^1$ is

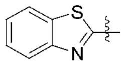,

$R^2$, $R^3$, $R^4$ and $R^5$ are all H, and $R^6$ is $CH_3$;
compound 759: n is 0, X is S, Y is O, $R^1$ is

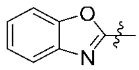,

$R^2$, $R^3$, $R^4$ and $R^5$ are all H, and $R^6$ is $CH_3$;
compound 760: n is 0, X is S, Y is O, $R^1$ is

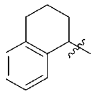,

$R^2$, $R^3$, $R^4$ and $R^5$ are all H, and $R^6$ is $CH_3$;
compound 761: n is 0, X is S, Y is O, $R^1$ is

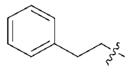,

$R^2$, $R^3$, $R^4$ and $R^5$ are all H, and $R^6$ is $CH_3$;
compound 762: n is 0, X is S, Y is O, $R^1$ is

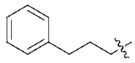,

$R^2$, $R^3$, $R^4$ and $R^5$ are all H, and $R^6$ is $CH_3$;
compound 763: n is 0, X is S, Y is O, $R^1$ is

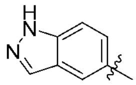,

$R^2$, $R^3$, $R^4$ and $R^5$ are all H, and $R^6$ is $CH_3$;
compound 764: n is 0, X is S, Y is O, $R^1$ is

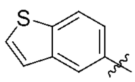,

$R^2$, $R^3$, $R^4$ and $R^5$ are all H, and $R^6$ is $CH_3$;
compound 765: n is 0, X is S, Y is O, $R^1$ is

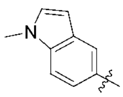,

$R^2$, $R^3$, $R^4$ and $R^5$ are all H, and $R^6$ is $CH_3$;
compound 766: n is 0, X is S, Y is O, $R^1$ is

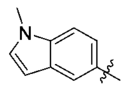,

$R^2$, $R^3$, $R^4$ and $R^5$ are all H, and $R^6$ is $CH_3$;
compound 767: n is 0, X is S, Y is O, $R^1$ is $CH_3(CH_2)_3$, $R^2$ is $CH_3(CH_2)_3$, $R^3$, $R^4$ and $R^5$ are all H, and $R^6$ is $CH_3$.

**8.** The compound of claim 1, wherein, in formula (I), X and Y are both O;
preferably, the compound of formula (I) has the structure of a compound of formula (I-A):

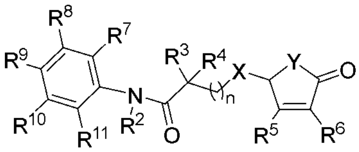 formula (I-A),

wherein, in formula (I-A), the substitution of n, X, Y, $R^2$, $R^3$, $R^4$, $R^5$, $R^6$, $R^7$, $R^8$, $R^9$, $R^{10}$ and $R^{11}$ is as follows:

compound 768: n is 0, X is O, Y is O, $R^6$ is Me, $R^2$, $R^3$, $R^4$, $R^5$, $R^7$, $R^8$, $R^9$, $R^{10}$ and $R^{11}$ are all H;
compound 769: n is 0, X is O, Y is O, $R^6$ is Me, $R^7$ is F, and $R^2$, $R^3$, $R^4$, $R^5$, $R^7$, $R^9$, $R^{10}$ and $R^{11}$ are all H;
compound 770: n is 0, X is O, Y is O, $R^6$ is Me, $R^8$ is F, and $R^2$, $R^3$, $R^4$, $R^5$, $R^7$, $R^9$, $R^{10}$ and $R^{11}$ are all H;
compound 771: n is 0, X is O, Y is O, $R^6$ is Me, $R^9$ is F, and $R^2$, $R^3$, $R^4$, $R^5$, $R^7$, $R^8$, $R^{10}$ and $R^{11}$ are all H;
compound 772: n is 0, X is O, Y is O, $R^6$ is Me, $R^7$ is Cl, and $R^2$, $R^3$, $R^4$, $R^5$, $R^8$, $R^9$, $R^{10}$ and $R^{11}$ are all H;
compound 773: n is 0, X is O, Y is O, $R^6$ is Me, $R^8$ is Cl, and $R^2$, $R^3$, $R^4$, $R^5$, $R^7$, $R^9$, $R^{10}$ and $R^{11}$ are all H;
compound 774: n is 0, X is O, Y is O, $R^6$ is Me, $R^9$ is Cl, and $R^2$, $R^3$, $R^4$, $R^5$, $R^7$, $R^8$, $R^{10}$ and $R^{11}$ are all H;
compound 775: n is 0, X is O, Y is O, $R^6$ is Me, $R^7$ is Br, and $R^2$, $R^3$, $R^4$, $R^5$, $R^8$, $R^9$, $R^{10}$ and $R^{11}$ are all H;
compound 776: n is 0, X is O, Y is O, $R^6$ is Me, $R^8$ is Br, and $R^2$, $R^3$, $R^4$, $R^5$, $R^7$, $R^9$, $R^{10}$ and $R^{11}$ are all H;
compound 777: n is 0, X is O, Y is O, $R^6$ is Me, $R^9$ is Br, and $R^2$, $R^3$, $R^4$, $R^5$, $R^7$, $R^8$, $R^{10}$ and $R^{11}$ are all H;
compound 778: n is 0, X is O, Y is O, $R^6$ is Me, $R^7$ is $NO_2$, and $R^2$, $R^3$, $R^4$, $R^5$, $R^8$, $R^9$, $R^{10}$ and $R^{11}$ are all H;
compound 779: n is 0, X is O, Y is O, $R^6$ is Me, $R^8$ is $NO_2$, and $R^2$, $R^3$, $R^4$, $R^5$, $R^7$, $R^9$, $R^{10}$ and $R^{11}$ are all H;
compound 780: n is 0, X is O, Y is O, $R^6$ is Me, $R^9$ is $NO_2$, and $R^2$, $R^3$, $R^4$, $R^s$, $R^7$, $R^3$, $R^{10}$ and $R^{11}$ are all H;
compound 781: n is 0, X is O, Y is O, $R^6$ is Me, $R^7$ is $CF_3$, and $R^2$, $R^3$, $R^4$, $R^5$, $R^3$, $R^9$, $R^{10}$ and $R^{11}$ are all H;
compound 782: n is 0, X is O, Y is O, $R^6$ is Me, $R^8$ is $CF_3$, and $R^2$, $R^3$, $R^4$, $R^5$, $R^7$, $R^9$, $R^{10}$ and $R^{11}$ are all H;
compound 783: n is 0, X is O, Y is O, $R^6$ is Me, $R^9$ is $CF_3$, and $R^2$, $R^3$, $R^4$, $R^5$, $R^7$, $R^8$, $R^{10}$ and $R^{11}$ are all H;
compound 784: n is 0, X is O, Y is O, $R^6$ is Me, $R^7$ is $OCH_3$, and $R^2$, $R^3$, $R^4$, $R^5$, $R^8$, $R^9$, $R^{10}$ and $R^{11}$ are all H;
compound 785: n is 0, X is O, Y is O, $R^6$ is Me, $R^8$ is $OCH_3$, and $R^2$, $R^3$, $R^4$, $R^5$, $R^7$, $R^9$, $R^{10}$ and $R^{11}$ are all H;
compound 786: n is 0, X is O, Y is O, $R^6$ is Me, $R^9$ is $OCH_3$, and $R^2$, $R^3$, $R^4$, $R^5$, $R^7$, $R^8$, $R^{10}$ and $R^{11}$ are all H;
compound 787: n is 0, X is O, Y is O, $R^6$ and $R^7$ are Me, and $R^2$, $R^3$, $R^4$, $R^5$, $R^8$, $R^9$, $R^{10}$ and $R^{11}$ are all H;
compound 788: n is 0, X is O, Y is O, $R^6$ and $R^8$ are Me, and $R^2$, $R^3$, $R^4$, $R^5$, $R^7$, $R^9$, $R^{10}$ and $R^{11}$ are all H;
compound 789: n is 0, X is O, Y is O, $R^6$ and $R^9$ are Me, and $R^2$, $R^3$, $R^4$, $R^5$, $R^7$, $R^8$, $R^{10}$ and $R^{11}$ are all H;
compound 790: n is 0, X is O, Y is O, $R^5$ is Me, $R^7$ is $CH_3CH_2$, and $R^2$, $R^3$, $R^4$, $R^6$, $R^8$, $R^9$, $R^{10}$ and $R^{11}$ are all H;
compound 791: n is 0, X is O, Y is O, $R^5$ is Me, $R^8$ is $CH_3CH_2$, and $R^2$, $R^3$, $R^4$, $R^6$, $R^7$, $R^9$, $R^{10}$ and $R^{11}$ are all H;
compound 792: n is 0, X is O, Y is O, $R^5$ is Me, $R^9$ is $CH_3CH_2$, and $R^2$, $R^3$, $R^4$, $R^6$, $R^7$, $R^8$, $R^{10}$ and $R^{11}$ are all H;
compound 793: n is 0, X is O, Y is O, $R^5$ is Me, $R^7$ is $CH(CH_3)_2$, and $R^2$, $R^3$, $R^4$, $R^6$, $R^8$, $R^9$, $R^{10}$ and $R^{11}$ are all H;
compound 794: n is 0, X is O, Y is O, $R^5$ is Me, $R^3$ is $CH(CH_3)_2$, and $R^2$, $R^3$, $R^4$, $R^6$, $R^7$, $R^9$, $R^{10}$ and $R^{11}$ are all H;
compound 795: n is 0, X is O, Y is O, $R^5$ is Me, $R^9$ is $CH(CH_3)_2$, and $R^2$, $R^3$, $R^4$, $R^6$, $R^7$, $R^8$, $R^{10}$ and $R^{11}$ are all H.

**9.** A process for producing the amide compound according to any one of claims 1 to 8, which comprises: carrying out contact reaction on the compound shown in a formula (V-1) and the compound shown in a formula (V-2);

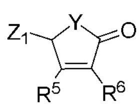 formula (V-1), 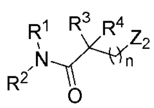 formula (V-2)

wherein the substitution of the substituents in the formulae (V-1) and (V-2) corresponds to the same substitution in the amide compound according to any one of claims 1 to 8, and one of $Z_1$ and $Z_2$ is -Br and the other is -XH, and X is as

defined in any one of claims 1 to 8.

**10.** A process for producing the amide compound according to any one of claims 1 to 8, which comprises: carrying out coupling reaction on a compound shown in a formula (II) and a compound shown in a formula (III);

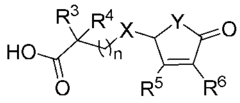 formula (II), 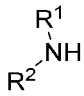 formula (III);

wherein the substitution of the substituents in the formula (III) and the formula (II) is the same as the corresponding substitution in the amide compound according to any one of claims 1 to 8.

**11.** Agricultural application of amide compounds according to any of claims 1 to 8;
preferably, the agricultural application is selected from at least one application of increasing crop yield, managing abiotic stress on plants, regulating plant growth, enhancing plant disease resistance, promoting seed germination, regulating soil microbial population structure, and increasing soil fertility.

**12.** A biostimulant comprising a plant growth regulating effective amount of at least one amide compound according to any one of claims 1 to 8, and optionally an adjuvant;
preferably, the content of the amide compound is 1-99.99wt%, or the content of the amide compound is 0.001 ppm to 100 ppm.

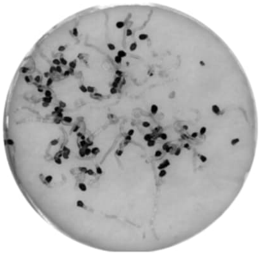

FIG.1

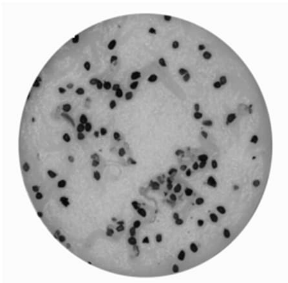

FIG.2

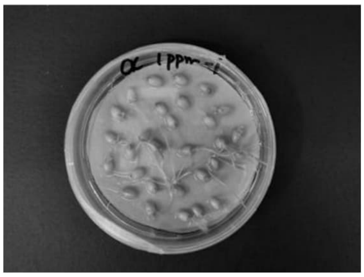

FIG.3

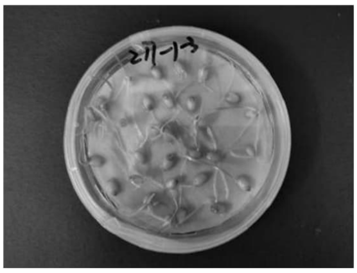

FIG.4

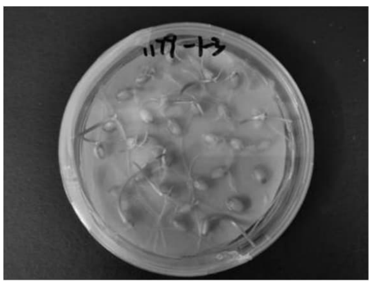

FIG.5

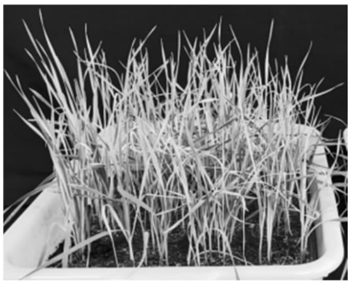

FIG.6

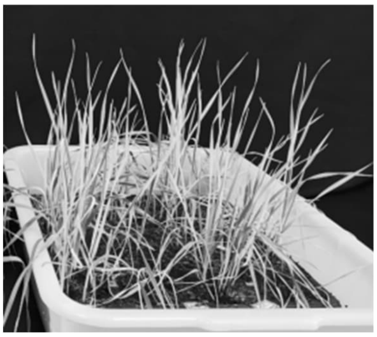

FIG.7

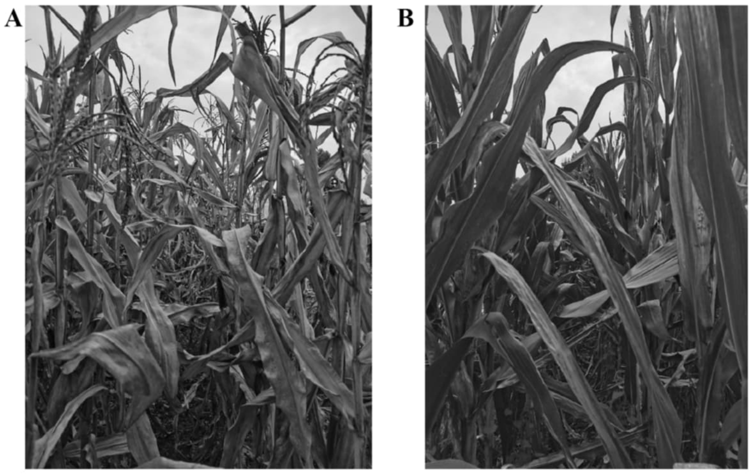

FIG.8

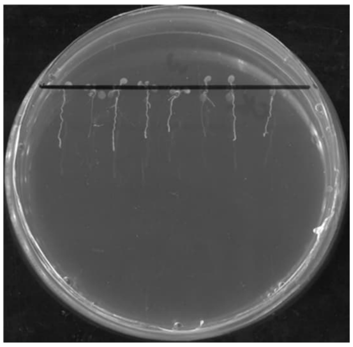

FIG.9

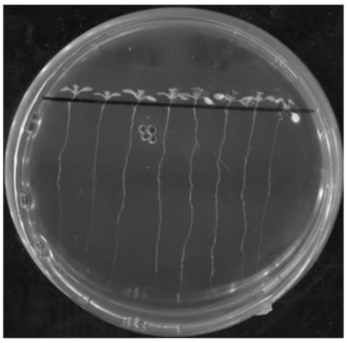

FIG.10

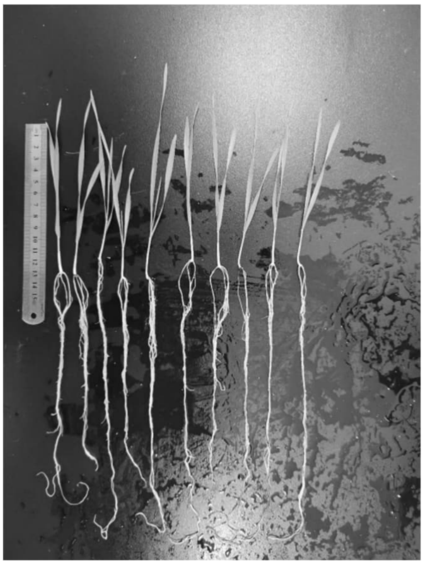

FIG.11

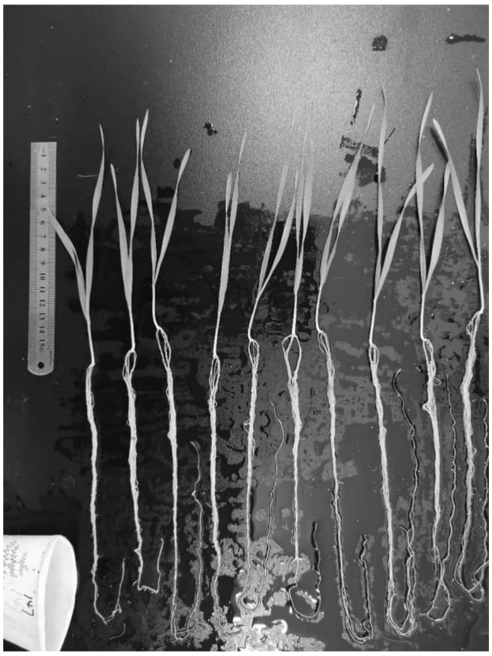

FIG.12

**INTERNATIONAL SEARCH REPORT**

International application No.
**PCT/CN2024/119810**

**A. CLASSIFICATION OF SUBJECT MATTER**

C07D307/64(2006.01)i; C07D307/60(2006.01)i; A01N43/08(2006.01)i; A01P21/00(2006.01)i

According to International Patent Classification (IPC) or to both national classification and IPC

**B. FIELDS SEARCHED**

Minimum documentation searched (classification system followed by classification symbols)

IPC: C07D, A01N, A01P

Documentation searched other than minimum documentation to the extent that such documents are included in the fields searched

Electronic data base consulted during the international search (name of data base and, where practicable, search terms used)

CNTXT, WPABS, ENTXT, DWPI, STN(REGISTRY, CAPLUS, MARPAT): 南开大学, 席真, 王大伟, 吴海翔, 酰胺, 独脚金内酯, 种子萌发, 根寄生杂草, amide, strigolacton+, seed germination, root parasitic weed? , 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 117534639 A (NANKAI UNIVERSITY) 09 February 2024 (2024-02-09) claims 1-12 | 1-12 |
| X | CN 110734417 A (NANKAI UNIVERSITY) 31 January 2020 (2020-01-31) claims 1-11, and description, paragraphs 0083-0089, and table 1 | 1-12 |
| A | WO 2018060865 A1 (KING ABDULLAH UNIVERSITY OF SCIENCE AND TECHNOLOGY et al.) 05 April 2018 (2018-04-05) claims 1-15 | 1-12 |
| A | WO 2012146374 A2 (ALBERT-LUDWIGS-UNFVERSITAT FREIBURG et al.) 01 November 2012 (2012-11-01) claims 1-17 | 1-12 |
| A | CN 101677545 A (BAYER CROPSCIENCE SA) 24 March 2010 (2010-03-24) claims 1-23 | 1-12 |
| A | CN 101730469 A (BAYER CROPSCIENCE SA) 09 June 2010 (2010-06-09) claims 1-21 | 1-12 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 December 2024** | **23 December 2024** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/119810**

| Patent document cited in search report | | | Publication date (day/month/year) | Patent family member(s) | | | Publication date (day/month/year) |
|---|---|---|---|---|---|---|---|
| CN | 117534639 | A | 09 February 2024 | None | | | |
| CN | 110734417 | A | 31 January 2020 | None | | | |
| WO | 2018060865 | A1 | 05 April 2018 | US | 2019357534 | A1 | 28 November 2019 |
| | | | | US | 11903386 | B2 | 20 February 2024 |
| | | | | WO | 2018060865 | A8 | 20 June 2019 |
| | | | | EP | 3519396 | A1 | 07 August 2019 |
| | | | | EP | 3519396 | B1 | 24 March 2021 |
| WO | 2012146374 | A2 | 01 November 2012 | EP | 2518062 | A1 | 31 October 2012 |
| | | | | WO | 2012146374 | A3 | 14 March 2013 |
| | | | | US | 2014287924 | A1 | 25 September 2014 |
| | | | | US | 8980795 | B2 | 17 March 2015 |
| | | | | US | 2015250170 | A1 | 10 September 2015 |
| | | | | US | 9326510 | B2 | 03 May 2016 |
| CN | 101677545 | A | 24 March 2010 | IL | 201833 | A0 | 16 June 2010 |
| | | | | NZ | 580799 | A | 30 March 2012 |
| | | | | WO | 2008152091 | A2 | 18 December 2008 |
| | | | | WO | 2008152091 | A8 | 26 February 2009 |
| | | | | WO | 2008152091 | A3 | 05 November 2009 |
| | | | | CL | 2008001767 | A1 | 19 December 2008 |
| | | | | BRPI | 0810956 | A2 | 08 September 2015 |
| | | | | BRPI | 0810956 | A8 | 27 October 2015 |
| | | | | KR | 20100020030 | A | 19 February 2010 |
| | | | | TW | 200904331 | A | 01 February 2009 |
| | | | | JP | 2010529974 | A | 02 September 2010 |
| | | | | JP | 5504157 | B2 | 28 May 2014 |
| | | | | CA | 2685608 | A1 | 18 December 2008 |
| | | | | US | 2010137373 | A1 | 03 June 2010 |
| | | | | EP | 2157857 | A2 | 03 March 2010 |
| | | | | EP | 2157857 | B1 | 18 September 2013 |
| | | | | AU | 2008263920 | A1 | 18 December 2008 |
| | | | | MX | 2009013508 | A | 27 January 2010 |
| | | | | RU | 2010101111 | A | 20 July 2011 |
| | | | | RU | 2442328 | C2 | 20 February 2012 |
| | | | | JP | 2014062098 | A | 10 April 2014 |
| | | | | ZA | 200907936 | B | 28 July 2010 |
| | | | | AR | 066156 | A1 | 29 July 2009 |
| CN | 101730469 | A | 09 June 2010 | WO | 2008152092 | A2 | 18 December 2008 |
| | | | | WO | 2008152092 | A3 | 05 November 2009 |
| | | | | AU | 2008263921 | A1 | 18 December 2008 |
| | | | | MX | 2009013509 | A | 27 January 2010 |
| | | | | EP | 2157858 | A2 | 03 March 2010 |
| | | | | EP | 2157858 | B1 | 22 August 2012 |
| | | | | US | 2010152270 | A1 | 17 June 2010 |
| | | | | CA | 2685576 | A1 | 18 December 2008 |
| | | | | BRPI | 0810958 | A2 | 08 September 2015 |
| | | | | BRPI | 0810958 | A8 | 13 September 2016 |
| | | | | TW | 200904330 | A | 01 February 2009 |
| | | | | ZA | 200907725 | B | 28 July 2010 |
| | | | | AR | 066157 | A1 | 29 July 2009 |

Form PCT/ISA/210 (patent family annex) (July 2022)

**INTERNATIONAL SEARCH REPORT**
**Information on patent family members**

International application No.
**PCT/CN2024/119810**

| Patent document cited in search report | Publication date (day/month/year) | Patent family member(s) | | | Publication date (day/month/year) |
|---|---|---|---|---|---|
| | | NZ | 580800 | A | 30 March 2012 |
| | | RU | 2010101073 | A | 20 July 2011 |
| | | RU | 2444194 | C2 | 10 March 2012 |
| | | CL | 2008001768 | A1 | 19 December 2008 |
| | | IL | 201827 | A0 | 16 June 2010 |
| | | KR | 20100020031 | A | 19 February 2010 |
| | | JP | 2010529975 | A | 02 September 2010 |
| | | JP | 5465665 | B2 | 09 April 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311268630
- WO 2018060865 A1
- WO 2012146374 A3


### Non-patent literature cited in the description


- **BOUWMEESTER, H. J.** Strigolactones: plant hormones with promising features. *Angew.Chem. Int.Ed.*, 2019, vol. 58, 12778-12786
- **MANGNUS, E. M.** ; **ZWANENBURG, B.** Tentative molecular mechanism for germination stimulation of striga and orobanche seeds by strigol and its synthetic analogs. *J. Agric. Food Chem.*, 1992, vol. 40, 1066-1070